(19) Europäisches Patentamt
European Patent Office
Office européen des brevets

(11) **EP 3 604 361 A1**

(12) **EUROPEAN PATENT APPLICATION**
published in accordance with Art. 153(4) EPC

(43) Date of publication:
**05.02.2020 Bulletin 2020/06**

(21) Application number: **18771295.5**

(22) Date of filing: **16.03.2018**

(51) Int Cl.:
***C08F 20/38*** *(2006.01)* ***C08F 20/34*** *(2006.01)*
***C09K 19/38*** *(2006.01)* ***G02B 1/11*** *(2015.01)*
***G02B 5/30*** *(2006.01)* ***G02F 1/1335*** *(2006.01)*
***G02F 1/13363*** *(2006.01)*

(86) International application number:
**PCT/JP2018/010481**

(87) International publication number:
**WO 2018/173954 (27.09.2018 Gazette 2018/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.03.2017 JP 2017058254**
**07.07.2017 JP 2017134139**

(71) Applicant: **Zeon Corporation**
**Tokyo 100-8246 (JP)**

(72) Inventors:
- **SAKAMOTO, Kei**
  **Tokyo 100-8246 (JP)**
- **OKUYAMA, Kumi**
  **Tokyo 100-8246 (JP)**
- **MIMA, Takanori**
  **Tokyo 100-8246 (JP)**

(74) Representative: **Beckmann, Claus**
**Kraus & Weisert**
**Patentanwälte PartGmbB**
**Thomas-Wimmer-Ring 15**
**80539 München (DE)**

(54) **POLYMERIZABLE COMPOUND AND PRODUCTION METHOD THEREFOR, POLYMERIZABLE COMPOSITION, POLYMER, OPTICAL FILM, OPTICALLY ANISOTROPIC OBJECT, POLARIZER, DISPLAY DEVICE, ANTIREFLECTION FILM, AND COMPOUND AND USE THEREOF**

(57) Disclosed is a polymerizable compound useful in the preparation of a polymer which is capable of producing, for example, an optical film having excellent in-plane thickness uniformity and improved in-plane uniformity in optical properties. The polymerizable compound of the present disclosure is represented by formula (I):

$$P^1-Y^7-G^1-Y^5\left[B^1-Y^3\right]_n A^1-Y^1-Ar-Y^2-A^2\left[Y^4-B^2\right]_m Y^6-G^2-Y^8-P^2$$

(I)

where Ar is represented by the following formula (II-1) or (II-2):

EP 3 604 361 A1

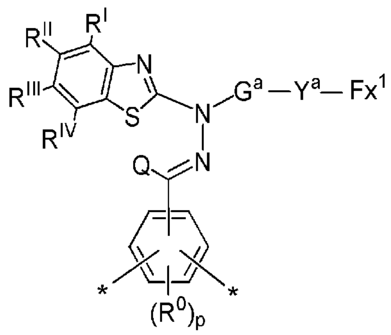

(II-1)

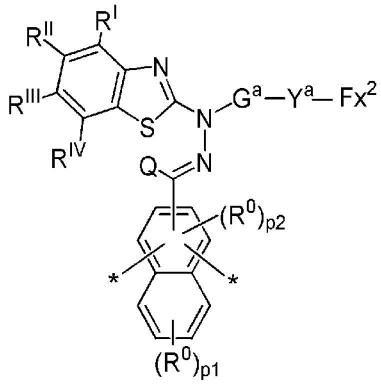

(II-2)

## Description

TECHNICAL FIELD

The present disclosure relates to an optical film and an optically anisotropic body which have excellent in-plane thickness uniformity and improved in-plane uniformity in optical properties, and to a polarizing plate, a display device and an antireflection film in which the optically anisotropic body is used.

The present disclosure also relates to a polymerizable compound, a polymerizable composition and a polymer, which may be used in the preparation of the aforementioned optical film and the optically anisotropic body, and to a compound which may be used in the preparation of the polymerizable compound.

The present disclosure also relates to a method for producing the aforementioned polymerizable compound, and, a method for using the aforementioned compound.

BACKGROUND

Examples of retardation plates used in various devices such as flat panel displays include quarter-wave plates that convert linearly polarized light to circularly polarized light and half-wave plates that perform 90° conversion of the plane of vibration of linearly polarized light. These retardation plates can accurately impart a retardation of 1/4λ or 1/2λ of the wavelength of light with respect to specific monochromatic light.

However, conventional retardation plates have a problem that polarized light that passes therethrough and is output therefrom is converted to colored polarized light. Since a constituent material of the retardation plate has a property of wavelength dispersion with respect to retardation, and a distribution arises in the polarization state of each wavelength for white light, which is a composite wave in which light in the visible region is mixed, it is impossible to achieve accurate adjustment to polarized light with a retardation of 1/4λ or 1/2λ over the entire wavelength region of input light.

In order to solve this problem, various retardation plates which are wideband retardation plates that can achieve uniform retardation with respect to light over a wide wavelength region having so-called reverse wavelength dispersion have been considered.

On the one hand, it has been desired to reduce the thickness of the flat panel display device as much as possible along with an improvement in functionality and widespread use of information terminals such as mobile personal computers and mobile phones. Therefore, a reduction in the thickness of the retardation plates which are components has also been desired.

In terms of methods for achieving thickness-reduction, a method in which a retardation plate is produced by applying a polymerizable composition comprising a low-molecular weight polymerizable compound onto a film substrate to form an optical film has been regarded as the most effective method in recent years. For this reason, polymerizable compounds capable of forming optical films having excellent reverse wavelength dispersion or polymerizable compositions containing such compounds have been widely developed.

Specifically, polymerizable compounds have been provided for use in the production of an optical film such as a polarizing plate or a retardation plate capable of uniform conversion of polarized light over a wide wavelength band (for example, refer to PTL 1).

CITATION LIST

Patent Literature

PTL 1: WO2014/010325

SUMMARY

(Technical Problem)

Here, in order to exert an excellent reverse wavelength dispersion over a wide wavelength band, optical films and the like are required to exhibit ideal retardation characteristics such that the retardation value increases in proportion to the wavelength on both the longer wavelength side and the short wavelength side. Further, accompanying the large area of a liquid crystal display (LCD) and an organic EL display (OLED), the demand for in-plane uniformity of an optical film or the like has been growing. However, as described in PTL 1, the conventional polymerizable compounds have room for improvement of the application properties, and improvement in the in-plane uniformity of the film thickness for the optical film to be obtained, and consequently, the in-plane uniformity in the optical properties such as the retardation.

The present disclosure was conceived in view of the above-described circumstances, and an object of the

present disclosure is to provide a polymerizable compound, a polymerizable composition and a polymer, which are able to form an optical film and an optically anisotropic body which have excellent in-plane thickness uniformity and improved in-plane uniformity in optical properties.

Another object of the present disclosure is to provide a compound which can be used in the preparation of the aforementioned polymerizable compound.

Still another object of the present disclosure is to provide a method for producing the aforementioned polymerizable compound, and, a method for using the aforementioned compound.

Yet another object of the present disclosure is to provide an optical film and an optically anisotropic body which have excellent in-plane thickness uniformity and improved in-plane uniformity in optical properties, and a polarizing plate, a display device and an antireflection film in which the optically anisotropic body is used.

(Solution to Problem)

The inventors performed keen research for solving the aforementioned problems, and as a result, it was discovered that an optical film and an optically anisotropic body which have excellent in-plane thickness uniformity and improved in-plane uniformity in optical properties can be formed when a predetermined polymerizable compound represented by the following formula (I) is used, and completed the present disclosure.

Accordingly, the present disclosure provides a polymerizable compound and a method for producing the same, a polymerizable composition, a polymer, an optical film, an optically anisotropic body, a polarizing plate, a display device, an antireflection film, and, a compound and a use of the same given below.

[1] A polymerizable compound represented by the following formula (I):

$$P^1-Y^7-G^1-Y^5\left[B^1-Y^3\right]_n A^1-Y^1-Ar-Y^2-A^2\left[Y^4-B^2\right]_m Y^6-G^2-Y^8-P^2$$

(I)

where in the formula (I), Ar is represented by the following formula (II-1) or (II-2),

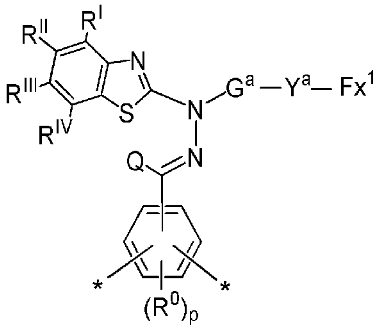

(II-1)

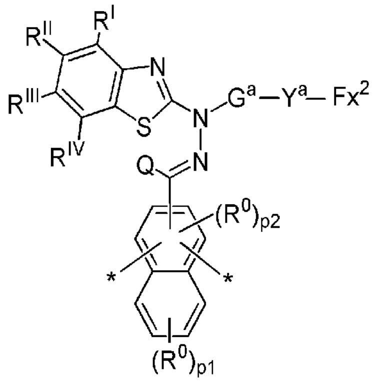

(II-2)

where in the formulas (II-1) and (II-2),

$Fx^1$ and $Fx^2$ each independently represent an organic group having at least one of an aromatic hydrocarbon ring and an aromatic heterocyclic ring,

$Y^a$ represents a chemical single bond, -O-, -C(=O)-, -C(=O)-O-, -O-C(=O)-, -O-C(=O)-O-, -C(=O)-S-, -S-C(=O)-, $-NR^{11}$-C(=O)-, -C(=O)-$NR^{11}$-, -O-C(=O)-$NR^{11}$-, $-NR^{11}$-C(=O)-O-, -S-, -N=N-, or -C=C-, where $R^{11}$ represents a hydrogen atom or an alkyl group having 1 to 6 carbon atoms,

$G^a$ is an organic group having 1 to 30 carbon atoms, preferably 3 to 30 carbon atoms, which may have a substituent,

Q represents a hydrogen atom, or an alkyl group having 1 to 6 carbon atoms which may have a substituent,

$R^I$ to $R^{IV}$ each independently represent a hydrogen atom, a halogen atom, an alkyl group having 1 to 6 carbon atoms, a cyano group, a nitro group, an alkyl group having 1 to 6 carbon atoms in which at least one hydrogen

atom is substituted with a halogen atom, an alkoxy group having 1 to 6 carbon atoms, -OCF3, -C(=O)-O-$R^a$, or, -O-C(=O)-$R^a$, where $R^a$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring having 5 to 12 carbon atoms which may have a substituent, where $R^I$ to $R^{IV}$ may be the same or different, and one or more ring constituents C-$R^I$ to C-$R^{IV}$ may be replaced by a nitrogen atom,

$R^0$ represents a halogen atom, an alkyl group having 1 to 6 carbon atoms, a cyano group, a nitro group, an alkyl group having 1 to 6 carbon atoms in which at least one hydrogen atom is substituted with a halogen atom, an alkoxy group having 1 to 6 carbon atoms, -OCF3, -C(=O)-O-$R^a$, or -O-C(=O)-$R^a$, where $R^a$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring having 5 to 12 carbon atoms which may have a substituent, and when there is a plurality of $R^0$, the plurality of $R^0$ may be the same or different from each other,

* represents a bond with $Y^1$ or $Y^2$, and

p represents an integer from 0 to 3, p1 represents an integer from 0 to 4, and p2 represents 0 or 1;

$Y^1$ to $Y^8$ each independently represent a chemical single bond, -O-, $-O-CH_2-$, $-CH_2-O-$, $-O-CH_2-CH_2-$, $-CH_2-CH_2-O-$, -C(=O)-O-, -O-C(=O)-, -O-C(=O)-O-, -C(=O)-S-, -S-C(=O)-, $-NR^{13}-C(=O)-$, $-C(=O)-NR^{13}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-C(=O)-O-$, $-O-C(=O)-CH_2-$, $-CH_2-O-C(=O)-$, $-C(=O)-O-CH_2-$, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or -C=C-, where $R^{13}$ represents a hydrogen atom or an alkyl group having 1 to 6 carbon atoms;

$A^1$, $A^2$, $B^1$ and $B^2$ each independently represent a cyclic aliphatic group which may have a substituent, or an aromatic group which may have a substituent;

$G^1$ and $G^2$ each independently represent an organic group which is either a divalent aliphatic hydrocarbon group having 1 to 30 carbon atoms, or a divalent aliphatic hydrocarbon group having 3 to 30 carbon atoms in which at least one $-CH_2-$ contained in the divalent aliphatic hydrocarbon group is substituted with -O-, -S-, -O-C(=O)-, -C(=O)-O-, -O-C(=O)-O-, $-NR^{14}-C(=O)-$, $-C(=O)-NR^{14}-$, $-NR^{14}-$, or -C(=O)-, with the proviso that cases where there are two or more contiguous -O- or -S- are excluded, where $R^{14}$ represents a hydrogen atom or an alkyl group having 1 to 6 carbon atoms, and the hydrogen atoms included in the organic group of $G^1$ and $G^2$ may be substituted by an alkyl group having 1 to 5 carbon atoms, an alkoxy group having 1 to 5 carbon atoms, a cyano group, or a halogen atom;

$P^1$ and $P^2$ each independently represent an alkenyl group having 2 to 10 carbon atoms which may be substituted by a halogen atom or a methyl group; and

n and m each independently represent 0 or 1.

[2] The polymerizable compound according to [1], wherein the number of $\pi$ electrons included in the ring structure in Ar is 22 or more.

[3] The polymerizable compound according to [1] or [2], wherein the number of $\pi$ electrons included in the ring structure in $Fx^1$ is 8 or more, and the number of $\pi$ electrons included in the ring structure in $Fx^2$ is 4 or more.

[4] The polymerizable compound according to any one of [1] to [3], wherein $G^a$ is an organic group which is either a divalent aliphatic hydrocarbon group having 1 to 30 carbon atoms (preferably 3 to 30 carbon atoms), or a divalent aliphatic hydrocarbon group having 3 to 30 carbon atoms in which at least one $-CH_2-$ contained in the divalent aliphatic hydrocarbon group is substituted with -O-, -S-, -O-C(=O)-, -C(=O)-O-, -O-C(=O)-O-, $-NR^{12}-C(=O)-$, $-C(=O)-NR^{12}-$, $-NR^{12}-$, or -C(=O)-, with the proviso that cases where there are two or more contiguous -O- or -S- are excluded, where $R^{12}$ represents a hydrogen atom or an alkyl group having 1 to 6 carbon atoms, and the hydrogen atoms included in the organic group of $G^a$ may be substituted by an alkyl group having 1 to 5 carbon atoms, an alkoxy group having 1 to 5 carbon atoms, a cyano group, or a halogen atom.

[5] The polymerizable compound according to any one of [1] to [4], wherein $G^a$ is an organic group which is either a divalent chain aliphatic hydrocarbon group having 1 to 18 carbon atoms (preferably 3 to 18 carbon atoms), or a divalent chain aliphatic hydrocarbon group having 3 to 18 carbon atoms in which at least one $-CH_2-$ contained in the divalent chain aliphatic hydrocarbon group is substituted with -O-, -S-, -O-C(=O)-, -C(=O)-O-, or -C(=O)-, with the proviso that cases where there are two or more contiguous -O- or -S- are excluded, and the hydrogen atoms included in the organic group of $G^a$ may be substituted by an alkyl group having 1 to 5 carbon atoms, an alkoxy group having 1 to 5 carbon atoms, a cyano group, or, a halogen atom.

[6] The polymerizable compound according to any one of [1] to [5], wherein the $G^a$ is an alkylene group having 1 to 18 carbon atoms, preferably 3 to 18 carbon atoms.

[7] The polymerizable compound according to any one of [1] to [6] represented by the following formula (III-1) or (III-2).

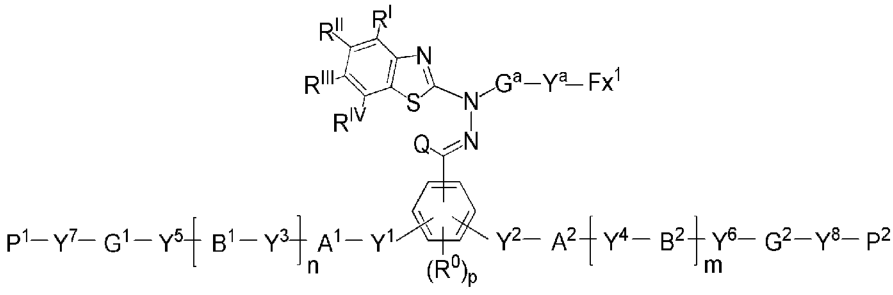

(III-1)

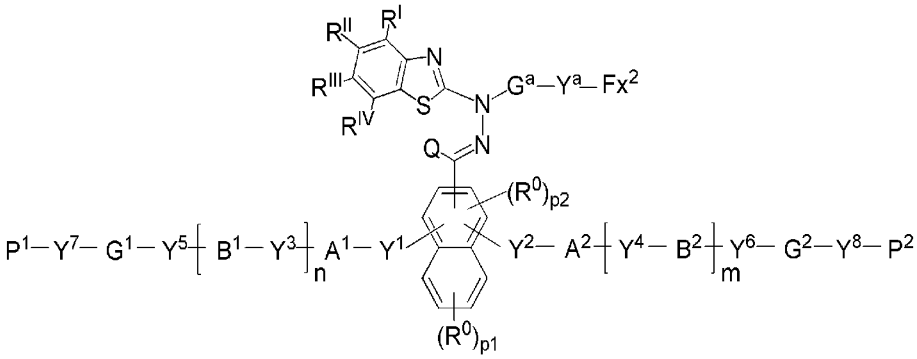

(III-2)

where in the formulas (III-1) and (III-2), $Y^1$ to $Y^8$, $A^1$, $A^2$, $B^1$, $B^2$, $G^1$, $G^2$, $P^1$, $P^2$, $R^I$ to $R^{IV}$, Q, $R^0$, n, m, p, p1 and p2 are the same as defined above;

$G^a$ represents an organic group which is either an alkylene group having 1 to 18 carbon atoms (preferably 3 to 18 carbon atoms) which may have a substituent, or an alkylene group having 3 to 18 carbon atoms in which at least one $-CH_2-$ contained in the alkylene group is substituted with -O-, -S-, -O-C(=O)-, -C(=O)-O-, or -C(=O)-, with the proviso that cases where there are two or more contiguous -O- or -S- are excluded;

$Y^a$ represents a chemical single bond, -O-, -C(=O)-, -C(=O)-O-, -O-C(=O)-, -O-C(=O)-O-, -C(=O)-S-, -S-C(=O)-, $-NR^{11}$-C(=O)-, -C(=O)-$NR^{11}$-, -O-C(=O)-$NR^{11}$-, $-NR^{11}$-C(=O)-O-, -S-, and

$R^{11}$ represents a hydrogen atom or an alkyl group having 1 to 6 carbon atoms;

$Fx^1$ and $Fx^2$ each independently represent an organic group having 2 to 30 carbon atoms having one of an aromatic hydrocarbon ring and an aromatic heterocyclic ring; and

the number of $\pi$ electrons included in the ring structure in $Fx^1$ is 8 or more, and the number of $\pi$ electrons included in the ring structure in $Fx^2$ is 4 or more.

[8] The polymerizable compound according to any one of [1] to [7], wherein the number of $\pi$ electrons included in the ring structure in $Fx^1$ is 10 or more, and the number of $\pi$ electrons included in the ring structure in $Fx^2$ is 6 or more.

[9] The polymerizable compound according to any one of [1] to [8], wherein the $Fx^1$ and $Fx^2$ are each independently an alkyl group having 1 to 18 carbon atoms in which at least one hydrogen atom is substituted with a ring-containing group having at least one of an aromatic hydrocarbon ring and an aromatic heterocyclic ring and which may have a substituent other than the ring-containing group; or a cyclic group having 2 to 20 carbon atoms having at least one of an aromatic hydrocarbon ring and an aromatic heterocyclic ring and which may have a substituent.

[10] The polymerizable compound according to any one of [1] to [9], wherein $Fx^1$ is represented by any of the following formulas (i-1) to (i-9),

the $Fx^2$ is represented by any of the following formulas (I-1) to (I-11), and

the groups represented by the following formulas (i-1) to (i-11) may have a substituent:

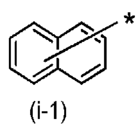
(i-1)

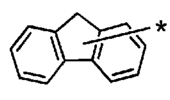
(i-2)

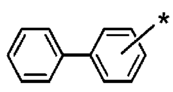
(i-3)

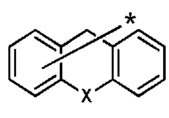
(i-4)

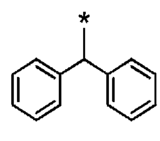
(i-5)

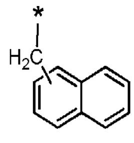
(i-6)

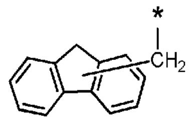
(i-7)

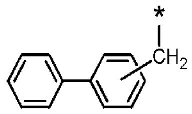
(i-8)

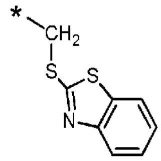
(i-9)

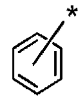
(i-10)

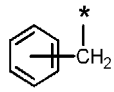
(i-11)

where in formula (i-4), X represents $-CH_2-$, $-NR^d-$, an oxygen atom, a sulfur atom, -SO- or $-SO_2-$, and $R^d$ represents a hydrogen atom or an alkyl group having 1 to 6 carbon atoms.

[11] The polymerizable compound according to any one of [1] to [10], wherein $G^1$ and $G^2$ each independently represent an organic group which is either a divalent aliphatic hydrocarbon group having 1 to 18 carbon atoms, or a divalent aliphatic hydrocarbon group having 3 to 18 carbon atoms in which at least one $-CH_2-$ contained in the divalent aliphatic hydrocarbon group is substituted with -O-, -S-, -O-C(=O)-, -C(=O)-O-, -O-C(=O)-O-, $-NR^{14}-C(=O)-$, $-C(=O)-NR^{14}-$, $-NR^{14}-$, or -C(=O)-, with the proviso that cases where there are two or more contiguous -O- or -S- are excluded, where $R^{14}$ represents a hydrogen atom or an alkyl group having 1 to 6 carbon atoms, the hydrogen atoms included in the organic group of $G^1$ and $G^2$ may be substituted by an alkyl group having 1 to 5 carbon atoms, an alkoxy group having 1 to 5 carbon atoms, a cyano group, or a halogen atom.

[12] The polymerizable compound according to any one of [1] to [11], wherein $G^1$ and $G^2$ are each independently an alkylene group having 1 to 18 carbon atoms which may have at least one substituent selected from the group consisting of an alkyl group having 1 to 5 carbon atoms, an alkoxy group having 1 to 5 carbon atoms, a cyano group and a halogen atom.

[13] The polymerizable compound according to any one of the [1] to [12], wherein $P^1$ and $P^2$ are each independently $-CH_2=CH-$,$-CH_2=C(CH_3)-$, or $-CH_2=C(Cl)-$.

[14] The polymerizable compound according to any one of [1] to [13], wherein $Y^1$ to $Y^8$ are each independently a chemical single bond, -O-, -O-C(=O)-, -C(=O)-O-, or -O-C(=O)-O-.

[15] The polymerizable compound according to any one of [1] to [14], wherein $A^1$ and $A^2$ are each independently a trans-1,4-cyclohexelene group which may have a substituent, and $B^1$ and $B^2$ are each independently a 1,4-phenylene group.

[16] The polymerizable compound according to any one of [1] to [6] represented by the following formula (iii-1):

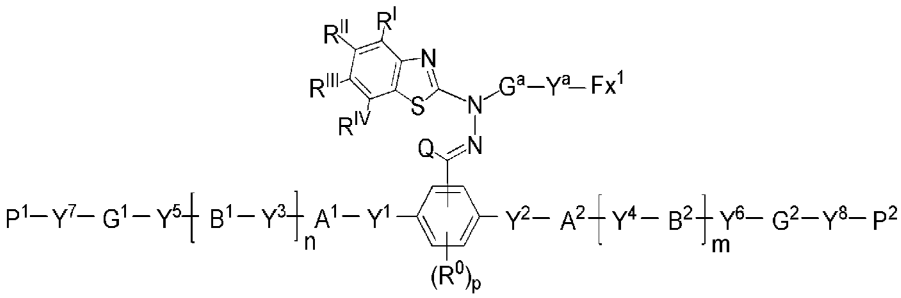
(iii-1)

where in the formula (iii-1), $Y^1$ to $Y^8$, $A^1$, $A^2$, $B^1$, $B^2$, $G^1$, $G^2$, $P^1$, $P^2$, $R^I$ to $R^{IV}$, Q, $G^a$, $Y^a$, $Fx^1$, $R^0$, m, n and p are the same as defined above.

[17] The polymerizable compound according to any one of [1] to [16] represented by any of the following formulas (1) to (21):

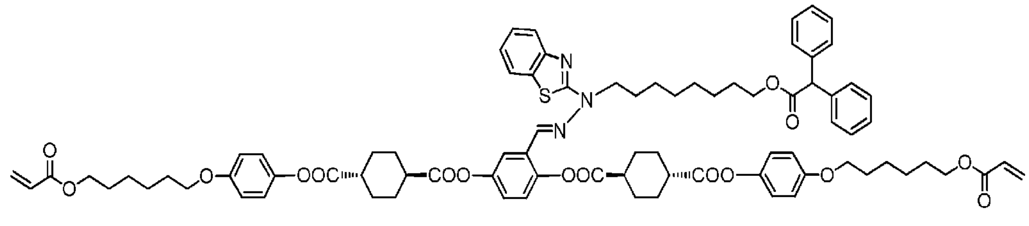
(1)

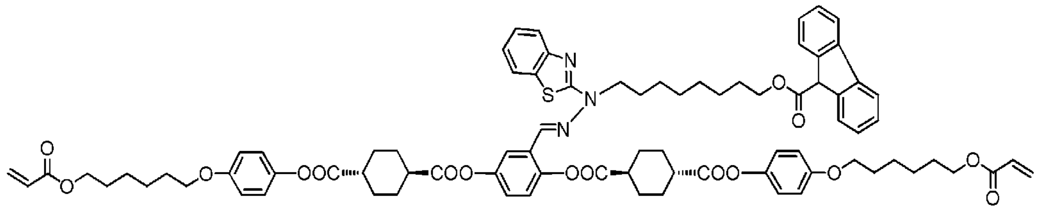
(2)

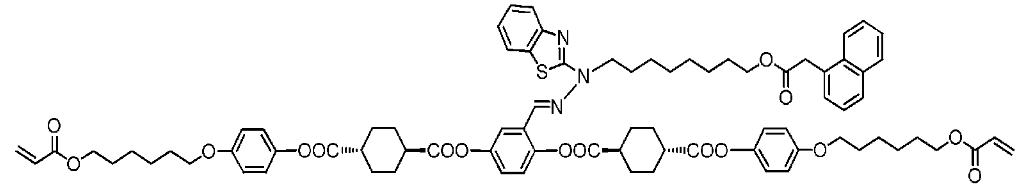
(3)

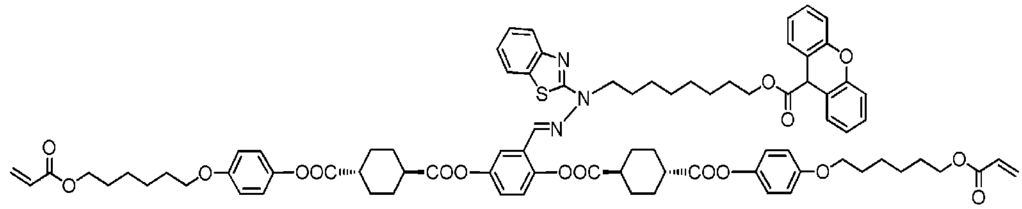
(4)

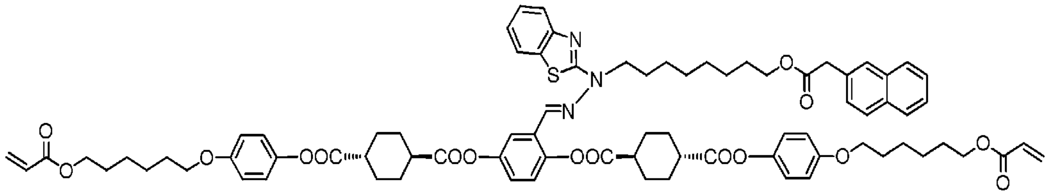
(5)

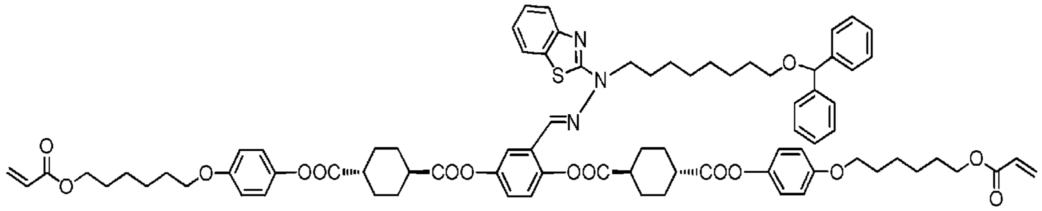
(6)

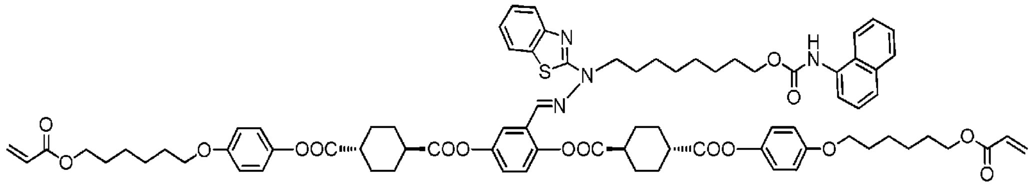
(7)

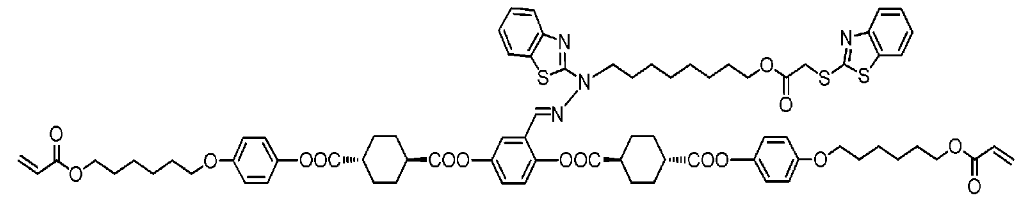
(8)

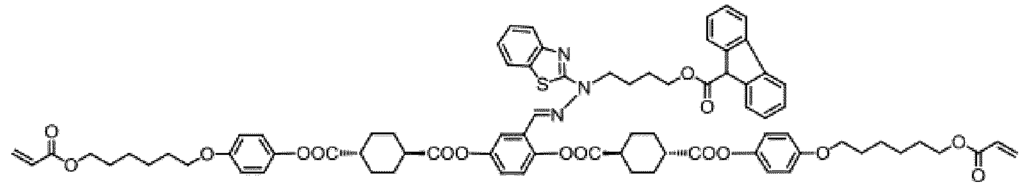

(9)

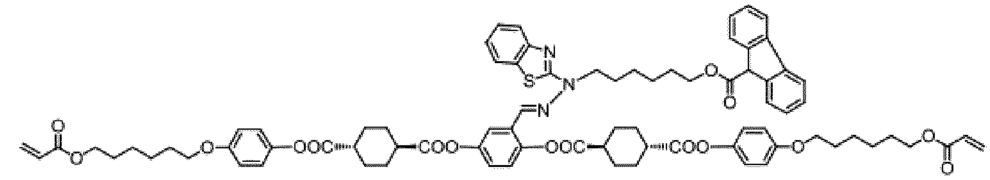

(10)

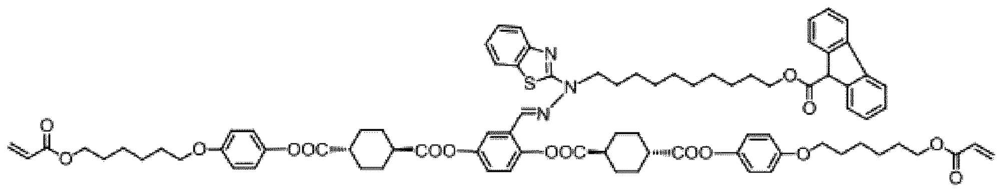

(11)

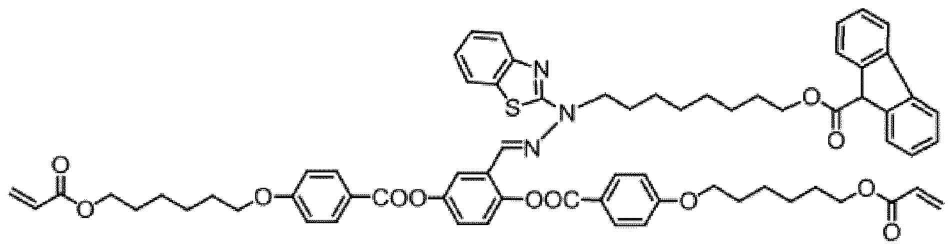

(12)

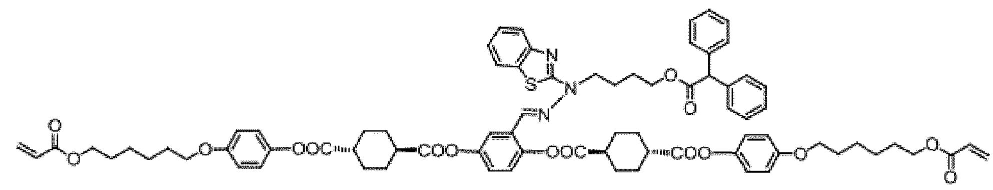

(13)

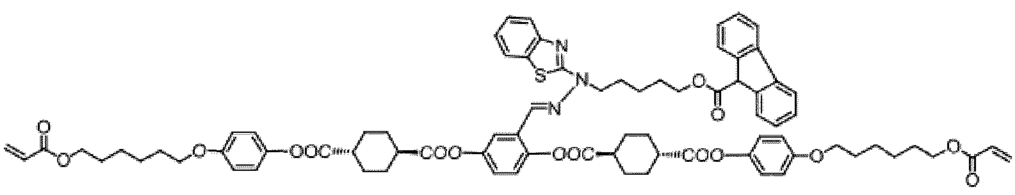

(14)

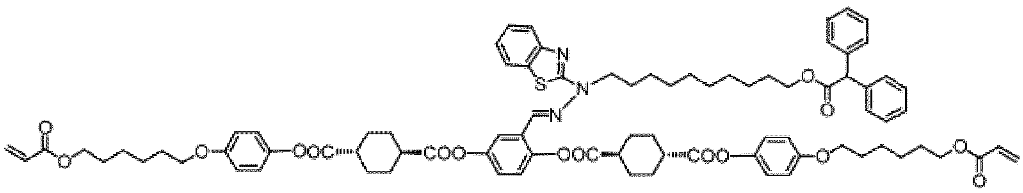

(15)

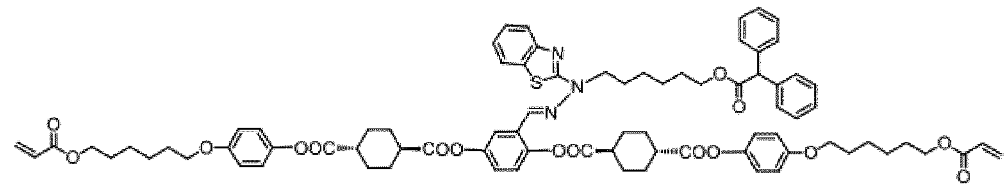
(16)

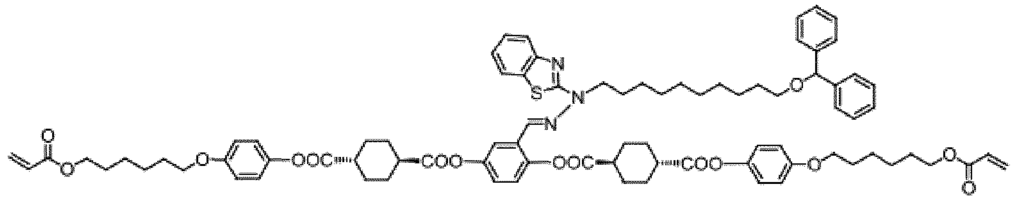
(17)

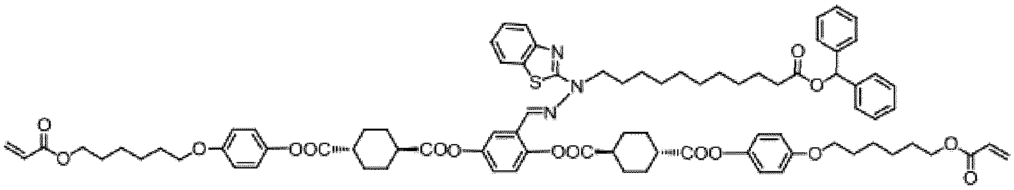
(18)

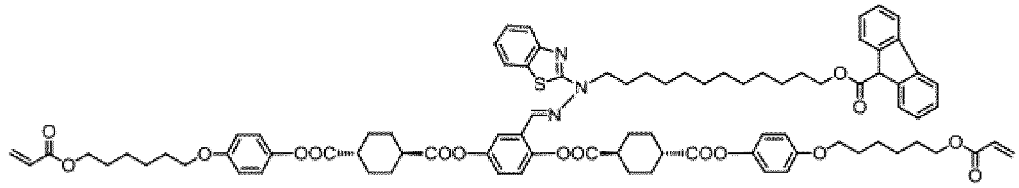
(19)

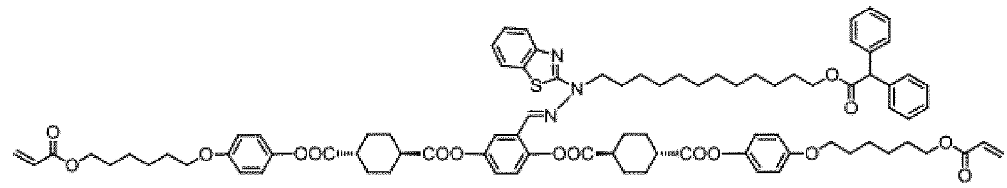
(20)

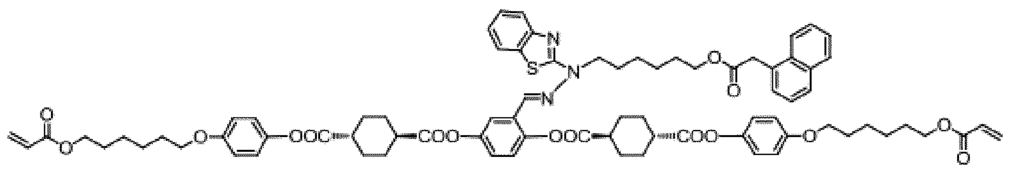
(21)

[18] A polymerizable composition comprising the polymerizable compound according to [1] to [17].
[19] A polymer obtainable by polymerizing the polymerizable compound according to [1] to [17].
[20] An optical film comprising the polymer according to [19] as a constituent material.
[21] An optically anisotropic body comprising a layer which comprises the polymer according to [19] as a constituent material.
[22] A polarizing plate comprising the optically anisotropic body according to [21] and a polarizing film.
[23] A display device comprising the polarizing plate according to [22].
[24] An antireflection film comprising the polarizing plate according to [22].
[25] A compound represented by the following formula (IV):

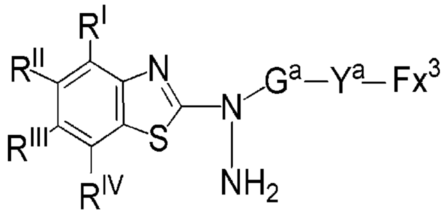

(IV)

where in the formula (IV), $R^I$ to $R^{IV}$ each independently represent a hydrogen atom, a halogen atom, an alkyl group having 1 to 6 carbon atoms, a cyano group, a nitro group, an alkyl group having 1 to 6 carbon atoms in which at least one hydrogen atom is substituted with a halogen atom, an alkoxy group having 1 to 6 carbon atoms, -OCF3, -C(=O)-O-$R^a$, or -O-C(=O)-$R^a$, where $R^a$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring having 5 to 12 carbon atoms which may have a substituent, where $R^I$ to $R^{IV}$ may be the same or different, and one or more ring constituents C-$R^I$ to C-$R^{IV}$ may be replaced by a nitrogen atom;

$Y^a$ represents a chemical single bond, -O-, -C(=O)-, -C(=O)-O-, -O-C(=O)-, -O-C(=O)-O-, -C(=O)-S-, -S-C(=O)-, -$NR^{11}$-C(=O)-, -C(=O)-$NR^{11}$-, -O-C(=O)-$NR^{11}$-, -$NR^{11}$-C(=O)-O-, -S-, -N=N-, or -C=C-, where $R^{11}$ represents a hydrogen atom or an alkyl group having 1 to 6 carbon atoms;

$G^a$ represents an organic group which is either an alkylene group having 3 to 18 carbon atoms which may have a substituent, or an alkylene group having 1 to 18 carbon atoms in which at least one -$CH_2$- contained in the alkylene group is substituted with -O-, -S-, -O-C(=O)-, -C(=O)-O-, or -C(=O)-, with the proviso that cases where there are two or more contiguous -O- or -S- are excluded;

$Fx^3$ is a hydrogen atom, or an organic group having 2 to 20 carbon atoms having at least one of an aromatic hydrocarbon ring and an aromatic heterocyclic ring; and

when $Fx^3$ has a ring structure, the number of $\pi$ electrons included in the ring structure in $Fx^3$ is 4 or more.

[26] The compound according to [25], wherein $G^a$ is an alkylene group having 1 to 18 carbon atoms (preferably 3 to 18 carbon atoms) which may have a substituent, $Y^a$ represents a chemical single bond, -O-, -C(=O)-, -C(=O)-O-, -O-C(=O)-, -O-C(=O)-O-, -O-C(=O)-$NR^{11}$-, -$NR^{11}$-C(=O)-O-, or -S-, where $R^{11}$ represents a hydrogen atom or an alkyl group having 1 to 6 carbon atoms.

[27] The compound according to [25] or [26] represented by the following formulas (A) to (O):

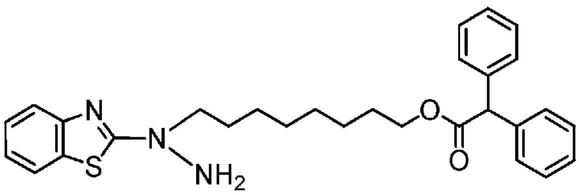

(A)

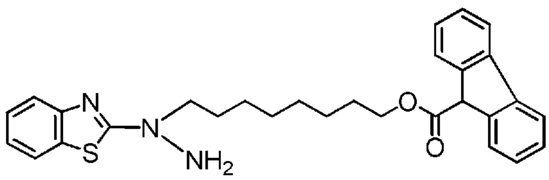

(B)

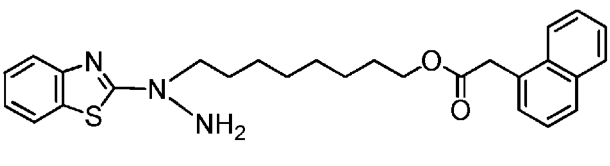

(C)

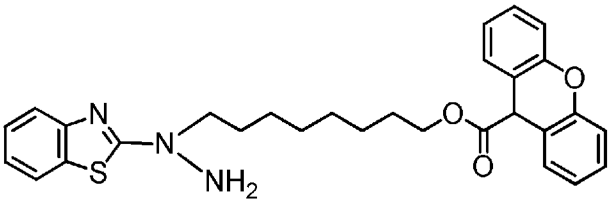

(D)

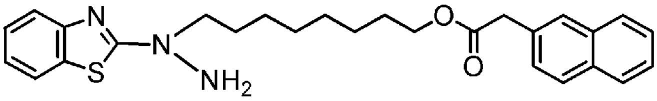

(E)

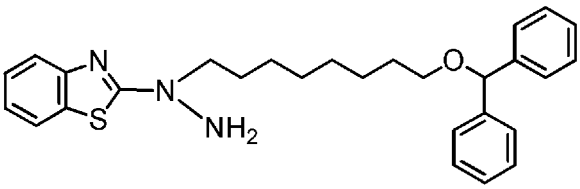

(F)

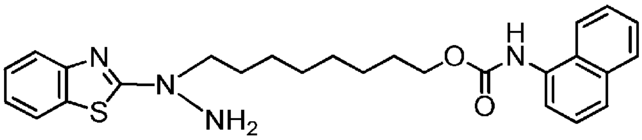

(G)

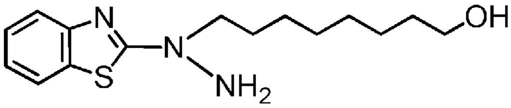

(H)

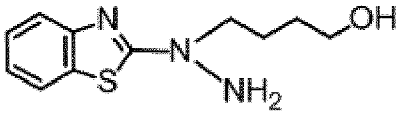

(I)

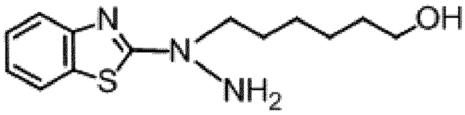

(J)

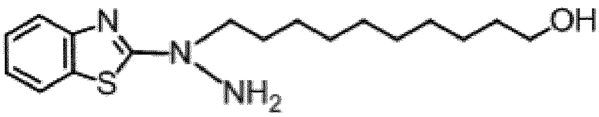

(K)

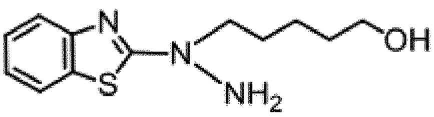

(L)

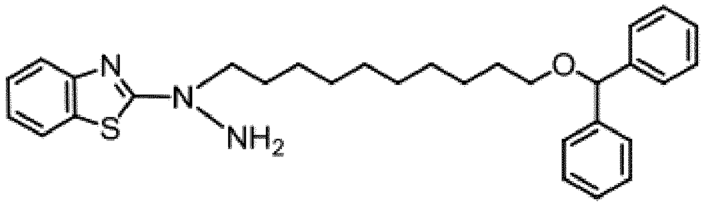

(M)

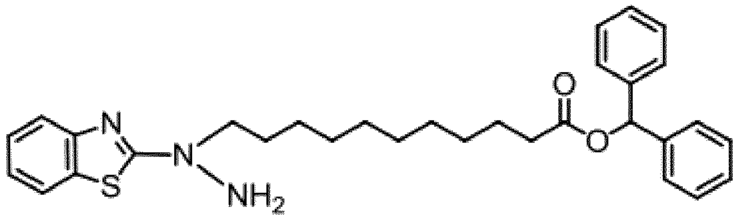

(N)

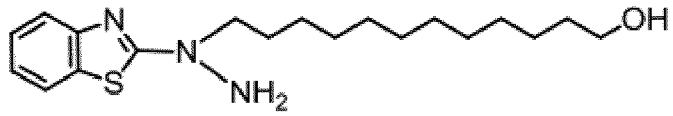

(O)

[28] A method for producing a polymerizable compound, comprising: reacting the compound according to any one of [25] to [27] with a compound represented by the following formula (V-1) or (V-2):

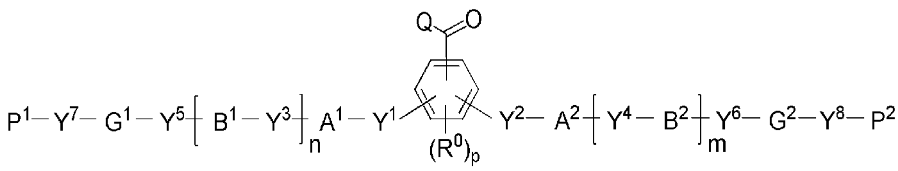

(V-1)

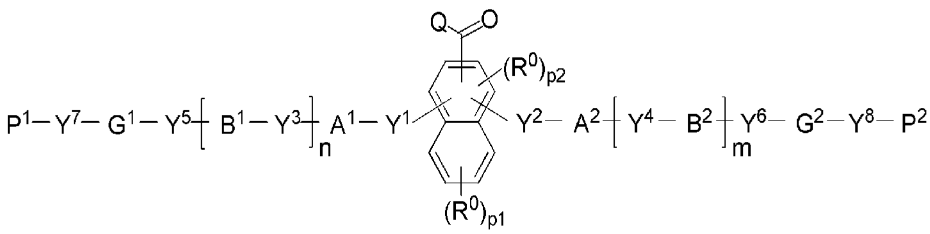

(V-2)

where in the formulas (V-1) and (V-2),

Q represents a hydrogen atom, or an alkyl group having 1 to 6 carbon atoms which may have a substituent;
$R^0$ represents a halogen atom, an alkyl group having 1 to 6 carbon atoms, a cyano group, a nitro group, an alkyl group having 1 to 6 carbon atoms in which at least one hydrogen atom is substituted with a halogen atom, an alkoxy group having 1 to 6 carbon atoms, -OCF3, -C(=O)-O-$R^a$, or -O-C(=O)-$R^a$, where $R^a$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring having 5 to 12 carbon atoms which may have a substituent, and when there is a plurality of $R^0$, the plurality of $R^0$ may be the same or different from each other;
p represents an integer from 0 to 3, p1 represents an integer from 0 to 4, and p2 represents 0 or 1;
$Y^1$ to $Y^8$ each independently represent a chemical single bond, -O-, -O-$CH_2$-, -$CH_2$-O-, -O-$CH_2$-$CH_2$-, -$CH_2$-$CH_2$-O-, -C(=O)-O-, -O-C(=O)-, -O-C(=O)-O-, -C(=O)-S-, -S-C(=O)-, -$NR^{13}$-C(=O)-, -C(=O)-$NR^{13}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-C(=O)-O-, -O-C(=O)-$CH_2$-, -$CH_2$-O-C(=O)-, -C(=O)-O-$CH_2$-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, or -C=C-, where $R^{13}$ represents a hydrogen atom or an alkyl group having 1 to 6 carbon atoms;

$A^1$, $A^2$, $B^1$ and $B^2$ each independently represent a cyclic aliphatic group which may have a substituent, or an aromatic group which may have a substituent;

$G^1$ and $G^2$ each independently represent an organic group which is either a divalent aliphatic hydrocarbon group having 1 to 30 carbon atoms or a divalent aliphatic hydrocarbon group having 3 to 30 carbon atoms in which at least one $-CH_2-$ contained in the divalent aliphatic hydrocarbon group is substituted with -O-, -S-, -O-C(=O)-, -C(=O)-O-, -O-C(=O)-O-, $-NR^{14}-C(=O)-$,

$-C(=O)-NR^{14}-$, $-NR^{14}-$, or -C(=O)-, with the proviso that cases where there are two or more contiguous -O- or -S- are excluded, where $R^{14}$ represents a hydrogen atom or an alkyl group having 1 to 6 carbon atoms, and the hydrogen atoms included in the organic group of $G^1$ and $G^2$ may be substituted by an alkyl group having 1 to 5 carbon atoms, an alkoxy group having 1 to 5 carbon atoms, a cyano group, or, a halogen atom;

$P^1$ and $P^2$ each independently represent an alkenyl group having 2 to 10 carbon atoms which may be substituted by a halogen atom or a methyl group; and

n and m each independently represent 0 or 1.

[29] A method for using the compound according to any one of [25] to [27] to obtain a polymerizable compound.

[30] A compound represented by the following formula (V-3) or (V-4):

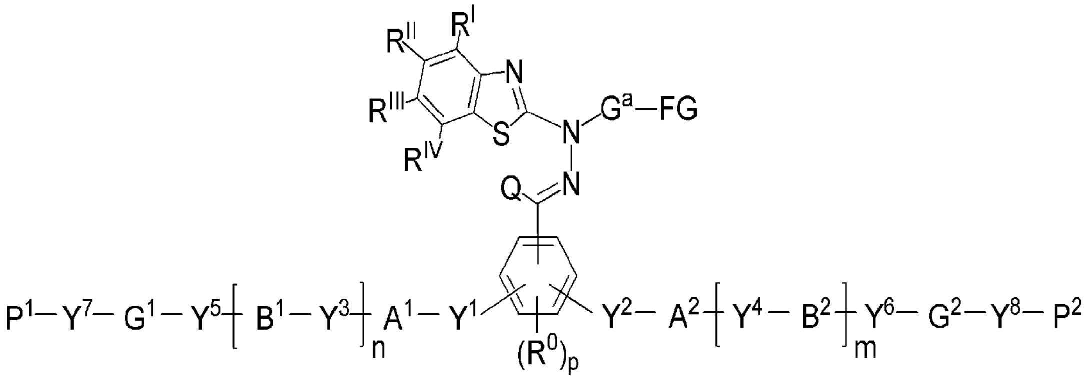

(V − 3)

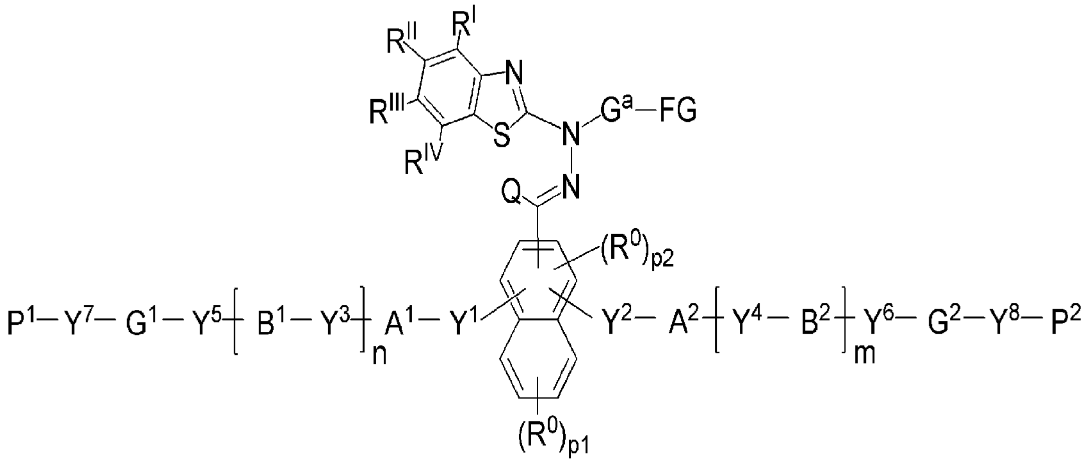

(V − 4)

where in the formulas (V-3) and (V-4),

Q represents a hydrogen atom, or an alkyl group having 1 to 6 carbon atoms which may have a substituent;

$R^0$ represents a halogen atom, an alkyl group having 1 to 6 carbon atoms, a cyano group, a nitro group, an alkyl group having 1 to 6 carbon atoms in which at least one hydrogen atom is substituted with a halogen atom, an alkoxy group having 1 to 6 carbon atoms, -OCF3, $-C(=O)-O-R^a$, or $-O-C(=O)-R^a$, where $R^a$ represents an alkyl group having 1 to 20 carbon atoms which may have a substituent, an alkenyl group having 2 to 20 carbon

atoms which may have a substituent, a cycloalkyl group having 3 to 12 carbon atoms which may have a substituent, or an aromatic hydrocarbon ring having 5 to 12 carbon atoms which may have a substituent, and when there is a plurality of $R^0$, the plurality of $R^0$ may be the same or different from each other;

p represents an integer from 0 to 3, p1 represents an integer from 0 to 4, and p2 represents 0 or 1;

$Y^1$ to $Y^8$ each independently represent a chemical single bond, -O-, $-O-CH_2-$, $-CH_2-O-$, $-O-CH_2-CH_2-$, $-CH_2-CH_2-O-$, -C(=O)-O-, -O-C(=O)-, -O-C(=O)-O-, -C(=O)-S-, -S-C(=O)-, $-NR^{13}-C(=O)-$, $-C(=O)-NR^{13}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-C(=O)-O-$, $-O-C(=O)-CH_2-$, $-CH_2-O-C(=O)-$, $-C(=O)-O-CH_2-$, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or, -C=C-, where $R^{13}$ represents a hydrogen atom or an alkyl group having 1 to 6 carbon atoms;

$A^1$, $A^2$, $B^1$ and $B^2$ each independently represent a cyclic aliphatic group which may have a substituent, or, an aromatic group which may have a substituent;

$G^1$ and $G^2$ each independently represent an organic group which is either a divalent aliphatic hydrocarbon group having 1 to 30 carbon atoms or a divalent aliphatic hydrocarbon group having 3 to 30 carbon atoms in which at least one $-CH_2-$ contained in the divalent aliphatic hydrocarbon group is substituted with -O-, -S-, -O-C(=O)-, -C(=O)-O-, -O-C(=O)-O-, $-NR^{14}-C(=O)-$,

$-C(=O)-NR^{14}-$, $-NR^{14}-$, or -C(=O)-, with the proviso that cases where there are two or more contiguous -O- or -S- are excluded, where $R^{14}$ represents a hydrogen atom or an alkyl group having 1 to 6 carbon atoms, and the hydrogen atoms included in the organic group of $G^1$ and $G^2$ may be substituted by an alkyl group having 1 to 5 carbon atoms, an alkoxy group having 1 to 5 carbon atoms, a cyano group, or, a halogen atom;

$P^1$ and $P^2$ each independently represent an alkenyl group having 2 to 10 carbon atoms which may be substituted by a halogen atom or a methyl group;

n and m each independently represent 0 or 1;

$R^I$ to $R^{IV}$ each independently represent a hydrogen atom, a halogen atom, an alkyl group having 1 to 6 carbon atoms, a cyano group, a nitro group, an alkyl group having 1 to 6 carbon atoms in which at least one hydrogen atom is substituted with a halogen atom, an alkoxy group having 1 to 6 carbon atoms, -OCF3, $-C(=O)-O-R^a$, or $-O-C(=O)-R^a$, where $R^a$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring having 5 to 12 carbon atoms which may have a substituent, where $R^I$ to $R^{IV}$ may be the same or different, and one or more ring constituents $C-R^I$ to $C-R^{IV}$ may be replaced by a nitrogen atom;

$G^a$ represents an organic group which is either an alkylene group having 1 to 18 carbon atoms (preferably 3 to 18 carbon atoms) which may have a substituent, or an alkylene group having 3 to 18 carbon atoms in which at least one $-CH_2-$ contained in the alkylene group is substituted with -O-, -S-, -O-C(=O)-, -C(=O)-O-, or -C(=O)-, with the proviso that cases where there are two or more contiguous -O- or -S- are excluded; and

FG represents -OH, -C(=O)-OH, -SH, or, $-NR^*R^{**}$, where$R^*$ and $R^{**}$ each independently represent a hydrogen atom or an alkyl group having 1 to 6 carbon atoms, with the proviso that $R^*$ and $R^{**}$ are not simultaneously an alkyl group having 1 to 6 carbon atoms.

[31] The compound according to [30] represented by the following formulas (a) to (g):

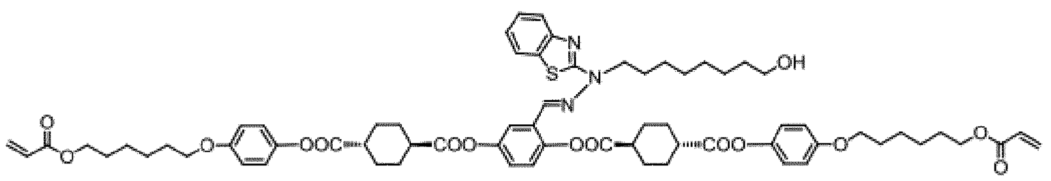

(a)

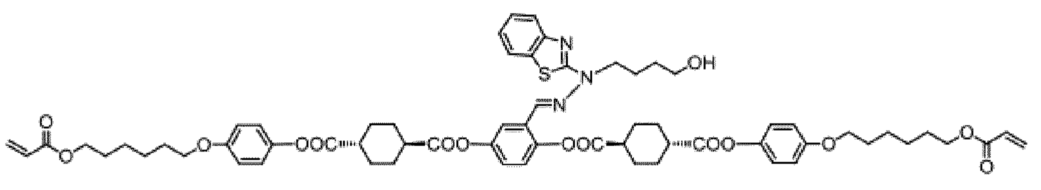

(b)

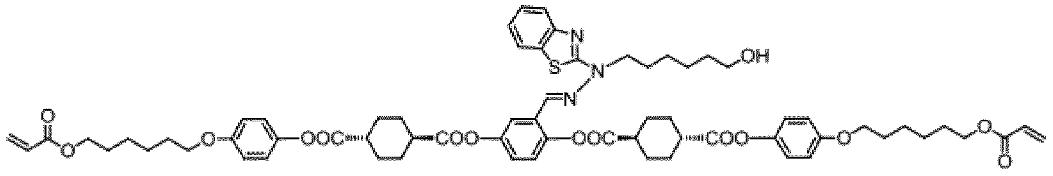
(c)

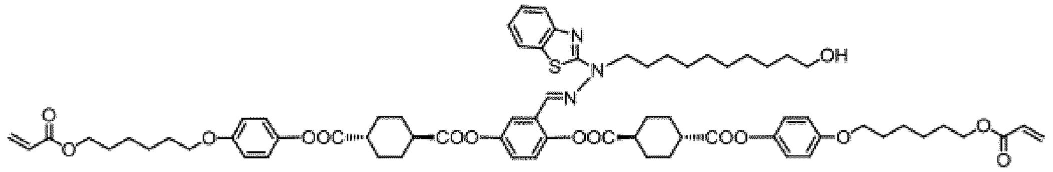
(d)

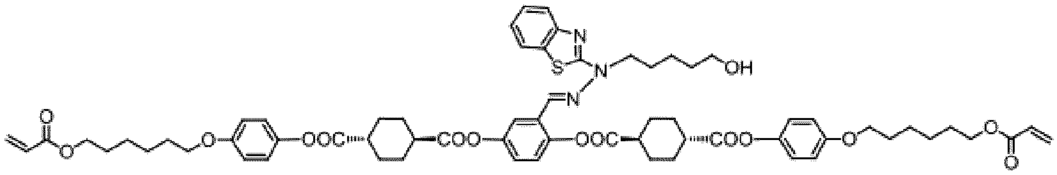
(e)

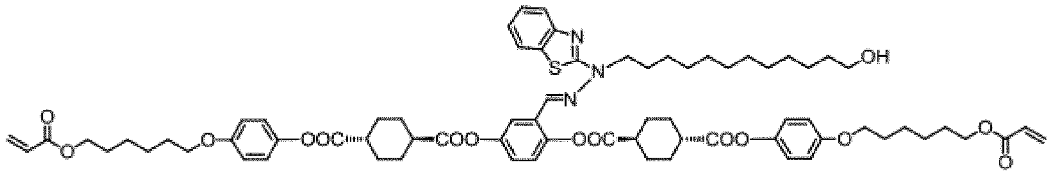
(f)

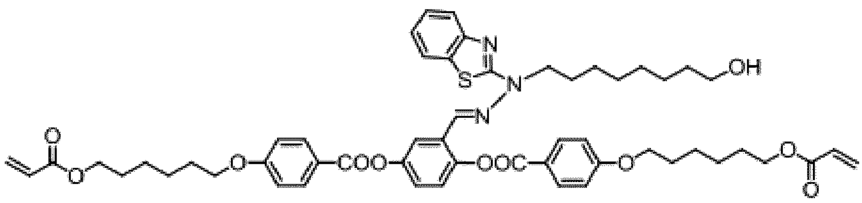
(g)

(Advantageous Effect)

The present disclosure provides a polymerizable compound, a polymerizable composition and a polymer, which are able to form an optical film and an optically anisotropic body having excellent in-plane thickness uniformity and improved in-plane uniformity in optical properties.

Further, the present disclosure provides provide a compound which is useful in the preparation of the aforementioned polymerizable compound.

Further, the present disclosure provides a method for producing the aforementioned polymerizable compound, and, a method for using the aforementioned compound.

Moreover, the present disclosure provides an optical film and an optically anisotropic body which have excellent in-plane thickness uniformity and improved in-plane uniformity in optical properties, and a polarizing plate, a display device and an antireflection film in which the optically anisotropic body is used.

BRIEF DESCRIPTION OF THE DRAWINGS

In the accompanying drawing:
FIG. 1 is an illustration for explaining the measurement position of retardation of the optically anisotropic body of the present disclosure.

DETAILED DESCRIPTION

The present disclosure will be described in detail below. Note that, in the present disclosure, "may have a

substituent" means "unsubstituted, or, having a substituent". Further, when an organic group such as an alkyl group or an aromatic hydrocarbon ring contained in the general formula has a substituent, the number of carbon atoms of the organic group having the substituent does not include the number of carbon atoms of the substituent. For example, when an aromatic hydrocarbon ring having 6 to 20 carbon atoms has a substituent, the number of carbon atoms of the aromatic hydrocarbon ring having 6 to 20 does not include the number of carbon atoms of such a substituent. On the one hand, the "number of $\pi$ electrons included in the ring structure in Ar", the "number of $\pi$ electrons included in the ring structure in $Fx^1$", the "number of $\pi$ electrons included in the ring structure in $Fx^2$" and the "number of $\pi$ electrons included in the ring structure in $Fx^3$" also includes the $\pi$ electron of the ring structure contained in the substituent. Furthermore, in the present disclosure, the phrase "alkyl group" means a chain (linear or branched) saturated hydrocarbon group, and the "alkyl group" does not include a "cyclic alkyl group" which is a cyclic saturated hydrocarbon group.

Here, the polymerizable compound and the polymerizable composition of the present disclosure are not specifically limited, and can be used, for example, when preparing the polymer of the present disclosure.

Furthermore, the polymer of the present disclosure is not specifically limited, and can be used, for example, as a constituent material of the optical film of the present disclosure and as a constituent material of a layer of the optically anisotropic body of the present disclosure. Further, the optically anisotropic body of the present disclosure is not specifically limited, and can be used in, for example, the production of a polarizing plate of the present disclosure. Furthermore, the polarizing plate of the present disclosure is not specifically limited, and can be used in, for example, the production of a display device and an antireflection film of the present disclosure.

Further, the compound (intermediate) of the present disclosure is not specifically limited, and can be used, for example, when preparing the polymerizable compound of the present disclosure.

(1) Polymerizable compound

The polymerizable compound of the present disclosure is the compound represented by Formula (I) (hereinafter, referred to as the "polymerizable compound (I)"), and can be used advantageously when preparing the polymer, the optical film and the optically anisotropic body which are described later.

$$P^1-Y^7-G^1-Y^5\left[B^1-Y^3\right]_n A^1-Y^1-Ar-Y^2-A^2\left[Y^4-B^2\right]_m Y^6-G^2-Y^8-P^2$$

(I)

Note that, as described later, a polymerizable composition having an excellent application property can be obtained by using the compound represented by the formula (I), and can advantageously produce an optical film or the like having excellent in-plane thickness uniformity, and improved in-plane uniformity in optical properties and an optically anisotropic body.

Here, in the formula (I), Ar is represented by the following formula (II-1) or (II-2), preferably the following formula (II-3) or (II-4). The total number of $\pi$ electrons contained in the ring structure of Ar is preferably 22 or more, more preferably 24 or more, and preferably 50 or less, more preferably 40 or less, and 30 or less is particularly preferable. Here, the "total number of $\pi$ electrons contained in the ring structure of Ar" means the number of $\pi$ electrons included in the one ring structure when the there is one ring structure contained in Ar, and when there is a plurality of ring structures in Ar, means the total number of $\pi$ electrons in the plurality of ring structures.

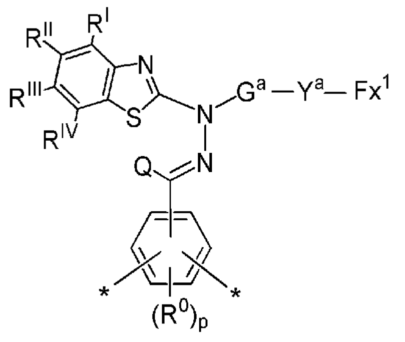

(II-1)

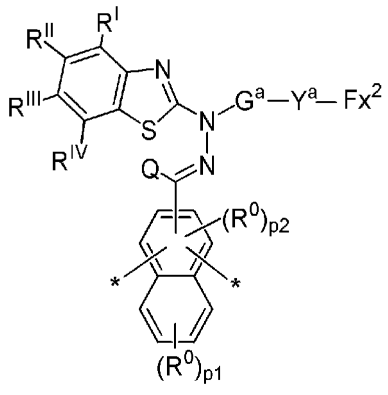

(II-2)

where in the formulas (II-1) and (II-2), * represents a bond with $Y^1$ or $Y^2$.

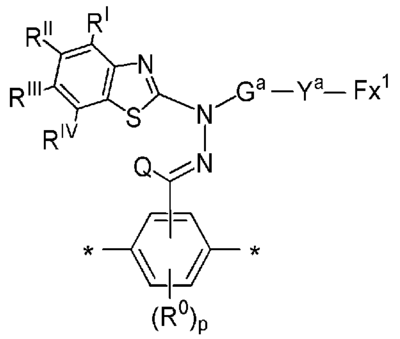

(II-3)

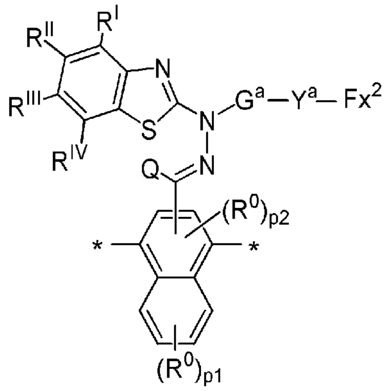

(II-4)

where in the formulas (II-3) and (II-4), * represents a bond with $Y^1$ or $Y^2$.

Further, $Fx^1$ and $Fx^2$ each independently represent an organic group having at least one of an aromatic hydrocarbon ring and an aromatic heterocyclic ring. The number of carbon atoms of the organic group of $Fx^1$ and $Fx^2$ is preferably 2 to 30, preferably 7 or more, even more preferably 8 or more, and 10 or more is particularly preferable. Further, $Fx^1$ and $Fx^2$ preferably have a fused ring structure, or, have two or more single rings of at least one type selected from the group consisting of an aromatic hydrocarbon single ring and an aromatic heterocyclic single ring. The organic groups of $Fx^1$ and $Fx^2$ may have one or more of only the aromatic hydrocarbon ring, may have one or more of only the aromatic heterocyclic ring, and may have one or more aromatic hydrocarbon ring and one or more aromatic heterocyclic ring. Further, when the organic groups of $Fx^1$ and $Fx^2$ have a plurality of aromatic hydrocarbon rings and/or aromatic heterocyclic rings, the rings may be the same or different.

Note that, examples of the aromatic hydrocarbon ring of $Fx^1$ and $Fx^2$ include aromatic hydrocarbon rings having 6 to 30 carbon atoms such as a benzene ring, a naphthalene ring, an anthracene ring, a phenanthrene ring, a pyrene ring, a fluorene ring.

Thereamong, a benzene ring, a naphthalene ring, an anthracene ring, and a fluorene ring are preferable as the aromatic hydrocarbon ring.

Further, examples of the aromatic heterocyclic ring of $Fx^1$ and $Fx^2$ include aromatic heterocycles having 2 to 30 carbon atoms such as a 1H-isoindole-1,3 (2H)-dione ring, a 1-benzofuran ring, a 2-benzofuran ring, an acridine ring, an isoquinoline ring, an imidazole ring, an indole ring, an oxadiazole ring, an oxazole ring, an oxazolopyrazine ring, an oxazolopyridine ring, an oxazolopyridazyl ring, an oxazolopyrimidine ring, a quinazoline ring, a quinoxaline ring, a quinoline ring, a cinnoline ring, a thiadiazole ring, a thiazole ring, a thiazolopyrazine ring, a thiazolopyridine ring, a thiazolopyridazine ring, a thiazolopyrimidine ring, a thiophene ring, a triazine ring, a triazole ring, a naphthyridine ring, a pyrazine ring, a pyrazole ring, a pyranone ring, a pyran ring, a pyridine ring, a pyridazine ring, a pyrimidine ring, a pyrrole ring, a phenanthridine ring, a phthalazine ring, a furan ring, a benzo[c]thiophene ring, a benzisoxazole ring, a benzisothiazole ring, a benzimidazole ring, a benzoxadiazole ring, a benzoxazole ring, a benzothiadiazole ring, a benzothiazole ring, a benzothiophene ring, a benzotriazine ring, a benzotriazole ring, a benzopyrazole ring, a benzopyranone ring.

Thereamong, a monocyclic aromatic heterocyclic ring such as a furan ring, a pyran ring, a thiophene ring, an oxazole ring, an oxadiazoyl ring, a thiazole ring, and a thiadiazole ring; a fused aromatic heterocyclic ring such as a benzothiazole ring, a benzoxazole ring, a quinoline ring, a 1 -benzofuran ring, a 2-benzofuran ring, a benzo[b]thiophen ring, a 1H-isoindole-1,3 (2H)-dione ring, a benzo[c]thiophene ring, a thiazolopyridine ring, a thiazolopyrazine ring, a benzoisoxazole ring, a benzoxadiazole ring, a benzothiadiazole ring and the like are preferable as the aromatic heterocyclic ring.

The aromatic hydrocarbon ring and an aromatic heterocyclic ring of $Fx^1$ and $Fx^2$ may have a substituent. Examples of such a substituent include a halogen atom such as a fluorine atom and a chlorine atom; a cyano group; an alkyl group having 1 to 6 carbon atoms such as a methyl group, an ethyl group, and a propyl group; an alkenyl group having 2 to 6 carbon atoms such as a vinyl group and an allyl group; an alkyl group having 1 to 6 carbon atoms in which at least one hydrogen atom is substituted with a halogen atom such as a trifluoromethyl group and a pentafluoroethyl group; an N-N-dialkylamino group having 2 to 12 carbon atoms such as a dimethylamino group; an alkoxy group having 1 to 6 carbon atoms such as a methoxy group, an ethoxy group and an isopropoxy group; a nitro group; an aromatic hydrocarbon ring having 6 to 20 carbon atoms such as a phenyl group and a naphthyl group; $-OCF_3$, $-C(=O)-R^a$; $-C(=O)-O-R^a$; $-O-C(=O)-R^a$; and $SO_2R^b$; and the like. Here, $R^a$ represents an alkyl group having 1 to 20 carbon atoms which may

have a substituent, an alkenyl group having 2 to 20 carbon atoms which may have a substituent, a cycloalkyl group having 3 to 12 carbon atoms which may have a substituent, or, an aromatic hydrocarbon ring having 5 to 12 carbon atoms which may have a substituent. Further, $R^b$ represents an alkyl group having 1 to 6 carbon atoms such as a methyl group and an ethyl group; or, an alkyl group having 1 to 6 carbon atoms or an aromatic hydrocarbon ring having 6 to 20 carbon atoms which may have an alkoxy group having 1 to 6 carbon atoms as a substituent such as a phenyl group, a 4-methylphenyl group, or a 4-methoxyphenyl group.

Thereamong, examples of the substituents of the aromatic hydrocarbon ring and an aromatic heterocyclic ring of $Fx^1$ and $Fx^2$ preferably include a halogen atom, a cyano group, an alkyl group having 1 to 6 carbon atoms, and, an alkoxy group having 1 to 6 carbon atoms.

Note that, $Fx^1$ and $Fx^2$ may have a plurality of substituents selected from the aforementioned substituents. When $Fx^1$ and $Fx^2$ have a plurality of substituents, the substituents may be the same or different.

Examples of the alkyl group having 1 to 20 carbon atoms of the alkyl group having 1 to 20 carbon atoms which may have a substituent of $R^a$ include a methyl group, an ethyl group, an n-propyl group, an isopropyl group, an n-butyl group, an isobutyl group, a 1-methylpentyl group, a 1-ethylpentyl group, a sec-butyl group, a t-butyl group, a n-pentyl group, an isopentyl group, a neopentyl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, and an n-icosyl group and the like. Note that, the number of carbon atoms of an alkyl group having 1 to 20 carbon atoms which may have a substituent is preferably 1 to 12, and even more preferably 4 to 10.

Examples of the alkenyl group having 2 to 20 carbon atoms of the alkenyl group having 2 to 20 carbon atoms which may have a substituent of $R^a$ include a vinyl group, a propenyl group, an isopropenyl group, a butenyl group, an isobutenyl group, a pentenyl group, a hexenyl group, a heptenyl group, an octenyl group, a decenyl group, an undecenyl group, a dodecenyl group, a tridecenyl group, a tetradecenyl group, a pentadecenyl group, a hexadecenyl group, a heptadecenyl group, an octadecenyl group, a nonadenyl group, and an icosenyl group and the like.

The number of carbons of the alkenyl group having 2 to 20 carbon atoms which may have a substituent is preferably 2 to 12.

Examples of the substituents of the alkyl group having 1 to 20 carbon atoms and the alkenyl group having 2 to 20 carbon atoms of $R^a$ preferably include a halogen atom such as a fluorine atom, and a chlorine atom, a cyano group; an N-N-dialkylamino group having 2 to 12 carbon atoms such as a dimethylamino group; an alkoxy group having 1 to 20 carbon atoms such as a methoxy group, an ethoxy group, an isopropoxy group; an alkoxy group having 1 to 12 carbon atoms substituted with an alkoxy group having 1 to 12 carbon atoms such as a methoxymethoxy group and a methoxyethoxy group; a nitro group; an aromatic hydrocarbon ring having 6 to 20 carbon atoms such as a phenyl group and a naphthyl group; an aromatic heterocyclic ring group having 2 to 20 carbon atoms such as a triazolyl group, a pyrrolyl group, a furanyl group, a thiophenyl group and a benzothiazole-2-yl group; a cycloalkyl group having 3 to 8 carbon atoms such as a cyclopropyl group, a cyclopentyl group and a cyclohexyl group; a cycloalkyloxy group having 3 to 8 carbon atoms such as a cyclopentyloxy group and a cyclohexyloxy group; a cyclic ether group having 2 to 12 carbon atoms such as a tetrahydrofuranyl group, a tetrahydropyranyl group, a dioxolanyl group and a dioxanyl group; an aryloxy group having 6 to 14 carbon atoms such as a phenoxy group and a naphthoxy group; a fluoroalkyl group having 1 to 12 carbon atoms in which at least one hydrogen atom is substituted with a fluorine atom such as a trifluoromethyl group, a pentafluoroethyl group, and $-CH_2CF_3$; a benzofuryl group; a benzopyranyl group; a benzodioxolyl group; a benzodioxanyl group and the like. Thereamong, examples of the substituents of the alkyl group having 1 to 20 carbon atoms and the alkenyl group having 2 to 20 carbon atoms of $R^a$ include a halogen atom such as a fluorine atom, and a chlorine atom, a cyano group; an alkoxy group having 1 to 20 carbon atoms such as a methoxy group, an ethoxy group, an isopropoxy group; a nitro group; an aromatic hydrocarbon ring having 6 to 20 carbon atoms such as a phenyl group and a naphthyl group; an aromatic heterocyclic ring group having 2 to 20 carbon atoms such as a furanyl group and a thiophenyl group; a cycloalkyl group having 3 to 8 carbon atoms such as a cyclopropyl group, a cyclopentyl group and a cyclohexyl group; a fluoroalkyl group having 1 to 12 carbon atoms in which at least one hydrogen atom is substituted with a fluorine atom such as a trifluoromethyl group, a pentafluoroethyl group, and $-CH_2CF_3$.

Note that, the alkyl group having 1 to 20 carbon atoms and the alkenyl group having 2 to 20 carbon atoms of $R^a$ may have a plurality of substituents selected from the aforementioned substituents. When the alkyl group having 1 to 20 carbon atoms or the alkenyl group having 2 to 20 carbon atoms of $R^a$ has a plurality of substituents, the plurality of substituents may be the same or different.

Examples of the cycloalkyl group having 3 to 12 carbon atoms of the cycloalkyl group having 3 to 12 carbon atoms which may have a substituent of $R^a$ include a cyclopropyl group, a cyclobutyl group, a cyclopentyl group, a cyclohexyl group, a cyclooctyl group and the like. Thereamong, a cyclopentyl group and a cyclohexyl group are preferable.

Examples of the substituents of the cycloalkyl group having 3 to 12 carbon atoms of $R^a$ include a halogen atom such as a fluorine atom, and a chlorine atom, a cyano group; an N-N-dialkylamino group having 2 to 12 carbon atoms such as a dimethylamino group;an alkyl group having 1 to 6 carbon atoms such as a methyl group, an ethyl group and

a propyl group; an alkoxy group having 1 to 6 carbon atoms such as a methoxy group, an ethoxy group and an isopropoxy group; a nitro group; and, an aromatic hydrocarbon ring having 6 to 20 carbon atoms such as a phenyl group and a naphthyl group and the like. Thereamong, examples of the cycloalkyl group having 3 to 12 carbon atoms substituents of $R^a$ preferably include a halogen atom such as a fluorine atom, and a chlorine atom; a cyano group; an alkyl group having 1 to 6 carbon atoms such as a methyl group, an ethyl group, and a propyl group; an alkoxy group having 1 to 6 carbon atoms such as a methoxy group, an ethoxy group and an isopropoxy group; a nitro group; and, an aromatic hydrocarbon ring having 6 to 20 carbon atoms such as a phenyl group and a naphthyl group.

Note that the cycloalkyl group having 3 to 12 carbon atoms of $R^a$ may have a plurality of substituents. When the cycloalkyl group having 3 to 12 carbon atoms of $R^a$ has a plurality of substituents, the plurality of substituents may be the same or different.

Examples of the aromatic hydrocarbon ring having 5 to 12 carbon atoms of the aromatic hydrocarbon ring having 5 to 12 carbon atoms which may have a substituent of $R^a$ include a phenyl group, a 1-naphthyl group, a 2-naphthyl group and the like. Thereamong, a phenyl group is preferable.

Examples of the substituent of the aromatic hydrocarbon ring having 5 to 12 carbon atoms which may have a substituent include a halogen atom such as a fluorine atom, and a chlorine atom, a cyano group; an N-N-dialkylamino group having 2 to 12 carbon atoms such as a dimethylamino group; an alkoxy group having 1 to 20 carbon atoms such as a methoxy group, an ethoxy group, an isopropoxy group ; an alkoxy group having 1 to 12 carbon atoms substituted with an alkoxy group having 1 to 12 carbon atoms such as a methoxymethoxy group and a methoxyethoxy group; a nitro group; an aromatic hydrocarbon ring having 6 to 20 carbon atoms such as a phenyl group and a naphthyl group; an aromatic heterocyclic ring group having 2 to 20 carbon atoms such as a triazolyl group, a pyrrolyl group, a furanyl group and a thiophenyl group; a cycloalkyl group having 3 to 8 carbon atoms such as a cyclopropyl group, a cyclopentyl group and a cyclohexyl group; a cycloalkyloxy group having 3 to 8 carbon atoms such as a cyclopentyloxy group and a cyclohexyloxy group; a cyclic ether group having 2 to 12 carbon atoms such as a tetrahydrofuranyl group, a tetrahydropyranyl group, a dioxolanyl group and a dioxanyl group; an aryloxy group having 6 to 14 carbon atoms such as a phenoxy group and a naphthoxy group; a fluoroalkyl group having 1 to 12 carbon atoms in which at least one hydrogen atom is substituted with a fluorine atom such as a trifluoromethyl group, a pentafluoroethyl group, and $-CH_2CF_3$; $-OCF_3$; a benzofuryl group; a benzopyranyl group; a benzodioxolyl group; a benzodioxanyl group and the like. Thereamong, the substituent of the aromatic hydrocarbon ring having 5 to 12 carbon atoms is preferably one or more substituents selected from a halogen atom such as a fluorine atom, and a chlorine atom, a cyano group; an alkoxy group having 1 to 20 carbon atoms such as a methoxy group, an ethoxy group, an isopropoxy group, a nitro group; an aromatic hydrocarbon ring having 6 to 20 carbon atoms such as a phenyl group and a naphthyl group; an aromatic heterocyclic ring group having 2 to 20 carbon atoms such as a triazolyl group, a pyrrolyl group, a furanyl group and a thiophenyl group; a cycloalkyl group having 3 to 8 carbon atoms such as a cyclopropyl group, a cyclopentyl group and a cyclohexyl group; a fluoroalkyl group having 1 to 12 carbon atoms in which at least one hydrogen atom is substituted with a fluorine atom such as a trifluoromethyl group, a pentafluoroethyl group, and $-CH_2CF_3$; and $-OCF_3$.

Note that, the aromatic hydrocarbon ring having 5 to 12 carbon atoms may have a plurality of substituents. When the aromatic hydrocarbon ring having 5 to 12 carbon atoms has a plurality of substituent, the substituents may be the same or different.

Here, the "number of carbon atoms" of the organic group having at least one of an aromatic hydrocarbon ring and an aromatic heterocyclic ring of $Fx^1$ and $Fx^2$ means the number of carbon atoms of the organic group itself having at least one of an aromatic hydrocarbon ring and an aromatic heterocyclic ring which does not include the carbon atoms of the substituent.

Preferably, $Fx^1$ and $Fx^2$ each independently are "an alkyl group having 1 to 18 carbon atoms in which at least one hydrogen atom is substituted with a ring-containing group having at least one of an aromatic hydrocarbon ring and an aromatic heterocyclic ring and which may have a substituent other than the ring-containing group", or, "a cyclic group having 2 to 20 carbon atoms having at least one of an aromatic hydrocarbon ring and an aromatic heterocyclic ring and which may have a substituent".

When $Fx^1$ and $Fx^2$ have a plurality of aromatic hydrocarbon rings and/or a plurality of an aromatic heterocyclic rings, the rings may be the same or different.

Here, an aromatic hydrocarbon ring having the aforementioned ring-containing group and a cyclic group is the same as the aforementioned "aromatic hydrocarbon ring of $Fx^1$ and $Fx^2$", and further, the aromatic heterocyclic ring having the aforementioned ring-containing group and the cyclic group is the same as the aromatic heterocyclic ring of the aforementioned "$Fx^1$ and $Fx^2$. Note that, these rings (the aromatic hydrocarbon ring and the aromatic heterocyclic ring) may be similarly substituted with the aforementioned "an aromatic hydrocarbon ring and an aromatic heterocyclic ring of $Fx^1$ and $Fx^2$".

Moreover, the specific examples of the "alkyl group having 1 to 18 carbon atoms" in "an alkyl group having 1 to 18 carbon atoms in which at least one hydrogen atom is substituted with a ring-containing group having at least one of an aromatic hydrocarbon ring and an aromatic heterocyclic ring and which may have a substituent other than the ring-

containing group" of $Fx^1$ and $Fx^2$ include a methyl group, an ethyl group, a propyl group, an isopropyl group and the like.

Further, the "alkyl group having 1 to 18 carbon atoms in which at least one hydrogen atom is substituted with a ring-containing group having at least one of an aromatic hydrocarbon ring and an aromatic heterocyclic ring and which may have a substituent other than the ring-containing group" may have one or a plurality of substituents other than a ring-containing group. When there are a plurality of substituents other than a ring-containing group, the plurality of substituents may be the same or different.

Note that, "at least one of an aromatic hydrocarbon ring and an aromatic heterocyclic ring" of the ring-containing group may be directly bonded to the carbon atom of an alkyl group having 1 to 18 carbon atoms, and may be bonded to the carbon atom of an alkyl group having 1 to 18 carbon atoms via a linking group such as -S-, -O-, -C(=O)-, -C(=O)-O-, -O-C(=O)-, -O-C(=O)-O-, -C(=O)-S-, -S-C(=O)-, $-NR^{11}-C(=O)-$, $-C(=O)-NR^{11}$. Here, $R^{11}$ represents a hydrogen atom or an alkyl group having 1 to 6 carbon atoms. That is, a ring-containing group may be an aromatic hydrocarbon ring which may be substituted and/or an aromatic heterocyclic ring group which may be substituted, and may be a group consisting of an optionally substituted aromatic hydrocarbon ring having a linking group and / or a group consisting of an optionally substituted aromatic heterocycle ring having a linking group.

Moreover, examples of the "aromatic hydrocarbon ring" of the ring-containing group of $Fx^1$ and $Fx^2$ include a phenyl group, a naphthyl group, an anthracenyl group, a phenanthrenyl group, a pyrenyl group, a fluorenyl group and the like. Thereamong, a phenyl group, a naphthyl group and a fluorenyl group are preferable.

Note that, the substituents which the "aromatic hydrocarbon ring" of the ring-containing group may have are the same as the substituent which the aforementioned aromatic hydrocarbon ring and an aromatic heterocyclic ring of $Fx^1$ and $Fx^2$" may have.

Further, examples of the "aromatic heterocyclic ring group" of the ring-containing group of $Fx^1$ and $Fx^2$ include a phthalimido group, a 1-benzofuranyl group, a 2-benzofuranyl group, an acridinyl group, an isoquinoryl group, an imidazolyl group, an indolinyl group, a furazanyl group, an oxazolyl group, an oxazolopyrazinyl group, an oxazolopyridinyl group, an oxazolopyridazinyl group, an oxazolopyrimidinyl group, a quinazolinyl group, a quinoxalinyl group, a quinolyl group, a cinnolinyl group, a thiadiazolyl group, a thiazolyl group, a thiazolopyrazinyl group, a thiazolopyridyl group, a thiazolopyridazinyl group, a thiazolopyrimidinyl group, a thienyl group, a triazinyl group, a triazolyl group, a naphthyridinyl group, a pyrazinyl group, a pyrazolyl group, a pyranonyl group, a pyranyl group, a pyridyl group, a pyridazinyl group, a pyrimidinyl group, a pyrrolyl group, a phenanthridinyl group, a phthalazinyl group, a furanyl group, a benzo[c]thienyl group, a benzisoxazolyl group, a benzisothiazolyl group, a benzimidazolyl group, a benzoxazolyl group, a benzoxazolyl group, a benzothiadiazolyl group, a benzothiazolyl group, a benzothienyl group, a benzotriazinyl group, a benzotriazolyl group, a benzopyrazolyl group, a benzopyranonyl group and the like. Thereamong, a single ring aromatic heterocyclic ring group such as a furanyl group, a pyranyl group, a thienyl group, an oxazolyl group, a furanizanyl group, a thiazolyl group, and a thiadiazolyl group, and a fused ring aromatic heterocyclic ring group such as a benzothiazolyl group, a benzoxazolyl group, a quinolyl group, 1-benzofuranyl group, a 2-benzofuranyl group, a benzo[b]a thienyl group, a phthalimide group, a benzo[c]a thienyl group, thiazolopyridyl group, a thiazolopyrazinyl group, a benzisoxazolyl group, and a benzothiadiazolyl group are preferable.

Note that, the substituent which "an aromatic heterocyclic ring group" of the ring-containing group may have is the same as the substituent which the aforementioned "aromatic hydrocarbon ring and an aromatic heterocyclic ring of $Fx^1$ and $Fx^2$" may have.

Examples of the "group consisting of an aromatic hydrocarbon ring having a linking group" and/or the "group consisting of an aromatic heterocyclic ring having a linking group" of the ring-containing group of $Fx^1$ and $Fx^2$ include a phenylthio group, a naphthylthio group, an anthracenylthio group, a phenanthrenylthio group, a pyrenylthio group, a fluorenylthio group, a phenyloxy group, a naphthyloxy group, an anthracenyloxy group, a phenanthrenyloxy group, a pyrenyloxy group, a fluorenyloxy group, a benzoisoxazolylthio group, a benzoisothiazolylthio group, a benzooxadiazolylthio group, a benzooxazolylthio group, a benzothiadiazolylthio group, a benzothiazolylthio group, a benzothienylthio, a benzisoxazolyloxy group, a benzoisothiazolyloxy group, a benzoxadiazolyloxy group, a benzoxazolyloxy group, a benzothiadiazolyloxy group, a benzothiazolyloxy group, a benzothienyloxy group, and the like. Thereamong, a benzothiazolylthio group is preferable.

Note that, the substituent which the "aromatic hydrocarbon ring having a linking group" and the "aromatic heterocyclic ring having a linking group" of a ring-containing group has is the same as the substituent which the aforementioned "aromatic hydrocarbon ring and an aromatic heterocyclic ring of $Fx^1$ and $Fx^2$" may have.

Preferred specific examples of the "alkyl group having 1 to 18 carbon atoms in which at least one hydrogen atom is substituted with a ring-containing group having at least one of an aromatic hydrocarbon ring and an aromatic heterocyclic ring and which may have a substituent other than the ring-containing group" of $Fx^1$ and $Fx^2$ preferably include the structures represented by the following formulas (3-1) to (3-10), with formulas (3-3), (3-6), (3-7), (3-9) and (3-10) being preferable. However, the present disclosure is not limited to the following examples. Note that, in the following formulas, the "-" represents a bond with $Y^a$ that extends from any position of the ring. Note that, the groups represented by the following formulas (3-1) to (3-10) may have a substituent, and the specific examples are the same

as the examples of the substituents that the aromatic hydrocarbon ring and an aromatic heterocyclic ring of $Fx^1$ and $Fx^2$ may have.

Further, the substituents other than the ring-containing group are the same as the example of the substituent of the alkyl group having 1 to 20 carbon atoms and the alkenyl group having 2 to 20 carbon atoms of $R^a$.

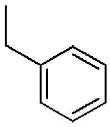
(3-1)
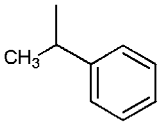
(3-2)
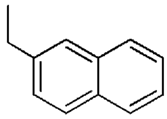
(3-3)
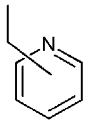
(3-4)
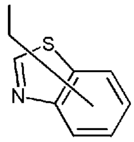
(3-5)
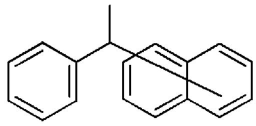
(3-6)

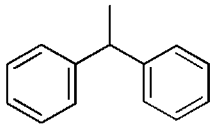
(3-7)
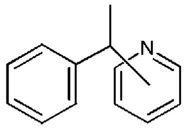
(3-8)
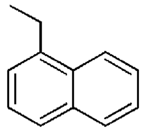
(3-9)
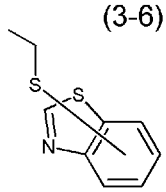
(3-10)

Moreover, the "cyclic group having 2 to 20 carbon atoms having at least one of an aromatic hydrocarbon ring and an aromatic heterocyclic ring an which may have a substituent" of $Fx^1$ and $Fx^2$ include the following 1) or 2).

1) an optionally substituted hydrocarbon ring group having 6 to 20 carbon atoms having at least an aromatic hydrocarbon ring having 6 to 18 carbon atoms, and
2) an optionally substituted heterocyclic ring group having 2 to 20 carbon atoms having at least one aromatic ring selected from the group consisting of an aromatic hydrocarbon ring having 6 to 18 carbon atoms and an aromatic heterocyclic ring having 2 to 18 carbon atoms.

Examples of the hydrocarbon ring group the aforementioned 1) include an aromatic hydrocarbon ring having 6 to 18 carbon atoms (a phenyl group (6 carbon atoms), a naphthyl group (10 carbon atoms), an anthracenyl group (14 carbon atoms), a phenanthrenyl group (14 carbon atoms), a pyrenyl group (16 carbon atoms), a fluorenyl group (13 carbon atoms), etc., an indanyl group (9 carbon atoms), a 1,2,3,4-tetrahydronaphthyl group (10 carbon atoms), a 1,4-dihydronaphthyl group (10 carbon atoms), and the like.

Examples of the heterocyclic ring group of the aforementioned 2) inlcude an aromatic heterocyclic ring group having 2 to 18 carbon atoms (a phthalimido group, a 1-benzofuranyl group, a 2-benzofuranyl group, an acridinyl group, an isoquinoryl group, an imidazolyl group, an indolinyl group, a furazanyl group, an oxazolyl group, an oxazolopyrazinyl group, an oxazolopyridinyl group, an oxazolopyridazinyl group, an oxazolopyrimidinyl group, a quinazolinyl group, a quinoxalinyl group, a quinolyl group, a cinnolinyl group, a thiadiazolyl group, a thiazolyl group, a thiazolopyrazinyl group, a thiazolopyridinyl group, a thiazolopyridazinyl group, a thiazolopyrimidinyl group, a thienyl group, a triazinyl group, a triazolyl group, a naphthyridinyl group, a pyrazinyl group, a pyrazolyl group, a pyranonyl group, a pyranyl group, a pyridyl group, a pyridazinyl group, a pyrimidinyl group, a pyrrolyl group, a phenanthridinyl group, a phthalazinyl group, a furanyl group, a benzo[c]thienyl group, a benzisoxazolyl group, a benzisothiazolyl group, a benzimidazolyl group, a benzoxazolyl group, a benzothiadiazolyl group, benzothiazolyl group, a benzothiophenyl group, a benzotriazinyl group, a benzotriazolyl group, a benzopyrazolyl group, benzopyranonyl group etc.), a xanthenyl group, a 2,3-dihydroindolyl group, a 9,10-dihydroacridinyl group, a 1,2,3,4-tetrahydroquinolyl group, a dihydropyranyl group, a tetrahydropyranyl group, a dihydrofuranyl group and a tetrahydrofuranyl group.

The preferred specific examples of the "cyclic group having 2 to 20 carbon atoms having at least one of an aromatic hydrocarbon ring and an aromatic heterocyclic ring an which may have a substituent" of $Fx^1$ and $Fx^2$ are shown below. However, the present disclosure is not limited to the following examples. Note that, in the following formulas, the "-" represents a bond with $Y^a$ that extends from any position of the ring.

1) Specific examples of an optionally substituted hydrocarbon ring group having 6 to 20 carbon atoms having at least an aromatic hydrocarbon ring having 6 to 18 carbon atoms includes the structures represented by the following formulas (1-1) to (1-21), and preferably the hydrocarbon ring group having 6 to 18 carbon atoms represented by formulas (1-8) to (1-21). Note that, the groups represented by the following formulas (1-1) to (1-21) may have substituents.

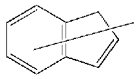 (1-1) 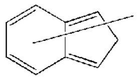 (1-2) 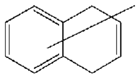 (1-3) 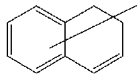 (1-4) 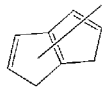 (1-5) 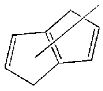 (1-6)

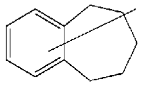 (1-7) 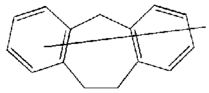 (1-8)

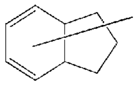 (1-9) 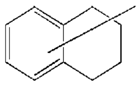 (1-10) 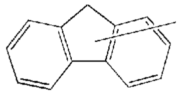 (1-11) 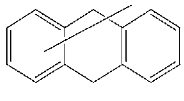 (1-12)

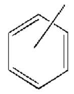 (1-13) 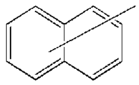 (1-14) 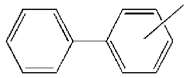 (1-15) 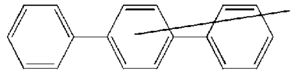 (1-16) 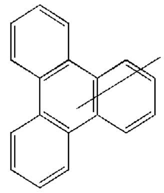 (1-21)

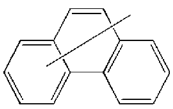 (1-17) 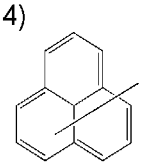 (1-18) 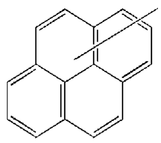 (1-19) 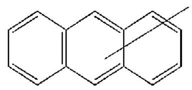 (1-20)

2) Specific examples of an optionally substituted heterocyclic ring group having 2 to 20 carbon atoms having at least one aromatic ring selected from the group consisting of an aromatic hydrocarbon ring having 6 to 18 carbon atoms and an aromatic heterocyclic ring having 2 to 18 carbon atoms include the structures presented by the following formulas (2-1) to (2-51) and the like, and preferably the heterocyclic ring group having 2 to 16 carbon atoms represented by formulas (2-11) to (2-51). Note that, the groups represented by the following formulas may have substituents.

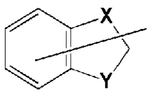 (2-1) 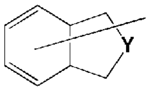 (2-2) 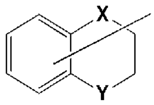 (2-3) 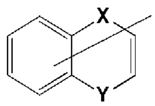 (2-4) 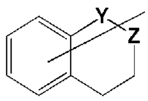 (2-5) 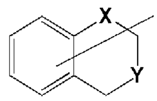 (2-6)

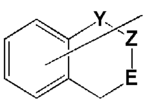 (2-7) 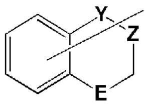 (2-8) 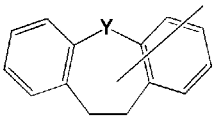 (2-9) 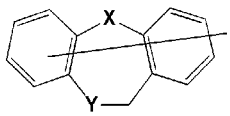 (2-10) 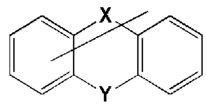 (2-11)

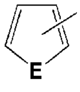 (2-12) 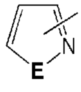 (2-13) 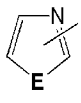 (2-14) 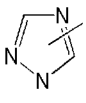 (2-15) 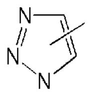 (2-16) 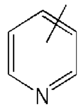 (2-17) 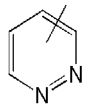 (2-18) 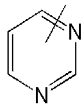 (2-19)

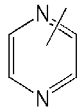
(2-20)
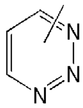
(2-21)
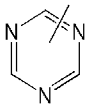
(2-22)
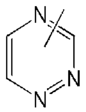
(2-23)
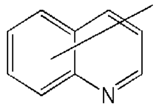
(2-24)
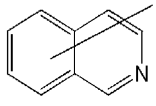
(2-25)
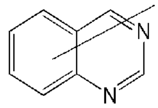
(2-26)

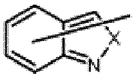
(2-27)
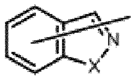
(2-28)
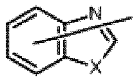
(2-29)
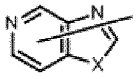
(2-30)
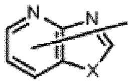
(2-31)
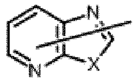
(2-32)
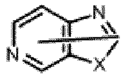
(2-33)

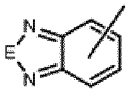
(2-34)
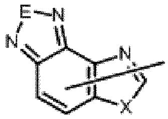
(2-35)
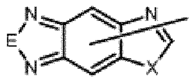
(2-36)
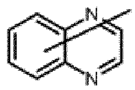
(2-37)
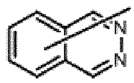
(2-38)
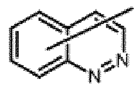
(2-39)

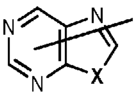
(2-40)
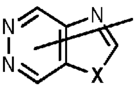
(2-41)
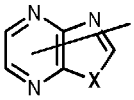
(2-42)
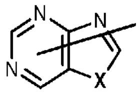
(2-43)
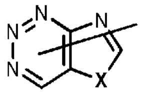
(2-44)
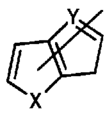
(2-45)
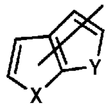
(2-46)

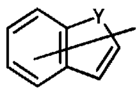
(2-47)
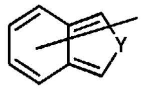
(2-48)
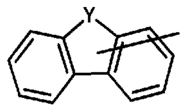
(2-49)
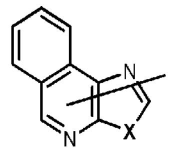
(2-50)
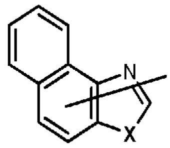
(2-51)

where in each of the formulas, X represents $-CH_2-$, $-NR^c-$, an oxygen atom, a sulfur atom, -SO- or $-SO_2-$; Y and Z each independently represent $-NR^c-$, an oxygen atom, a sulfur atom, -SO- or $-SO_2-$; and E represents, $-NR^c-$, an oxygen atom or a sulfur atom, where $R^c$ represents a hydrogen atom, or, an alkyl group having 1 to 6 carbon atoms such as a methyl group, an ethyl group and a propyl group (where, in each of the formulas, an oxygen atom, a sulfur atom, -SO-, $-SO_2-$ may not be adjacent to each other).

Among these described above, $Fx^1$ and $Fx^2$ are preferably any of the groups represented by the aforementioned formula (1-8), formula (1-11), formula (1-12), formula (1-13), formula (1-14), formula (1-15), formula (1-20), formula (2-9) to formula (2-11), formula (2-24) to formula (2-33), formula (2-35) to formula (2-43), formula (2-47) and, formulas (2-49) to (2-51).

Note that, the total number of $\pi$ electrons contained in the ring structure in $Fx^1$ is preferably 8 or more, more preferably 10 or more, and is preferably 20 or less, and more preferably 18 or less. The total number of $\pi$ electrons contained in the ring structure in $Fx^2$ is preferably 4 or more, more preferably 6 or more, and is preferably 20 or less, and more preferably 18 or less.

Furthermore, $Fx^1$ is preferably any of the following formulas (i-1) to (i-9), and $Fx^2$ is preferably any of the following (i-1) to (i-11). Note that, the groups represented by the following formulas (i-1) to (i-11) may have substituents.

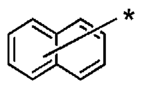
(i-1)
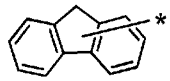
(i-2)
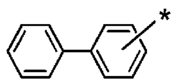
(i-3)
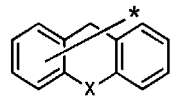
(i-4)
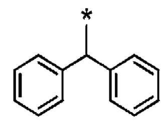
(i-5)
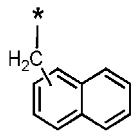
(i-6)
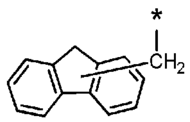
(i-7)
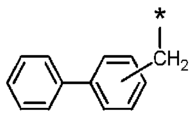
(i-8)
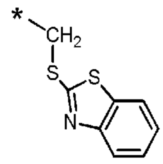
(i-9)
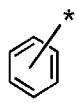
(i-10)
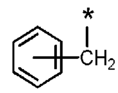
(i-11)

where in the formula (i-4), X represents $-CH_2-$, $-NR^d-$, an oxygen atom, a sulfur atom, -SO- or $SO_2-$, and $R^d$ represents a hydrogen atom or an alkyl group having 1 to 6 carbon atoms.

Note that, the "alkyl group having 1 to 18 carbon atoms in which at least one hydrogen atom is substituted with a ring-containing group having at least one of an aromatic hydrocarbon ring and an aromatic heterocyclic ring and which may have a substituent other than the ring-containing group" and the "cyclic group having 2 to 20 carbon atoms having at least one of an aromatic hydrocarbon ring and an aromatic heterocyclic ring and which may have a substituent" of $Fx^1$ and $Fx^2$ may have one or more substituents. When there is a plurality of substituents, the plurality of substituents may be the same or different.

Examples of the substituent which the "alkyl group having 1 to 18 carbon atoms in which at least one hydrogen atom is substituted with a ring-containing group having at least one of an aromatic hydrocarbon ring and an aromatic heterocyclic ring and which may have a substituent other than the ring-containing group" and the "cyclic group having 2 to 20 carbon atoms having at least one of an aromatic hydrocarbon ring and an aromatic heterocyclic ring and which may have a substituent" of $Fx^1$ and $Fx^2$ have include a halogen atom such as a fluorine atom, and a chlorine atom; a cyano group; an alkyl group having 1 to 6 carbon atoms such as a methyl group, an ethyl group, and a propyl group; an alkenyl group having 2 to 6 carbon atoms such as a vinyl group and an allyl group; an alkyl group having 1 to 6 carbon atoms in which at least one hydrogen atom is substituted with a halogen atom such as a trifluoromethyl group and a pentafluoroethyl group; an N-N-dialkylamino group having 2 to 12 carbon atoms such as a dimethylamino group; an alkoxy group having 1 to 6 carbon atoms such as a methoxy group, an ethoxy group and an isopropoxy group; a nitro group; an aromatic hydrocarbon ring having 6 to 20 carbon atoms such as a phenyl group and a naphthyl group; -OCF3; $-C(=O)-R^a$; $-C(=O)-O-R^a$; $-O-C(=O)-R^a$; $-SO_2R^b$; and the like. Here, $R^a$ and $R^b$ are the same as defined above, and the preferred examples are also the same as stated above. Moreover, when there is a plurality of substituents, the plurality of substituents may be the same or different.

Thereamong, at least one substituent is preferably selected from a halogen atom, a cyano group, an alkyl group having 1 to 6 carbon atoms, and, an alkoxy group having 1 to 6 carbon atoms.

$Y^a$ represents a chemical single bond, -O-, -C(=O)-, -C(=O)-O-, -O-C(=O)-, -O-C(=O)-O-, -C(=O)-S-, -S-C(=O)-, $-NR^{11}-C(=O)-$, $-C(=O)-NR^{11}-$, $-O-C(=O)-NR^{11}-$, $-NR^{11}-C(=O)-O-$, -S-, -N=N-, or, -C=C-. Here, $R^{11}$ represents a hydrogen atom or an alkyl group having 1 to 6 carbon atoms. Note that, $Y^a$ may be -O-, -C(=O)-O-, -O-C(=O)-, -O-C(=O)-O-, -C(=O)-S-, -S-C(=O)-, $-NR^{11}-C(=O)-$, $-C(=O)-NR^{11}-$, $-O-C(=O)-NR^{11}-$, $-NR^{11}-C(=O)-O-$, -S-, -N=N-, or, -C=C-.

Moreover, $Y^a$ preferably represents a chemical single bond, -O-, -C(=O)-, -C(=O)-O-, -O-C(=O)-, -O-C(=O)-O-, -C(=O)-S-, -S-C(=O)-, $-NR^{11}-C(=O)-$, $-C(=O)-NR^{11}-$, $-O-C(=O)-NR^{11}-$, $-NR^{11}-C(=O)-O-$, or -S-, and more preferably a chemical single bond, -O-, -C(=O)-, -C(=O)-O-, -O-C(=O)-, -O-C(=O)-O-, $-NR^{11}-C(=O)-$, $-C(=O)-NR^{11}-$, $-O-C(=O)-NR^{11}-$, $-NR^{11}-C(=O)-O-$, or -S-, and even more preferably a chemical single bond, -O-, -C(=O)-, -C(=O)-O-, -O-C(=O)-, -O-C(=O)-O-, $-O-C(=O)-NR^{11}-$, $-NR^{11}-C(=O)-O-$, -S-, and -O-, -C(=O)-O-, -O-C(=O)-, $-O-C(=O)-NR^{11}-$, $-NR^{11}-C(=O)-O-$ is particularly preferable.

$G^a$ is a divalent organic group having 1 to 30 carbon atoms which may have a substituent, and preferably is a divalent organic group having 3 to 30 carbon atoms which may have a substituent.

$G^a$ is more preferably an organic group which is either a divalent aliphatic hydrocarbon group having 1 to 30 carbon atoms which may have a substituent, or a divalent aliphatic hydrocarbon group having 3 to 30 carbon atoms in which at least one $-CH_2-$ contained in the divalent aliphatic hydrocarbon group is substituted with -O-, -S-, -O-C(=O)-, -C(=O)-O-, -O-C(=O)-O-, $-NR^{12}-C(=O)-$, $-C(=O)-NR^{12}-$, $-NR^{12}-$, or -C(=O)-, with the proviso that cases where there are two or more contiguous -O- or -S- are excluded. Here, $R^{12}$ represents a hydrogen atom or an alkyl group having 1 to 6 carbon atoms, and examples of the substituent which the organic group of $G^a$ has include an alkyl group having 1 to 5 carbon atoms such as a methyl group, an ethyl group, a propyl group; an alkoxy group having 1 to 5 carbon atoms such as a methoxy group, an ethoxy group, and a propoxy group; a cyano group; a halogen atom such as a fluorine atom,

and a chlorine atom.

Here, with respect to $G^a$, the "divalent aliphatic hydrocarbon group" is preferably a divalent chain aliphatic hydrocarbon group, and more preferably an alkylene group. Further, the number of carbon atoms of the "divalent aliphatic hydrocarbon group" is preferably 3 to 30, and more preferably 3 to 18. Moreover, the "divalent aliphatic hydrocarbon group" is preferably a divalent aliphatic hydrocarbon group having 3 to 30 carbon atoms, more preferably a divalent chain aliphatic hydrocarbon group having 3 to 18 carbon atoms, and more preferably an alkylene group having 3 to 18 carbon atoms.

The number of carbon atoms of Ga is preferably 4 to 16 carbon atoms, even more preferably 5 to 14 carbon atoms, particularly preferably 6 to 12 carbon atoms, and most preferably 6 to 10 carbon atoms.

The structure of Ga is preferably an unsubstituted alkylene group having 4 to 16 carbon atoms, more preferably an unsubstituted alkylene group having 5 to 14 carbon atoms, particularly preferably an unsubstituted alkylene group having 6 to 12 carbon atoms, and most preferably an unsubstituted alkylene group having 6 to 10 carbon atoms.

Note that, when the number of carbon atoms of Ga is 3 or more, both ends of Ga are preferably $-CH_2-$ (both ends of Ga are unsubstituted). Further, in the "group in which at least one $-CH_2-$ contained in a divalent aliphatic hydrocarbon group having 3 to 30 carbon atoms is substituted with -O-, -S-, -O-C(=O)-, -C(=O)-O-, -O-C(=O)-O-, $-NR^{12}-C(=O)-$, $-C(=O)-NR^{12}-$, $-NR^{12}-$or -C(=O)-", -O- and -S- preferably do not substitute consecutive $-CH_2-$ in the aliphatic hydrocarbon group (i.e., the -O-O- and -S-S- configurations are not formed) (in short, cases where there are two or more contiguous -O- or -S- are preferably excluded), and -C(=O)- preferably do not substitute consecutive $-CH_2-$ in the aliphatic hydrocarbon group (i.e., the -C(=O)-C(=O)-configuration is not formed).

$G^a$ is preferably (i) "an organic group which is either a divalent chain aliphatic hydrocarbon group having 1 to 18 carbon atoms (preferably 3 to 18 carbon atoms) which may have a substituent, or a divalent chain aliphatic hydrocarbon group having 3 to 18 carbon atoms which may have a substituent in which at least one $-CH_2-$ contained in the divalent chain aliphatic hydrocarbon group is substituted with -O-, -S-, -O-C(=O)-, -C(=O)-O-, or -C(=O), with the proviso that cases where there are two or more contiguous -O- or -S- are excluded", more preferably (ii) "a divalent chain aliphatic hydrocarbon group having 3 to 18 carbon atoms which may have a substituent", even more preferably (iii) "an alkylene group having 3 to 18 carbon atoms which may have a substituent", even more preferably (iv) "an unsubstituted alkylene group having 4 to 16 carbon atoms", even more preferably (v) "an unsubstituted alkylene group having 5 to 14 carbon atoms", particularly preferably (vi) "an unsubstituted alkylene group having 6 to 12 carbon atoms", and most preferably (vii) "an unsubstituted alkylene group having 6 to 10 carbon atoms". Examples of the aforementioned substituents of $G^a$ include an alkyl group having 1 to 5 carbon atoms such as a methyl group, an ethyl group, and a propyl group; an alkoxy group having 1 to 5 carbon atoms such as a methoxy group, an ethoxy group, or an isopropoxy group; a cyano group; a halogen atom such as a fluorine atom, and a chlorine atom.

Q represents a hydrogen atom, or an alkyl group having 1 to 6 carbon atoms which may have a substituent. Examples of an alkyl group having 1 to 6 carbon atoms of an alkyl group having 1 to 6 carbon atoms which may have a substituent include a methyl group, an ethyl group, a propyl group, or an isopropyl group and the like, and examples of the substituent include an aromatic hydrocarbon group having 6 to 12 carbon atoms such as a phenyl group and a naphthalene group.

Further, in the above stated formulas (II-1) and (II-2), $R^I$ to $R^{IV}$ each independently represent a hydrogen atom; a halogen atom such as a fluorine atom, and a chlorine atom; an alkyl group having 1 to 6 carbon atoms such as a methyl group, an ethyl group, a propyl group, a cyano group, a nitro group; an alkyl group having 1 to 6 carbon atoms in which at least one hydrogen atom is substituted with a halogen atom such as a trifluoromethyl group and a pentafluoroethyl group; an alkoxy group having 1 to 6 carbon atoms such as a methoxy group, an ethoxy group, or an isopropoxy group; $-OCF_3$; $-C(=O)-O-R^a$; or $-O-C(=O)-R^a$, where $R^a$ is the same as defined above, and the preferred examples are also the same as stated above.

Thereamong, preferably (i) all of $R^I$ to $R^{IV}$ are hydrogen atoms, or, (ii) at least one among $R^I$ to $R^{IV}$ is an alkoxy group having 1 to 6 carbon atoms which may have a substituent, and, the remainder are hydrogen atoms.

$R^I$ to $R^{IV}$ may be the same or different, and one or more ring constituents $C-R^I$ to $C-R^{IV}$ may be replaced by a nitrogen atom.

Specific examples of the group in which at least one among $C-R^I$ to $C-R^{IV}$ is substituted with a nitrogen atom are shown below. However, the group in which at least one among $C-R^I$ to $C-R^{IV}$ is substituted with a nitrogen atom is not limited to these examples.

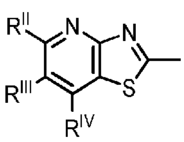 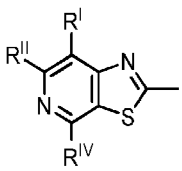 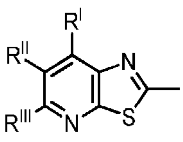 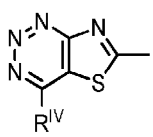

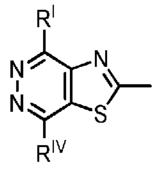 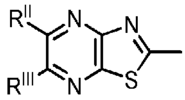 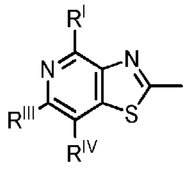 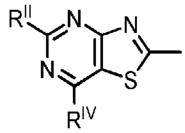

where in each of the formulas, $R^I$ to $R^{IV}$ are the same as defined above, and the preferred examples are also the same as stated above.

In the above stated formulas (II-1) and (II-2), $R^0$ represents a halogen atom, an alkyl group having 1 to 6 carbon atoms such as a methyl group, an ethyl group and a propyl group, a cyano group, a nitro group; an alkyl group having 1 to 6 carbon atoms in which at least one hydrogen atom is substituted with a halogen atom such as a trifluoromethyl group and a pentafluoroethyl group; an alkoxy group having 1 to 6 carbon atoms such as a methoxy group, an ethoxy group, and a propoxy group; -OCF3; -C(=O)-O-$R^a$; or -O-C(=O)-$R^a$, where $R^a$ is the same as defined above, and the preferred examples are also the same as stated above.

From the viewpoint of solubility improvement, examples of $R^0$ preferably include a halogen atom such as a fluorine atom, and a chlorine atom, an alkyl group having 1 to 6 carbon atoms such as a methyl group, an ethyl group and a propyl group, a cyano group, a nitro group; an alkyl group having 1 to 6 carbon atoms in which at least one hydrogen atom is substituted with a halogen atom such as a trifluoromethyl group and a pentafluoroethyl group, an alkoxy group having 1 to 6 carbon atoms such as a methoxy group, an ethoxy group, and a propoxy group. Note that, when there is a plurality of $R^0$, the plurality of $R^0$ may be the same or different from each other. Furthermore, p represents an integer from 0 to 3, p1 represents an integer from 0 to 4, p2 represents 0 or 1, and preferably all of p, p1 and p2 are 0.

Further, in the aforementioned Formula (I), $Y^1$ to $Y^8$ each independently represent a chemical single bond, -O-, -O-$CH_2$-, -$CH_2$-O-, -O-$CH_2$-$CH_2$, -$CH_2$-$CH_2$-O-, -C(=O)-O-, -O-C(=O)-, -O-C(=O)-O-, -C(=O)-S-, -S-C(=O)-, -$NR^{13}$-C(=O)-, -C(=O)-$NR^{13}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-C(=O)-O-, -O-C(=O)-$CH_2$-, -$CH_2$-O-C(=O)-, -C(=O)-O-$CH_2$-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, or, -C=C-, where $R^{13}$ represents a hydrogen atom or an alkyl group having 1 to 6 carbon atoms.

Thereamong, $Y^1$ to $Y^8$ each preferably independently represent a chemical single bond, -O-, -O-C(=O)-, -C(=O)-O-, or, -O-C(=O)-O-.

Further, in the aforementioned formula (I), $A^1$, $A^2$, $B^1$ and $B^2$ each independently represent a cyclic aliphatic group which may have a substituent, or, an aromatic group which may have a substituent.

Thereamong, $A^1$, $A^2$, $B^1$ and $B^2$ each preferably independently represent a cyclic aliphatic group having 5 to 20 carbon atoms which may have a substituent, or, an aromatic group having 2 to 20 carbon atoms which may have a substituent.

Specific examples of the cyclic aliphatic group include a cycloalkanediyl group having 5 to 20 carbon atoms such as cyclopentane-1,3-diyl, cyclohexane-1,4-diyl, cycloheptane-1,4-diyl, and cyclooctane-1,5-diyl; a bicycloalkanediyl group having 5 to 20 carbon atoms such as decahydronaphthalene-1,5-diyl and decahydronaphthalene-2,6-diyl and the like. Thereamong, a cycloalkanediyl group having 5 to 20 carbon atoms which may have a substituent is preferable as the cyclic aliphatic group, and cyclohexanediol group is more preferable, and specifically, a 1,4-cyclohexylene group is preferable, and a trans-1,4-cyclohexelene group is more preferable.

Specific examples of the aromatic group include an aromatic hydrocarbon ring having 6 to 20 carbon atoms such as a 1,2-phenylene group, a 1,3-naphthylene group, a 1,4-phenylene group, a 1,4-naphthylene group, a 1,5-naphthylene group, a 2,6-naphthylene group, and a 4,4'-biphenylene group; an aromatic heterocyclic ring group having 2 to 20 carbon atoms such as furan-2,5-diyl, thiophene-2,5-diyl, pyridine-2,5-diyl, pyrazine-2,5-diyl; and the like. Thereamong, an aromatic hydrocarbon ring having 6 to 20 carbon atoms is preferable as the aromatic group, a phenylene group is more preferable, and specifically, a 1,4-phenylene group is preferable.

Examples of the substituents of the cyclic aliphatic group and the aromatic group include a halogen atom such as a fluorine atom, a chlorine atom, and a bromine atom, an alkyl group having 1 to 6 carbon atoms such as a methyl group and an ethyl group; an alkoxy group having 1 to 5 carbon atoms such as a methoxy group and an isopropoxy group; a nitro group; a cyano group; and the like. The cyclic aliphatic group, the cyclic aliphatic group having 5 to 20 carbon atoms, the aromatic group, and the aromatic group having 2 to 20 carbon atoms may have at least one substituent selected from the aforementioned substituents. Note that, when there is a plurality of substituents, each substituent may be the same or different.

$A^1$ and $A^2$ are cyclic aliphatic groups which may have a substituent, and $B^1$ and $B^2$ are aromatic groups which may have a substituent.

A combination in which $A^1$ and $A^2$, independently of each other, are a trans-1,4-cyclohexelene group which may have a substituent represented by formula (a), and $B^1$ and $B^2$ are a 1,4-phenylene group which may have a

substituent represented by formula (b), and a combination in which $A^1$ and $A^2$, independently of each other, are a 1,4-phenylene group which may have a substituent represented by formula (b) and n and m, independently of each other are 0, are more preferable.

(a)

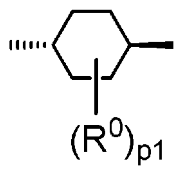

(b)

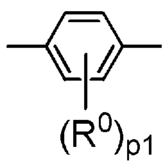

where $R^0$ and P1 are the same as defined above, and the preferred examples are also the same as stated above.

Further, in the aforementioned formula (I), $G^1$ and $G^2$ each independently represent an organic group which is either a divalent aliphatic hydrocarbon group having 1 to 30 carbon atoms, or a divalent aliphatic hydrocarbon group having 3 to 30 carbon atoms in which at least one $-CH_2-$contained in the divalent aliphatic hydrocarbon group is substituted with -O-, -S-, -O-C(=O)-, -C(=O)-O-, -O-C(=O)-O-, $-NR^{14}-C(=O)-$, $-C(=O)-NR^{14}-$, $-NR^{14}-$, or -C(=O)-, with the proviso that cases where there are two or more contiguous -O- or -S- are excluded. Here, $R^{14}$ represents a hydrogen atom or an alkyl group having 1 to 6 carbon atoms, the hydrogen atom included in the organic group of $G^1$ and $G^2$ may be substituted with an alkyl group having 1 to 5 carbon atoms such as a methyl group, an ethyl group, and a propyl group; an alkoxy group having 1 to 5 carbon atoms such as a methoxy group, an ethoxy group, and a propoxy group; a cyano group; or a halogen atom such as a fluorine atom, and a chlorine atom.

Note that, when $G^1$ and $G^2$ each have 3 or more carbon atoms, both ends of $G^1$ and $G^2$ are preferably $-CH_2-$ (both ends of $G^1$ and $G^2$ are unsubstituted). Further, in "at least $-CH_2-$ contained in a divalent aliphatic hydrocarbon group having 3 to 30 carbon atoms is substituted with -O-, -S-, -O-C(=O)-, -C(=O)-O-, -O-C(=O)-O-, $-NR^{14}-C(=O)-$, $-C(=O)-NR^{14}-$, $-NR^{14}-$, or, -C(=O)-", -O- and -S- preferably do not substitute consecutive $-CH_2-$ in the aliphatic hydrocarbon group (i.e., the -O-O- and -S-S- configurations are not formed) (in short, cases where there are two or more contiguous -O- or -S- are preferably excluded), and -C(=O)- preferably does not substitute consecutive $-CH_2-$ in the aliphatic hydrocarbon group (i.e., the -C(=O)-C(=O)-configuration is not formed). Note that, $R^{14}$ is the same as defined above.

$G^1$ and $G^2$ preferably each independently represent (i) "an organic group which is either a divalent aliphatic hydrocarbon group having 1 to 18 carbon atoms, or a divalent aliphatic hydrocarbon group having 3 to 18 carbon atoms in which at least one $-CH_2-$ contained in the divalent aliphatic hydrocarbon group is substituted with -O-, -S-, -O-C(=O)-, -C(=O)-O-, -O-C(=O)-O-, $-NR^{14}-C(=O)-$, $-C(=O)-NR^{14}-$, $-NR^{14}-$, or -C(=O)- (preferably with -O-, -S-, -O-C(=O)-, -C(=O)-O-, or -C(=O)-)", and more preferably (ii) "an alkylene group having 1 to 18 carbon atoms which may have a substituent". Note that, $R^{14}$ is the same as defined above.

The hydrogen atom of $G^1$ and $G^2$ included in the organic group includes an alkyl group having 1 to 5 carbon atoms such as a methyl group, an ethyl group, and a propyl group; an alkoxy group having 1 to 5 carbon atoms such as a methoxy group, an ethoxy group, and a propoxy group; a cyano group; or; may be substituted by a halogen atom such as a fluorine atom, and a chlorine atom.

Examples of the substituent of the alkylene group having 1 to 18 carbon atoms include an alkyl group having 1 to 5 carbon atoms such as a methyl group, an ethyl group, and a propyl group; an alkoxy group having 1 to 5 carbon atoms such as a methoxy group, an ethoxy group, and a propoxy group; a cyano group; or; a halogen atom such as a fluorine atom, and a chlorine atom.

Further, in the aforementioned formula (I), $P^1$ and $P^2$ each independently represent an alkenyl group having 2 to 10 carbon atoms which may be substituted by a halogen atom or a methyl group.

Examples of the alkenyl group having 2 to 10 carbon atoms of the alkenyl group having 2 to 10 carbon atoms which may have a substituent include a vinyl group, a propenyl group, an isopropenyl group, a butenyl group, an isobutenyl group, a pentenyl group, a hexenyl group, a heptenyl group, an octenyl group, a decenyl group and the like. $P^1$ and $P^2$ are preferably each independently $CH_2=CH-$ (vinyl group), $CH_2=C(CH_3)-$, or, $CH_2=C(Cl)-$, and $CH_2=CH-$ (vinyl group) is more preferable.

Here, in the formula (I), n and m are each independently 0 or 1, and more preferably are each independently 1.

When both of n and m are 1, $B^1$ and $B^2$ in the aforementioned formula (I) each independently preferably are a cyclic aliphatic group which may have a substituent, and more preferably are a cyclic aliphatic group having 5 to 20 carbon atoms which may have a substituent.

Further, the polymerizable compound (I) is not specifically limited, but preferably has a symmetrical structure around Ar (that is, $Y^1$ and $Y^2$, $A^1$ and $A^2$, $Y^3$ and $Y^4$, $B^1$ and $B^2$, n and m, $Y^5$ and $Y^6$, $G^1$ and $G^2$, $Y^7$ and $Y^8$, and $P^1$ and $P^2$ are respectively the same (symmetrical around Ar)).

Here, the polymerizable compound of the present disclosure is preferably a polymerizable compound represented by any of the following formulas (III-1) and (III-2), and more preferably the following formula (III-1).

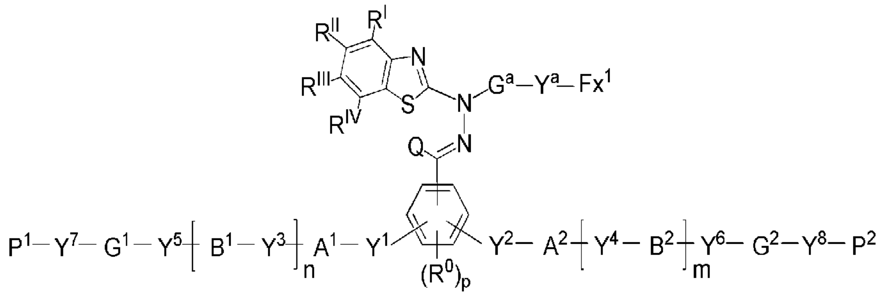

(III-1)

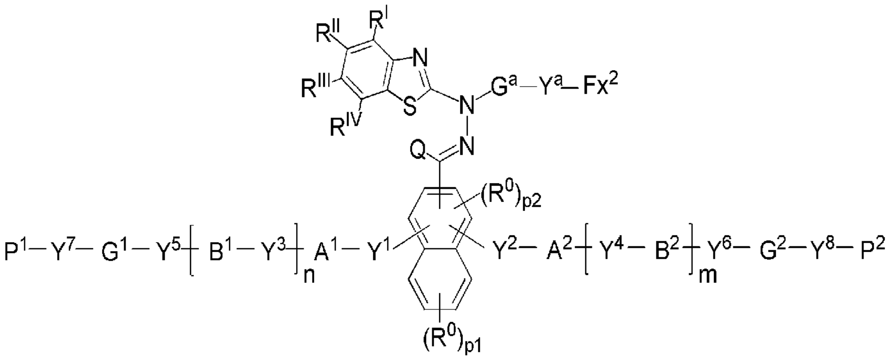

(III-2)

where in the formulas (III-1) and (III-2), $Y^1$ to $Y^8$, $A^1$, $A^2$, $B^1$, $B^2$, $G^1$, $G^2$, $P^1$, $P^2$, $R^I$ to $R^{IV}$, Q, $R^0$, n, m, p, p1 and p2 are the same as defined above, and the preferred examples are also the same as stated above.

$G^a$ represents an organic group which is either an alkylene group having 1 to 18 carbon atoms (preferably 3 to 18 carbon atoms) which may have a substituent, or an alkylene group having 3 to 18 carbon atoms in which at least one $-CH_2-$ contained in the alkylene group is substituted with -O-, -S-, -O-C(=O)-, -C(=O)-O-, or -C(=O)-, with the proviso that cases where there are two or more contiguous -O- or -S- are excluded. Here, the substituent which the organic group of $G^a$ has includes an alkyl group having 1 to 5 carbon atoms such as a methyl group, an ethyl group, and a propyl group; an alkoxy group having 1 to 5 carbon atoms such as a methoxy group, an ethoxy group, and a propoxy group; a cyano group; a halogen atom such as a fluorine atom, and a chlorine atom.

Note that, when the number of carbon atoms of Ga is 3 or more, both ends of Ga are preferably $-CH_2-$ (both ends of Ga are unsubstituted), and further, -C(=O)- preferably does not substitute consecutive $-CH_2-$ in the Ga (i.e., the -C(=O)-C(=O)- configuration is not formed).

Furthermore, $G^a$ is preferably an unsubstituted alkylene group having 4 to 16 carbon atoms, more preferably an unsubstituted alkylene group having 5 to 14 carbon atoms, particularly preferably an unsubstituted alkylene group having 6 to 12 carbon atoms, and most preferably an unsubstituted alkylene group having 6 to 10 carbon atoms.

$Y^a$ represents a chemical single bond, -O-, -C(=O)-, -C(=O)-O-, -O-C(=O)-, -O-C(=O)-O-, -C(=O)-S-, -S-C(=O)-, $-NR^{11}-C(=O)-$, $-C(=O)-NR^{11}-$, $-O-C(=O)-NR^{11}-$, $-NR^{11}-C(=O)-O-$, or , -S-, and $R^{11}$ represents a hydrogen atom or an alkyl group having 1 to 6 carbon atoms.

$Fx^1$ and $Fx^2$ are each independently an organic group having 2 to 20 carbon atoms having one of an aromatic hydrocarbon ring and an aromatic heterocyclic ring, and

the number of $\pi$ electrons included in the ring structure in $Fx^1$ is 8 or more, and the number of $\pi$ electrons included in the ring structure in $Fx^2$ is preferably 4 or more, the number of $\pi$ electrons included in the ring structure in $Fx^1$ is preferably 10 or more, and the number of $\pi$ electrons included in the ring structure in $Fx^2$ is preferably 6 or more.

The preferred examples of $Fx^1$ and $Fx^2$ are the same as defined above.

The combination of $G^a$, $Y^a$ and $Fx^1$ is preferably

(I) a combination in which $G^a$ is an organic group which is either an alkylene group having 1 to 18 carbon atoms (preferably 3 to 18 carbon atoms), or an alkylene group having 3 to 18 carbon atoms in which at least one $-CH_2-$contained in the alkylene group is substituted with -O-, -S-, -O-C(=O)-, -C(=O)-O-, or -C(=O)- (with the proviso that cases where there are two or more contiguous -O- or -S- are excluded),

$Y^a$ is a chemical single bond, -O-, -C(=O)-O-, -O-C(=O)-, $-C(=O)-NR^{11}-$, or $-O-C(=O)-NR^{11}-$ (with the proviso that

$R^{11}$ represents a hydrogen atom or an alkyl group having 1 to 6 carbon atoms), and
$Fx^1$ is an organic group in which the number of π electrons contained in the ring structure is 10 or more,
more preferably (II) a combination in which $G^a$ is an organic group which is either an alkylene group having 1 to 18 carbon atoms (preferably 3 to 18 carbon atoms), or an alkylene group having 3 to 18 carbon atoms in which at least one $-CH_2-$ contained in the alkylene group is substituted with -O-, -C(=O)-, or -S- (with the proviso that cases where there are two or more contiguous -O- or -S- are excluded),
$Y^a$ is a chemical single bond, -O-, -C(=O)-O-, -O-C(=O)-, $-C(=O)-NR^{11}-$, or $-O-C(=O)-NR^{11}-$ (with the proviso that $R^{11}$ represents a hydrogen atom or an alkyl group having 1 to 6 carbon atoms), and
$Fx^1$ is an organic group in which the number of π electrons contained in the ring structure is 10 or more,
particularly preferably (III) a combination in which $G^a$ is an alkylene group having 1 to 18 carbon atoms (preferably 3 to 18 carbon atoms),
$Y^a$ is a chemical single bond, -O-, -C(=O)-O-, -O-C(=O)-, $-C(=O)-NR^{11}-$, or $-O-C(=O)-NR^{11}-$ (with the proviso that $R^{11}$ represents a hydrogen atom or an alkyl group having 1 to 6 carbon atoms), and
$Fx^1$ is an organic group in which the number of π electrons contained in the ring structure is 10 or more, and
most preferably (IV) a combination in which $G^a$ is an alkylene group having 1 to 18 carbon atoms (preferably 3 to 18 carbon atoms),
$Y^a$ is a chemical single bond, -O-, -C(=O)-O-, or, -O-C(=O)-, and
$Fx^1$ is any of the following formulas (i-1) to (i-9):

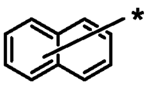
(i-1)
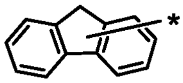
(i-2)
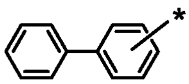
(i-3)
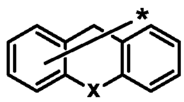
(i-4)
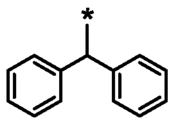
(i-5)
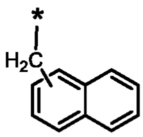
(i-6)
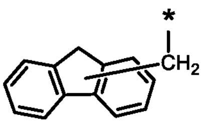
(i-7)
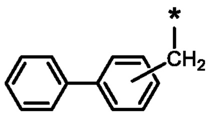
(i-8)
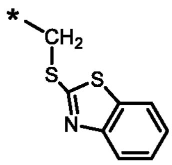
(i-9)

The combination of $G^a$, $Y^a$ and $Fx^2$ is preferably

(I) a combination in which $G^a$ is an organic group which is either an alkylene group having 1 to 18 carbon atoms (preferably 3 to 18 carbon atoms), or an alkylene group having 3 to 18 carbon atoms in which at least one -CH2- contained in the alkylene group is substituted with -O-, -S-, -O-C(=O)-, -C(=O)-O-, or -C(=O)- (with the proviso that cases where there are two or more contiguous -O- or -S- are excluded),
$Y^a$ is a chemical single bond, -O-, -C(=O)-O-, -O-C(=O)-, $-C(=O)-NR^{11}-$, or, $-O-C(=O)-NR^{11}-$ (where $R^{11}$ represents a hydrogen atom or an alkyl group having 1 to 6 carbon atoms), and
$Fx^2$ is an organic group in which the number of π electrons contained in the ring structure is 6 or more,
more preferably (II) a combination in which $G^a$ is an organic group which is either an alkylene group having 1 to 18 carbon atoms (preferably 3 to 18 carbon atoms), or an alkylene group having 3 to 18 carbon atoms in which at least one $-CH_2-$ contained in the alkylene group is substituted with -O-, -C(=O)-, or -S- (with the proviso that cases where there are two or more contiguous -O- or -S- are excluded),
$Y^a$ is a chemical single bond, -O-, -C(=O)-O-, -O-C(=O)-, $-C(=O)-NR^{11}-$, or, $-O-C(=O)-NR^{11}-$ (where $R^{11}$ represents a hydrogen atom or an alkyl group having 1 to 6 carbon atoms), and
$Fx^2$ is an organic group in which the number of π electrons contained in the ring structure is 6 or more,
particularly preferably (III) a combination in which $G^a$ is an alkylene group having 1 to 18 carbon atoms (preferably 3 to 18 carbon atoms),
$Y^a$ is a chemical single bond, -O-, -C(=O)-O-, -O-C(=O)-, $-C(=O)-NR^{11}-$, or, $-O-C(=O)-NR^{11}-$ (where $R^{11}$ represents a hydrogen atom or an alkyl group having 1 to 6 carbon atoms), and
$Fx^2$ is an organic group in which the number of π electrons contained in the ring structure is 6 or more, and
most preferably (IV) a combination in which $G^a$ is an alkylene group having 1 to 18 carbon atoms (preferably 3 to 18 carbon atoms),
$Y^a$ is a chemical single bond, -O-, -C(=O)-O-, or, -O-C(=O)-, and
$Fx^2$ is any of the following formulas (i-1) to (i-11).

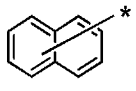
(i-1)
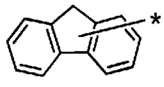
(i-2)
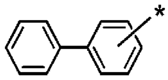
(i-3)
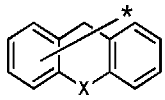
(i-4)
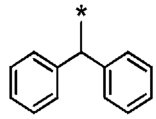
(i-5)
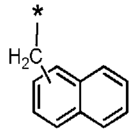
(i-6)
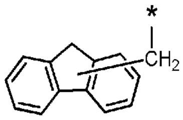
(i-7)
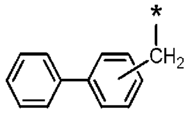
(i-8)
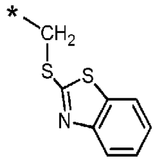
(i-9)
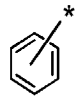
(i-10)
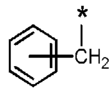
(i-11)

Here, the polymerizable compound represented by the aforementioned formula (III-1) is preferably the polymerizable compound represented by the following formula (iii-1), more preferably the polymerizable compound represented by the following formula (iii-2), and the polymerizable compounds represented by any of the following formulas (1) to (21) are particularly preferable.

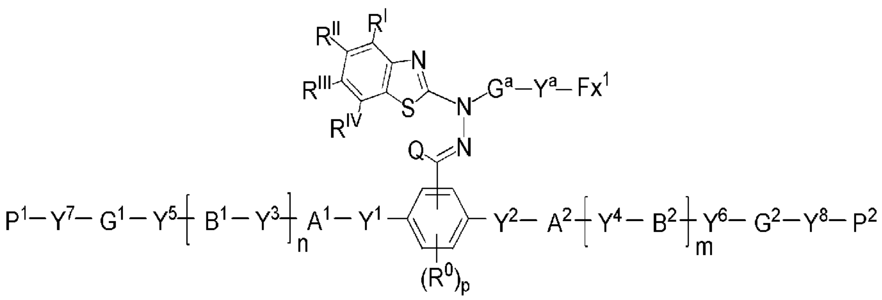
(iii-1)

where in the formula (iii-1), $Y^1$ to $Y^8$, $A^1$, $A^2$, $B^1$, $B^2$, $G^1$, $G^2$, $P^1$, $P^2$, $R^I$ to $R^{IV}$, Q, $G^a$, $Y^a$, $Fx^1$, $R^0$, m, n and p are the same as defined above, and the preferred examples are also the same as stated above.

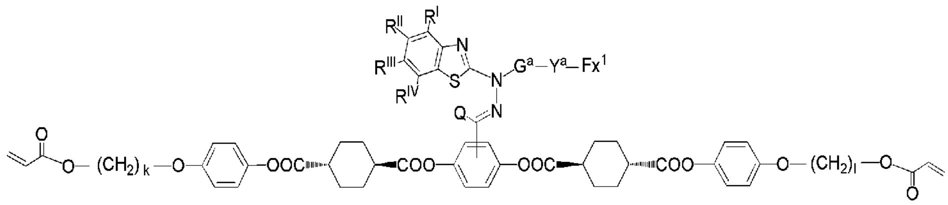
(iii-2)

where in the formula (iii-2), $R^I$ to $R^{IV}$, Q, $G^a$, $Y^a$ and $Fx^1$ are the same as defined above and the preferred examples are also the same as stated above, and k and 1 each independently represent an integer from 1 to 18.

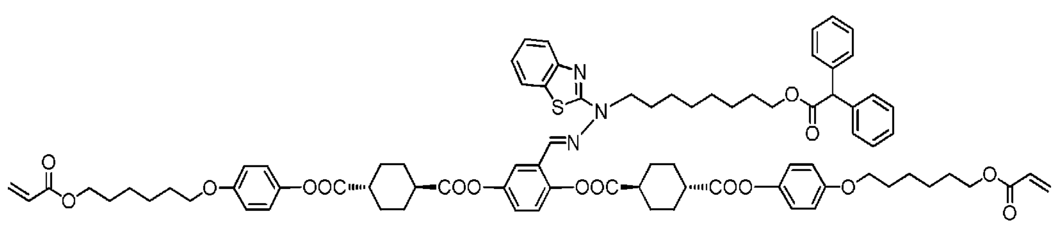
(1)

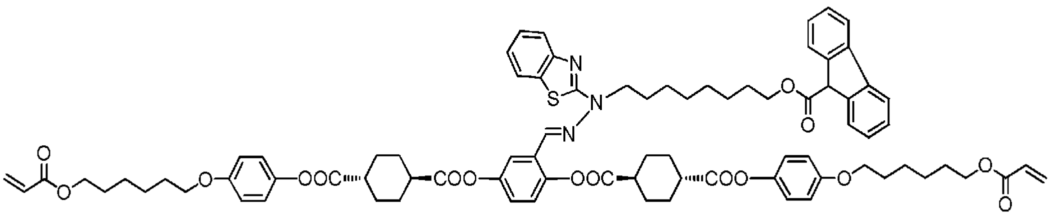

( 2 )

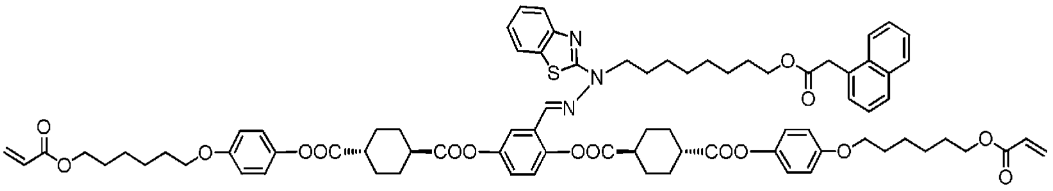

( 3 )

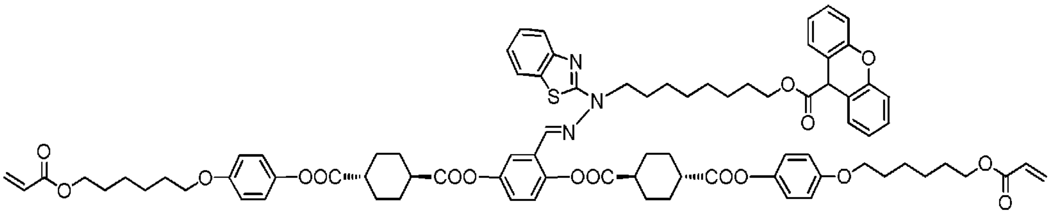

( 4 )

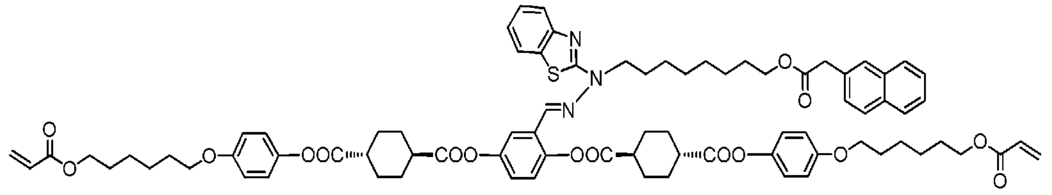

( 5 )

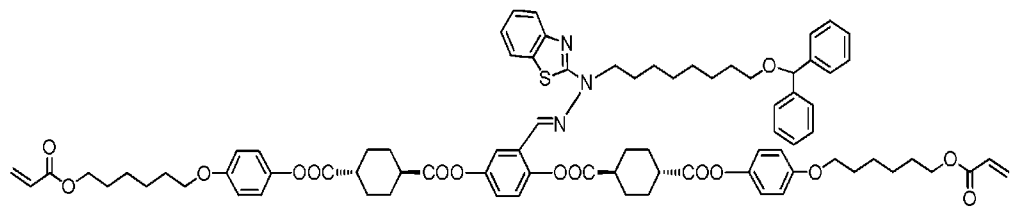

( 6 )

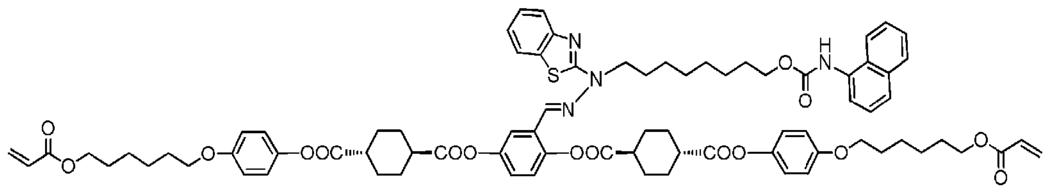

( 7 )

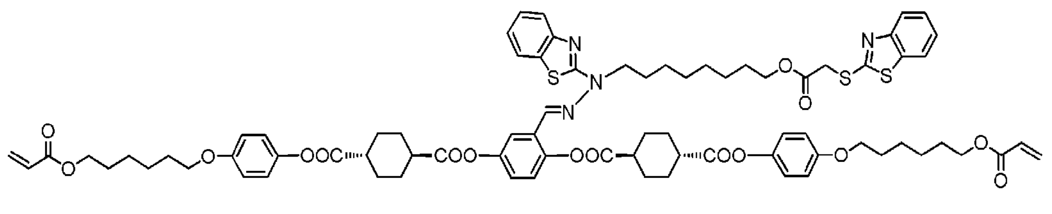

( 8 )

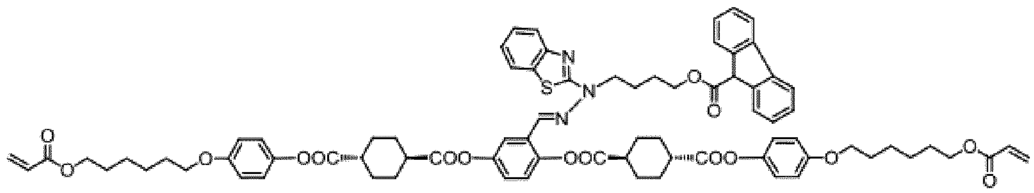
(9)

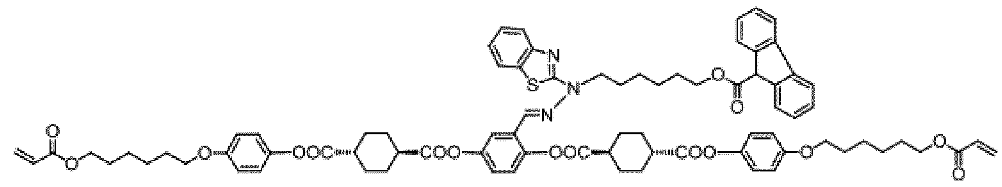
(10)

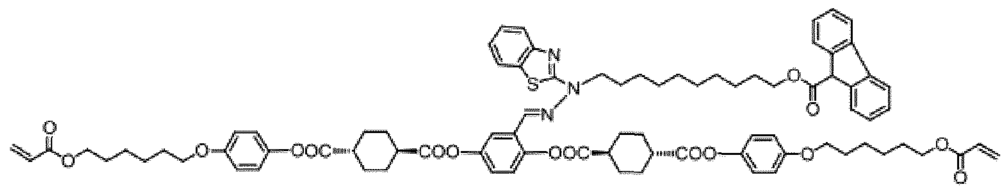
(11)

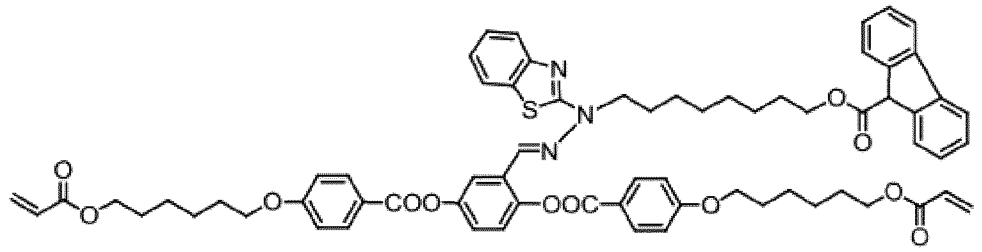
(12)

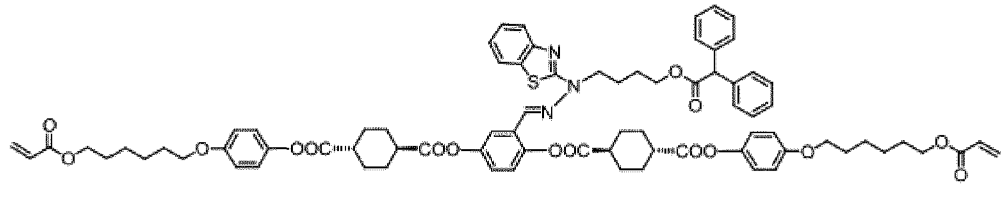
(13)

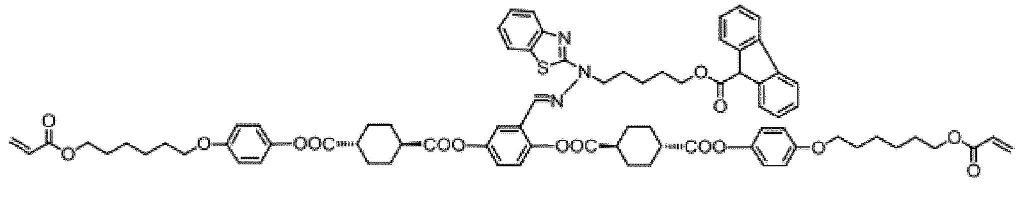
(14)

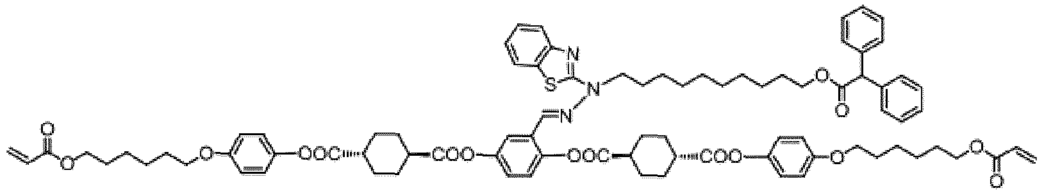
(15)

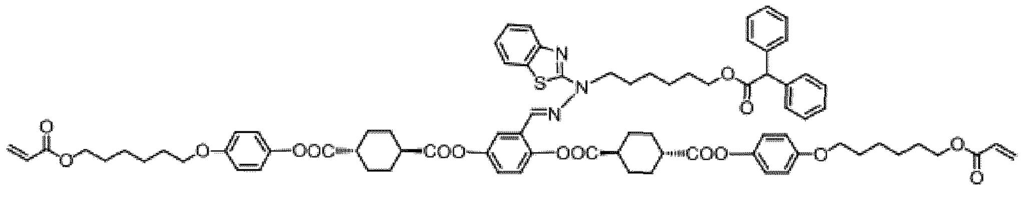

(1 6)

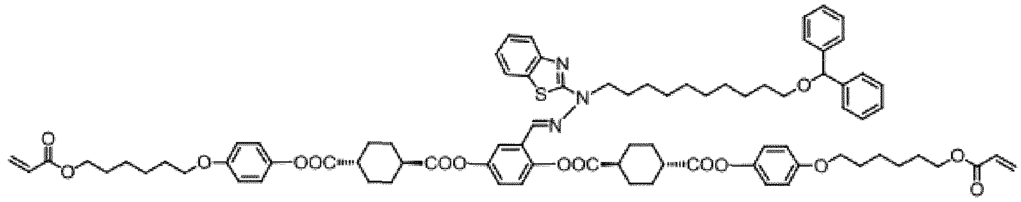

(1 7)

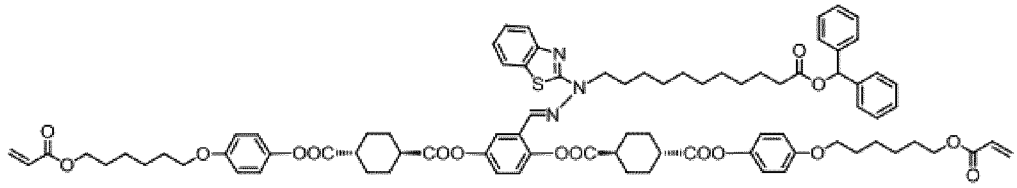

(1 8)

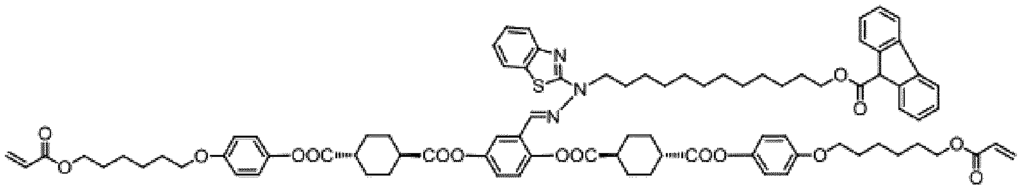

(1 9)

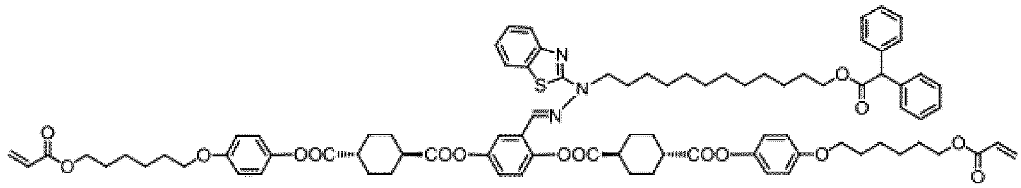

(2 0)

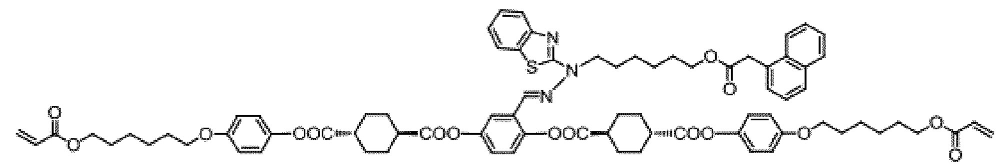

(2 1)

The aforementioned polymerizable compound (I) described above may be produced by a combination of known synthesis reactions. That is, the polymerizable compound (I) may be synthesized by referring to the methods described in various literatures (e.g., WO2012/141245, WO2012/147904, WO2014/010325, WO2013/046781, WO2014/061709, WO2014/126113, WO2015/064698, WO2015/140302, WO2015/129654, WO2015/141784, WO2016/159193, WO2012/169424, WO2012/176679, and WO2015/122385).

(2) Polymerizable composition

The aforementioned polymerizable composition comprises at least polymerizable compound (I) and a polymerization initiator.

Note that, as described later, the aforementioned polymerizable composition is useful as a material for producing

the polymer, the optical film, and the optically anisotropic body of the present disclosure. Moreover, the polymerizable composition of the present disclosure can suitably produce an optical film or the like having excellent in-plane thickness uniformity and improved in-plane uniformity in optical properties.

Here, the polymerization initiator is used to more efficiently perform the polymerization reaction of the polymerizable compound (I) contained in the polymerizable composition.

Moreover, examples of the polymerization initiator to be used include a radical polymerization initiator, an anionic initiator, a cationic polymerization initiator and the like.

Examples of the radical initiator include a thermal radical generator which is a compound that generates an active species that initiates the polymerization of the polymerizable compound upon heating; and a photo-radical generator which is a compound that generates an active species that initiate the polymerization of the polymerizable compound upon exposure to light such as visible light, ultraviolet rays (e.g., i-line), deep ultraviolet rays, electron beams, and X-rays, but it is preferable to use the photo-radical generator.

Examples of the photo-radical generator include an acetophenone-based compound, a biimidazole-based compound, a triazine-based compound, an O-acyloxime-based compound, an onium salt-based compound, a benzoin-based compound, a benzophenone-based compound, an $\alpha$-diketone-based compound, a polynuclearquinone-based compound, a xanthone-based compound, a diazo-based compound, an imide sulfonate-based compound, and the like. These compounds generate either or both of active radicals and an active acid upon exposure. These photo-radical generators may be used either alone or in combination.

Specific examples of the acetophenone-based compound include 2-hydroxy-2-methyl-1-phenylpropan-1-one, 2-methyl-1-[4-(methylthio)phenyl]-2-morpholinopropan-1-one, 2-benzyl-2-dimethylamino-1-(4-morpholinophenyl)butan-1-one, 1-hydroxycyclohexyl phenyl ketone, 2,2-dimethoxy-1,2-diphenylethan-1-one, 1-[4-(phenylthio)phenyl]-octane-1,2-dione-2-(O-benzoyloxime) and the like.

Specific examples of the biimidazole-based compound include 2,2'-bis(2-chlorophenyl)-4,4',5,5'-tetrakis(4-ethoxycarbonylphenyl)-1,2'-biim idazole, 2,2'-bis(2-bromophenyl)-4,4',5,5'-tetrakis(4-ethoxycarbonylphenyl)-1,2'-biim idazole, 2,2'-bis(2-chlorophenyl)-4,4',5,5'-tetraphenyl-1,2'-biimidazole, 2,2'-bis(2,4-dichlorophenyl)-4,4',5,5'-tetraphenyl-1,2'-biimidazole, 2,2'-bis(2,4,6-trichlorophenyl)-4,4',5,5'-tetraphenyl-1,2'-biimidazole, 2,2'-bis(2-bromophenyl)-4,4',5,5'-tetraphenyl-1,2'-biimidazole, 2,2'-bis(2,4-dibromophenyl)-4,4',5,5'-tetraphenyl-1,2'-biimidazole, 2,2'-bis(2,4,6-tribromophenyl)-4,4',5,5'-tetraphenyl-1,2'-biimidazole, and the like.

Note that, in the present disclosure, when using a biimidazole-based compound as a photoinitiator (photo-radical generator), it is preferable to use a hydrogen donor in combination with the biimidazole-based compound in order to further improve sensitivity.

Here, the term "hydrogen donor" refers to a compound which can donate a hydrogen atom to radicals generated by the biimidazole-based compound upon exposure to light. A mercaptan-based compound, an amine-based compound and the like defined below are preferable as the hydrogen donor.

Examples of the mercaptan-based compound include 2-mercaptobenzothiazole, 2-mercaptobenzoxazole, 2-mercaptobenzimidazole, 2,5-dimercapto-1,3,4-thiadiazole, 2-mercapto-2,5-dimethylaminopyridine, and the like. Examples of the amine-based compound include 4,4'-bis(dimethylamino)benzophenone, 4,4'-bis(diethylamino)benzophenone, 4-diethylaminoacetophenone, 4-dimethylaminopropiophenone, ethyl-4-dimethylaminobenzoate, 4-dimethylaminobenzoic acid and 4-dimethylaminobenzonitrile.

Specific examples of the triazine-based compound include a triazine-based compound that includes a halomethyl group, such as 2,4,6-tris(trichloromethyl)-s-triazine, 2-methyl-4,6-bis(trichloromethyl)-s-triazine, 2-[2-(5-methylfuran-2-yl)ethenyl)ethenyl]-4,6-bis(trichloromethyl)-s-triazine, 2-[2-(4-diethylamino-2-methylphenyl)ethenyl]-4,6-bis(trichloromethyl)-s-tria zine, 2-[2-(3,4-dimethoxyphenyl)ethenyl]-4,6-bis(trichloromethyl)-s-triazine, 2-(4-methoxyphenyl)-4,6-bis(trichloromethyl)-s-triazine, 2-(4-ethoxystyryl)-4,6-bis(trichloromethyl)-s-triazine, 2-(4-n-butoxyphenyl)-4,6-bis(trichloromethyl)-s-triazine and the like.

Specific examples of the O-acyloxime-based compound include 1-[4-(phenylthio)phenyl]heptane-1,2-dione-2-(O-benzoyloxime), 1-[4-(phenylthio)phenyl]octane-1,2-dione-2-(O-benzoyloxime), 1-[4-(benzoyl)phenyl]octane-1,2-dione-2-(O-benzoyloxime), 1-[9-ethyl-6-(2-methylbenzoyl)-9H-carbazol-3-yl]ethanone-1-(O-acetyloxime ), 1-[9-ethyl-6-(3-methylbenzoyl)-9H-carbazol-3-yl]ethanone-1-(O-acetyloxime ), 1-(9-ethyl-6-benzoyl-9H-carbazol-3-yl)ethanone-1-(O-acetyloxime), ethanone-1-[9-ethyl-6-(2-methyl-4-tetrahydrofuranylbenzoyl)-9H-carbazol-3-yl]-1-(O-acetyloxime), ethanone-1-[9-ethyl-6-(2-methyl-4-tetrahydropyranylbenzoyl)-9H-carbazol-3-yl]-1-(O-acetyloxime), ethanone-1-[9-ethyl-6-(2-methyl-5-tetrahydrofuranylbenzoyl)-9H-carbazol-3-yl]-1-(O-acetyloxime), ethanone-1-[9-ethyl-6-(2-methyl-5-tetrahydropyranylbenzoyl)-9H-carbazol-3-yl]-1-(O-acetyloxime), ethanone-1-[9-ethyl-6-{2-methyl-4-(2,2-dimethyl-1,3-dioxolanyl)benzoyl}-9 H-carbazol-3-yl]-1-(O-acetyloxime), ethanone-1-[9-ethyl-6-(2-methyl-4-tetrahydrofuranylmethoxybenzoyl)-9H-car bazol-3-yl]-1-(O-acetyloxime), ethanone-1-[9-ethyl-6-(2-methyl-4-tetrahydropyranylmethoxybenzoyl)-9H-ca rbazol-3-yl]-1-(O-acetyloxime), ethanone-1-[9-ethyl-6-(2-methyl-5-tetrahydrofuranylmethoxybenzoyl)-9H-car bazol-3-yl]-1-(O-acetyloxime), ethanone-1-[9-ethyl-6-(2-methylbenzoyl)-9H-carbazol-3-yl]-1-(O-acetyloxim e), ethanone-1-[9-ethyl-6-(2-methyl-5-tetrahydropyranylmethoxybenzoyl)-9H-ca rbazol-3-yl]-1-(O-acetyloxime), ethanone-1-[9-ethyl-6-{2-methyl-4-(2,2-dimethyl-1,3-dioxolanyl)methoxyben zoyl}-9H-carbazol-3-yl]-1-(O-acetyloxime) and the like.

Further, a commercially available product may be used directly as the photo-radical generator. Specific examples of a commercially available product that may be used as the photo-radical generator include Irgacure 907, Irgacure 184, Irgacure 369, Irgacure 651, Irgacure 819, Irgacure 907, and Irgacure OXE02 (manufactured by BASF); Adeka Arkls N1919T (manufactured by Adeka Corporation); and the like.

Examples of the anionic initiator include an alkyllithium compound; a monolithium salt or a monosodium salt of biphenyl, naphthalene, pyrene and the like; a polyfunctional initiator such as a dilithium salt and a trilithium salt; and the like.

Further, examples of the cationic polymerization initiator include a proton acid such as sulfuric acid, phosphoric acid, perchloric acid, and trifluoromethanesulfonic acid; a Lewis acid such as boron trifluoride, aluminum chloride, titanium tetrachloride, and tin tetrachloride; an aromatic onium salt or a combination of an aromatic onium salt and a reducing agent; and the like.

These polymerization initiators may be used either alone or in combination.

Note that, in the aforementioned polymerizable composition, the blending ratio of the polymerization initiator is normally 0.1 to 30 parts by weight, and preferably 0.5 to 10 parts by weight, based on 100 parts by weight of the polymerizable compound in the polymerizable composition.

Further, a surfactant is preferably added to the aforementioned polymerizable composition in order to adjust the surface tension. The surfactant is not particularly limited, but a nonionic surfactant is normally preferable as the surfactant. Examples of a commercially available product that may be used as the nonionic surfactant include a nonionic surfactant which is a fluorine-containing group, a hydrophilic group, and a lipophilic group-containing oligomer, for example, the SURFLON series (S242, S243, S386, S611, S651, etc) manufactured by AGC Seimi Chemical Co., Ltd, MEGAFACE SERIES (F251, F554, F556, F562, RS-75, RS-76-E, etc) manufactured by DIC Corporation, the Ftargent series (FTX601AD, FTX602A, FTX601ADH2, FTX650A, etc) manufactured by Neos Co., Ltd. and the like. Further, as the surfactant, one type thereof may be solely used, and two or more types thereof may also be used in combination at any ratio.

Here, in the aforementioned polymerizable composition, the blending ratio of the surfactant is normally 0.01 to 10 parts by weight, and preferably 0.01 to 2 parts by weight, based on 100 parts by weight of the polymerizable compound in the polymerizable composition.

Furthermore, in addition to the polymerizable compound, the polymerization initiator and the surfactant, other components may be further included to the extent that the effect of the present disclosure is not affected. Examples of the other components include a metal, a metal complex, a dye, a pigment, a fluorescent material, a phosphorescent material, a leveling agent, a thixotropic agent, a gelling agent, a polysaccharide, an ultraviolet absorber, an infrared absorber, an antioxidant, and an ion exchange resin and titanium oxide.

Further, examples of the other components include also include other copolymerizable monomers. These are not specifically limited, and examples include 4'-methoxyphenyl 4-(2-methacryloyloxyethyloxy)benzoate, biphenyl 4-(6-methacryloyloxyhexyloxy)benzoate, 4'-cyanobiphenyl 4-(2-acryloyloxyethyloxy)benzoate, 4'-cyanobiphenyl 4-(2-methacryloyloxyethyloxy)benzoate, 3',4'-difluorophenyl 4-(2-methacryloyloxyethyloxy)benzoate, naphthyl 4-(2-methacryloyloxyethyloxy)benzoate, 4-acryloyloxy-4'-decylbiphenyl, 4-acryloyloxy-4'-cyanobiphenyl, 4-(2-acryloyloxyethyloxy)-4'-cyanobiphenyl, 4-(2-methacryloyloxyethyloxy)-4'-methoxybiphenyl, 4-(2-methacryloyloxyethyloxy)-4'-(4"-fluorobenzyloxy)-biphenyl, 4-acryloyloxy-4'-propylcyclohexylphenyl, 4-methacryloyl-4'-butylbicyclohexyl, 4-acryloyl-4'-amyltolane, 4-acryloyl-4'-(3,4-difluorophenyl)bicyclohexyl, (4-amylphenyl) 4-(2-acryloyloxyethyl)benzoate, (4-(4'-propylcyclohexyl)phenyl) 4-(2-acryloyloxyethyl)benzoate, Product name: "LC-242" (BASF), trans-1,4-bis[4-[6-(acryloyloxy)hexyloxy]phenyl]cyclohexane dicarboxylate, and polymerizable monomers such as the compounds disclosed in JP2007-002208A, JP2009-173893A, JP2009-274984A, JP2010-030979A, JP2010-031223A, JP2011-006360A, JP2010-24438A, WO2012/141245, WO2012/147904, WO2012/169424, WO2012/76679, WO2013/180217, WO2014/010325, WO2014/061709, WO2014/065176, WO2014/126113, WO2015/025793, WO2015/064698, WO2015/122384, and WO2015/122385.

The blending ratio of these other components is normally 0.005 to 50 parts by weight based on 100 parts by weight of the polymerizable compound contained in the polymerizable composition.

The aforementioned polymerizable composition can normally be prepared by mixing and dissolving the polymerizable compound, the polymerization initiator, and, a predetermined amount of the other components to be blended according to need in an appropriate solvent.

Examples of the organic solvent a ketone such as cyclopentanone, cyclohexanone, and methyl ethyl ketone; an acetate such as butyl acetate and amyl acetate; a halogenated hydrocarbon such as chloroform, dichloromethane, and dichloroethane; an ether such as 1,4-dioxane, cyclopentyl methyl ether, tetrahydrofuran, tetrahydropyran, and 1,3-dioxolane; and the like.

(3) Polymer

The polymer of the present disclosure is obtained by polymerizing the aforementioned polymerizable compound (I) or the aforementioned polymerizable composition.

Here, the term "polymerization" means a chemical reaction in a broad sense including a normal polymerization reaction and a crosslinking reaction.

Moreover, the polymer of the present disclosure normally has a monomer unit (for example, a repeating unit (I)') derived from the polymerizable compound (I).

The structure of the repeating unit (I)' when using the polymerizable compound (I) having a polymerizable group represented by $CH_2$=CR- as $P^1$ and $P^2$, -C(=O)-O- as $Y^7$, and -O-C(=O)- as $Y^8$ is shown as one example below.

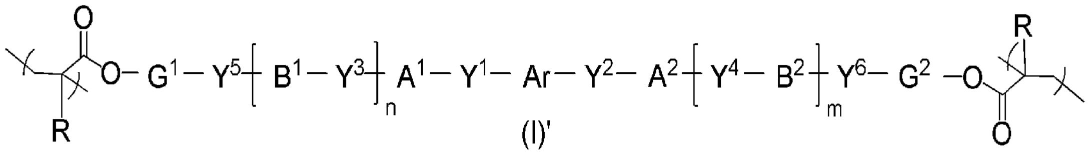

(I)'

where in the formula (I)', Ar, $Y^1$ to $Y^6$, $A^1$, $A^2$, $B^1$, $B^2$, $G^1$, $G^2$, n and m are defined as stated above and the preferred example are the same, and R is a hydrogen atom, a halogen atom such as a fluorine atom or a chlorine atom, or a methyl group, and thereamong, a hydrogen atom, a chlorine atom or a methyl group are preferable.

Note that, the polymer of the present disclosure is prepared using the aforementioned polymerizable compound (I), or, the aforementioned polymerizable composition, and thus, can be suitably used as the constituent material of the optical film or the like.

Further, the polymer of the present disclosure is not specifically limited, and can be used in any shape or form according to its intended use, including film, powder or layer made of an aggregation of powder.

Specifically, a film of the polymer can be suitably used as the constituent material of the optical film and the optically anisotropic body which are described later, powders of the polymer can be utilized for paints, anti-forgery items, security items and the like, and layers made of the polymer powder can be suitably used as the constituent material for the optically anisotropic body.

Moreover, the polymer of the present disclosure can be suitably produced for example by ($\alpha$) a method for polymerizing the aforementioned polymerizable compound (I), or, the aforementioned polymerizable composition, isolating the target polymer, dissolving the obtained polymer in the presence of a suitable organic solvent to prepare a solution, applying the solution on a suitable substrate to form thereon a coating film, and then drying the coating film followed by optional heating, or ($\beta$) a method for performing a polymerization reaction by dissolving the aforementioned polymerizable compound (I), or, the aforementioned polymerizable composition in an organic solvent, applying the solution on a substrate by a coating method known in the art, and then removing the solvent, and by heating or irradiation with actinic radiation and the like. Note that, the aforementioned polymerizable compound (I) may be polymerized alone.

The organic solvent which can be used for the polymerization by method ($\alpha$) is not specifically limited as long as it is inert. Examples of the organic solvent include aromatic hydrocarbons such as toluene, xylene, and mesitylene; ketones such as cyclohexanone, cyclopentanone, and methyl ethyl ketone; acetates such as butyl acetate and amyl acetate; halogenated hydrocarbons such as chloroform, dichloromethane, and dichloroethane; and ethers such as cyclopentyl methyl ether, tetrahydrofuran, and tetrahydropyran.

Thereamong, an organic solvent having a boiling point of 60°C to 250°C is preferable, and those having a boiling point of 60°C to 150°C are more preferable from the viewpoint of handling capability.

Further, examples of the organic solvents used to dissolve the isolated polymer in method ($\alpha$) and the organic solvents used in method ($\beta$) include ketone solvents such as acetone, methyl ethyl ketone, methyl isobutyl ketone, cyclopentanone, and cyclohexanone; ester solvents such as butyl acetate and amyl acetate; halogenated hydrocarbon solvents such as dichloromethane, chloroform, and dichloroethane; halogenated hydrocarbon solvents such as dichloromethane, chloroform, and dichloroethane; ether solvents such as tetrahydrofuran, tetrahydropyran, 1,2-dimethoxyethane, 1,4-dioxane, cyclopentyl methyl ether, and 1,3-dioxolane; and aprotic polar solvents such as N,N-dimethylformamide, N,N-dimethylacetamide, dimethyl sulfoxide, $\gamma$-butyrolactone, and N-methylpyrrolidone (N-methyl-2-pyrrolidone); and the like. Thereamong, an organic solvent having a boiling point of the solvent of 60°C to 200°C is preferable from the viewpoint of handling capability. These solvents can be used alone or in combination.

Substrates made of any organic or inorganic material known in the art can be used in the methods ($\alpha$) and ($\beta$). Examples of the organic material include polycycloolefins such as Zeonex and Zeonor (Zeonex and Zeonor are registered trademarks in Japan, other countries, or both) manufactured by Zeon Corporation, Arton (Arton is a registered trademark in Japan, other countries, or both) manufactured by JSR Corporation, and Apel (Apel is a registered trademark in Japan, other countries, or both) manufactured by Mitsui Chemicals Inc.), polyethylene terephthalates; polycarbonates; polyimides; polyamides; polymethyl methacrylates; polystyrenes, polyvinyl chlorides; polytetrafluoroethylene, celluloses, cellulose triacetate; polyethersulfones and the like. Examples of the inorganic material include silicon, glass, calcite and the like.

Further, the substrate may be a monolayer or a laminate.

The substrate is preferably made of an organic material, more preferably a resin film formed to a film shape with an organic material.

Note that, the substrate includes those used for the production of the optically anisotropic body which is described later.

Further, methods known in the art can be used for applying the polymer solution on the substrate in method ($\alpha$) and for applying the solution for the polymerization reaction on the substrate in method ($\beta$). Specific examples of usable coating methods include curtain coating, extrusion coating, roll coating, spin coating, dip coating, bar coating, spray coating, slide coating, print coating, gravure coating, die coating and cap coating.

Furthermore, drying or solvent removal in methods ($\alpha$) and ($\beta$) can be effected by natural drying, drying by heating, drying under reduced pressure, drying by heating under reduced pressure, or the like.

The drying temperature is not specifically limited as long as the solvent can be removed, but the lower limit temperature is preferably 50°C or more, and more preferably 70°C or more from the viewpoint of stably obtaining a constant temperature.

The upper limit of the drying temperature is preferably 200°C or less, and more preferably 195°C or less from the viewpoint of not adversely affecting the substrate.

Further, the method for polymerizing the aforementioned polymerizable compound (I) or the aforementioned polymerizable composition includes irradiation with actinic radiation, thermal polymerization and the like, but irradiation with actinic radiation is preferable as the reaction progresses at room temperature without requiring heating. Thereamong, irradiation with light such as UV light is preferable as the operation is simple.

The temperature for irradiating light such as UV is not specifically limited as long as long as the liquid crystal phase can be maintained, but the lower limit temperature is preferably 15°C or more, and more preferably 20°C or more from the viewpoint that the photopolymerization can stably progress.

The upper limit of the temperature for irradiating light such as UV is preferably 200°C or less, and more preferably 195°C or less from the viewpoint of not adversely affecting the substrate.

Here, the temperature during light irradiation is preferably set to 100°C or less. The irradiation intensity is normally in a range from 1 $W/m^2$ to 10 $kW/m^2$, preferably in a range from 5 $W/m^2$ to 2 $kW/m^2$. The irradiation amount of the UV rays is preferably 0.1 $mJ/cm^2$ or more, more preferably 0.5 $mJ/cm^2$ or more, and preferably 5000 $mJ/cm^2$ or less, and more preferably 4000 $mJ/cm^2$ or less.

The polymer obtained as described above can be transferred from the substrate for use, removed from the substrate for single use, or used as is as the constituent material for optical film etc. without being removed from the substrate.

Further, the polymer removed from the substrate can also be made into powder form by a grinding method known in the art prior to use.

The number-average molecular weight of the polymer of the present disclosure obtained as described above is preferably 500 to 500,000, more preferably 5,000 to 300,000. When the number-average molecular weight is within these ranges, a high hardness can be obtained, and the handling capability is excellent which is desirable. The number-average molecular weight of the polymer can be determined by gel permeation chromatography (GPC) using monodisperse polystyrene as a standard with tetrahydrofuran as an eluant.

Moreover, the polymer of the present disclosure can obtain an optical film or the like having excellent in-plane thickness uniformity, and improved in-plane uniformity in optical properties.

(4) Optical film

The optical film of the present disclosure is formed using the polymer of the present disclosure and/or the polymerizable compound, and includes a layer having an optical function. An optical function means a simple transmittance, reflection, refraction, birefringence, or the like. Moreover, the optical film of the present disclosure uses the polymer of the present invention as a main constituent material of the layer having an optical function, or, the layer having an optical function includes the polymerizable compound of the present disclosure. Preferably, the optical film which uses the polymer of the present disclosure as a constituent material has an occupancy ratio of the polymer of the present disclosure in excess of 50 mass% when all of the components of the layer having the optical function are 100 mass%. Further, the optical film containing the polymerizable compound of the present disclosure preferably comprises 0.01 mass% or more of the polymerizable compound of the present disclosure when all of the components of the layer having the optical function are 100 mass%.

Here, the optical film of the present disclosure may be used in any of the following configurations: alignment

substrate / (alignment film) / optical film configuration where the optical film remains formed on an alignment substrate which may have an alignment film; transparent substrate film / optical film configuration where the optical film has been transferred to a transparent substrate film or the like which is different from the alignment substrate; and a single optical film configuration (optical film) when the optical film is self-supportive.

Note that, the alignment film and the alignment substrate can use the same substrate and alignment film as the optically anisotropic body which is described later.

Moreover, the optical film of the present disclosure can be produced by (A) the method for applying on an alignment substrate a solution containing the polymerizable compound of the present disclosure, or, of the solution of the polymerizable composition, drying the resulting coating film, subjecting the film to heat treatment (for alignment of liquid crystals), and irradiation of light and/or heating treatment (for polymerization); (B) the method for applying on an alignment substrate a solution of a liquid crystal polymer obtained by polymerization of the polymerizable compound or the polymerizable composition of the present disclosure, and optionally drying the resulting coated film, or (C) the method for applying on an alignment substrate a solution containing the polymerizable compound of the present disclosure and resin, and drying the resulting coated film.

The optical film of the present disclosure can be used for an optically anisotropic body, alignment films for liquid crystal display devices, color filters, low-pass filters, polarization prisms, and various optical filters.

Note that, the optical film of the present disclosure was determined from the retardation at wavelengths from 400 nm to 800 nm measured by a Mueller Matrix Polarimeter Axoscan. It is preferable that the following $\alpha$-value and $\beta$-value are within a predetermined range. Specifically, the $\alpha$-value is preferably 0.70 to 0.99, and more preferably 0.75 to 0.90. Further, the $\beta$-value is preferably 1.00 to 1.25, and more preferably 1.01 to 1.20.

$$\alpha = (\text{retardation at 450 nm}) / (\text{retardation at 550 nm})$$

$$\beta = (\text{retardation at 650 nm}) / (\text{retardation at 550 nm})$$

(5) Optically anisotropic body

The optically anisotropic body of the present disclosure has a layer which makes the polymer of the present disclosure as the constituent material.

The optically anisotropic body of the present disclosure can be obtained, for example, by forming an alignment film on a substrate and forming a layer (liquid crystal layer) made of the polymer of the present disclosure on the alignment film. Note that, the optically anisotropic body of the present disclosure may be obtained by directly forming a layer (liquid crystal layer) made the polymer of the present disclosure on the substrate, or may consist only of a layer (liquid crystal layer) made of the polymer of the present disclosure.

Note that, the layer forming the polymer may be formed of a polymer film or may be an aggregate of powdery polymer.

Here, the alignment film is formed on the surface of the substrate to align and regulate the polymerizable compound in one direction in the plane.

The alignment film can be obtained by applying a solution (alignment film composition) containing a polymer such as polyimide, polyvinyl alcohol, polyester, polyarylate, polyamideimide, or polyetherimide on the substrate, drying the film, and rubbing the film in one direction.

The thickness of the alignment film is preferably 0.001 to 5 $\mu$m, and more preferably 0.001 to 1.0 $\mu$m.

The method of the rubbing treatment is not specifically limited, but, for example, the alignment film may be rubbed in a predetermined direction using a roll around which a cloth or felt formed of a synthetic fiber such as nylon or a natural fiber such as cotton is wound. The alignment film is preferably washed with isopropyl alcohol or the like after the rubbing treatment in order to remove fine powders (foreign substances) formed during the rubbing treatment to clean the surface of the alignment film.

Further, other than the rubbing treatment, the alignment film can be provided with a function for aligning and regulating in one direction in the plane even by a method for irradiating the surface of the alignment film with polarized UV light.

Examples of substrates on which the alignment film is to be formed include glass substrates and substrates formed of synthetic resin films. Examples of synthetic resins include thermoplastic resins such as acrylic resins, polycarbonate resins, polyethersulfone resins, polyethylene terephthlate resins, polyimide resins, polymethyl methacrylate resins, polysulfone resins, polyarylate resins, polyethylene resins, polystyrene resins, polyvinyl chloride resins, cellulose diacetate, cellulose triacetate and alicyclic olefin polymers.

Examples of the alicyclic olefin polymers include the cyclic olefin random multi-component copolymers described

in JPH05-310845A and US5,179,171; the hydrogenated polymers described in JPH05-97978A and US5,202,388, and the thermoplastic dicyclopentadiene open-ring polymers and the hydrogenated products thereof described in JPH11-124429A (WO99/20676) and the like.

In the present disclosure, examples of methods of forming a liquid crystal layer made of the polymer of the present disclosure on the alignment film are the same as the methods described in the above chapter for the polymer of the present disclosure (methods ($\alpha$) and ($\beta$)).

The thickness of the resulting liquid crystal layer is not specifically limited, but normally is 1 to 10 $\mu$m.

Note that, examples of the optically anisotropic body of the present disclosure are not specifically limited, and may include a retardation plate, a viewing-angle enhancing film and the like.

Note that, the optically anisotropic body of the present disclosure was determined from the retardation at wavelengths from 400 nm to 800 nm measured by a Mueller Matrix Polarimeter Axoscan. It is preferable that the following $\alpha$-value and $\beta$-value are within a predetermined range. Specifically, the $\alpha$-value is preferably 0.70 to 0.99, and more preferably 0.75 to 0.90. Further, the $\beta$-value is preferably 1.00 to 1.25, and more preferably 1.01 to 1.20.

$$\alpha = (\text{retardation at 450 nm}) / (\text{retardation at 550 nm})$$

$$\beta = (\text{retardation at 650 nm}) / (\text{retardation at 550 nm})$$

(6) Polarizing plate and the like

The polarizing plate of the present disclosure includes the optically anisotropic body of the present disclosure and a polarizing film.

A specific example of the polarizing plate of the present disclosure is obtained by laminating the optically anisotropic body of the present disclosure on a polarizing film either directly or with other layer(s) (a glass plate, etc).

The method for producing the polarizing film is not specifically limited. Examples of methods for producing a PVA polarizing film include a method wherein iodine ions are adsorbed onto a PVA film followed by uniaxial stretching of the PVA film; a method wherein a PVA film is uniaxially stretched followed by adsorption of iodine ions; a method wherein adsorption of iodine ions to a PVA film and uniaxial stretching are simultaneously performed; a method wherein a PVA film is dyed with dichroic dye followed by uniaxial stretching; a method wherein a PVA film is uniaxially stretched followed by dying with dichroic dye; and a method wherein dying of a PVA film with dichroic dye and uniaxial stretching are simultaneously performed. Further, examples of methods of manufacturing a polyene polarizing film include known methods in the art such as a method wherein a PVA film is uniaxially stretched followed by heating and dehydration in the presence of a dehydration catalyst, and a method wherein a polyvinyl chloride film is uniaxially stretched followed by heating and dechlorination in the presence of a dechlorination catalyst.

In the polarizing plate of the present disclosure, the polarizing film and optically anisotropic body of the present disclosure may be bonded with an adhesive layer consisting of an adhesive (including tackifier). The average thickness of the adhesive layer is normally 0.01 to 30 $\mu$m, preferably 0.1 to 15 $\mu$m. The adhesive layer preferably has a tensile fracture strength of 40 MPa or less as measured in accordance with JIS K7113.

Examples of adhesives for the adhesive layer include acrylic adhesives, urethane adhesives, polyester adhesives, polyvinyl alcohol adhesives, polyolefin adhesives, modified polyolefin adhesives, polyvinyl alkyl ether adhesives, rubber adhesives, vinyl chloride-vinyl acetate adhesives, styrene-butadiene-styrene copolymer (SBS copolymer) adhesives and their hydrogenated product (SEBS copolymer) adhesives, ethylene adhesives such as ethylene-vinyl acetate copolymers and ethylene-styrene copolymers, and acrylate adhesives such as ethylene-methyl methacrylate copolymer, ethylene-methyl acrylate copolymer, ethylene-ethyl methacrylate copolymer and ethylene-ethyl acrylate copolymer.

The polarizing plate of the present disclosure uses the optically anisotropic body of the present disclosure, and thus, has a reverse wavelength dispersion while having an excellent in-plane uniformity in optical properties.

Further, a display device having a panel and an antireflection film can be preferably produced using the polarizing plate of the present disclosure. Examples of the panel include a liquid crystal panel, and an organic electroluminescence panel. Examples of the display device include a flat panel display device having a polarizing plate and a liquid crystal panel, and an organic electroluminescence display device having a liquid crystal panel and an organic electroluminescence panel.

(7) Compound

The compound of the present disclosure is used as a production intermediate of the aforementioned polymerizable compound (I). An example of this type of compound includes the compound (referred to as "compound (IV)")

represented by the following formula (IV).

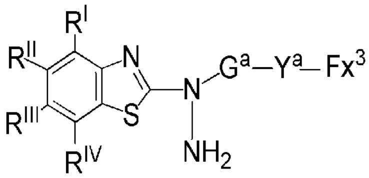

( I V )

In the formula (IV), $R^I$ to $R^{IV}$, $G^a$ and $Y^a$ are the same as defined above. $Fx^3$ is a hydrogen atom, or, an organic group having at least one aromatic hydrocarbon ring or aromatic heterocyclic ring. Examples of the "organic group having at least one aromatic hydrocarbon ring or aromatic heterocyclic ring" of $Fx^3$ include the same examples as the organic groups of the aforementioned $Fx^1$ and $Fx^2$.

Furthermore, the following are included as the preferred combinations.

$Y^a$ represents a chemical single bond, -O-, -C(=O)-, -C(=O)-O-, -O-C(=O)-, -O-C(=O)-O-, -C(=O)-S-, -S-C(=O)-, $-NR^{11}-C(=O)-$, $-C(=O)-NR^{11}-$, $-O-C(=O)-NR^{11}-$, $-NR^{11}-C(=O)-O-$, -S-, -N=N-, or -C=C-, where $R^{11}$ represents a hydrogen atom or an alkyl group having 1 to 6 carbon atoms, and,

$G^a$ represents an organic group which is either an alkylene group having 1 to 18 carbon atoms (preferably 3 to 18 carbon atoms) which may have a substituent, or an alkylene group having 3 to 18 carbon atoms in which at least one $-CH_2-$ contained in the alkylene group is substituted with -O-, -S-, -O-C(=O)-, -C(=O)-O-, or -C(=O)-, with the proviso that cases where there are two or more contiguous -O- or -S- are excluded, where both ends of Ga are preferably -CH2- (both ends of Ga are unsubstituted), and further, -C(=O)-preferably does not substitute consecutive $-CH_2-$ in Ga (i.e., the -C(=O)-C(=O)- configuration is not formed).

Furthermore, $G^a$ is preferably an unsubstituted alkylene group having 4 to 16 carbon atoms, more preferably an unsubstituted alkylene group having 5 to 14 carbon atoms, particularly preferably an unsubstituted alkylene group having 6 to 12 carbon atoms, and most preferably an unsubstituted alkylene group having 6 to 10 carbon atoms.

Furthermore, the following are included as more preferable combinations.

$Y^a$ represents a chemical single bond, -O-, -C(=O)-, -C(=O)-O-, -O-C(=O)-, -O-C(=O)-O-, $-O-C(=O)-NR^{11}-$, $-NR^{11}-C(=O)-O-$, or, -S-, $R^{11}$ represents a hydrogen atom or an alkyl group having 1 to 6 carbon atoms, and, $G^a$ is preferably an alkylene group having 1 to 18 carbon atoms which may have a substituent, and more preferably 3 to 18 carbon atoms.

$Fx^3$ is a hydrogen atom, or an organic group having 2 to 30 carbon atoms having at least an aromatic hydrocarbon ring and an aromatic heterocyclic ring, and

when $Fx^3$ has a ring structure, the number of $\pi$ electrons included in the ring structure in $Fx^3$ is 4 or more, preferably 6 or more, more preferably 8 or more, and 10 or more is particularly preferable. When $Fx^3$ is a hydrogen atom, Ya is preferably -O-.

Examples of the "organic group having 2 to 30 carbon atoms having at least an aromatic hydrocarbon ring and an aromatic heterocyclic ring" of $Fx^3$ include the same compounds as the organic group having 2 to 30 carbon atoms of the aforementioned $Fx^1$ and $Fx^2$.

In this case, the preferred examples of $Fx^3$ are the same as the preferred examples of the $Fx^1$ and $Fx^2$.

The compound (IV) is preferably represented by any of the following formulas (A) to (O):

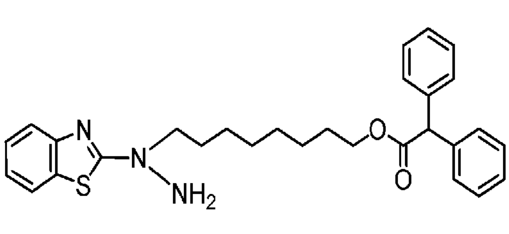

(A)

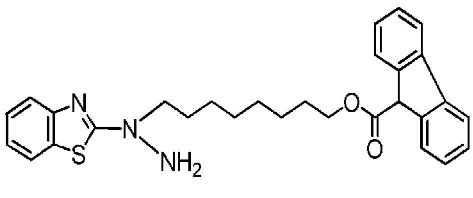

(B)

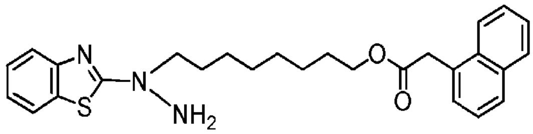

(C)

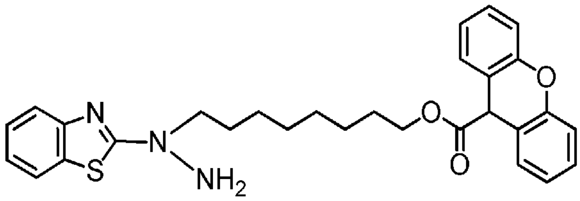

(D)

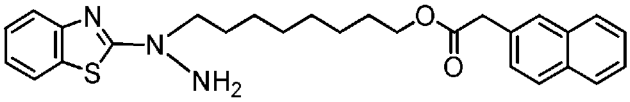

(E)

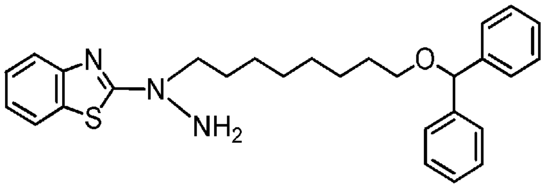

(F)

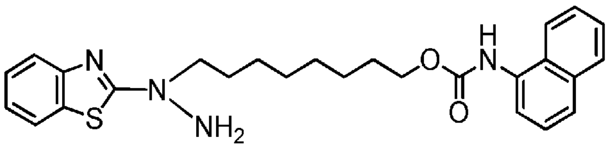

(G)

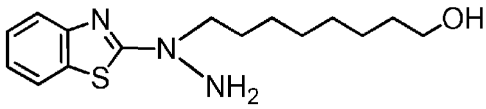

(H)

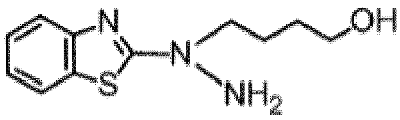

(I)

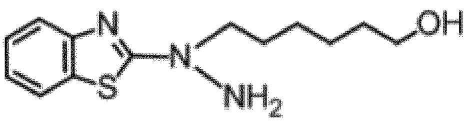

(J)

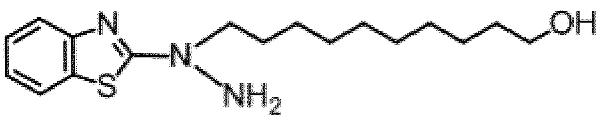

(K)

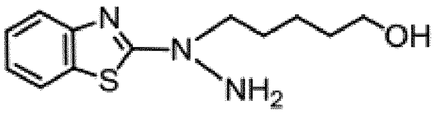

(L)

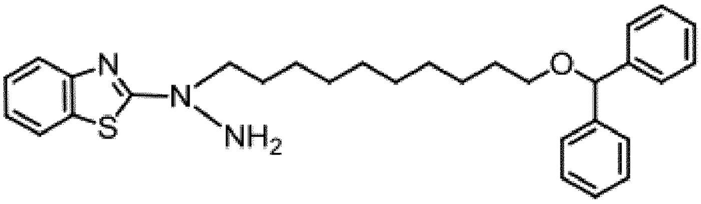

(M)

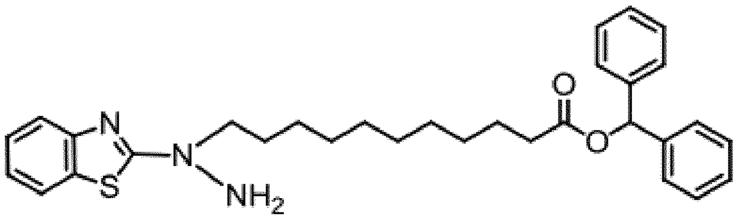

(N)

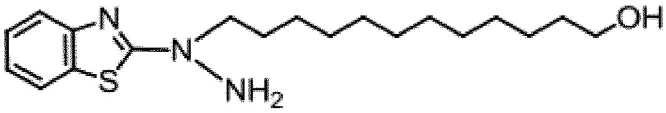

(O)

The compound represented by the aforementioned formula (IV) can be used to produce the polymerizable compound.

For example, the compound can be used to produce the polymerizable compound (I) of the present disclosure.

Examples of the method for producing the polymerizable compound (I) of the present disclosure include a method which can react the $NH_2$ portion in the compound represented by the aforementioned formula (IV) with the -C(=O)Q portion in the following formulas (V-1) and (V-2) by a known synthesis reaction.

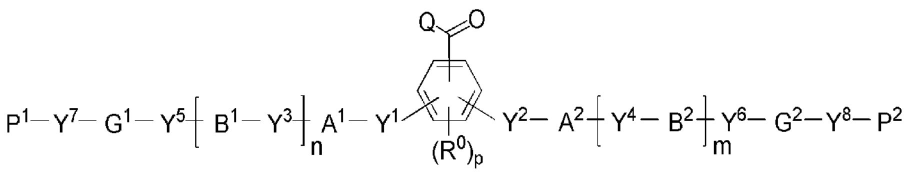

(V-1)

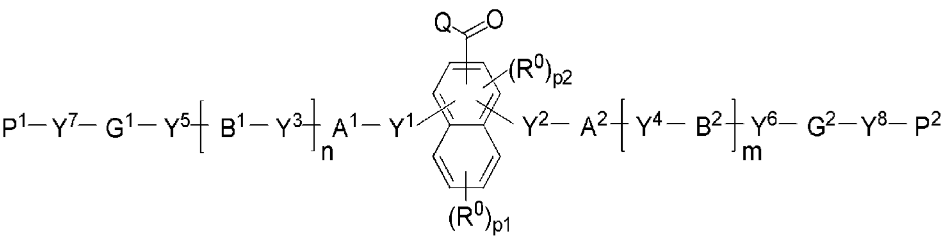

(V-2)

Here, in the formulas (V-1) and (V-2), Q, $Y^1$ to $Y^8$, $A^1$, $A^2$, $B^1$, $B^2$, $G^1$, $G^2$, $P^1$, $P^2$ n, m, $R^0$, p, p1 and p2 are the same as defined above, and the preferred examples are also the same as stated above.

However, when a plurality of $R^0$ are present, these may be the same or different.

Note that, the compounds represented by formulas (V-1) and (V-2) may be synthesized by a combination of known synthesis reactions. That is, the compounds represented by formulas (V-1) and (V-2) may be synthesized by referring to the methods described in various literatures (e.g., WO2012/141245, WO2012/147904, WO2014/010325, WO2013/046781, WO2014/061709, WO2014/126113, WO2015/064698, WO2015-140302, WO2015/129654, WO2015/141784, WO2016/159193, WO2012/169424, WO2012/176679 and WO2015/122385.

The compound represented by formula (V-3) or (V-4) can also be used to produce the polymerizable compound.

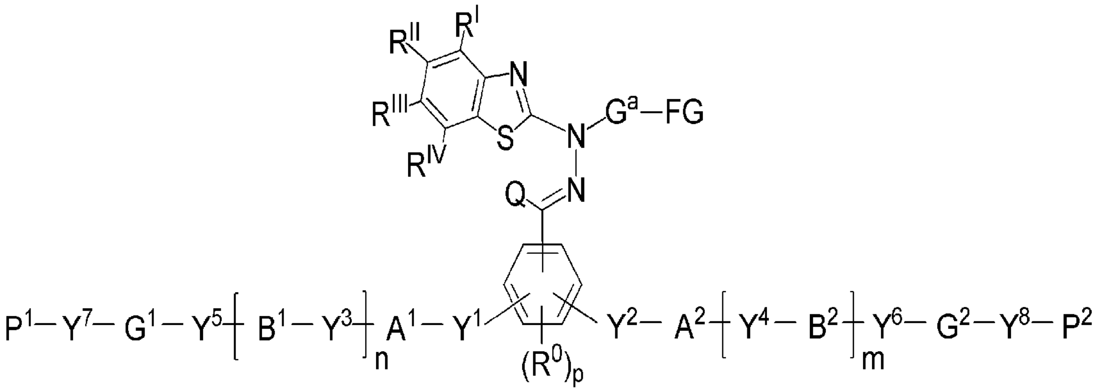

(V-3)

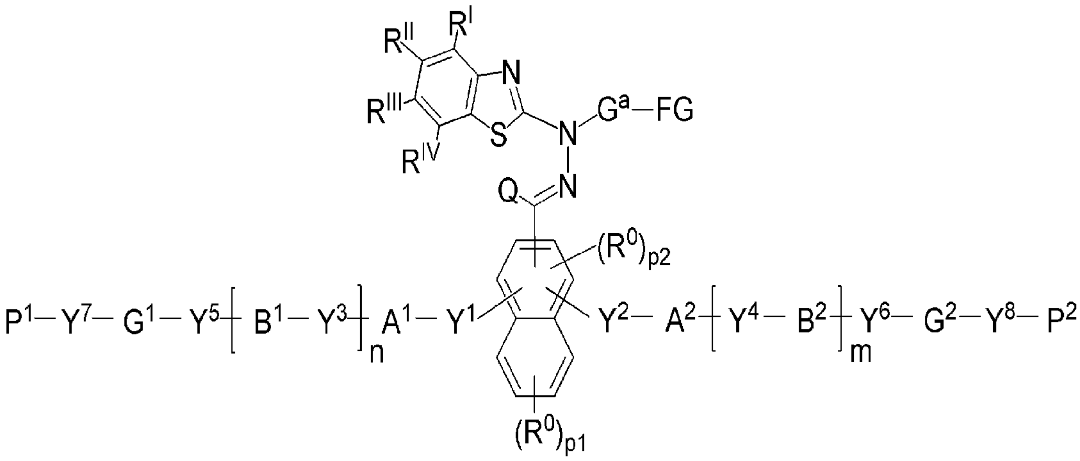

(V-4)

where in the formulas (V-3) and (V-4),
$Y^1$ to $Y^8$, $A^1$, $A^2$, $B^1$, $B^2$, $G^1$, $G^2$, $G^a$, $P^1$, $P^2$, $R^I$ to $R^{IV}$, Q, $R^0$, n, m, p, p1, and p2 are the same as defined above, and the preferred examples are the same. FG represents -OH, -C(=O)-OH, -SH, or NR*R**. Here, R* and R** each independently represent a hydrogen atom or an alkyl group having 1 to 6 carbon atoms (with the proviso that R* and R** are not simultaneously an alkyl group having 1 to 6 carbon atoms).

The combination of $G^a$ and FG is preferably

(I) a combination in which $G^a$ is an organic group which is either an alkylene group having 1 to 18 carbon atoms (preferably 3 to 18 carbon atoms), or an alkylene group having 3 to 18 carbon atoms in which at least one $-CH_2-$ contained in the alkylene group is substituted with -O-, -S-, -O-C(=O)-, -C(=O)-O-, or -C(=O)- (with the proviso that cases where there are two or more contiguous -O- or -S- are excluded), and
FG is -OH or C(=O)-OH,
more preferably (II) a combination in which $G^a$ is an organic group which is either an alkylene group having 1 to 18 carbon atoms (preferably 3 to 18 carbon atoms), or an alkylene group having 3 to 18 carbon atoms in which at least one $-CH_2-$ contained in the alkylene group is substituted with -O-, -C(=O)-, or -S- (with the proviso that cases where there are two or more contiguous -O- or -S- are excluded), and
FG is -OH or C(=O)-OH, and
particularly preferably (III) a combination in which $G^a$ is an alkylene group having 1 to 18 carbon atoms (preferably 3 to 18 carbon atoms), and
FG is -OH or -C(=O)-OH.

The compound represented by the formula (V-3) or (V-4) is preferably any of the following formulas (a) to (g).

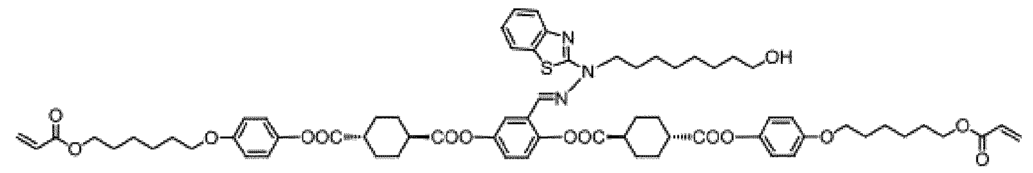
(a)

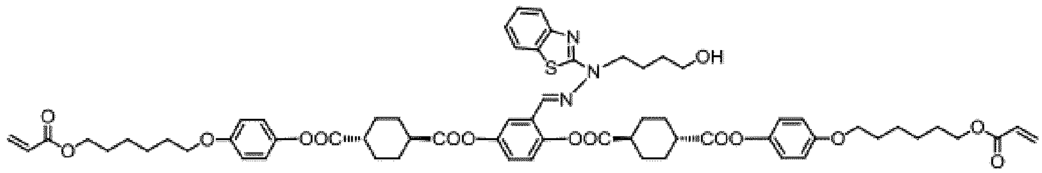
(b)

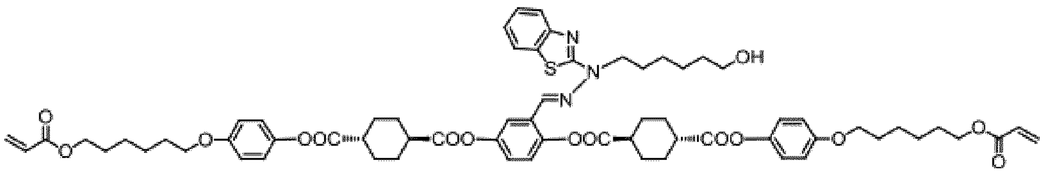
(c)

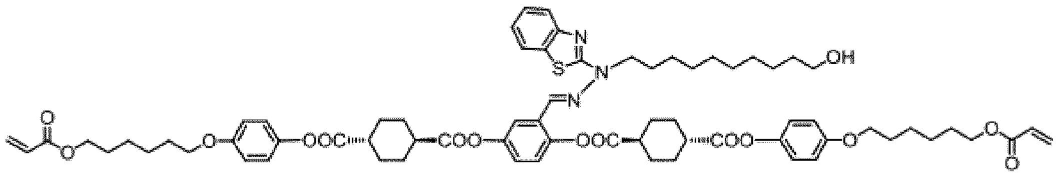
(d)

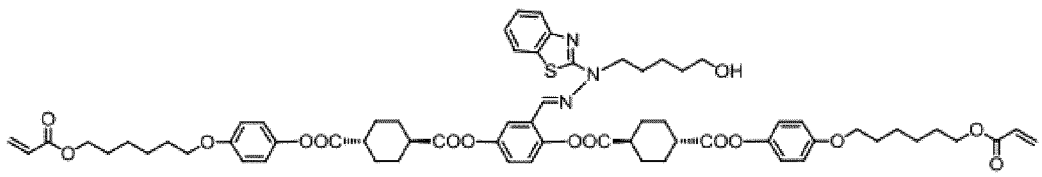
(e)

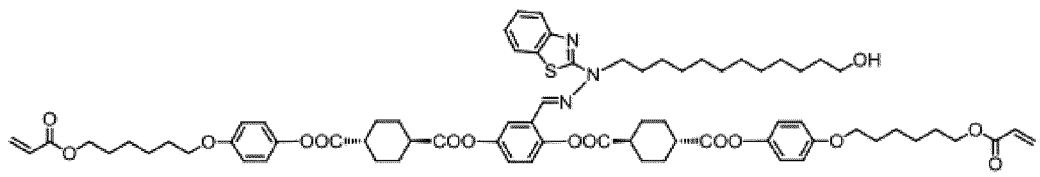
(f)

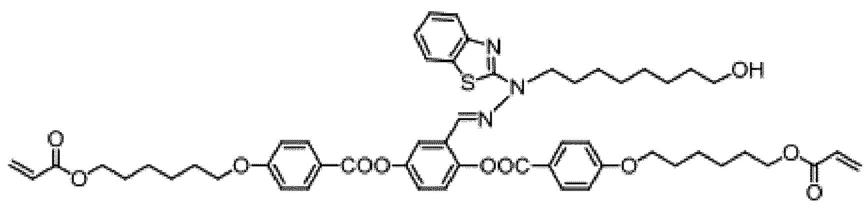
(g)

Examples of the compound which is useful in the production of the formula (IV) which is the production intermediate of the aforementioned polymerizable compound (I) include the compound (referred to as "compound (VI)") represented by the following formula (VI):

$$\text{Hal-G}^a\text{-Y}^a\text{-Fx}^a \qquad \text{(VI)}$$

where $G^a$ and $Y^a$ are the same as defined above, and $Fx^a$ is an organic group having at least one aromatic hydrocarbon ring or aromatic heterocyclic ring. The "organic group having at least one aromatic hydrocarbon ring or aromatic heterocyclic ring" of $Fx^a$ is the same as the organic groups of the aforementioned $Fx^1$ and $Fx^2$. Hal represents a halogen atom.

Furthermore, the following are included as the preferred combinations.

$Y^a$ represents a chemical single bond, -O-, -C(=O)-, -C(=O)-O-, -O-C(=O)-, -O-C(=O)-O-, -C(=O)-S-, -S-C(=O)-, $-NR^{11}-C(=O)-$, $-C(=O)-NR^{11}-$, $-O-C(=O)-NR^{11}-$, $-NR^{11}-C(=O)-O-$, -S-, -N=N-, or -C≡C-, and $R^{11}$ represents a hydrogen atom or an alkyl group having 1 to 6 carbon atoms, and,

$G^a$ represents an organic group which is either an alkylene group having 1 to 18 carbon atoms (preferably 3 to 18 carbon atoms) which may have a substituent, or an alkylene group having 3 to 18 carbon atoms in which at least one $-CH_2-$ contained in the alkylene group is substituted with -O-, -S-, -O-C(=O)-, -C(=O)-O-, or -C(=O)-, with the proviso that cases where there are two or more contiguous -O- or -S- are excluded, where both ends of $G^a$ are preferably $-CH_2-$ (both ends of Ga are unsubstituted), and further, -C(=O)-preferably does not substitute consecutive $-CH_2-$ in $G^a$ (i.e., the -C(=O)-C(=O)- configuration is not formed).

Furthermore, $G^a$ is preferably an unsubstituted alkylene group having 4 to 16 carbon atoms, more preferably an unsubstituted alkylene group having 5 to 14 carbon atoms, particularly preferably an unsubstituted alkylene group having 6 to 12 carbon atoms, and most preferably an unsubstituted alkylene group having 6 to 10 carbon atoms.

Furthermore, the following are included as more preferred combinations.

$Y^a$ represents a chemical single bond, -O-, -C(=O)-, -C(=O)-O-, -O-C(=O)-, -O-C(=O)-O-, $-O-C(=O)-NR^{11}-$, $-NR^{11}-C(=O)-O-$, or, -S-, and $R^{11}$ represents a hydrogen atom or an alkyl group having 1 to 6 carbon atoms, and, $G^a$ is preferably an alkylene group having 1 to 18 carbon atoms which may have a substituent, and more preferably 3 to 18 carbon atoms.

$Fx^a$ is an organic group having 2 to 30 carbon atoms having at least an aromatic hydrocarbon ring and an aromatic heterocyclic ring, and

when $Fx^a$ is a ring structure, the number of $\pi$ electrons included in the ring structure in $Fx^a$ is 4 or more, preferably 6 or more, more preferably 8 or more, and 10 or more is particularly preferable.

Examples of the "organic group having 2 to 30 carbon atoms having at least an aromatic hydrocarbon ring and an aromatic heterocyclic ring" of $Fx^a$ include the same compounds as the organic group having 2 to 30 carbon atoms of the aforementioned $Fx^1$ and $Fx^2$.

In this case, the preferred examples of $Fx^a$ are the same as the preferred examples of the $Fx^1$ and $Fx^2$.

The compound (VI) is preferably represented by any of the following general formulas (VI-1) to (VI-6):

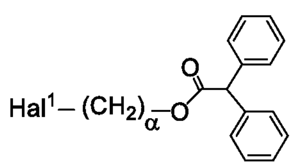
(VI-1)
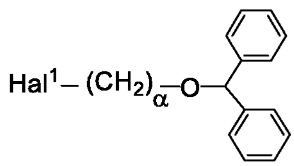
(VI-2)

where $\alpha$ represents an integer from 4 to 16, and $Hal^1$ represents a halogen atom.

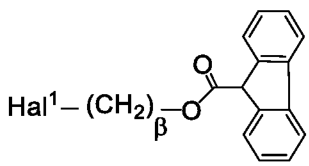
(VI-3)
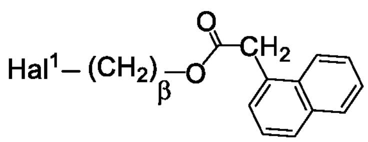
(VI-4)

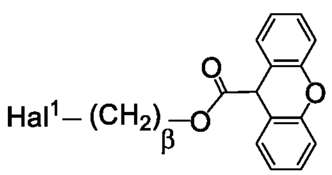
(VI-5)
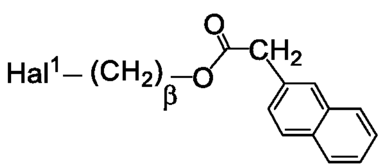
(VI-6)

where $\beta$ represents an integer from 4 to 16, and $Hal^1$ represents a halogen atom.

Furthermore, $\alpha$ is preferably an integer from 6 to 12, and an integer from 7 to 12 is particularly preferable. Further, $\beta$ is preferably an integer from 6 to 12, and an integer from 6 to 10 is particularly preferable. Furthermore, $Hal^1$ is preferably a chlorine atom or a bromine atom.

The compounds represented by the aforementioned formula (VI-1) to (VI-6) can be used in the production of the formula (IV). Examples of the method for producing formula (IV) include the method for reacting with the following

formula (VII) in the presence of a base, as described in WO2015/129654.

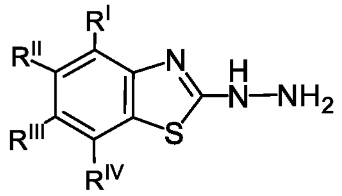 (VII)

where in the formula, $R^I$ to $R^{IV}$ are the same as defined above.

EXAMPLES

The present disclosure will be further described in detail by way of examples. However, the present disclosure is not limited to the following examples.

(Synthesis Example 1) Synthesis of Polymerizable compound 1 (example of a compound represented by formula (III-1))

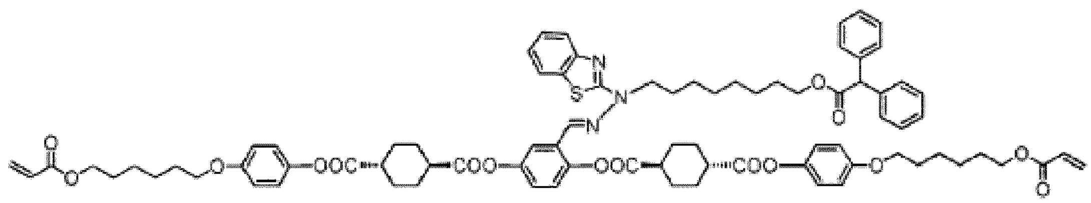

・・・Polymerizable compound 1

<Step 1: Synthesis of Intermediate A>

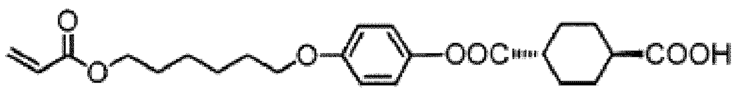 ・・・Intermediate A

83.05 g (0.40 mol) of trans-1,4-cyclohexanedicarboxylic acid dichloride was added to 600 g of cyclopentyl methyl ether in a three-necked reactor equipped with a thermometer under a nitrogen stream, and cooled to 5°C in an ice bath. 100 g (0.38 mol) of 4-(6-acryloyloxy-hex-1-yloxy)phenol (manufactured by DKSH Management Ltd.), 1.67 g of 2,6-di-tert-butyl-4-methylphenol, and, 230 g of tetrahydrofuran (THF) were added to the solution. While vigorously stirring, 40.2 g (0.40 mol) of triethylamine was gradually dropped therein. After the dropwise addition, the reaction was performed at 5°C for 1 hour. After completion of the reaction, 250 g of water was added, the temperature was raised to 50°C and stirred for 4 hours. Then, after adding 416 g of a 1 mol/L concentration of acetic acid / sodium acetate buffer solution to the organic layer obtained by extracting the water layer and stirring for 30 minutes, the water layer was extracted. Furthermore, the organic layer was collected after washing with 250 g of water, and dried with anhydrous sodium sulfate, and the sodium sulfate was filtered off. After the solvent was evaporated from the filtrate using a rotary evaporator, the obtained residue was purified by silica gel column chromatography (THF:toluene = 1:9 (volume ratio)) to obtain 75 g of Intermediate A as a white solid. The yield was 47.4 mol%. The structure of Intermediate A was identified by $^1H$-NMR. The $^1H$-NMR spectral data is shown below.

$^1H$-NMR (500 MHz, DMSO-$d_6$, TMS, δppm): 12.12 (s,1H), 6.99 (d,2H, J = 9.0 Hz), 6.92 (d,2H, J = 9.0 Hz), 6.32 (dd,1H, J = 1.5 Hz,17.5 Hz), 6.17 (dd, 1H, J = 10.0 Hz,17.5 Hz), 5.93 (dd,1H, J = 1.5 Hz, 10.0 Hz), 4.11 (t,2H, J = 6.5 Hz), 3.94 (t,2H, J = 6.5 Hz), 2.48-2.56 (m,1H), 2.18-2.26 (m,1H), 2.04-2.10 (m,2H), 1.93-2.00 (m,2H), 1.59-1.75 (m,4H), 1.35-1.52 (m,8H).

<Step 2: Synthesis of Intermediate B (Example of a compound represented by formula (V-1))>

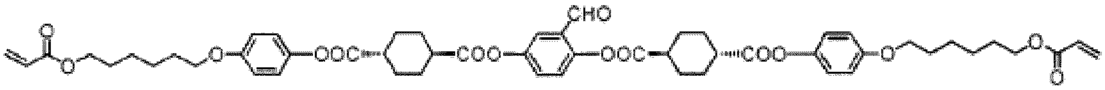

· · · Intermediate B

10.00 g (23.90 mmol) of Intermediate A synthesized in Step 1, 1.32 g (9.56 mmol) of 2,5-dihydroxybenzaldehyde, and 234 mg (1.92 mmol) of 4-(dimethylamino)pyridine was added to 80 ml of chloroform in a three-necked reactor equipped with a thermometer under a nitrogen stream. 3.2 g (25.36 mmol) of N-N'-diisopropylcarbodiimide was gradually dropped therein at room temperature. After the dropwise addition, the solution was stirred at 23°C for 3 hours. After completion of the reaction, the reaction solution was directly purified by a silica gel column chromatography (gradient from only chloroform to chloroform:THF = 9:1 (volume ratio)) to obtain 6.80 g of Intermediate B as a white solid. The yield was 75.7 mol%. The structure of Intermediate B was identified by $^1$H-NMR. The $^1$H-NMR spectral data is shown below.

$^1$H-NMR (500 MHz,DMSO-$d_6$, TMS, δppm): 10.02 (s, 1H), 7.67 (d,1H, J = 3.0 Hz), 7.55 (dd, 1H, J = 3.0 Hz,8.5 Hz), 7.38 (d,1H, J = 8.5 Hz), 6.99-7.04 (m,4H), 6.91-6.96 (m,4H), 6.32 (dd,2H, J = 1.5 Hz,17.5 Hz), 6.17 (dd,2H, J = 10.0 Hz,17.5 Hz), 5.93 (dd,2H, J = 1.5 Hz,10.0 Hz), 4.11 (t,4H, J = 6.5 Hz), 3.95 (t,4H, J = 6.5 Hz), 2.56-2.81 (m,4H), 2.10-2.26 (m,8H), 1.50-1.76 (m,16H), 1.33-1.49 (m,8H).

<Step 3: Synthesis of Intermediate C>

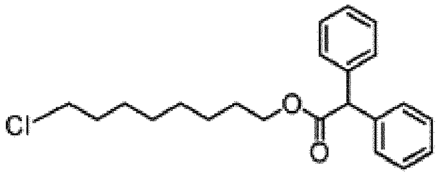

· · · Intermediate C

11.60 g (54.65 mmol) of diphenyl acetic acid and 75 ml of N-methyl-2-pyrrolidone were charged in a three-necked reactor equipped with a thermometer, under a nitrogen stream to prepare a uniform solution. 7.50 g (45.55 mmol) of 8-chloro-1-n-octanol was added thereto. Next, 1.33 g (10.89 mmol) of N-N-dimethyl-4-aminopyridine was added. Next, after 12.57 g (65.59 mmol) of 1-ethyl-3-(3-dimethylaminopropyl)carbodiimide hydrochloride was added to the reaction solution over 5 minutes while maintaining the temperature of the reaction solution at 20 to 30°C, the solution was further stirred at 25°C for 4 hours. After completion of the reaction, 250 ml of saturated saline solution was added to the reaction solution, followed by extraction twice with 250 ml of ethyl acetate. The organic layer was collected, dried with anhydrous sodium sulfate, and the sodium sulfate was filtered off. After the solvent was evaporated from the filtrate using a rotary evaporator, the obtained residue was purified by silica gel column chromatography (ethyl acetate:hexane = 1:20 (volume ratio)) to obtain 15.94 g of Intermediate C as a colorless oil. The yield was 97.5 mol%. The structure of Intermediate C was identified by $^1$H-NMR. The $^1$H-NMR spectral data is shown below.

$^1$H-NMR (500 MHz, $CDCl_3$, TMS, δppm): 7.32-7.30 (m,10H), 5.01 (s, 1H), 4.14 (t,2H, J = 6.5 Hz), 3.50 (t,2H, J = 6.5 Hz), 1.73 (tt,7H, J = 7.0 Hz, 7.0 Hz), 1.63-1.58 (m,2H), 1.34-1.40 (m,2H), 1.23-1.27 (m,6H).

<Step 4: Synthesis of Intermediate D (Example of a compound represented by formula (IV))>

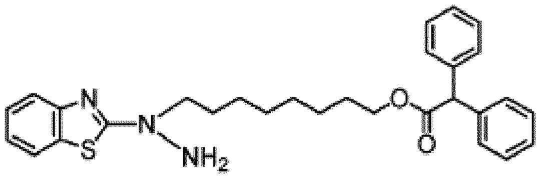

· · · Intermediate D

6.00 g (36.32 mmol) of 2-hydrazinobenzothiazole was dissolved in 60.0 ml of N-N-dimethylformamide in a three-necked reactor equipped with a thermometer under a nitrogen stream. 23.67 g (72.63 mmol) of cesium carbonate and 15.64 g (43.58 mmol) of Intermediate C synthesized in the Step 3 were added to the solution, and the solution was stirred at 25°C for 14 hours. After completion of the reaction, the reaction solution was charged with 250 ml of distilled water, followed by extraction twice with 250 ml of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 80:20 (volume ratio)) to obtain 9.90 g of Intermediate D as a gray solid. The yield was 55.9 mol%. The structure of Intermediate D was identified by $^1$H-NMR. The $^1$H-NMR spectral data is shown below.

$^1$H-NMR (500 MHz, $CDCl_3$, TMS, δppm): 7.59-7.57 (m, 1H), 7.53-7.51 (m, 1H), 7.31-7.30 (m,11H), 7.04 (ddd,1H, J = 1.0 Hz, 8.0 Hz, 8.0 Hz), 5.01 (s, 1H), 4.19 (br,2H), 4.12 (t,2H, J = 6.5 Hz), 3.70 (t,2H,7.5 Hz), 1.68-1.57 (m,4H), 1.33-1.21 (m,8H).

<Step 5: Synthesis of Polymerizable compound 1 (Example of a compound represented by formula (III-1))>

4.36 g (8.95 mmol) of Intermediate D synthesized in the Step 4, and, 6.00 g (6.39 mmol) of Intermediate B synthesized in Step 2 were dissolved in 12.0 mL of ethanol and 120 mL of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 mL of distilled water 200 mL was charged into the reaction solution, followed with extraction twice with 200 ml of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 7.76 g of polymerizable compound 1 as a yellow solid. The yield was 86.2 mol%. The structure of the target product (polymerizable compound 1) was identified by $^1$H-NMR. The $^1$H-NMR spectral data is shown below.

$^1$H-NMR (500 MHz, $CDCl_3$, TMS, δppm): 7.75 (dd,1H, J = 1.0 Hz, 2.0 Hz), 7.70-7.66 (m,3H), 7.35-7.21 (m,11H), 7.17 (ddd,1H, J = 1.0 Hz, 8.0 Hz, 8.0 Hz), 7.11-7.12 (m,2H), 7.00-6.95 (m,4H), 6.90-6.85 (m,4H), 6.405 (dd, 1H, J = 1.5 Hz, 17.5 Hz), 6.402 (dd, 1H, J = 1.5 Hz, 17.5 Hz), 6.127 (dd, 1H, J = 10.5 Hz, 17.5 Hz), 6.125 (dd, 1H, J = 10.5 Hz, 17.5 Hz), 5.823 (dd, 1H, J = 1.5 Hz, 10.5 Hz), 5.821 (dd,1H, J = 1.5 Hz, 10.5 Hz), 4.99 (s, 1H), 4.30 (t,2H, J = 7.5 Hz), 4.18 (t,2H, J = 6.5 Hz), 4.17 (t,2H, J = 6.5 Hz), 4.10 (t,2H, J = 6.5 Hz), 3.95 (t,2H, J = 6.5 Hz), 3.93 (t,2H, J = 6.5 Hz), 2.70-2.56 (m,4H), 2.35-2.25 (m,8H), 1.83-1.24 (m,36H).

(Synthesis Example 2) Synthesis of Polymerizable compound 2 (Another example of a compound represented by formula (III-1))

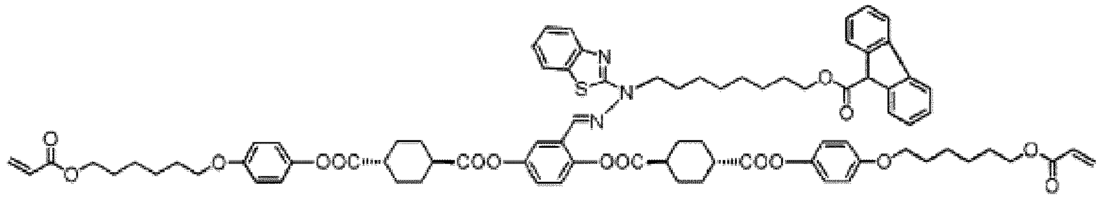

・・・Polymerizable compound 2

<Step 1: Synthesis of Intermediate E>

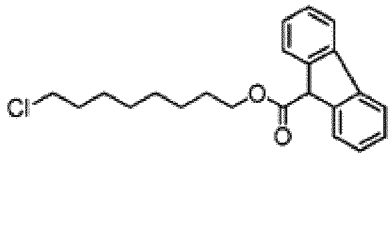

・・・Intermediate E

11.49 g (54.65 mmol) of 9-fluorenecarboxylic acid and 75 mL of N-methyl-2-pyrrolidone were charged in a three-necked reactor equipped with a thermometer, under a nitrogen stream to prepare a uniform solution. 7.50 g (45.55 mmol) of 8-chloro-1-n-octanol was added thereto. Next, 1.33 g (10.89 mmol) of N-N-dimethyl-4-aminopyridine was added. Next, after 12.57 g (65.59 mmol) of 1-ethyl-3-(3-dimethylaminopropyl)carbodiimide hydrochloride was added to the reaction solution over 5 minutes while maintaining the temperature of the reaction solution at 20 to 30°C, the solution was further stirred at 25°C for 4 hours. After completion of the reaction, 250 ml of saturated saline solution was added to the reaction solution, followed by extraction twice with 250 mL of ethyl acetate. The organic layer was collected, dried with anhydrous sodium sulfate, and the sodium sulfate was filtered off. After the solvent was evaporated from the filtrate using a rotary evaporator, the obtained residue was purified by silica gel column chromatography (ethyl acetate:hexane = 1:20 (volume ratio)) to obtain 14.22 g of Intermediate E as a yellow oil. The yield was 87.5 mol%. The structure of Intermediate E was identified by $^1$H-NMR. The $^1$H-NMR spectral data is shown below.

$^1$H-NMR (500 MHz, $CDCl_3$, TMS, δppm): 7.64 (d,2H, J = 7.5 Hz), 7.61 (dd,2H, J = 0.5 Hz, 7.5 Hz), 7.32 (dd,2H,7.5 Hz,7.5 Hz), 7.26 (ddd,2H, J = 1.5 Hz,7.5 Hz,7.5 Hz), 4.77 (s, 1H), 4.06 (t,2H,6.5 Hz), 3.41 (t,2H, J = 6.5 Hz), 1.64 (tt,2H, J = 7.5 Hz, 7.5 Hz), 1.55-1.50 (m,2H), 1.30-1.24 (m,2H), 1.24-1.12 (m,6H).

<Step 2: Synthesis of Intermediate F (Another example of a compound represented by formula (IV))>

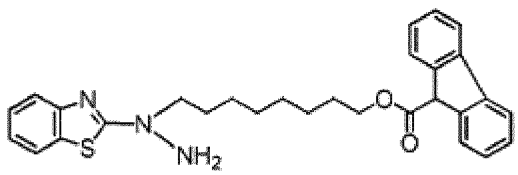

• • • Intermediate F

6.00 g (36.32 mmol) of 2-hydrazinobenzothiazole was dissolved in 60.0 mL of N-N-dimethylformamide in a three-necked reactor equipped with a thermometer under a nitrogen stream. 23.67 g (72.63 mmol) of cesium carbonate and 15.55 g (43.58 mmol) of Intermediate E synthesized in Step 1 were added to the solution, and the solution was stirred at 25°C for 14 hours. After completion of the reaction, the reaction solution was charged with 250 ml of distilled water, followed by extraction twice with 250 mL of ethyl acetate. After the ethyl acetate layer was dried with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 80:20 (volume ratio)) to obtain 10.79 g of Intermediate F as a yellow oil. The yield was 61.2 mol%. The structure of Intermediate F was identified by $^1$H-NMR. The $^1$H-NMR spectral data is shown below.

$^1$H-NMR (500 MHz, $CDCl_3$, TMS, δppm): 7.74 (d,2H, J = 7.5 Hz), 7.65 (dd, 1H, J = 0.5 Hz,7.5 Hz), 7.59 (dd, 1H, J = 0.5 Hz,7.5 Hz), 7.53 (d, 1H, J = 7.5 Hz), 7.41 (dd,2H, J = 7.5 Hz,7.5 Hz), 7.33 (ddd,2H, J = 1.5 Hz,7.5 Hz,7.5 Hz), 7.28-7.25 (m,2H), 7.05 (ddd,1H, J = 1.5 Hz,7.5 Hz,7.5 Hz), 4.85 (s, 1H), 4.20 (br,2H), 4.14 (t,2H, J = 6.5 Hz), 3.74 (t,2H, J = 6.5 Hz), 1.84 (tt,2H, J = 6.5 Hz,6.5 Hz), 1.72-1.59 (m,4H), 1.36-1.26 (m,6H).

<Step 3: Synthesis of Polymerizable compound 2 (Another example of a compound represented by formula (III-1))>

4.36 g (8.95 mmol) of Intermediate F synthesized in Step 2, and, 6.00 g (6.39 mmol) of Intermediate B synthesized in the Synthesis Step 2 of Synthesis Example 1 were dissolved in 12.0 ml of ethanol and 120 ml of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 mL of distilled water was charged into the reaction solution, followed with extraction twice with 200 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 7.55 g of Polymerizable compound 2 as a yellow solid. The yield was 84.0 mol%. The structure of the target product (polymerizable compound 2) was identified by $^1$H-NMR. The $^1$H-NMR spectral data is shown below.

$^1$H-NMR (500 MHz, $CDCl_3$, TMS, δppm): 7.75-7.73 (m,3H), 7.69 (ddd,2H, J = 1.0 Hz,7.5 Hz,7.5 Hz), 7.63 (dd,2H, J = 0.5 Hz,7.5 Hz), 7.40 (dd,2H, J = 7.5 Hz,7.5 Hz), 7.35-7.30 (m,4H), 7.17 (ddd,1H, J = 1.0 Hz,7.5 Hz,7.5 Hz), 7.11-7.10 (m,2H), 6.99 (d,2H, J = 9.0 Hz), 6.95 (d,2H, J = 9.0 Hz), 6.88 (d,2H, J = 9.0 Hz), 6.85 (d,2H, J = 9.0 Hz), 6.405 (dd,1H, J = 1.5 Hz,17.5 Hz), 6.402 (dd, 1H, J = 1.5 Hz,17.5 Hz), 6.127 (dd, 1H, J = 10.5 Hz,17.5 Hz), 6.124 (dd, 1H, J = 10.5 Hz,17.5 Hz), 5.823 (dd, 1H, J = 1.5 Hz,10.5 Hz), 5.820 (dd, 1H, J = 1.5 Hz,10.5 Hz), 4.83 (s, 1H), 4.31 (t,2H, J

= 7.5 Hz), 4.176 (t,2H, J = 6.5 Hz), 4.170 (t,2H, J = 6.5 Hz), 4.102 (t,2H, J = 6.5 Hz), 3.947 (t,2H, J = 6.5 Hz), 3.912 (t,2H, J = 6.5 Hz), 2.70-2.54 (m,4H), 2.35-2.26 (m,8H), 1.81-1.28 (m,36H).

(Synthesis Example 3) Synthesis of Polymerizable compound 3 (Still another example of the compound represented by formula (III-1))

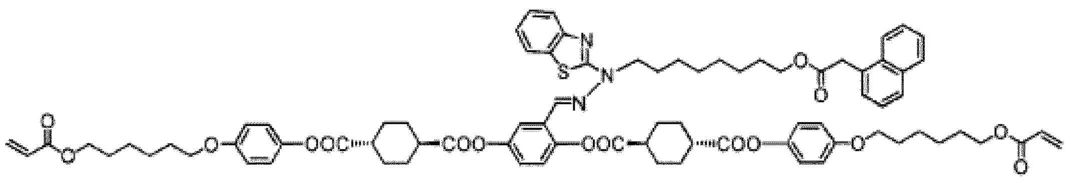

· · · Polymerizable compound 3

<Step 1: Synthesis of Intermediate G>

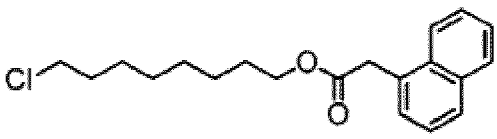

· · · Intermediate G

10.18 g (54.65 mmol) of 1-naphthylacetic acid and 75 mL of N-methyl-2-pyrrolidone were charged in a three-necked reactor equipped with a thermometer, under a nitrogen stream to prepare a uniform solution. 7.50 g (45.55 mmol) of 8-chloro-1-n-octanol was added thereto. Next, 1.33 g (10.89 mmol) of N-N-dimethyl-4-aminopyridine was added. Next, after 12.57 g (65.59 mmol) of 1-ethyl-3-(3-dimethylaminopropyl)carbodiimide hydrochloride was added to the reaction solution over 5 minutes while maintaining the temperature of the reaction solution at 20 to 30°C, the solution was further stirred at 25°C for 4 hours. After completion of the reaction, 250 mLof saturated saline solution was added to the reaction solution, followed by extraction twice with 250 mL of ethyl acetate. The organic layer was collected, dried with anhydrous sodium sulfate, and the sodium sulfate was filtered off. After the solvent was evaporated from the filtrate using a rotary evaporator, the obtained residue was purified by silica gel column chromatography (ethyl acetate:hexane = 1:20 (volume ratio)) to obtain 12.88 g of Intermediate G as a colorless oil. The yield was 85.0 mol%. The structure of the Intermediate G was identified by $^1$H-NMR. The $^1$H-NMR spectral data is shown below.

$^1$H-NMR (500 MHz, $CDCl_3$, TMS, δppm): 8.01 (dd, 1H, J = 1.0 Hz,8.5 Hz), 7.86 (dd, 1H, J = 1.0 Hz,8.5 Hz), 7.79 (dd, 1H, J = 1.5 Hz,7.5 Hz), 7.54-7.47 (m,2H), 7.45-7.40 (m,2H), 4.07 (t,2H, J = 6.5 Hz), 4.06 (s,2H), 3.51 (t,2H, J = 6.5 Hz), 1.73 (tt,2H, J = 7.5 Hz,7.5 Hz), 1.58-1.52 (m,2H), 1.38-1.32 (m,2H), 1.23-1.12 (m,6H).

<Step 2: Synthesis of Intermediate H (Still another example of the compound represented by formula (IV))>

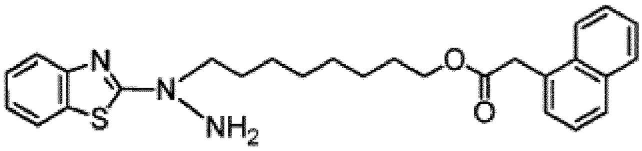

· · · Intermediate H

6.00 g (36.32 mmol) of 2-hydrazinobenzothiazole was dissolved in 60.0 ml of N-N-dimethylformamide in a three-necked reactor equipped with a thermometer under a nitrogen stream. 23.67 g (72.63 mmol) of cesium carbonate and 20.12 g (43.58 mmol) of Intermediate G synthesized in Step 1 were added to the solution, and the solution was stirred at 25°C for 14 hours. After completion of the reaction, the reaction solution was charged with 250 mL of distilled water, followed by extraction twice with 250 mL of ethyl acetate. After drying the ethyl acetate layer with anhydrous sodium sulfate, the sodium sulfate was filtered off. The organic layer was collected, dried with anhydrous sodium sulfate,

and the sodium sulfate was filtered off. After the solvent was evaporated from the filtrate using a rotary evaporator, the obtained residue was purified by silica gel column chromatography (hexane:THF = 80:20 (volume ratio)) to obtain 10.67 g of Intermediate H as a yellow oil. The yield was 63.6 mol%. The structure of Intermediate H was identified by $^1$H-NMR. The $^1$H-NMR spectral data is shown below.

$^1$H-NMR (500 MHz, $CDCl_3$, TMS, δppm): 8.00 (dd, 1H, J = 1.0 Hz,8.5 Hz), 7.86 (dd, 1H, J = 1.0 Hz,8.5 Hz), 7.79 (dd, 1H, J = 1.5 Hz,7.5 Hz), 7.61-7.59 (m, 1H), 7.54-7.39 (m,5H), 7.28 (dd,1H, J = 1.0 Hz,7.0 Hz), 7.06 (ddd,1H, J = 1.0 Hz,7.5 Hz,7.5 Hz), 4.20 (s,2H), 4.07 (t,2H, J = 6.5 Hz), 4.06 (s,2H), 3.71 (t,2H, J = 7.5 Hz), 1.69 (tt,2H, J = 7.5 Hz,7.5 Hz), 1.58-1.52 (m,2H), 1.34-1.14 (m,8H).

<Step 3: Synthesis of Polymerizable compound 3 (Still another example of the compound represented by formula (III-1))>

4.13 g (8.95 mmol) of Intermediate H synthesized in Step 2, and, 6.00 g (6.39 mmol) of Intermediate B synthesized in the Synthesis Step 2 of Synthesis Example 1 were dissolved in 12.0 mL of ethanol and 120 mL of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 mL of distilled water 200 mL was charged into the reaction solution, followed with extraction twice with 200 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 6.84 g of polymerizable compound 3 as a yellow solid. The yield was 77.4 mol%. The structure of the target product (polymerizable compound 3) was identified by $^1$H-NMR. The $^1$H-NMR spectral data is shown below.

$^1$H-NMR (500 MHz, $CDCl_3$, TMS, δppm): 7.98 (d, 1H, J = 8.5 Hz), 7.85 (dd, 1H, J = 1.5 Hz,8.5 Hz), 7.78 (d, 1H, J = 7.5 Hz), 7.75 (dd, 1H, J = 0.5 Hz,2.5 Hz), 7.70-7.67 (m,3H), 7.52-7.45 (m,2H), 7.43-7.38 (m,2H), 7.34 (ddd,1H, J = 1.0 Hz,7.5 Hz,7.5 Hz), 7.17 (ddd,1H, J = 1.0 Hz,7.5 Hz,7.5 Hz), 7.13-7.09 (m,2H), 7.00-6.94 (m,4H), 6.90-6.85 (m,4H), 6.405 (dd,1H, J = 1.5 Hz,17.5 Hz), 6.402 (dd, 1H, J = 1.5 Hz,17.5 Hz), 6.13 (dd, 1H, J = 10.5 Hz,17.5 Hz), 6.12 (dd, 1H, J = 10.5 Hz,17.5 Hz), 5.823 (dd, 1H, J = 1.5 Hz,10.5 Hz), 5.820 (dd, 1H, J = 1.5 Hz,10.5 Hz), 4.30 (t,2H, J = 7.5 Hz), 4.176 (t,2H, J = 6.5 Hz), 4.170 (t,2H, J = 6.5 Hz), 4.05-4.02 (m,4H), 3.95 (t,2H, J = 6.5 Hz), 3.92 (t,2H, J = 6.5 Hz), 2.70-2.55 (m,4H), 2.37-2.26 (m,8H), 1.83-1.18 (m,36H).

(Synthesis Example 4) Synthesis of Polymerizable compound 4 (Still another example of the compound represented by formula (III-1))

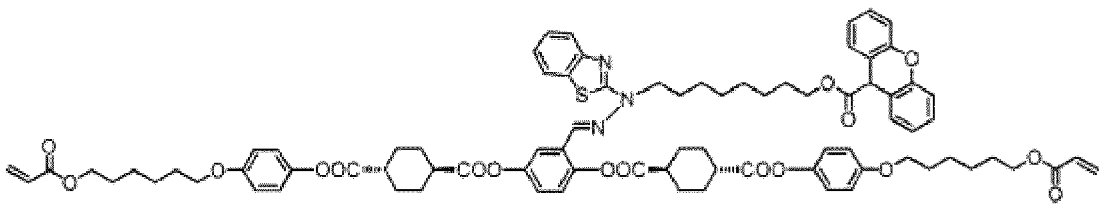

・・・Polymerizable compound 4

<Step 1: Synthesis of Intermediate I>

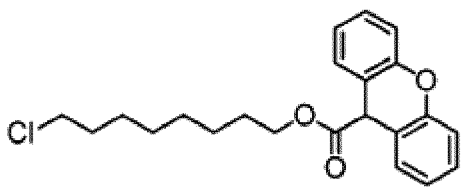

・・・Intermediate I

12.36 g (54.65 mmol) of xanthene-9-carboxylic acid and 75 mL of N-methyl-2-pyrrolidone were charged in a three-necked reactor equipped with a thermometer under a nitrogen stream to prepare a uniform solution. 7.50 g (45.55 mmol) of 8-chloro-1-n-octanol was added thereto. Next, 1.33 g (10.89 mmol) of N-N-dimethyl-4-aminopyridine was

added. Next, after 12.57 g (65.59 mmol) of 1-ethyl-3-(3-dimethylaminopropyl)carbodiimide hydrochloride was added to the reaction solution over 5 minutes while maintaining the temperature of the reaction solution at 20 to 30°C, the solution was further stirred at 25°C for 4 hours. After completion of the reaction, 250 mL of saturated saline solution was added to the reaction solution, followed by extraction twice with 250 mL of ethyl acetate. The organic layer was collected, dried with anhydrous sodium sulfate, and the sodium sulfate was filtered off. After the solvent was evaporated from the filtrate using a rotary evaporator, the obtained residue was purified by silica gel column chromatography (ethyl acetate:hexane = 1:20 (volume ratio)) to obtain 14.20 g of Intermediate I as a colorless oil. The yield was 83.6 mol%. The structure of Intermediate I was identified by $^1$H-NMR. The $^1$H-NMR spectral data is shown below.

$^1$H-NMR (500 MHz, $CDCl_3$, TMS, δppm): 7.30-7.25 (m,4H), 7.13 (dd,2H, J = 1.0 Hz,8.5 Hz), 7.07 (ddd, 2H, J = 1.0 Hz,7.5 Hz,7.5 Hz), 4.98 (s, 1H), 4.02 (t,2H, J = 6.5 Hz), 3.52 (t,2H, J = 6.5 Hz), 1.74 (tt,2H, J = 7.5 Hz,7.5 Hz), 1.49 (tt,2H, J = 7.5 Hz,7.5 Hz), 1.39-1.33 (m,2H), 1.23-1.10 (m,6H).

<Step 2: Synthesis of Intermediate J (Still another example of the compound represented by formula (IV))>

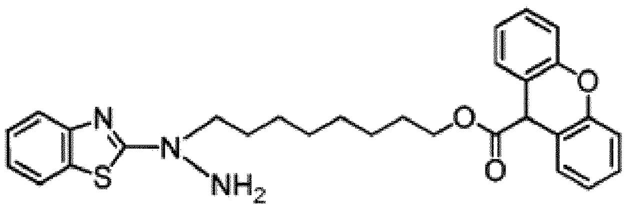

・・・Intermediate J

6.00 g (36.32 mmol) of 2-hydrazinobenzothiazole was dissolved in 60.0 mL of N-N-dimethylformamide in a three-necked reactor equipped with a thermometer under a nitrogen stream. 23.67 g (72.63 mmol) of cesium carbonate and 16.25 g (43.58 mmol) of Intermediate I synthesized in Step 1 were added to the solution, and the solution was stirred at 25°C for 14 hours. After completion of the reaction, the reaction solution was charged with 250 mL of distilled water, followed by extraction twice with 250 mL of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 80:20 (volume ratio)) to obtain 10.18 g of Intermediate J as a yellow oil. The yield was 55.9 mol%. The structure of Intermediate J was identified by $^1$H-NMR. The $^1$H-NMR spectral data is shown below.

$^1$H-NMR (500 MHz, $CDCl_3$, TMS, δppm): 8.01 (s, 1H), 7.60 (dd,1H, J = 1.0 Hz,7.5 Hz), 7.53 (dd,1H, J = 1.0 Hz,7.5 Hz), 7.30-7.25 (m,5H), 7.13 (dd,2H, J = 1.0 Hz,8.5 Hz), 7.09-7.06 (m,2H), 4.98 (s, 1H), 4.23 (s,2H), 4.02 (t,2H, J = 6.5 Hz), 3.73 (t,2H, J = 7.5 Hz), 1.70 (tt,2H, J = 7.5 Hz,7.5 Hz), 1.48 (tt,2H, J = 7.5 Hz,7.5 Hz), 1.35-1.09 (m,8H).

<Step 3: Synthesis of Polymerizable compound 4 (Still another example of the compound represented by formula (III-1))>

4.49 g (8.95 mmol) of Intermediate J synthesized in Step 2, and, 6.00 g (6.39 mmol) of Intermediate B synthesized in the Synthesis Step 2 of Example 1 were dissolved in 12.0 mL of ethanol and 120 mL of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 ml of distilled water 200 mL was charged into the reaction solution, followed with extraction twice with 200 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 7.39 g of polymerizable compound 4 as a yellow solid. The yield was 81.3 mol%. The structure of the target product (polymerizable compound 4) was identified by $^1$H-NMR. The $^1$H-NMR spectral data is shown below.

$^1$H-NMR (500 MHz, $CDCl_3$, TMS, δppm): 7.75 (dd, 1H, J = 1.0 Hz,2.0 Hz), 7.70-7.66 (m,3H), 7.34 (ddd,1H, J = 1.0 Hz,7.5 Hz,7.5 Hz), 7.29-7.25 (m,4H), 7.17 (ddd,1H, J = 1.0 Hz,7.5 Hz,7.5 Hz), 7.14-7.11 (m,4H), 7.06 (ddd,2H, J = 1.0 Hz,7.5 Hz,7.5 Hz), 7.00 (d,2H, J = 9.0 Hz), 6.95 (d,2H, J = 9.0 Hz), 6.88 (d,2H, J = 9.0 Hz), 6.86 (d,2H, J = 9.0 Hz), 6.405 (dd,1H, J = 1.5 Hz,17.5 Hz), 6.402 (dd, 1H, J = 1.5 Hz,17.5 Hz), 6.127 (dd, 1H, J = 10.5 Hz,17.5 Hz), 6.124 (dd, 1H, J = 10.5 Hz,17.5 Hz), 5.823 (dd, 1H, J = 1.5 Hz,17.5 Hz), 5.820 (dd, 1H, J = 1.5 Hz,17.5 Hz), 4.95 (s,1H), 4.31 (t,2H, J = 7.5 Hz), 4.176 (t,2H, J = 6.5 Hz), 4.172 (t,2H, J = 6.5 Hz), 3.980 (t,2H, J = 6.5 Hz), 3.947 (t,2H, J = 6.5 Hz), 3.928 (t,2H, J = 6.5 Hz), 2.72-2.56 (m,4H), 2.35-2.28 (m,8H), 1.83-1.12 (m,36H).

(Synthesis Example 5) Synthesis of Polymerizable compound 5 (Still another example of the compound represented by formula (III-1))

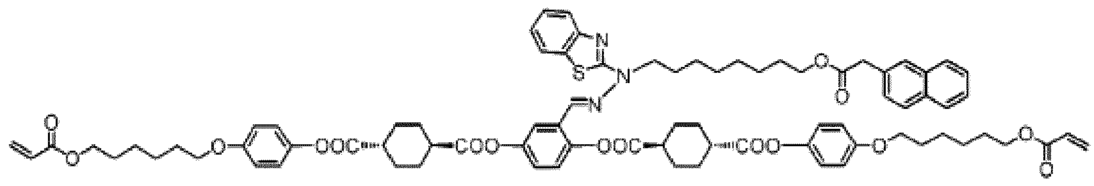

・・・Polymerizable compound 5

Step 1: Synthesis of Intermediate K>

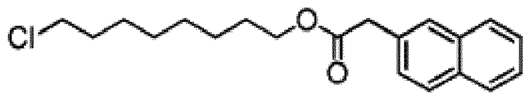

・・・Intermediate K

10.18 g (54.65 mmol) of 2-naphthylacetic acid and 75 mL of N-methyl-2-pyrrolidone was charged into a three-necked reactor equipped with a thermometer, under a nitrogen stream to prepare a uniform solution. 7.50 g (45.55 mmol) of 8-chloro-1-n-octanol was added thereto. Next, 1.33 g (10.89 mmol) of N-N-dimethyl-4-aminopyridine was added. Next, after 12.57 g (65.59 mmol) of 1-ethyl-3-(3-dimethylaminopropyl)carbodiimide hydrochloride was added to the reaction solution over 5 minutes while maintaining the temperature of the reaction solution at 20 to 30°C, the solution was further stirred at 25°C for 4 hours. After completion of the reaction, 250 mL of saturated saline solution was added to the reaction solution, followed by extraction twice with 250 mL of ethyl acetate. The organic layer was collected, dried with anhydrous sodium sulfate, and the sodium sulfate was filtered off. After the solvent was evaporated from the filtrate using a rotary evaporator, the obtained residue was purified by silica gel column chromatography (ethyl acetate:hexane = 1:20 (volume ratio)) to obtain 14.33 g of Intermediate K as a colorless oil. The yield was 94.5 mol%. The structure of Intermediate K was identified by $^1$H-NMR. The $^1$H-NMR spectral data is shown below.

$^1$H-NMR (500 MHz, $CDCl_3$, TMS, $\delta$ppm): 7.83-7.79 (m,3H), 7.73 (s, 1H), 7.49-7.45 (m,2H), 7.42 (dd, 1H, J = 1.5 Hz,8.5 Hz), 4.10 (t,2H, J = 6.5 Hz), 3.78 (s,2H), 3.50 (t,2H, J = 6.5 Hz), 1.72 (tt,2H, J = 7.5 Hz,7.5 Hz), 1.61 (tt,2H, J = 7.5 Hz,7.5 Hz), 1.39-1.21 (m,8H).

<Step 2: Synthesis of Intermediate L (Still another example of the compound represented by formula (IV))>

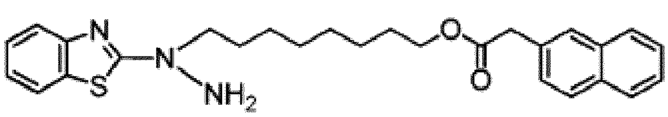

・・・Intermediate L

6.00 g (36.32 mmol) of 2-hydrazinobenzothiazole was dissolved in 60.0 mL of N-N-dimethylformamide in a three-necked reactor equipped with a thermometer under a nitrogen stream. 23.67 g (72.63 mmol) of cesium carbonate and 14.51 g (43.58 mmol) of Intermediate K synthesized in Step 1 were added to the solution, and the solution was stirred at 25°C for 14 hours. After completion of the reaction, the reaction solution was charged with 250 mL of distilled water, followed by extraction twice with 250 mL of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 80:20 (volume ratio)) to obtain 10.21 g of Intermediate L as a yellow oil. The yield was 60.9 mol%. The structure of Intermediate L was identified by $^1$H-NMR. The $^1$H-NMR spectral data is shown below.

$^1$H-NMR (500 MHz, $CDCl_3$, TMS, $\delta$ppm): 7.82-7.79 (m,3H), 7.73 (s, 1H), 7.60 (dd, 1H, J = 1.0 Hz,7.5 Hz), 7.53

(dd, 1H, J = 1.0 Hz,7.5 Hz), 7.48-7.44 (m,2H), 7.42 (dd, 1H, J = 1.5 Hz,8.5 Hz), 7.29-7.27 (m, 1H), 7.06 (ddd,1H, J = 1.0 Hz,7.5 Hz,7.5 Hz), 4.20 (s,2H), 4.09 (t,2H, J = 6.5 Hz), 3.77 (s,2H), 3.71 (t,2H, J = 7.5 Hz), 1.69-1.54 (m,4H), 1.34-1.25 (m,8H).

<Step 3: Synthesis of Polymerizable compound 5 (Still another example of the compound represented by formula (III-1))>

4.13 g (8.95 mmol) of Intermediate L synthesized in Step 2, and, 6.00 g (6.39 mmol) of Intermediate B synthesized in the Synthesis Step 2 of Synthesis Example 1 were dissolved in 12.0 mL of ethanol and 120 mL of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 mL of distilled water 200 mL was charged into the reaction solution, followed with extraction twice with 200 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 7.41 g of polymerizable compound 5 as a yellow solid. The yield was 83.9 mol%. The structure of the target product (polymerizable compound 5) was identified by $^1$H-NMR. The $^1$H-NMR spectral data is shown below.

$^1$H-NMR (500 MHz, $CDCl_3$, TMS, δppm): 7.81-7.77 (m,3H), 7.75 (dd, 1H, J = 0.5 Hz,2.0 Hz), 7.71-7.66 (m,4H), 7.45-7.42 (m,2H), 7.39 (dd, 1H, J = 2.0 Hz,8.5 Hz), 7.34 (ddd,1H, J = 1.5 Hz,7.5 Hz,7.5 Hz), 7.17 (ddd,1H, J = 1.5 Hz,7.5 Hz,7.5 Hz), 7.13-7.09 (m,2H), 7.00-6.95 (m,4H), 6.90-6.85 (m,4H), 6.404 (dd, 1H, J = 1.5 Hz,17.5 Hz), 6.402 (dd, 1H, J = 1.5 Hz,17.5 Hz), 6.127 (dd, 1H, J = 10.5 Hz,17.5 Hz), 6.124 (dd, 1H, J = 10.5 Hz,17.5 Hz), 5.823 (dd, 1H, J = 1.5 Hz,10.5 Hz), 5.820 (dd, 1H, J = 1.5 Hz,10.5 Hz), 4.28 (t,2H, J = 7.5 Hz), 4.18 (t,2H, J = 6.5 Hz), 4.17 (t,2H, J = 6.5 Hz), 4.06 (t,2H, J = 6.5 Hz), 3.95 (t,2H, J = 6.5 Hz), 3.92 (t,2H, J = 6.5 Hz), 3.74 (s,2H), 2.70-2.56 (m,4H), 2.36-2.28 (m,8H), 1.82-1.28 (m,36H).

(Synthesis Example 6) Synthesis of Polymerizable compound 6 (Still another example of the compound represented by formula (III-1))

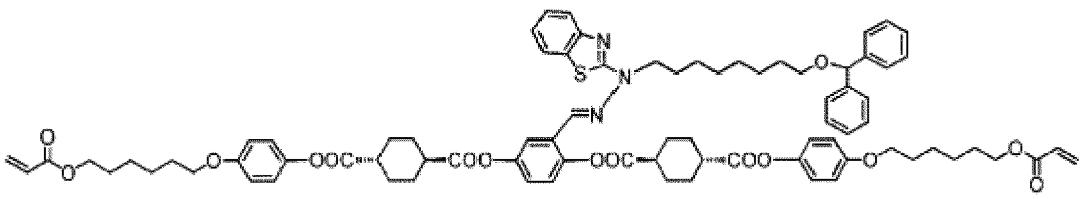

・・・Polymerizable compound 6

<Step 1: Synthesis of Intermediate M>

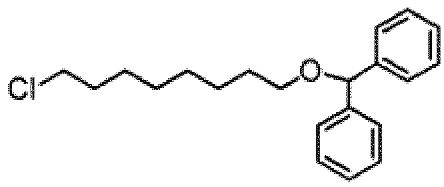

・・・Intermediate M

9.83 g (59.70 mmol) of 8-chloro-1-n-octanol and 100 mL of toluene were charged three-necked reactor equipped with a thermometer, under a nitrogen stream to prepare a uniform solution. 10.0 g (54.28 mmol) of benzhydrol was added thereto. Next, 1.26 g (5.43 mmol) of (±)-10-camphorsulfonic acid was added, and the solution was stirred at 110°C for 5 hours. After completion of the reaction, 250 mL of saturated saline solution was added to the reaction solution, followed by extraction twice with 250 mL of ethyl acetate. The organic layer was collected, dried with anhydrous sodium sulfate, and the sodium sulfate was filtered off. After the solvent was evaporated from the filtrate using a rotary evaporator, the obtained residue was purified by silica gel column chromatography (ethyl acetate:hexane = 1:20 (volume ratio)) to obtain 16.1 g of Intermediate M as a colorless oil. The yield was 89.6 mol%. The structure of Intermediate M was identified

by $^1$H-NMR. The $^1$H-NMR spectral data is shown below.

$^1$H-NMR (500 MHz, $CDCl_3$, TMS, δppm): 7.35-7.21 (m,10H), 5.32 (s, 1H), 3.51 (t,2H, J = 6.5 Hz), 3.44 (t,2H, J = 6.5 Hz), 1.75 (tt,2H, J = 7. Hz,7.5 Hz), 1.67-1.61 (m,2H), 1.49-1.25 (m,8H).

<Step 2: Synthesis of Intermediate N (Still another example of the compound represented by formula (IV))>

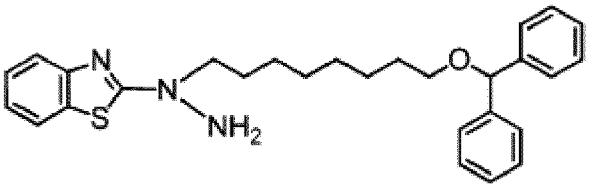

・・・Intermediate N

6.00 g (36.32 mmol) of 2-hydrazinobenzothiazole was dissolved in 60.0 mL of N-N-dimethylformamide in a three-necked reactor equipped with a thermometer under a nitrogen stream. 23.67 g (72.63 mmol) of cesium carbonate and 14.42 g (43.58 mmol) of Intermediate M synthesized in Step 1 were added to the solution, and the solution was stirred at 25°C for 14 hours. After completion of the reaction, the reaction solution was charged with 250 mL of distilled water, followed by extraction twice with 250 mL of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 80:20 (volume ratio)) to obtain 7.61 g of Intermediate N as a gray solid. The yield was 45.6 mol%. The structure of Intermediate N was identified by $^1$H-NMR. The $^1$H-NMR spectral data is shown below.

$^1$H-NMR (500 MHz, $CDCl_3$, TMS, δppm): 7.59 (dd,1H, J = 1.0 Hz,8.0 Hz), 7.53 (dd,1H, J = 1.0 Hz,8.0 Hz), 7.35-7.21 (m,11H), 7.058 (ddd,1H, J = 1.0 Hz,7.5 Hz,7.5 Hz), 5.32 (s, 1H), 4.20 (s,2H), 3.73 (t,2H, J = 7.5 Hz), 3.43 (t,2H, J = 6.5 Hz), 1.74-1.59 (m,4H), 1.41-1.24 (m,8H).

<Step 3: Synthesis of Polymerizable compound 6 (Still another example of the compound represented by formula (III-1))>

4.11 g (8.95 mmol) of Intermediate N synthesized in Step 2, and, 6.00 g (6.39 mmol) of Intermediate B synthesized in the Synthesis Step 2 of Synthesis Example 1 were dissolved in 12.0 mL of ethanol and 120 mL of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 mL of distilled water 200 mL was charged into the reaction solution, followed with extraction twice with 200 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 5.87 g of polymerizable compound 6 as a yellow solid. The yield was 66.5 mol%. The structure of the target product (polymerizable compound 6) was identified by $^1$H-NMR. The $^1$H-NMR spectral data is shown below.

$^1$H-NMR (500 MHz, $CDCl_3$, TMS, δppm): 7.75 (dd, 1H, J = 1.0 Hz,2.0 Hz), 7.69-7.66 (m,3H), 7.35-7.19 (m,11H), 7.16 (ddd,1H, J = 1.0 Hz,7.5 Hz,7.5 Hz), 7.12-7.10 (m,2H), 7.00-6.95 (m,4H), 6.90-6.85 (m,4H), 6.40 (dd,2H, J = 1.5 Hz,17.5 Hz), 6.13 (dd,2H, J = 10.5 Hz,17.5 Hz), 5.823 (dd, 1H, J = 1.5 Hz,10.5 Hz), 5.820 (dd, 1H, J = 1.5 Hz,10.5 Hz), 5.29 (s, 1H), 4.30 (t,2H, J = 7.5 Hz), 4.18 (t,4H, J = 6.5 Hz), 3.95 (t,2H, J = 6.5 Hz), 3.93 (t,2H, J = 6.5 Hz), 3.40 (t,2H, J = 6.5 Hz), 2.71-2.55 (m,4H), 2.38-2.25 (m,8H), 1.81-1.28 (m,36H).

(Synthesis Example 7) Synthesis of Polymerizable compound 7 (Still another example of the compound represented by formula (III-1))

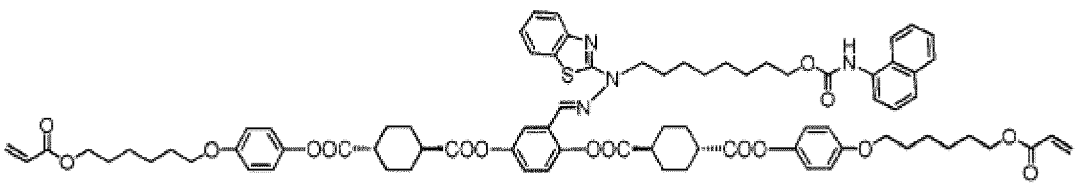

・・・Polymerizable compound 7

<Step 1: Synthesis of Intermediate O>

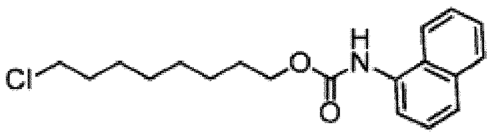

・・・Intermediate O

14.9 g (90.48 mmol) of 8-chloro-1-n-octanol and 150 mL of dichloromethane were charged in a three-necked reactor equipped with a thermometer, under a nitrogen stream to prepare a uniform solution. 13.9 g (82.16 mmol) of 1-naphthyl isocyanate was added thereto. Next, 21.2 g (164.02 mmol) of N-N- diisopropylethylamine was added, and the solution was stirred at 25°C for 5 hours. After completion of the reaction, 500 mL of saturated saline solution was added to the reaction solution, followed by extraction twice with 500 mL of ethyl acetate. The organic layer was collected, dried with anhydrous sodium sulfate, and the sodium sulfate was filtered off. After the solvent was evaporated from the filtrate using a rotary evaporator, the obtained residue was purified by silica gel column chromatography (ethyl acetate:hexane = 20:80 (volume ratio)) to obtain 15.6 g of Intermediate O was a white solid. The yield was 57.1 mol%. The structure of Intermediate O was identified by $^1$H-NMR. The $^1$H-NMR spectral data is shown below.

$^1$H-NMR (500 MHz, $CDCl_3$, TMS, δppm): 7.89-7.86 (m,3H), 7.67 (d,1H, J = 8.0 Hz), 7.55-7.46 (m,3H), 6.92 (br,1H), 4.22 (t,2H, J = 6.5 Hz), 3.54 (t,2H, J = 6.5 Hz), 1.78 (tt,2H, J = 7.5 Hz,7.5 Hz), 1.74-1.68 (m,2H), 1.45-1.31 (m,8H).

<Step 2: Synthesis of Intermediate P (Still another example of the compound represented by formula (IV))>

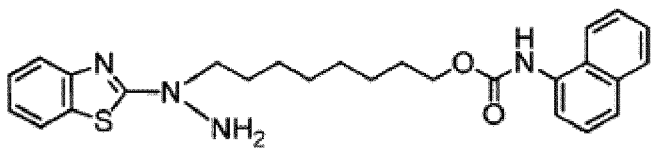

・・・Intermediate P

6.00 g (36.32 mmol) of 2-hydrazinobenzothiazole was dissolved in 60.0 mL of N-N-dimethylformamide in a three-necked reactor equipped with a thermometer under a nitrogen stream. 23.67 g (72.63 mmol) of cesium carbonate and 14.55 g (43.58 mmol) of Intermediate O synthesized in Step 1 were added to the solution, and the solution was stirred at 25°C for 14 hours. After completion of the reaction, the reaction solution was charged with 250 mL of distilled water, followed by extraction twice with 250 mL of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 70:30 (volume ratio)) to obtain 8.87 g of Intermediate P as a red oil. The yield was 52.8 mol%. The structure of Intermediate P was identified by $^1$H-NMR. The $^1$H-NMR spectral data is shown below.

$^1$H-NMR (500 MHz, $CDCl_3$, TMS, δppm): 7.89-7.85 (m,3H), 7.66 (d, 1H, J = 7.5 Hz), 7.59 (dd, 1H, J = 1.0 Hz,7.5 Hz), 7.54-7.46 (m,4H), 7.29-7.25 (m, 1H), 7.06 (ddd,1H, J = 1.0 Hz,7.5 Hz,7.5 Hz), 6.96 (br,1H), 4.22 (s,2H), 4.21 (t,2H, J = 6.5 Hz), 3.75 (t,2H, J = 6.5 Hz), 1.77-1.65 (m,4H), 1.43-1.35 (m,8H).

<Step 3: Synthesis of Polymerizable compound 7 (Still another example of the compound represented by formula (III-1))>

4.14 g (8.95 mmol) of Intermediate P synthesized in Step 2, and, 6.00 g (6.39 mmol) of Intermediate B synthesized in the Synthesis Step 2 of Example 1 were dissolved in 12.0 mL of ethanol and 120 mL of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 mL of distilled water was charged into the reaction solution, followed with extraction twice with 200 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 5.93 g of polymerizable compound 7 as a yellow solid. The yield was 67.1 mol%. The structure of the target product (polymerizable compound 7) was identified by $^1$H-NMR. The $^1$H-NMR spectral data is shown below.

$^1$H-NMR (500 MHz, $CDCl_3$, TMS, δppm): 7.86-7.83 (m,3H), 7.75 (d, 1H, J = 2.0 Hz), 7.69-7.64 (m,4H), 7.50-7.44 (m,3H), 7.34 (ddd,1H, J = 1.0 Hz,7.5 Hz,7.5 Hz), 7.16 (ddd,1H, J = 1.0 Hz,7.5 Hz,7.5 Hz), 7.13-7.08 (m,2H), 7.00-6.93 (br,1H), 6.99 (d,2H, J = 9.0 Hz), 6.95 (d,2H, J = 9.0 Hz), 6.88 (d,2H, J = 9.0 Hz), 6.83 (d,2H, J = 9.0 Hz), 6.405 (dd, 1H, J = 1.5 Hz,17.5 Hz), 6.402 (dd, 1H, J = 1.5 Hz,17.5 Hz), 6.127 (dd, 1H, J = 10.5 Hz,17.5 Hz), 6.124 (dd, 1H, J = 10.5 Hz,17.5 Hz), 5.823 (dd, 1H, J = 1.5 Hz,10.5 Hz), 5.820 (dd, 1H, J = 1.5 Hz,10.5 Hz), 4.31 (t,2H, J = 7.5 Hz), 4.19-4.15 (m,6H), 3.95 (t,2H, J = 6.5 Hz), 3.87 (t,2H, J = 6.5 Hz), 2.71-2.58 (m,4H), 2.36-2.30 (m,8H), 1.81-1.39 (m,36H).

(Synthesis Example 8) Synthesis of Polymerizable compound 8 (Still another example of the compound represented by formula (III-1))

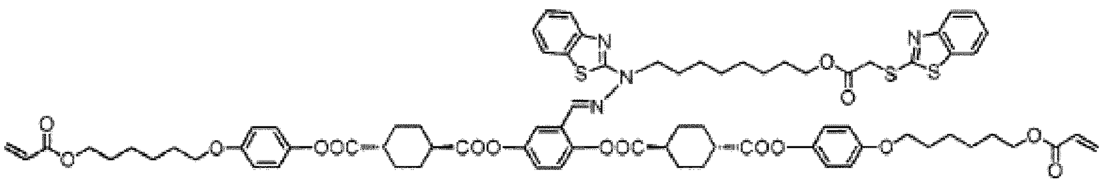

・・・Polymerizable compound 8

Step 1: Synthesis of Intermediate Q (Still another example of the compound represented by formula (IV))>

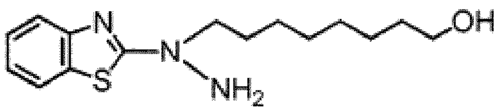

・・・Intermediate Q

6.00 g (36.32 mmol) of 2-hydrazinobenzothiazole was dissolved in 60.0 mL of N-N-dimethylformamide in a three-necked reactor equipped with a thermometer under a nitrogen stream. 23.67 g (72.63 mmol) of cesium carbonate and 7.18 g (43.58 mmol) of 8-chloro-1-n-octanol were added to the solution, and the solution was stirred at 25°C for 14 hours. After completion of the reaction, the reaction solution was charged with 250 mL of distilled water, followed by extraction twice with 250 mL of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 70:30 (volume ratio)) to obtain 5.97 g of Intermediate Q as a gray solid. The yield was 56.0 mol%. The structure of intermediate Q was identified by $^1$H-NMR. The $^1$H-NMR spectral data is shown below.

$^1$H-NMR (500 MHz, $CDCl_3$, TMS, δppm):7.60 (dd, 1H, J = 1.0 Hz,7.5 Hz), 7.53 (dd, 1H, J = 1.0 Hz,7.5 Hz), 7.29 (dd, 1H, J = 1.0 Hz,7.5 Hz), 7.06 (ddd,1H, J = 1.0 Hz,7.5 Hz,7.5 Hz), 4.23 (s,2H), 3.75 (t,2H, J = 7.5 Hz), 3.63 (t,2H, J = 6.5 Hz), 1.77-1.71 (m,2H), 1.58-1.53 (m,4H), 1.40-1.32 (m, 6H).

<Step 2: Synthesis of Intermediate R>

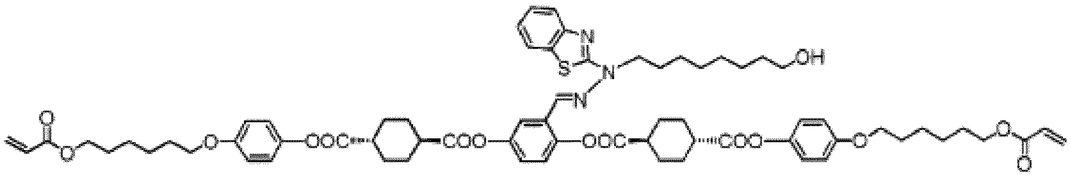

・・・Intermediate R

2.44 g (8.31 mmol) of Intermediate Q synthesized in Step 1, and, 6.00 g (6.39 mmol) of Intermediate B synthesized in the Synthesis Step 2 of Synthesis Example 1 were dissolved in 12.0 mL of ethanol and 120 mL of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 mL of distilled water was charged into the reaction solution, followed with extraction twice with 200 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 6.23 g of Intermediate R as a yellow solid. The yield was 80.3 mol%. The structure of Intermediate R was identified by $^1$H-NMR. The $^1$H-NMR spectral data is shown below.

$^1$H-NMR (500 MHz, $CDCl_3$, TMS, δppm): 7.75 (d, 1H, J = 2.5 Hz), 7.70-7.66 (m,3H), 7.34 (ddd,1H, J = 1.0 Hz,7.5 Hz,7.5 Hz), 7.17 (ddd,1H, J = 1.0 Hz,7.5 Hz,7.5 Hz), 7.13-7.09 (m,2H), 7.00-6.96 (m,4H), 6.88 (d,4H, J = 9.0 Hz), 6.40 (dd,2H, J = 1.5 Hz,17.5 Hz), 6.13 (dd,2H, J = 10.5 Hz,17.5 Hz), 5.82 (dd,2H, J = 1,5 Hz,10.5 Hz), 4.30 (t,2H, J = 7.5 Hz), 4.18 (t,4H, J = 6.5 Hz), 3.947 (t,2H, J = 6.5 Hz), 3.944 (t,2H, J = 6.5 Hz), 3.58 (t,2H, J = 6.5 Hz), 2.72-2.57 (m,4H), 2.37-2.29 (m,9H), 1.82-1.34 (m,36H).

<Step 3: Synthesis of Polymerizable compound 8 (Still another example of the compound represented by formula (III-1))>

5.00 g (4.12 mmol) of Intermediate R synthesized in Step 2 and 100 mL of chloroform were charged in a three-necked reactor equipped with a thermometer, under a nitrogen stream to prepare a uniform solution. 1.11 g (4.94 mmol) of (2-benzothiazolylthio)acetic acid was added to the solution. Next, 0.121 g (0.99 mmol) of N-N-dimethyl-4-aminopyridine was added. Next, after 0.685 g (5.43 mmol) of N-N'-diisopropylcarbodiimide was added to the reaction solution over 5 minutes while maintaining the temperature of the reaction solution at 20 to 30°C, the solution was further stirred at 25°C for 4 hours. After completion of the reaction, 250 mL of saturated saline solution was added to the reaction solution, followed by extraction twice with 250 mL of chloroform. The organic layer was collected, dried with anhydrous sodium sulfate, and the sodium sulfate was filtered off. After the solvent was evaporated from the filtrate using a rotary evaporator, the obtained residue was purified by silica gel column chromatography (chloroform:THF = 95:5) to obtain 4.38 g of polymerizable compound 8 as a yellow solid. The yield was 74.8 mol%. The structure of the target product (polymerizable compound 8) was identified by $^1$H-NMR. The $^1$H-NMR spectral data is shown below.

$^1$H-NMR (500 MHz, $CDCl_3$, TMS, δppm):7.83 (dd, 1H, J = 1.0 Hz,7.5 Hz), 7.75 (d, 1H, J = 2.0 Hz), 7.73 (dd, 1H, J = 1.0 Hz,7.5 Hz), 7.70-7.66 (m,3H), 7.38 (ddd,1H, J = 1.0 Hz,7.5 Hz,7.5 Hz), 7.34 (ddd,1H, J = 1.0 Hz,7.5 Hz,7.5 Hz), 7.27 (ddd,1H, J = 1.0 Hz,7.5 Hz,7.5 Hz), 7.17 (ddd,1H, J = 1.0 Hz,7.5 Hz,7.5 Hz), 7.13-7.09 (m,2H), 6.99 (d,2H, J = 9.0 Hz), 6.97 (d,2H, J = 9.0 Hz), 6.90-6.87 (m,4H), 6.405 (dd, 1H, J = 1.5 Hz,17.5 Hz), 6.402 (dd, 1H, J = 1.5 Hz,17.5 Hz), 6.127 (dd, 1H, J = 10.5 Hz,17.5 Hz), 6.125 (dd, 1H, J = 10.5 Hz,17.5 Hz), 5.824 (dd, 1H, J = 1.5 Hz,10.5 Hz), 5.821 (dd, 1H, J = 1.5 Hz,10.5 Hz), 4.29 (t,2H, J = 7.5 Hz), 4.176 (t,2H, J = 6.5 Hz), 4.170 (t,2H, J = 6.5 Hz), 4.125 (s,2H), 4.125 (t,2H, J = 7.5 Hz), 3.96-3.92 (m,4H), 2.72-2.58 (m,4H),2.36-2.29 (m,8H), 1.83-1.27 (m, 36H).

(Synthesis Example 9) Synthesis of Polymerizable compound 9 (Still another example of the compound represented by formula (III-1))

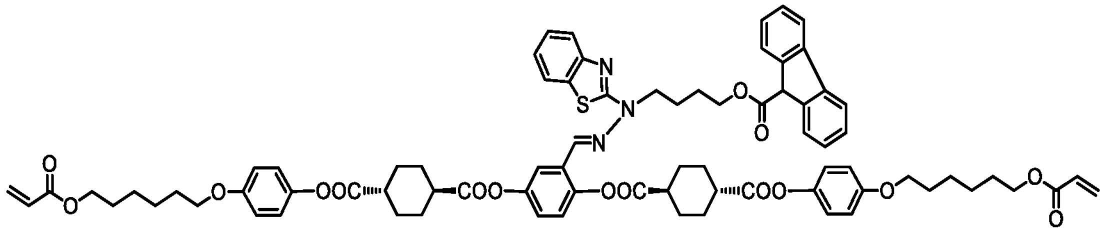

...Polymerizable compound 9

<Step 1: Synthesis of Intermediate S (Still another example of the compound represented by formula (IV))>

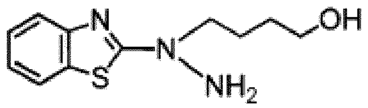

・・・Intermediate S

6.00 g (36.32 mmol) of 2-hydrazinobenzothiazole was dissolved in 60.0 mL of N-N-dimethylformamide in a three-necked reactor equipped with a thermometer under a nitrogen stream. 23.67 g (72.63 mmol) of cesium carbonate and 4.73 g (43.58 mmol) of 4-chloro-1-butanol were added to the solution, and the solution was stirred at 25°C for 14 hours. After completion of the reaction, the reaction solution was charged with 250 mL of distilled water, followed by extraction twice with 250 mL of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 30:70 (volume ratio)) to obtain 2.98 g of Intermediate S as a gray solid. The yield was 34.6 mol%. The structure of intermediate S was identified by $^1$ H-NMR. The $^1$ H-NMR spectral data is shown below.

$^1$H-NMR (500 MHz, $CDCl_3$, TMS, δppm):7.60 (dd, 1H, J = 1.0 Hz,8.0 Hz), 7.52 (d, 1H, J = 8.0 Hz), 7.28 (ddd,1H, J = 1.0 Hz,7.5 Hz,8.0 Hz), 7.07 (ddd,1H, J = 1.0 Hz,7.5 Hz,8.0 Hz), 4.28 (br,2H), 3.86 (t,2H, J = 7.0 Hz), 3.75 (t,2H, J = 6.0 Hz), 2.51 (br,1H), 1.88 (tt,2H, J = 7.0 Hz,7.0 Hz), 1.66 (tt,2H, J = 6.0 Hz,7.0 Hz).

<Step 2: Synthesis of Intermediate T>

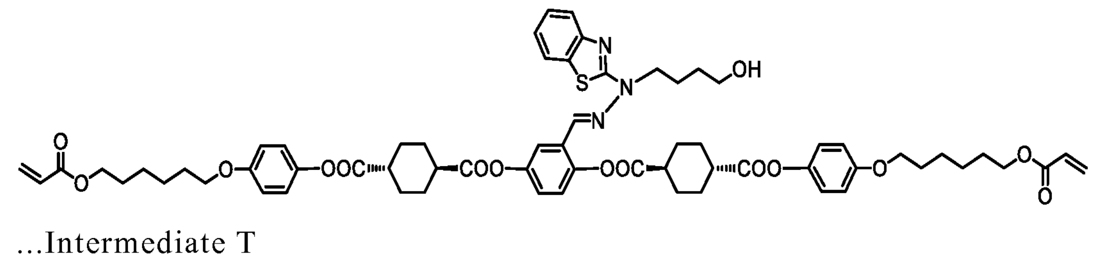

...Intermediate T

1.97 g (8.31 mmol) intermediate S synthesized in Step 1, and, 6.00 g (6.39 mmol) of Intermediate B synthesized in the Synthesis Step 2 of Example Synthesis 1 were dissolved in 12.0 mL of ethanol and 120 mL of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 mL of distilled water was charged into the reaction solution, followed by extraction twice with 200 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 6.21 g of Intermediate T as a yellow solid. The yield was 83.9 mol%. The structure of intermediate T was identified by $^1$H-NMR. The $^1$H-NMR spectral data is shown below.

$^1$ H-NMR (500 MHz, $CDCl_3$, TMS, δppm):7.74 (dd, 1H, J = 1.0 Hz,2.0 Hz), 7.73 (s,1H), 7.69 (dd, 1H, J = 0.5 Hz,8.0 Hz), 7.67 (d,1H, J = 7.5 Hz), 7.35 (ddd,1H, J = 1.0 Hz,7.5 Hz,8.0 Hz), 7.18 (ddd,1H, J = 1.0 Hz,7.5 Hz,8.0 Hz), 7.10-7.14 (m,2H), 6.96-7.00 (m,4H), 6.87-6.90 (m,4H), 6.40 (dd,2H, J = 1.5 Hz,17.5 Hz), 6.13 (dd,2H, J = 10.5 Hz,17.5 Hz), 5.82 (dd,2H, J = 1.5 Hz,10.5 Hz), 4.42 (t,2H, J = 7.0 Hz), 4.18 (t,4H, J = 6.5 Hz), 3.95 (t,4H, J = 6.5 Hz), 3.77-3.79 (m,2H), 2.59-2.71 (m,4H), 2.31-2.35 (m,8H), 1.88 (tt,2H, J = 7.0 Hz,7.5 Hz), 1.55-1.78 (m,19H), 1.42-1.53 (m,8H).

<Step 3: Synthesis of Polymerizable compound 9 (Still another example of the compound represented by formula (III-1))>

6.00 g (5.18 mmol) of Intermediate T synthesized in Step 2 and 120 mL of chloroform was charged in a three-necked reactor equipped with a thermometer, under a nitrogen stream to prepare a uniform solution. 1.31 g (6.22 mmol) of 9-fluorenecarboxylic acid was added to the solution. Next, 0.152 g (1.24 mmol) of N-N-dimethyl-4-aminopyridinewas added. Next, after 0.863 g (6.84 mmol) of N-N'-diisopropylcarbodiimide was added to the reaction solution over 5 minutes while maintaining the temperature of the reaction solution at 20 to 30°C, the solution was further stirred at 25°C for 4 hours. After completion of the reaction, 250 mL of saturated saline solution was added to the reaction solution, followed by extraction twice with 250 mL of chloroform. The organic layer was collected, dried with anhydrous sodium sulfate, and the sodium sulfate was filtered off. After the solvent was evaporated from the filtrate using a rotary evaporator, the obtained residue was purified by silica gel column chromatography (chloroform:THF = 95:5) to obtain 5.68 g of polymerizable compound 9 as a yellow solid. The yield was 81.2 mol%. The structure of the target product (polymerizable compound 9) was identified by $^1$ H-NMR. The $^1$ H-NMR spectral data is shown below.

$^1$ H-NMR (500 MHz, $CDCl_3$, TMS, δppm):7.70-7.73 (m,2H), 7.66-7.67 (m,3H), 7.59 (dd,2H, J = 0.5 Hz,7.5 Hz), 7.52 (s, 1H), 7.35 (ddd,1H, J = 1.5 Hz,7.5 Hz,8.0 Hz), 7.30 (dd,2H, J = 7.5 Hz,7.5 Hz), 7.17-7.23 (m,3H), 7.11-7.14 (m,2H), 6.94-7.00 (m,4H), 6.86-6.90 (m,4H), 6.40 (dd,2H, J = 1.5 Hz,17.5 Hz), 6.13 (dd,2H, J = 10.5 Hz,17.5 Hz), 5.824 (dd, 1H, J = 1.5 Hz,10.5 Hz), 5.823 (dd, 1H, J = 1.5 Hz,10.5 Hz), 4.85 (s,1H), 4.24-4.26 (m,4H), 4.18 (t,4H, J = 7.0 Hz), 3.95 (t,2H, J = 6.5 Hz), 3.94 (t,2H, J = 6.5 Hz), 2.45-2.69 (m,4H), 2.28-2.37 (m,4H), 2.18-2.23 (m,4H), 1.42-1.83 (m,28H).

(Synthesis Example 10) Synthesis of Polymerizable compound 10 (Still another example of the compound represented by formula (III-1))

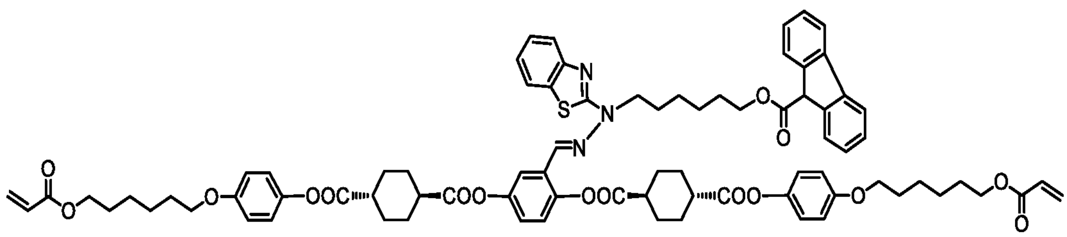

...Polymerizable compound 10

<Step 1: Synthesis of Intermediate U (Still another example of the compound represented by formula (IV))>

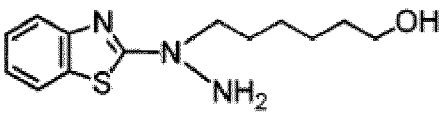

・・・Intermediate U

6.00 g (36.32 mmol) of 2-hydrazinobenzothiazole was dissolved in 60.0 mL of N-N-dimethylformamide in a three-necked reactor equipped with a thermometer under a nitrogen stream. 23.67 g (72.63 mmol) of cesium carbonate and 5.95 g (43.58 mmol) of 6-chloro-1-hexanol were added to the solution, and the solution was stirred at 25°C for 14 hours. After completion of the reaction, the reaction solution was charged with 250 mL of distilled water, followed by extraction twice with 250 mL of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue

was purified by silica gel column chromatography (hexane:THF = 40:60 (volume ratio)) to obtain 3.89 g of Intermediate U as a gray solid. The yield was 40.4 mol%. The structure of Intermediate U was identified by $^1$ H-NMR. The $^1$ H-NMR spectral data is shown below.

$^1$H-NMR (500 MHz, $CDCl_3$, TMS, δppm):7.60 (dd, 1H, J = 1.0 Hz,8.0 Hz), 7.53 (d, 1H, J = 8.0 Hz), 7.28 (ddd,1H, J = 1.0 Hz,7.5 Hz,8.0 Hz), 7.07 (ddd,1H, J = 1.0 Hz,7.5 Hz,8.0 Hz), 4.23 (br,2H), 3.77 (t,2H, J = 7.5 Hz), 3.65 (t,2H, J = 6.5 Hz), 1.76 (tt,2H, J = 7.0 Hz,7.5 Hz), 1.56-1.61 (m,2H), 1.39-1.50 (m,5H).

<Step 2: Synthesis of Intermediate V>

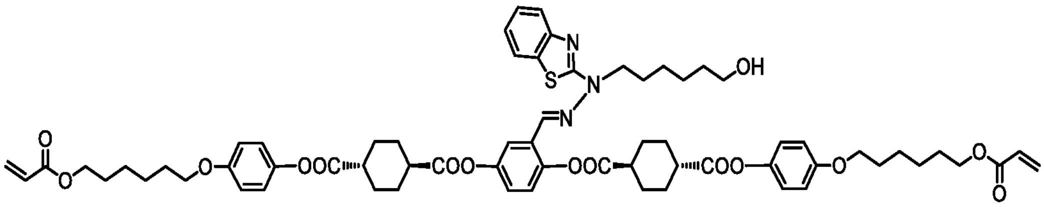

...Intermediate V

2.20 g (8.31 mmol) of Intermediate U synthesized in Step 1, and, 6.00 g (6.39 mmol) of Intermediate B synthesized in the Synthesis Step 2 of Synthesis Example 1 were dissolved in 12.0 mL of ethanol and 120 mL of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 mL of distilled water was charged into the reaction solution, followed with extraction twice with 200 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 6.73 g of Intermediate V as a yellow solid. The yield was 88.7 mol%. The structure of Intermediate V was identified by $^1$ H-NMR. The $^1$ H-NMR spectral data is shown below.

$^1$ H-NMR (500 MHz, $CDCl_3$, TMS, δppm):7.74 (d,1H, J = 3.0 Hz), 7.67-7.69 (m,3H), 7.34 (ddd,1H, J = 1.0 Hz,7.5 Hz,8.0 Hz), 7.17 (ddd,1H, J = 1.0 Hz,7.5 Hz,8.0 Hz), 7.09-7.14 (m,2H), 6.96-7.00 (m,4H), 6.87-6.90 (m,4H), 6.42 (dd,2H, J = 1.5 Hz,17.5 Hz), 6.13 (dd,2H, J = 10.5 Hz,17.5 Hz), 5.82 (dd,2H, J = 1.5 Hz,10.5 Hz), 4.32 (t,2H, J = 7.5 Hz), 4.18 (t,4H, J = 7.0 Hz), 3.95 (t,2H, J = 6.5 Hz), 3.94 (t,2H, J = 6.5 Hz), 3.61-3.64 (m,2H), 2.60-2.72 (m,4H), 2.28-2.35 (m,8H), 1.66-1.82 (m,18H), 1.42-1.62 (m,15H).

<Step 3: Synthesis of Polymerizable compound 10 (Still another example of the compound represented by formula (III-1))>

6.00 g (5.06 mmol) of Intermediate V synthesized in Step 2 and 120 mL of chloroform was charged in a three-necked reactor equipped with a thermometer under a nitrogen stream to prepare a uniform solution. 1.28 g (6.07 mmol) of 9-fluorene carboxylic acid was added to the solution. Next, 0.148 g (1.21 mmol) of N-N-dimethyl-4-aminopyridine was added. Next, after 0.842 g (6.68 mmol) of N-N'-diisopropylcarbodiimidewas added to the reaction solution over 5 minutes while maintaining the temperature of the reaction solution at 20 to 30°C, the solution was further stirred at 25°C for 4 hours. After completion of the reaction, 250 mL of saturated saline solution was added to the reaction solution, followed by extraction twice with 250 mL of chloroform. The organic layer was collected, dried with anhydrous sodium sulfate, and the sodium sulfate was filtered off. After the solvent was evaporated from the filtrate using a rotary evaporator, the obtained residue was purified by silica gel column chromatography (chloroform:THF = 95:5) to obtain 6.23 g of polymerizable compound 10 as a yellow solid. The yield was 89.4 mol%. The structure of the target product (polymerizable compound 10) was identified by $^1$ H-NMR. The $^1$ H-NMR spectral data is shown below.

$^1$ H-NMR (500 MHz, $CDCl_3$, TMS, δppm):7.62-7.75 (m,8H), 7.28-7.38 (m,5H), 7.17 (ddd,1H, J = 1.0 Hz,7.5 Hz,8.0 Hz), 7.10-7.14 (m,2H), 6.93-7.00 (m,4H), 6.84-6.90 (m,4H), 6.405 (dd, 1H, J = 1.0 Hz,17.5 Hz), 6.403 (dd, 1H, J = 1.0 Hz,17.5 Hz), 6.127 (dd, 1H, J = 10.5 Hz,17.5 Hz), 6.125 (dd, 1H, J = 10.5 Hz,17.5 Hz), 5.823 (dd, 1H, J = 1.0 Hz,10.5 Hz), 5.820 (dd, 1H, J = 1.0 Hz,10.5 Hz), 4.82 (s, 1H), 4.28 (t,2H, J = 7.5 Hz), 4.13-4.19 (m,6H), 3.91-3.96 (m,4H), 2.55-2.68 (m,4H), 2.28-2.36 (m,8H), 1.64-1.81 (m,20H), 1.39-1.55 (m,12H).

(Synthesis Example 11) Synthesis of Polymerizable compound 11 (Still another example of the compound represented by formula (III-1))

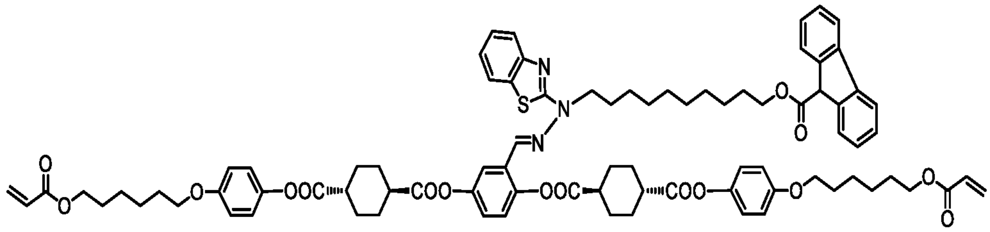

...Polymerizable compound 11

<Step 1: Synthesis of Intermediate W (Still another example of the compound represented by formula (IV))>

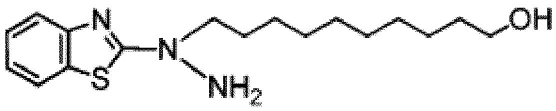

・・・Intermediate W

6.00 g (36.32 mmol) of 2-hydrazinobenzothiazole was dissolved in 60.0 mL of N-N-dimethylformamide in a three-necked reactor equipped with a thermometer under a nitrogen stream. 23.67 g (72.63 mmol) of cesium carbonate and 10.34 g (43.58 mmol) of 10-bromo-1-decanol were added to the solution, and the solution was stirred at 25°C for 14 hours. After completion of the reaction, the reaction solution was charged with 250 mL of distilled water, followed by extraction twice with 250 mL of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 70:30 (volume ratio)) to obtain 3.68 g of Intermediate W as a gray solid. The yield was 31.5 mol%. The structure of Intermediate W was identified by $^1$ H-NMR. The $^1$ H-NMR spectral data is shown below.

$^1$H-NMR (500 MHz, $CDCl_3$, TMS, δppm):7.60 (dd, 1H, J = 1.0 Hz,8.0 Hz), 7.53 (dd, 1H, J = 0.5 Hz,8.0 Hz), 7.28 (ddd,1H, J = 1.0 Hz,7.5 Hz,8.0 Hz), 7.06 (ddd,1H, J = 1.0 Hz,7.5 Hz,8.0 Hz), 4.23 (br,2H), 3.75 (t,2H, J = 7.5 Hz), 3.63 (br,2H), 1.73 (tt,2H, J = 7.0 Hz,7.5 Hz), 1.55 (tt,2H, J = 7.0 Hz,7.5 Hz), 1.25-1.41 (m,13H).

<Step 2: Synthesis of Intermediate Y>

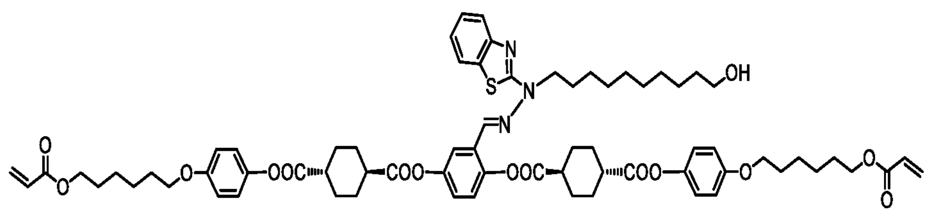

...Intermediate Y

2.67 g (8.31 mmol) of Intermediate W synthesized in Step 1, and, 6.00 g (6.39 mmol) of Intermediate B synthesized in the Synthesis Step 2 of Synthesis Example 1 were dissolved in 12.0 mL of ethanol and 120 mL of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 mL of distilled water was charged into the reaction solution, followed with extraction twice with 200 mL of ethyl

acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 6.77 g of Intermediate Y as a yellow solid. The yield was 85.3 mol%. The structure of Intermediate Y was identified by $^1$H-NMR. The $^1$H-NMR spectral data is shown below.

$^1$H-NMR (500 MHz, $CDCl_3$, TMS, δppm): 7.75 (d,1H, J = 2.0 Hz), 7.66-7.70 (m,3H), 7.34 (ddd,1H, J = 1.0 Hz,7.5 Hz,8.0 Hz), 7.17 (ddd,1H, J = 1.0 Hz,7.5 Hz,8.0 Hz), 7.09-7.13 (m,2H), 6.96-7.00 (m,4H), 6.87-6.90 (m,4H), 6.40 (dd,2H, J = 1.5 Hz,17.5 Hz), 6.13 (dd,2H, J = 10.5 Hz,17.5 Hz), 5.82 (dd,2H, J = 1.5 Hz,10.5 Hz), 4.30 (t,2H, J = 7.5 Hz), 4.18 (t,4H, J = 7.0 Hz), 3.95 (t,4H, J = 6.5 Hz), 3.56-3.59 (m,2H), 2.59-2.71 (m,4H), 2.32-2.35 (m,8H), 1.70-1.82 (m,18H), 1.28-1.54 (m,23H).

<Step 3: Synthesis of Polymerizable compound 11 (Still another example of the compound represented by formula (III-1))>

6.00 g (4.83 mmol) of Intermediate Y synthesized in Step 2 and 120 mL of chloroform was charged in a three-necked reactor equipped with a thermometer, under a nitrogen stream to prepare a uniform solution. 1.22 g (5.79 mmol) of 9-fluorenecarboxylic acid was added to the solution. Next, 0.142 g (1.16 mmol) of N-N-dimethyl-4-aminopyridine was added. Next, after 0.804 g (6.37 mmol) of N-N'-diisopropylcarbodiimide was added to the reaction solution over 5 minutes while maintaining the temperature of the reaction solution at 20 to 30°C, the solution was further stirred at 25°C for 4 hours. After completion of the reaction, 250 mL of saturated saline solution was added to the reaction solution, followed by extraction twice with 250 mL of chloroform. The organic layer was collected, dried with anhydrous sodium sulfate, and the sodium sulfate was filtered off. After the solvent was evaporated from the filtrate using a rotary evaporator, the obtained residue was purified by silica gel column chromatography (chloroform:THF = 95:5) to obtain 6.26 g of polymerizable compound 11 as a yellow solid. The yield was 90.3 mol%. The structure of the target product (polymerizable compound 11) was identified by $^1$H-NMR. The $^1$H-NMR spectral data is shown below.

$^1$H-NMR (500 MHz, $CDCl_3$, TMS, δppm): 7.73-7.75 (m,3H), 7.63-7.69 (m,5H), 7.39-7.42 (m,2H), 7.30-7.35 (m,3H), 7.16 (ddd,1H, J = 1.0 Hz,7.5 Hz,8.0 Hz), 7.09-7.13 (m,2H), 6.94-7.00 (m,4H), 6.84-6.90 (m,4H), 6.404 (dd,1H, J = 1.5 Hz,17.5 Hz), 6.401 (dd,1H, J = 1.5 Hz,17.5 Hz), 6.127 (dd,1H, J = 10.5 Hz,17.5 Hz), 6.123 (dd,1H, J = 10.5 Hz,17.5 Hz), 5.823 (dd,1H, J = 1.5 Hz,10.5 Hz), 5.819 (dd,1H, J = 1.5 Hz,10.5 Hz), 4.84 (s,1H), 4.31 (t,2H, J = 7.5 Hz), 4.15-4.19 (m,4H), 4.10 (t,2H, J = 6.5 Hz), 3.95 (t,2H, J = 6.5 Hz), 3.91 (t,2H, J = 6.5 Hz), 2.58-2.70 (m,4H), 2.31-2.33 (m,8H), 1.66-1.81 (m,18H), 1.25-1.59 (m,22H).

(Synthesis Example 12) Synthesis of Polymerizable compound 12 (Still another example of the compound represented by formula (III-1))

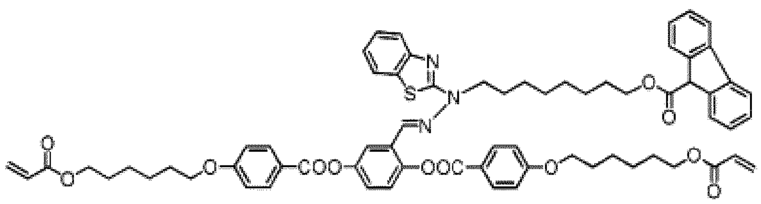

・・・Polymerizable compound 12

<Step 1: Synthesis of Intermediate Z>

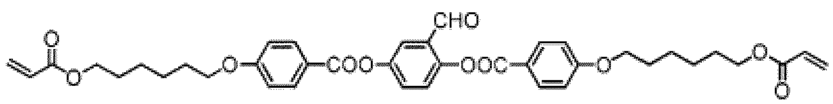

・・・Intermediate Z

20 g (144.8 mmol) of 2,5-dihydroxybenzaldehyde, 105.8 g (362.0 mmol) of 4-(6-acryloylhex-1-yloxy)benzoic acid (manufactured by DKSH Management Ltd.) and 5.3 g (43.4 mmol) of N-N-dimethylaminopyridine (N-N-dimethyl-4-aminopyridine)) were dissolved in 200 mL of N-methylpyrrolidone (N-methyl-2-pyrrolidone) in a four-necked reactor equipped with a thermometer under a nitrogen stream. 83.3 g (434.4 mmol) of 1-ethyl-3-(3-dimethylaminopropyl)carbodiimide hydrochloride (WSC) was added to the solution, and the solution was stirred at room temperature for 12 hours. After completion of the reaction, the reaction solution was charged in 1.5 liters of water, followed by extraction with 500 mL of ethyl acetate. The ethyl acetate layer was dried with anhydrous sodium sulfate. After the sodium sulfate was filtered off, and ethyl acetate was evaporated under reduced pressure using a rotary evaporator to obtain a light yellow solid. The light yellow solid was purified by silica gel column chromatography (toluene:ethyl acetate = 9:1) to obtain 75 g of Intermediate Z as a white solid (yield: 75.4%). The structure of Intermediate Z was identified by $^1$H-NMR. The $^1$H-NMR spectral data is shown below.

$^1$H-NMR (500 MHz, $CDCl_3$, TMS, δppm):10.20 (s, 1H), 8.18-8.12 (m, 4H), 7.78 (d, 1H, J = 2.8 Hz), 7.52 (dd, 1H, J = 2.8 Hz, 8.7 Hz), 7.38 (d, 1H, J = 8.7 Hz), 7.00-6.96 (m, 4H), 6.40 (dd, 2H, J = 1.4 Hz, 17.4 Hz), 6.12 (dd, 2H, J = 10.6 Hz, 17.4 Hz), 5.82 (dd, 2H, J = 1.4 Hz, 10.6 Hz), 4.18 (t, 4H, J = 6.4 Hz), 4.08-4.04 (m, 4H), 1.88-1.81 (m, 4H), 1.76-1.69 (m, 4H), 1.58-1.42 (m, 8H).

<Step 2: Synthesis of Intermediate A1>

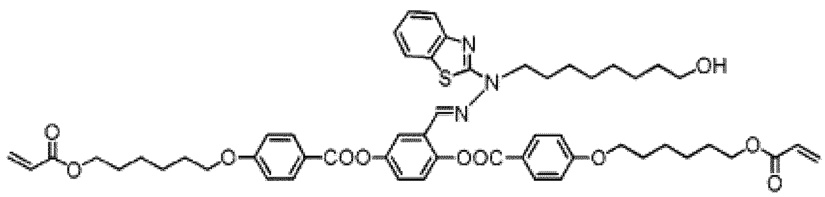

・・・Intermediate A1

6.00 g (8.74 mmol) of Intermediate Z synthesized in Step 1, and, 3.33 g (11.36 mmol) of Intermediate Q synthesized in Step 1 of the Synthesis Example 8 were dissolved in 12.0 mL of ethanol and 120 mL of THF in a three-necked reactor equipped with a thermometer under a nitrogen stream. 0.41 g (1.75 mmol) of (±)-10-camphorsulfonic acid was added to the solution, and the solution was stirred at 50°C for 4 hours. After completion of the reaction, 200 mL of distilled water was charged into the reaction solution, followed with extraction twice with 200 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 7.13 g of Intermediate A1 as a yellow solid. The yield was 84.8 mol%. The structure of Intermediate A1 was identified by $^1$H-NMR. The $^1$H-NMR spectral data is shown below.

$^1$H-NMR (500 MHz, $CDCl_3$, TMS, δppm):8.18-8.21 (m,4H), 7.90 (d,2H, J = 2.5 Hz), 7.76 (s,1H), 7.61-7.64 (m,2H), 7.29-7.32 (m,1H), 7.25-7.28 (m,1H), 7.13 (ddd,1H, J = 0.5 Hz,7.5 Hz,8.0 Hz), 6.99-7.03 (m,4H), 6.418 (dd,1H, J = 1.0 Hz,17.5 Hz), 6.416 (dd,1H, J = 1.0 Hz,17.5 Hz), 6.14 (dd,2H, J = 10.5 Hz,17.5 Hz), 5.837 (dd,1H, J = 1.0 Hz,10.5 Hz), 5.835 (dd,1H, J = 1.0 Hz,10.5 Hz), 4.18-4.21 (m,6H), 4.079 (t,2H, J = 6.5 Hz), 4.074 (t,2H, J = 6.5 Hz), 3.58-3.62 (m,2H), 1.87 (tt,4H, J = 6.5 Hz,6.5 Hz), 1.74 (tt,4H, J = 7.0 Hz,7.0 Hz), 1.46-1.62 (m,12H), 1.36 (br,1H), 1.25-1.60 (m,8H).

<Step 3: Synthesis of Polymerizable compound 12 (Still another example of the compound represented by formula (III-1))>

6.00 g (6.24 mmol) of Intermediate A1 synthesized in Step 2 and 120 mL of chloroform was charged in a three-necked reactor equipped with a thermometer, under a nitrogen stream to prepare a uniform solution. 1.57 g (7.48 mmol) of 9-fluorenecarboxylic acid was added to the solution. Next, 0.183 g (1.50 mmol) of N-N-dimethyl-4-aminopyridine was added. Next, after 1.04 g (8.23 mmol) of N-N'-diisopropylcarbodiimide was added to the reaction solution over 5 minutes while maintaining the temperature of the reaction solution at 20 to 30°C, the solution was further stirred at 25°C for 4 hours. After completion of the reaction, 250 mL of saturated saline solution was added to the reaction solution, followed by extraction twice with 250 mL of chloroform. The organic layer was collected, dried with anhydrous sodium sulfate, and the sodium sulfate was filtered off. After the solvent was evaporated from the filtrate using a rotary evaporator, the obtained residue was purified by silica gel column chromatography (chloroform:THF = 95:5) to obtain 6.07g of polymerizable compound 12 as a yellow solid. The yield was 84.3 mol%. The structure of the target product (polymerizable compound 12) was identified by $^1$H-NMR. The $^1$H-NMR spectral data is shown below.

$^1$H-NMR (500 MHz, $CDCl_3$, TMS, δppm):8.15-8.21 (m,4H), 7.90 (dd,1H, J = 1.0 Hz,2.0 Hz), 7.74-7.76 (m,3H), 7.61-7.65 (m,4H), 7.39-7.43 (m,3H), 7.25-7.35 (m,4H), 7.12 (ddd,1H, J = 1.0 Hz,7.0 Hz,8.0 Hz), 6.99-7.02 (m,2H), 6.94-6.96 (m,2H), 6.412 (dd,1H, J = 1.5 Hz,17.5 Hz), 6.409 (dd,1H, J = 1.5 Hz,17.5 Hz), 6.137 (dd,1H, J = 10.5 Hz,17.5 Hz), 6.123 (dd,1H, J = 10.5 Hz,17.5 Hz), 5.834 (dd,1H, J = 1.5 Hz,10.5 Hz), 5.828 (dd,1H, J = 1.5 Hz,10.5 Hz), 4.85 (s,1H), 4.15-4.21 (m,6H), 4.07-4.12 (m,4H), 3.98 (t,2H, J = 6.5 Hz), 1.87 (tt,2H, J = 6.5 Hz,6.5 Hz), 1.67-1.81 (m,6H),

1.40-1.61 (m,12H), 1.10-1.19 (m,8H).

(Synthesis Example 13) Synthesis of Polymerizable compound 13 (Still another example of the compound represented by formula (III-1))

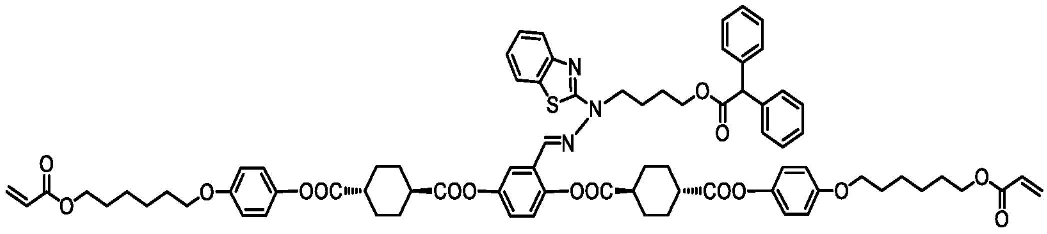

...Polymerizable compound 13

<Step 1: Synthesis of Polymerizable compound 13 (Still another example of the compound represented by formula (III-1))>

6.00 g (5.18 mmol) of Intermediate T synthesized in Step 2 of the Synthesis Example 9 and 120 mL of chloroform were charged in a three-necked reactor equipped with a thermometer, under a nitrogen stream to prepare a uniform solution. 1.32 g (6.22 mmol) of diphenyl acetic acid was added to the solution. Next, 0.152 g (1.24 mmol) of N-N-dimethyl-4-aminopyridine was added. Next, after 0.863 g (6.84 mmol) of N-N'-diisopropylcarbodiimide was added to the reaction solution over 5 minutes while maintaining the temperature of the reaction solution at 20 to 30°C, the solution was further stirred at 25°C for 4 hours. After completion of the reaction, 250 mL of saturated saline solution was added to the reaction solution, followed by extraction twice with 250 mL of chloroform. The organic layer was collected, dried with anhydrous sodium sulfate, and the sodium sulfate was filtered off. After the solvent was evaporated from the filtrate using a rotary evaporator, the obtained residue was purified by silica gel column chromatography (chloroform:THF = 95:5) to obtain 6.28 g of polymerizable compound 13 as a yellow solid. The yield was 89.7 mol%. The structure of the target product (polymerizable compound 13) was identified by $^1$H-NMR. The $^1$H-NMR spectral data is shown below.

$^1$H-NMR (500 MHz, $CDCl_3$, TMS, δppm):7.74 (dd,1H, J = 1.5 Hz,1.5 Hz), 7.69 (dd,1H, J = 0.5 Hz,7.5 Hz), 7.65 (d,1H, J = 8.0 Hz), 7.60 (s,1H), 7.35 (ddd,1H, J = 1.0 Hz,7.0 Hz,8.0 Hz), 7.22-7.28 (m,8H), 7.16-7.20 (m,3H), 7.10-7.14 (m,2H), 6.96-7.00 (m,4H), 6.86-6.90 (m,4H), 6.404 (dd,1H, J = 1.5 Hz,17.5 Hz), 6.403 (dd,1H, J = 1.5 Hz,17.5 Hz), 6.126 (dd,1H, J = 10.5 Hz,17.5 Hz), 6.125 (dd,1H, J = 10.5 Hz,17.5 Hz), 5.822 (dd,1H, J = 1.5 Hz,10.5 Hz), 5.820 (dd,1H, J = 1.5 Hz,10.5 Hz), 5.01 (s,1H), 4.28 (t,2H, J = 7.0 Hz), 4.24 (t,2H, J = 6.0 Hz), 4.18 (t,4H, J = 7.0 Hz), 3.946 (t,2H, J = 6.0 Hz), 3.945 (t,2H, J = 6.0 Hz), 2.55-2.67 (m,4H), 2.24-2.36 (m,8H), 1.62-1.83 (m,20H), 1.42-1.55 (m,8H).

(Synthesis Example 14) Synthesis of Polymerizable compound 14 (Still another example of the compound represented by formula (III-1))

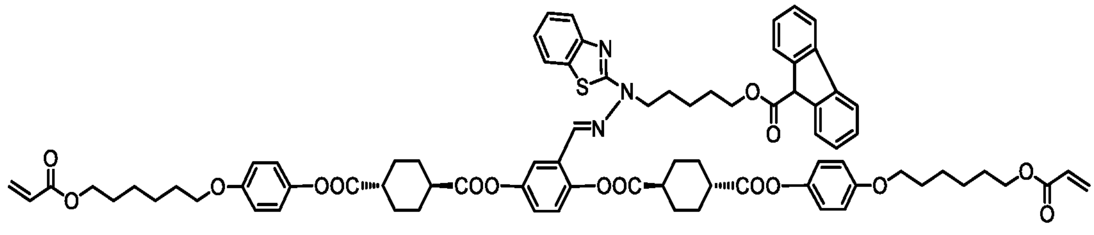

...Polymerizable compound 14

<Step 1: Synthesis of Intermediate D1 (Still another example of the compound represented by formula (IV))>

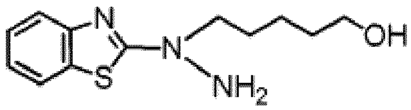

• • • Intermediate D1

6.00 g (36.32 mmol) of 2-hydrazinobenzothiazole was dissolved in 60.0 mL of N-N-dimethylformamide in a three-necked reactor equipped with a thermometer under a nitrogen stream. 23.67 g (72.63 mmol) of cesium carbonate and 5.34 g (43.58 mmol) of 5-chloro-1-pentanol were added to the solution, and the solution was stirred at 25°C for 14 hours. After completion of the reaction, the reaction solution was charged with 250 mL of distilled water, followed by extraction twice with 250 mL of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 40:60 (volume ratio)) to obtain 4.77 g of Intermediate D1 as a gray solid. The yield was 52.3 mol%. The structure of Intermediate D1 was identified by $^1$H-NMR. The $^1$H-NMR spectral data is shown below.

$^1$H-NMR (500 MHz, $CDCl_3$, TMS, δppm):7.60 (dd,1H, J = 1.0 Hz,7.5 Hz), 7.53 (dd,1H, J = 0.5 Hz,8.0 Hz), 7.28 (ddd,1H, J = 1.5 Hz,7.5 Hz,7.5 Hz), 7.07 (ddd,1H, J = 1.0 Hz,7.5 Hz,8.0 Hz), 4.25 (s,1H), 3.78 (t,2H, J = 7.5 Hz), 3.67 (t,2H, J = 6.5 Hz), 1.79 (tt,2H, J = 7.5 Hz,7.5 Hz), 1.63-1.69 (m,4H), 1.46-1.52 (m,2H).

<Step 2: Synthesis of Intermediate E1>

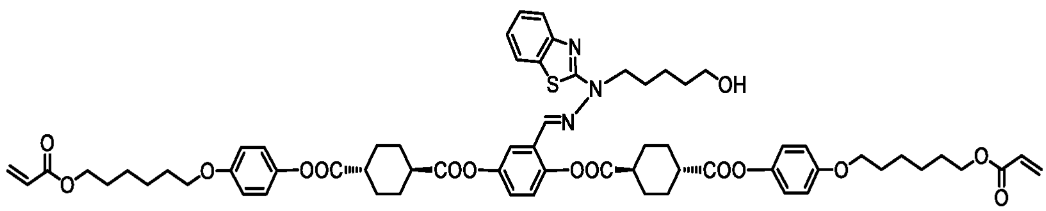

...Intermediate E1

2.09 g (8.31 mmol) of Intermediate D1 synthesized in Step 1, and, 6.00 g (6.39 mmol) of Intermediate B synthesized in the Synthesis Step 2 of Synthesis Example 1 were dissolved in 12.0 mL of ethanol and 120 mL of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 mL of distilled water was charged into the reaction solution, followed with extraction twice with 200 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 6.13 g of Intermediate E1 as a yellow solid. The yield was 81.9 mol%. The structure of Intermediate E1 was identified by $^1$H-NMR. The $^1$H-NMR spectral data is shown below.

$^1$H-NMR (500 MHz, $CDCl_3$, TMS, δppm): 7.75 (d,1H, J = 2.5 Hz), 7.66-7.69 (m,3H), 7.34 (ddd,1H, J = 1.0 Hz,7.5 Hz,7.5 Hz), 7.17 (ddd,1H, J = 1.0 Hz,7.5 Hz,8.0 Hz), 7.09-7.14 (m,2H), 6.96-7.00 (m,4H), 6.87-6.90 (m,4H), 6.40 (dd,2H, J = 1.5 Hz,17.5 Hz), 6.13 (dd,2H, J = 10.5 Hz,17.5 Hz), 5.82 (dd,2H, J = 1.5 Hz,10.5 Hz), 4.33 (t,2H, J = 7.5 Hz), 4.18 (t,4H, J = 7.0 Hz), 3.946 (t,2H, J = 6.5 Hz), 3.945 (t,2H, J = 6.5 Hz), 3.67 (t,2H, J = 6.5 Hz), 2.58-2.73 (m,4H), 2.28-2.35 (m,8H), 1.65-1.82 (m,20H), 1.42-1.55 (m,11H).

<Step 3: Synthesis of Polymerizable compound 14 (Still another example of the compound represented by formula (III-1))>

6.00 g (5.12 mmol) of Intermediate E1 synthesized in Step 2 and 120 mL of chloroform was charged in a three-necked reactor equipped with a thermometer, under a nitrogen stream to prepare a uniform solution. 1.29 g (6.14 mmol) of 9-fluorenecarboxylic acid was added to the solution. Next, 0.150 g (1.23 mmol) of N-N-dimethyl-4-aminopyridine was added. Next, after 0.853 g (6.76 mmol) of N-N'-diisopropylcarbodiimide was added to the reaction solution over 5 minutes while maintaining the temperature of the reaction solution at 20 to 30°C, the solution was further stirred at 25°C for 4 hours. After completion of the reaction, 250 mL of saturated saline solution was added to the reaction solution, followed by extraction twice with 250 mL of chloroform. The organic layer was collected, dried with anhydrous sodium sulfate,

and the sodium sulfate was filtered off. After the solvent was evaporated from the filtrate using a rotary evaporator, the obtained residue was purified by silica gel column chromatography (chloroform:THF = 95:5) to obtain 5.30 g of polymerizable compound 14 as a yellow solid. The yield was 75.9 mol%. The structure of the target product (polymerizable compound 14) was identified by $^1$H-NMR. The $^1$H-NMR spectral data is shown below.

$^1$H-NMR (500 MHz, $CDCl_3$, TMS, δppm): 7.75 (dd,1H, J = 1.5 Hz,1.5 Hz), 7.60-7.71 (m,7H), 7.33-7.38 (m,3H), 7.27-7.30 (m,2H), 7.18 (ddd,1H, J = 1.0 Hz,7.5 Hz,7.5 Hz), 7.10-7.13 (m,2H), 6.97-7.00 (m,2H), 6.92-6.95 (m,2H), 6.84-6.90 (m,4H), 6.40 (dd,2H, J = 1.5 Hz,17.5 Hz), 6.13 (dd,2H, J = 10.5 Hz,17.5 Hz), 5.82 (dd,2H, J = 1.5 Hz,10.5 Hz), 4.82 (s,1H), 4.30 (t,2H, J = 7.0 Hz), 4.15-4.19 (m,6H), 3.95 (t,2H, J = 6.5 Hz), 3.93 (t,2H, J = 6.5 Hz), 2.55-2.70 (m,4H), 2.20-2.35 (m,8H), 1.60-1.82 (m,20H), 1.42-1.55 (m,10H).

(Synthesis Example 15) Synthesis of Polymerizable compound 15 (Still another example of the compound represented by formula (III-1))

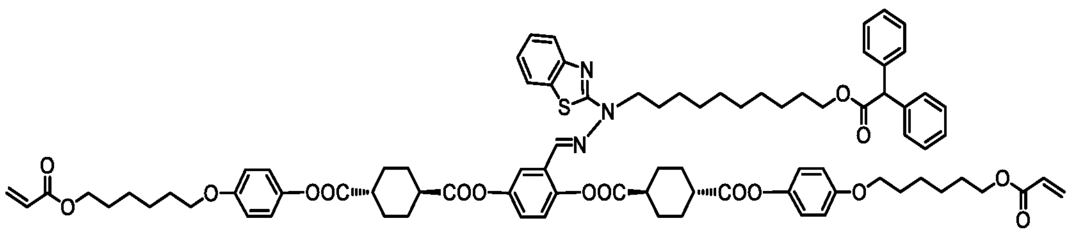

...Polymerizable compound 15

<Step 1: Synthesis of Polymerizable compound 15 (Still another example of the compound represented by formula (III-1))>

6.00 g (4.83 mmol) of Intermediate Y in Step 2 of the Synthesis Example 11 and 120 mL of chloroform were charged in a three-necked reactor equipped with a thermometer, under a nitrogen stream to prepare a uniform solution. 1.23 g (5.80 mmol) of diphenyl acetic acid was added to the solution Next, 0.142 g (1.16 mmol) of N-N-dimethyl-4-aminopyridine was added. Next, after 0.805 g (6.38 mmol) of N-N'-diisopropylcarbodiimide was added to the reaction solution over 5 minutes while maintaining the temperature of the reaction solution at 20 to 30°C, the solution was further stirred at 25°C for 4 hours. After completion of the reaction, 250 mL of saturated saline solution was added to the reaction solution, followed by extraction twice with 250 mL of chloroform. The organic layer was collected, dried with anhydrous sodium sulfate, and the sodium sulfate was filtered off. After the solvent was evaporated from the filtrate using a rotary evaporator, the obtained residue was purified by silica gel column chromatography (chloroform:THF = 95:5) to obtain 6.18 g of polymerizable compound 15 as a yellow solid. The yield was 89.0 mol%. The structure of the target product (polymerizable compound 15) was identified by $^1$H-NMR. The $^1$H-NMR spectral data is shown below.

$^1$H-NMR (500 MHz, $CDCl_3$, TMS, δppm): 7.75 (dd,1H, J = 0.5 Hz,2.0 Hz), 7.66-7.69 (m,3H), 7.27-7.35 (m,8H), 2.25-7.26 (m,3H), 7.16 (ddd,1H, J = 1.0 Hz,8.0 Hz,8.0 Hz), 7.09-7.13 (m,2H), 6.95-7.00 (m,4H), 6.85-6.90 (m,4H), 6.404 (dd,1H, J = 1.5 Hz,17.5 Hz), 6.402 (dd,1H, J = 1.5 Hz,17.5 Hz), 6.127 (dd,1H, J = 10.5 Hz,17.5 Hz), 6.125 (dd,1H, J = 10.5 Hz,17.5 Hz), 5.823 (dd,1H, J = 1.5 Hz,10.5 Hz), 5.820 (dd,1H, J = 1.5 Hz,10.5 Hz), 4.99 (s,1H), 4.30 (t,2H, J = 7.5 Hz), 4.18 (t,2H, J = 6.5 Hz), 4.17 (t,2H, J = 6.5 Hz), 4.09 (t,2H, J = 6.5 Hz), 3.95 (t,2H, J = 6.5 Hz), 3.93 (t,2H, J = 6.5 Hz), 2.58-2.70 (m,4H), 2.31-2.34 (m,8H), 1.68-1.81 (m,18H), 1.35-1.55 (m,14H), 1.21-1.26 (m,8H).

(Synthesis Example 16) Synthesis of Polymerizable compound 16 (Still another example of the compound represented by formula (III-1))

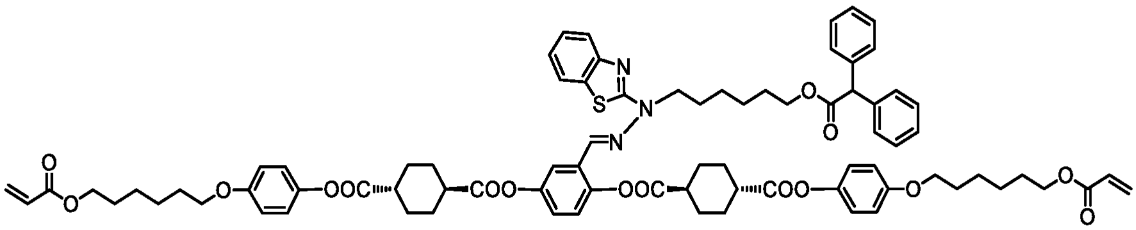

...Polymerizable compound 16

<Step 1: Synthesis of Polymerizable compound 16 (Still another example of the compound represented by formula (III-1))>

6.00 g (5.06 mmol) of Intermediate V synthesized in Step 2 of the Synthesis Example 10 and 120 mL of chloroform were charged in a three-necked reactor equipped with a thermometer, under a nitrogen stream to prepare a uniform solution. 1.29 g (6.07 mmol) of diphenyl acetic acid was added thereto. Next, 0.148 g (1.21 mmol) of N-N-dimethyl-4-aminopyridine was added. Next, after 0.842 g (6.68 mmol) of N-N'-diisopropylcarbodiimide was added to the reaction solution over 5 minutes while maintaining the temperature of the reaction solution at 20 to 30°C, the solution was further stirred at 25°C for 4 hours. After completion of the reaction, 250 mL of saturated saline solution was added to the reaction solution, followed by extraction twice with 250 mL of chloroform. The organic layer was collected, dried with anhydrous sodium sulfate, and the sodium sulfate was filtered off. After the solvent was evaporated from the filtrate using a rotary evaporator, the obtained residue was purified by silica gel column chromatography (chloroform:THF = 95:5) to obtain 6.14 g of polymerizable compound 16 as a yellow solid. The yield was 87.9 mol%. The structure of the target product (polymerizable compound 16) was identified by $^1$H-NMR. The $^1$H-NMR spectral data is shown below.

$^1$H-NMR (500 MHz, $CDCl_3$, TMS, δppm): 7.75 (dd,1H, J = 0.5 Hz,2.0 Hz), 7.69 (dd,1H, J = 0.5 Hz,8.0 Hz), 7.67 (d,1H, J = 8.0 Hz), 7.64 (s,1H), 7.34 (ddd,1H, J = 1.0 Hz,8.0 Hz,8.0 Hz), 7.27-7.30 (m,7H), 7.16-7.24 (m,4H), 7.09-7.13 (m,2H), 6.96-7.00 (m,4H), 6.86-6.90 (m,4H), 6.404 (dd,1H, J = 1.5 Hz,17.5 Hz), 6.402 (dd,1H, J = 1.5 Hz,17.5 Hz), 6.127 (dd,1H, J = 10.5 Hz,17.5 Hz), 6.126 (dd,1H, J = 10.5 Hz,17.5 Hz), 5.823 (dd,1H, J = 1.5 Hz,10.5 Hz), 5.821 (dd,1H, J = 1.5 Hz,10.5 Hz), 4.99 (s,1H), 4.26 (t,2H, J = 7.5 Hz), 4.18 (t,4H, J = 6.5 Hz), 4.14 (t,2H, J = 6.5 Hz), 3.95 (t,2H, J = 6.5 Hz), 3.94 (t,2H, J = 6.5 Hz), 2.58-2.67 (m,4H), 2.29-2.35 (m,8H), 1.59-1.82 (m,20H), 1.27-1.53 (m,12H).

(Synthesis Example 17) Synthesis of Polymerizable compound 17 (Still another example of the compound represented by formula (III-1))

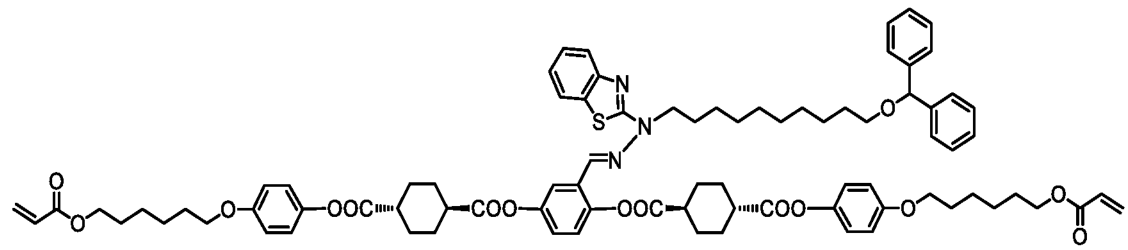

...Polymerizable compound 17

<Step 1: Synthesis of Intermediate F1>

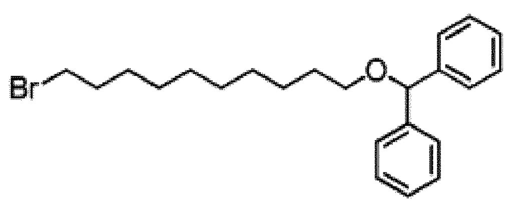

・・・Intermediate F1

10.00 g (42.16 mmol) of 10-bromo-1-decanol and 100 ml of toluene were placed in a three-necked reactor equipped with a thermometer, under a nitrogen stream to prepare a uniform solution. 8.86 g (42.16 mmol) of benzhydrol was added to the solution. Next, 1.06 g (8.43 mmol) of (±)-10-camphorsulfonic acid was added, and the solution was stirred at 110°C for 5 hours. After completion of the reaction, 250 mL of saturated saline solution was added to the reaction solution, followed by extraction twice with 250 mL of ethyl acetate. The organic layer was collected, dried with anhydrous sodium sulfate, and the sodium sulfate was filtered off. After the solvent was evaporated from the filtrate using a rotary evaporator, the obtained residue was purified by silica gel column chromatography (ethyl acetate:hexane = 1:20 (volume ratio)) to obtain 10.93 g of Intermediate F1 as a colorless oil. The yield was 64.3 mol%. The structure of Intermediate F1 was identified by $^1$H-NMR. The $^1$H-NMR spectral data is shown below.

$^1$H-NMR (500 MHz, $CDCl_3$, TMS, δppm):7.29-7.37 (m,8H), 7.22-7.27 (m,2H), 5.33 (s,1H), 3.44 (t,2H, J = 6.5 Hz), 3.40 (t,2H, J = 6.5 Hz), 1.85 (tt,2H, J = 7.0 Hz,7.0 Hz), 1.64 (tt,2H, J = 7.0 Hz,7.0 Hz), 1.36-1.43 (m,4H), 1.25-1.28 (m,8H).

<Step 2: Synthesis of Intermediate G1 (Still another example of the compound represented by formula (IV))>

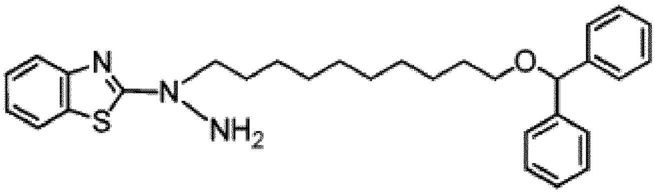

・・・Intermediate G1

3.00 g (18.16 mmol) of 2-hydrazinobenzothiazole was dissolved in 30.0 mL of N-N-dimethylformamide in a three-necked reactor equipped with a thermometer under a nitrogen stream. 11.83 g (36.32 mmol) of cesium carbonate and 8.79 g (21.79 mmol) of Intermediate F1 synthesized in Step 1 were added to the solution, and the solution was stirred at 25°C for 14 hours. After completion of the reaction, the reaction solution was charged with 250 mL of distilled water, followed by extraction twice with 250 mL of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 70:30 (volume ratio)) to obtain 5.20 g of Intermediate G1 as a yellow oil. The yield was 58.7 mol%. The structure of Intermediate G1 was identified by $^1$H-NMR. The $^1$H-NMR spectral data is shown below.

$^1$H-NMR (500 MHz, $CDCl_3$, TMS, δppm):7.59 (dd,1H, J = 0.5 Hz,8.0 Hz), 7.53 (dd,1H, J = 0.5 Hz,8.0 Hz), 7.21-7.35 (m,11H), 7.06 (ddd,1H, J = 1.0 Hz,8.0 Hz,8.0 Hz), 5.32 (s,1H), 4.21 (br,2H), 3.74 (t,2H, J = 7.5 Hz), 3.45 (t,2H, J = 6.5 Hz), 1.72 (tt,2H, J = 7.5 Hz,7.5 Hz), 1.63 (tt,2H, J = 6.5 Hz,6.5 Hz), 1.27-1.43 (m,12H).

<Step 3: Synthesis of Polymerizable compound 17 (Still another example of the compound represented by formula (III-1))>

4.05 g (8.31 mmol) of Intermediate G1 synthesized in Step 2, and, 6.00 g (6.39 mmol) of Intermediate B synthesized in the Synthesis Step 2 of Synthesis Example 1 were dissolved in 12.0 mL of ethanol and 120 mL of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 mL of distilled water was charged into the reaction solution, followed with extraction twice with 200 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 6.43 g of polymerizable compound 17 as a yellow solid. The yield was 71.5 mol%. The structure of the target product (polymerizable compound 17) was identified by $^1$H-NMR. The $^1$H-NMR spectral data is shown below.

$^1$H-NMR (500 MHz, $CDCl_3$, TMS, δppm): 7.75 (dd,1H, J = 0.5 Hz,2.0 Hz), 7.66-7.69 (m,3H), 7.28-7.35 (m,9H), 7.20-7.22 (m,2H), 7.16 (ddd,1H, J = 1.0 Hz,8.0 Hz,8.0 Hz), 7.09-7.13 (m,2H), 6.95-7.00 (m,4H), 6.85-6.90 (m,4H), 6.404 (dd,1H, J = 1.0 Hz,17.5 Hz), 6.403 (dd,1H, J = 1.0 Hz,17.5 Hz), 6.127 (dd,1H, J = 10.5 Hz,17.5 Hz), 6.125 (dd,1H, J = 10.5 Hz,17.5 Hz), 5.822 (dd,1H, J = 1.0 Hz,10.5 Hz), 5.820 (dd,1H, J = 1.0 Hz,10.5 Hz), 5.30 (s,1H), 4.30 (t,2H, J = 7.5 Hz), 4.18 (t,2H, J = 6.5 Hz), 4.17 (t,2H, J = 6.5 Hz), 3.94 (t,2H, J = 6.5 Hz), 3.93 (t,2H, J = 6.5 Hz), 3.40 (t,2H, J = 6.5 Hz), 2.58-2.71 (m,4H), 2.31-2.35 (m,8H), 1.65-1.81 (m,18H), 1.27-1.61 (m,22H).

(Synthesis Example 18) Synthesis of Polymerizable compound 18 (Still another example of the compound represented by formula (III-1))

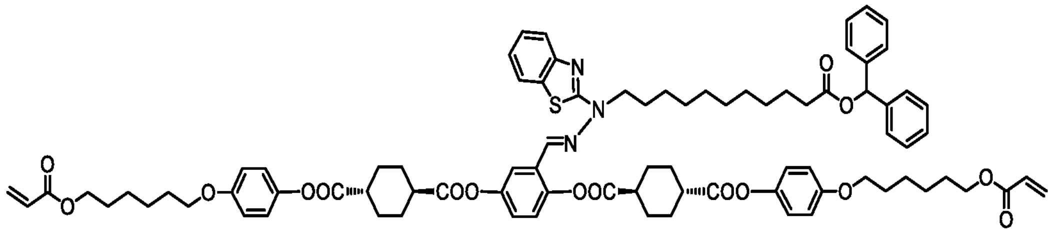

...Polymerizable compound 18

<Step 1: Synthesis of Intermediate H1>

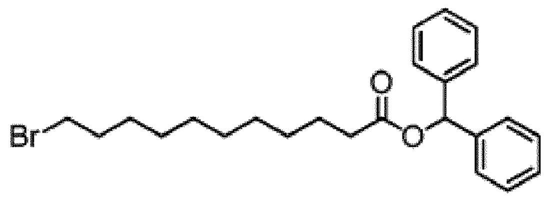

・・・Intermediate H1

9.00 g (33.94 mmol) of 11-bromoundecanoic acid and 90.0 mL of N-methylpyrrolidone (N-methyl-2-pyrrolidone) were placed in a three-necked reactor equipped with a thermometer, under a nitrogen stream to prepare a uniform solution. 6.25 g (33.94 mmol) of benzhydrol and 0.829 g (6.79 mmol) of N-N-dimethylaminopyridine (N-N-dimethyl-4-aminopyridine) were added thereto. 7.81 g (40.73 mmol) of 1-ethyl-3-(3-dimethylaminopropyl)carbodiimide hydrochloride was added thereto while gently stirring at 25°C. After completion of the reaction, 500 mL of saturated saline solution was added to the reaction solution, followed by extraction twice with 500 ml of ethyl acetate. The organic layer was collected, dried with anhydrous sodium sulfate, and the sodium sulfate was filtered off. After the solvent was evaporated from the filtrate using a rotary evaporator, the obtained residue was purified by silica gel column chromatography (ethyl acetate:hexane = 1:20 (volume ratio)) to obtain 9.21 g of Intermediate H1 as a colorless oil. The yield was 62.9 mol%. The structure of Intermediate H1 was identified by $^1$H-NMR. The $^1$H-NMR spectral data is shown below.

$^1$H-NMR (500 MHz, $CDCl_3$, TMS, δppm):7.24-7.35 (m,10H), 6.89 (s,1H), 3.51 (t,1H, J = 7.0 Hz), 3.39 (t,1H, J = 7.0 Hz), 2.41 (t,2H, J = 7.5 Hz), 1.83 (tt,1H, J = 7.0 Hz,7.5 Hz), 1.75 (tt,1H, J = 7.0 Hz,7.5 Hz), 1.65 (tt,2H, J = 7.0 Hz,7.5 Hz), 1.40 (tt,2H, J = 7.0 Hz,7.5 Hz), 1.24-1.26 (m,10H).

<Step 2: Synthesis of Intermediate I1 (Still another example of the compound represented by formula (IV))>

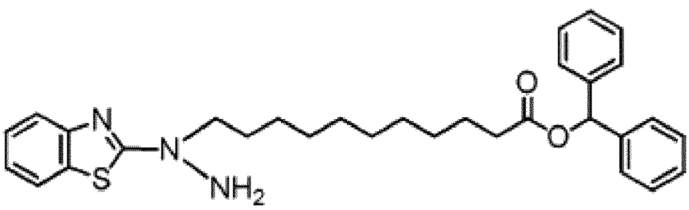

・・・Intermediate I1

2.50 g (15.13 mmol) of 2-hydrazinobenzothiazole was dissolved in 25.0 mL of N-N-dimethylformamide in a three-necked reactor equipped with a thermometer under a nitrogen stream. 9.86 g (30.26 mmol) of cesium carbonate and 7.83 g (18.16 mmol) of Intermediate H1 synthesized in Step 1 were added to the solution, and the solution was stirred at 25°C for 14 hours. After completion of the reaction, the reaction solution was charged with 250 mL of distilled

water, followed by extraction twice with 250 mL of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 70:30 (volume ratio)) to obtain 4.49 g of Intermediate I1 as a red oil. The yield was 57.5 mol%. The structure of Intermediate I1 was identified by $^1$H-NMR. The $^1$H-NMR spectral data is shown below.

$^1$H-NMR (500 MHz, $CDCl_3$, TMS, δppm):7.59 (dd,1H, J = 1.0 Hz,8.0 Hz), 7.53 (dd,1H, J = 0.5 Hz,8.0 Hz), 7.25-7.35 (m,11H), 7.06 (ddd,1H, J = 1.0 Hz,8.0 Hz,8.0 Hz), 6.88 (s,1H), 4.22 (br,2H), 3.74 (t,2H, J = 7.5 Hz), 2.41 (t,2H, J = 7.5 Hz), 1.72 (tt,2H, J = 7.5 Hz,7.5 Hz), 1.61-1.66 (m,2H, J = 5 Hz), 1.25-1.40 (m,12H).

<Step 3: Synthesis of Polymerizable compound 18 (Still another example of the compound represented by formula (III-1))>

4.28 g (8.31 mmol) of Intermediate I1 synthesized in Step 2, and, 6.00 g (6.39 mmol) of Intermediate B synthesized in the Synthesis Step 2 of Synthesis Example 1 were dissolved in 12.0 mL of ethanol and 120 mL of THF in a three-necked reactor equipped with a thermometer under a nitrogen stream. 0.30 g (1.28 mmol) of (±)-10-camphorsulfonic acid was added to the solution, and the solution was stirred at 50°C for 4 hours. After completion of the reaction, 200 mL of distilled water was charged into the reaction solution, followed with extraction twice with 200 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 6.43 g of polymerizable compound 18 as a yellow solid. The yield was 70.1 mol%. The structure of the target product (polymerizable compound 18) was identified by $^1$H-NMR. The $^1$H-NMR spectral data is shown below.

$^1$H-NMR (500 MHz, $CDCl_3$, TMS, δppm): 7.75 (d,1H, J = 2.0 Hz), 7.66-7.69 (m,3H), 7.25-7.35 (m,11H), 7.16 (ddd,1H, J = 1.0 Hz,7.5 Hz,7.5 Hz), 7.09-7.13 (m,2H), 6.95-7.00 (m,4H), 6.85-6.90 (m,5H), 6.404 (dd,1H, J = 1.5 Hz,17.5 Hz), 6.403 (dd,1H, J = 1.5 Hz,17.5 Hz), 6.127 (dd,1H, J = 10.5 Hz,17.5 Hz), 6.125 (dd,1H, J = 10.5 Hz,17.5 Hz), 5.823 (dd,1H, J = 1.5 Hz,10.5 Hz), 5.820 (dd,1H, J = 1.5 Hz,10.5 Hz), 4.30 (t,2H, J = 7.5 Hz), 4.18 (t,2H, J = 6.5 Hz), 4.17 (t,2H, J = 6.5 Hz), 3.95 (t,2H, J = 6.5 Hz), 3.93 (t,2H, J = 6.5 Hz), 2.59-2.70 (m,4H), 2.31-2.33 (m,8H), 1.64-1.81 (m,18H), 1.24-1.61 (m,24H).

(Synthesis Example 19) Synthesis of Polymerizable compound 19 (Still another example of the compound represented by formula (III-1))

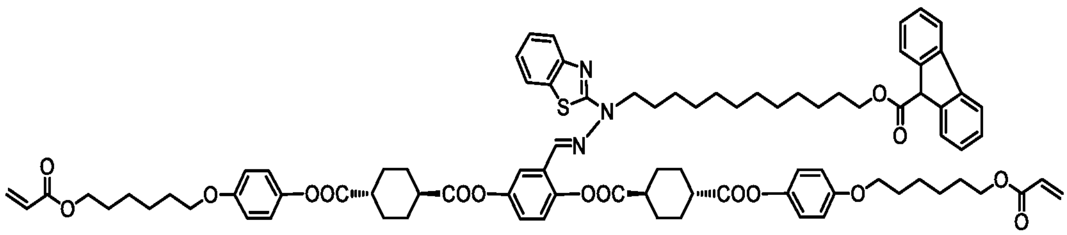

...Polymerizable compound 19

<Step 1: Synthesis of Intermediate J1 (Still another example of the compound represented by formula (IV))>

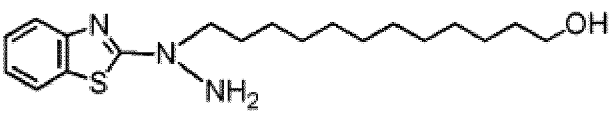

・・・Intermediate J1

6.00 g (36.32 mmol) of 2-hydrazinobenzothiazole was dissolved in 60.0 mL of N-N-dimethylformamide in a three-necked reactor equipped with a thermometer under a nitrogen stream, 23.67 g (72.63 mmol) of cesium carbonate and 11.56 g (43.58 mmol) of 12-bromo-1-dodecanol was added to the solution, and the solution was stirred at 25°C for 14 hours. After completion of the reaction, the reaction solution was charged with 250 mL of distilled water, followed by

extraction twice with 250 mL of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 80:20 (volume ratio)) to obtain 6.34 g of Intermediate J1 as a gray solid. The yield was 50.0 mol%. The structure of Intermediate J1 was identified by $^1$H-NMR. The $^1$H-NMR spectral data is shown below.

$^1$H-NMR (500 MHz, $CDCl_3$, TMS, δppm):7.60 (dd,1H, J = 0.5 Hz,8.0 Hz), 7.53 (dd,1H, J = 0.5 Hz,8.0 Hz), 7.28 (ddd,1H, J = 1.0 Hz,7.5 Hz,8.0 Hz), 7.06 (ddd,1H, J = 1.0 Hz,7.5 Hz,8.0 Hz), 4.23 (br,2H), 3.75 (t,2H, J = 7.5 Hz), 3.64 (br,2H), 1.73 (tt,2H, J = 7.5 Hz,7.5 Hz), 1.50-1.58 (m,2H), 1.27-1.41 (m,17H).

<Step 2: Synthesis of Intermediate K1>

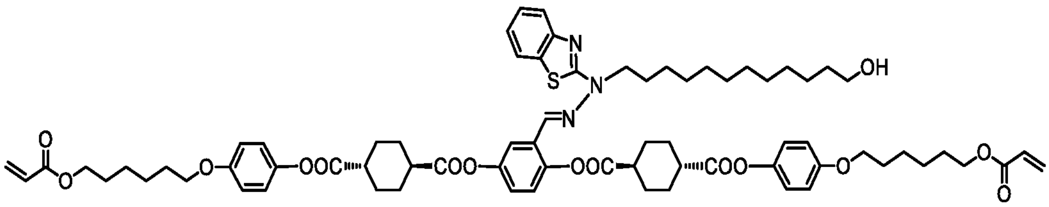

...Intermediate K1

5.81 g (16.61 mmol) of Intermediate J synthesized in Step 1, and, 12.00 g (12.78 mmol) of Intermediate B synthesized in the Synthesis Step 2 of Synthesis Example 1 were dissolved in 24.0 mL of ethanol and 240 mL of THF in a three-necked reactor equipped with a thermometer under a nitrogen stream. 0.59 g (2.56 mmol) of (±)-10-camphorsulfonic acid was added to the solution, and the solution was stirred at 50°C for 4 hours. After completion of the reaction, the reaction solution was charged into 400 mL of distilled water followed by extraction twice with 400 mL of ethyl acetate. After the ethyl acetate layer was dried with anhydrous sodium sulfate, the sodium sulfate was filtered off. The ethyl acetate was removed from the filtrate under reduced pressure using a rotary evaporator to obtain a yellow solid. The yellow solid was purified by silica gel column chromatography (chloroform:THF = 95:5) to obtain 13.36 g of Intermediate K1 as a yellow solid. The yield was 82.3 mol%. The structure of Intermediate K1 was identified by $^1$H-NMR. The $^1$H-NMR spectral data is shown below.

$^1$H-NMR (500 MHz, $CDCl_3$, TMS, δppm): 7.75 (d,1H, J = 2.5 Hz), 7.66-7.70 (m,3H), 7.34 (ddd,1H, J = 1.0 Hz,8.0 Hz,8.0 Hz), 7.17 (ddd,1H, J = 1.0 Hz,8.0 Hz,8.0 Hz), 7.09-7.13 (m,2H), 6.96-7.00 (m,4H), 6.87-6.90 (m,4H), 6.40 (dd,2H, J = 1.5 Hz,17.5 Hz),
6.13 (dd,2H, J = 10.5 Hz,17.5 Hz), 5.82 (dd,2H, J = 1.5 Hz,10.5 Hz), 4.30 (t,2H, J = 7.5 Hz), 4.18 (t,4H, J = 6.5 Hz), 3.95 (t,4H, J = 6.5 Hz), 3.60 (t,2H, J = 6.5 Hz), 2.58-2.70 (m,4H), 2.31-2.35 (m,8H), 1.66-1.83 (m,18H), 1.25-1.58 (m,27H).

<Step 3: Synthesis of Polymerizable compound 19 (Still another example of the compound represented by formula (III-1))>

6.00 g (5.06 mmol) of Intermediate K1 synthesized in Step 2 and 120 mL of chloroform were charged in a three-necked reactor equipped with a thermometer, under a nitrogen stream to prepare a uniform solution. 1.28 g (6.07 mmol) of 9-fluorene carboxylic acid was added to the solution. Next, 0.148 g (1.21 mmol) of N-N-dimethyl-4-aminopyridine was added. Next, after 0.842 g (6.68 mmol) of N-N'-diisopropylcarbodiimide was added to the reaction solution over 5 minutes while maintaining the temperature of the reaction solution at 20 to 30°C, the solution was further stirred at 25°C for 4 hours. After completion of the reaction, 250 mL of saturated saline solution was added to the reaction solution, followed by extraction twice with 250 mL of chloroform. The organic layer was collected, dried with anhydrous sodium sulfate, and the sodium sulfate was filtered off. After the solvent was evaporated from the filtrate using a rotary evaporator, the obtained residue was purified by silica gel column chromatography (chloroform:THF = 95:5) to obtain 5.05 g of polymerizable compound 19 as a yellow solid. The yield was 68.3 mol%. The structure of the target product (polymerizable compound 19) was identified by $^1$H-NMR. The $^1$H-NMR spectral data is shown below.

$^1$H-NMR (500 MHz, $CDCl_3$, TMS, δppm): 7.74-7.75 (m,3H), 7.64-7.69 (m,5H), 7.39-7.42 (m,2H), 7.31-7.35 (m,3H), 7.16 (ddd,1H, J = 1.0 Hz,8.0 Hz,8.0 Hz), 7.09-7.13 (m,2H), 6.94-7.00 (m,4H), 6.84-6.90 (m,4H), 6.404 (dd,1H, J = 1.5 Hz,17.5 Hz), 6.400 (dd,1H, J = 1.5 Hz,17.5 Hz), 6.127 (dd,1H, J = 10.5 Hz,17.5 Hz), 6.122 (dd,1H, J = 10.5 Hz,17.5 Hz), 5.822 (dd,1H, J = 1.5 Hz,10.5 Hz), 5.818 (dd,1H, J = 1.5 Hz,10.5 Hz), 4.85 (s,1H), 4.30 (t,2H, J = 7.0 Hz), 4.18 (t,2H, J = 6.5 Hz), 4.17 (t,2H, J = 6.5 Hz), 4.12 (t,2H, J = 6.5 Hz), 3.95 (t,2H, J = 6.5 Hz), 3.91 (t,2H, J = 6.5 Hz),

2.58-2.70 (m,4H), 2.31-2.33 (m,8H), 1.66-1.78 (m,18H), 1.22-1.63 (m,26H).

(Synthesis Example 20) Synthesis of Polymerizable compound 20 (Still another example of the compound represented by formula (III-1))

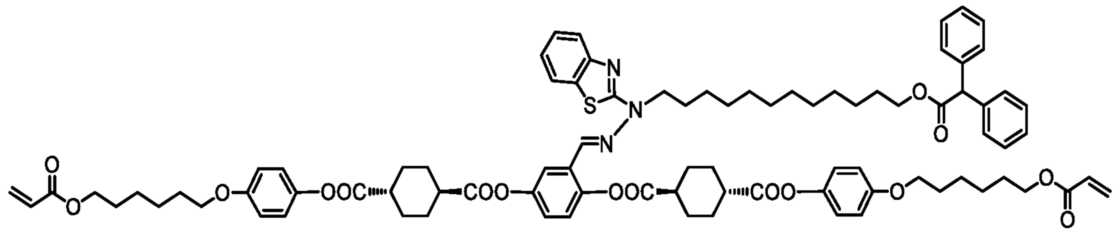

...Polymerizable compound 20

<Step 1: Synthesis of Polymerizable compound 20 (Still another example of the compound represented by formula (III-1))>

6.00 g (5.06 mmol) of Intermediate K1 synthesized in Synthesis Example 19 and 120 mL of chloroform were charged into a three-necked reactor equipped with a thermometer, under a nitrogen stream to prepare a uniform solution. 1.29 g (6.07 mmol) of diphenyl acetic acid was added thereto. Next, 0.148 g (1.21 mmol) of N-N-dimethyl-4-aminopyridine was added. Next, after 0.842 g (6.68 mmol) of N-N'-diisopropylcarbodiimide was added to the reaction solution over 5 minutes while maintaining the temperature of the reaction solution at 20 to 30°C, the solution was further stirred at 25°C for 4 hours. After completion of the reaction, 250 mL of saturated saline solution was added to the reaction solution, followed by extraction twice with 250 mL of chloroform. The organic layer was collected, dried with anhydrous sodium sulfate, and the sodium sulfate was filtered off. After the solvent was evaporated from the filtrate using a rotary evaporator, the obtained residue was purified by silica gel column chromatography (chloroform:THF = 95:5) to obtain 5.95 g of polymerizable compound 20 as a yellow solid. The yield was 80.3 mol%. The structure of the target product (polymerizable compound 20) was identified by $^1$H-NMR. The $^1$H-NMR spectral data is shown below.

$^1$H-NMR (500 MHz, $CDCl_3$, TMS, δppm): 7.75 (dd,1H, J = 0.5 Hz,2.5 Hz), 7.66-7.69 (m,3H), 7.25-7.35 (m,11H), 7.16 (ddd,1H, J = 1.0 Hz,7.5 Hz,7.5 Hz), 7.09-7.13 (m,2H), 6.95-7.00 (m,4H), 6.85-6.90 (m,4H), 6.404 (dd,1H, J = 1.5 Hz,17.5 Hz), 6.402 (dd,1H, J = 1.5 Hz,17.5 Hz), 6.127 (dd,1H, J = 10.5 Hz,17.5 Hz), 6.124 (dd,1H, J = 10.5 Hz, 17.5 Hz), 5.822 (dd,1H, J = 1.5 Hz,10.5 Hz), 5.819 (dd,1H, J = 1.5 Hz,10.5 Hz), 5.00 (s,1H), 4.30 (t,2H, J = 7.5 Hz), 4.18 (t,2H, J = 6.5 Hz), 4.17 (t,2H, J = 6.5 Hz), 4.11 (t,2H, J = 6.5 Hz), 3.95 (t,2H, J = 6.5 Hz), 3.93 (t,2H, J = 6.5 Hz), 2.58-2.70 (m,4H), 2.31-2.33 (m,8H), 1.68-1.81 (m,18H), 1.19-1.58 (m,26H).

(Synthesis Example 21) Synthesis of Polymerizable compound 21 (Still another example of the compound represented by formula (III-1))

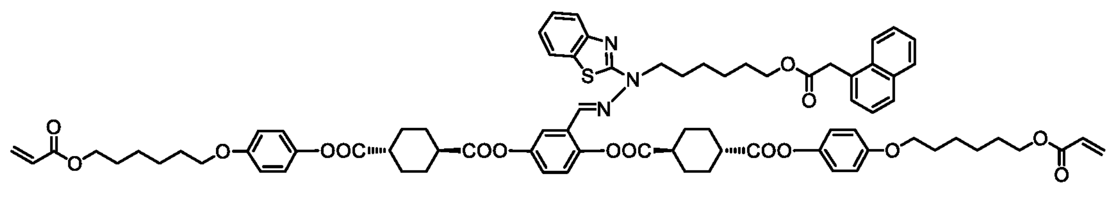

...Polymerizable compound 21

<Step 1: Synthesis of Polymerizable compound 21 (Still another example of the compound represented by formula (III-1))>

6.00 g (5.06 mmol) of Intermediate V synthesized in Step 2 of the Synthesis Example 10 and 120 mL of chloroform were charged in a three-necked reactor equipped with a thermometer, under a nitrogen stream to prepare a uniform solution. 1.13 g (6.07 mmol) of 1-naphthaleneacetic acid was added thereto. Next, 0.148 g (1.21 mmol) of N-N-dimethyl-4-aminopyridine was added. Next, after 0.842 g (6.68 mmol) of N-N'-diisopropylcarbodiimide was added to

the reaction solution over 5 minutes while maintaining the temperature of the reaction solution at 20 to 30°C, the solution was further stirred at 25°C for 6 hours. After completion of the reaction, 250 mL of saturated saline solution was added to the reaction solution, followed by extraction twice with 250 mL of chloroform. The organic layer was collected, dried with anhydrous sodium sulfate, and the sodium sulfate was filtered off. After the solvent was evaporated from the filtrate using a rotary evaporator, the obtained residue was purified by silica gel column chromatography (chloroform:THF = 95:5) to obtain 5.61 g of polymerizable compound 21 as a light yellow solid. The yield was 81.9 mol%. The structure of the target product (polymerizable compound 21) was identified by $^1$H-NMR. The $^1$H-NMR spectral data is shown below.

$^1$H-NMR (500 MHz, $CDCl_3$, TMS, δppm):7.97 (dd,1H, J = 0.5 Hz,8.5 Hz), 7.80 (ddd,1H, J = 0.5 Hz,0.5 Hz,8.0 Hz), 7.73-7.76 (m,2H), 7.67-7.71 (m,2H), 7.61 (s,1H), 7.49 (ddd,1H, J = 1.0 Hz,6.5 Hz,8.5 Hz), 7.42 (ddd,1H, J = 1.5 Hz,7.0 Hz,7.0 Hz), 7.33-7.39 (m,3H), 7.18 (ddd,1H, J = 1.0 Hz,7.5 Hz,8.0 Hz), 7.10-7.14 (m,2H), 6.95-7.01 (m,4H), 6.85-6.90 (m,4H), 6.405 (dd,1H, J = 1.5 Hz,17.5 Hz), 6.402 (dd,1H, J = 1.5 Hz,17.5 Hz), 6.127 (dd,1H, J = 10.5 Hz,17.5 Hz), 6.124 (dd,1H, J = 10.5 Hz,17.5 Hz), 5.822 (dd,1H, J = 1.5 Hz,10.5 Hz), 5.819 (dd,1H, J = 1.5 Hz,10.5 Hz), 4.16-4.22 (m,6H), 4.08 (t,2H, J = 6.5 Hz), 4.03 (s,2H), 3.95 (t,2H, J = 6.5 Hz), 3.93 (t,2H, J = 6.5 Hz), 2.56-2.67 (m,4H), 2.28-2.36 (m,8H), 1.59-1.83 (m,20H), 1.42-1.56 (m,8H), 1.24-1.36 (m,4H).

(Synthesis Example 22) Synthesis of Polymerizable compound 21 (Still another example of the compound represented by formula (III-1))

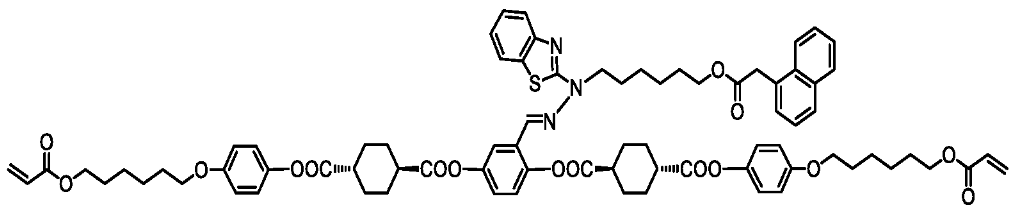

...Polymerizable compound 21

<Step 1: Synthesis of Intermediate L1>

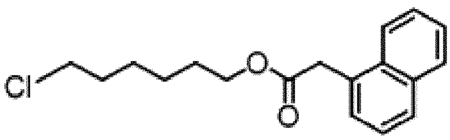

・・・Intermediate L1

50 g (268.5 mmol) of 1-naphthylacetic acid was added to 110 g of toluene in a three-necked reactor equipped with a thermometer, under a nitrogen stream. Furthermore, 34.8 g (255 mol) of 6-chloro-1-hexanol and 4.09 g (21.5 mmol) of p-toluenesulfonic acid mono hydrate were added to prepare the solution. A Dean Stark apparatus was used to heat the prepared solution, and azeotropic dehydration (internal temperature approximately 95°C) was performed for 5 hours while discharging the generated water out of the reaction system. After completion of the reaction, 75 g of a 6 wt% sodium bicarbonate water was added to the reaction solution cooled to 25°C to separate and wash. After the separation, the organic layer was further washed with 80 g of water. After the washing, the organic layer was filtered off. The solvent was removed from the organic layer using a rotary evaporator to obtain 75 g of a light brown oil containing Intermediate L1. Purification of the light brown oil was not performed, and the light brown oil was used in the following reaction (Step 2: Synthesis of Intermediate M1) as is. The structure of Intermediate L1 was identified by $^1$H-NMR. The $^1$H-NMR spectral data is shown below.

$^1$H-NMR (500 MHz, $CDCl_3$, TMS, δppm):8.00 (dd,1H, J = 1.0 Hz, 8.5 Hz), 7.86 (dd,1H, J = 1.5 Hz,8.5 Hz), 7.79 (dd,1H, J = 1.5 Hz,7.5 Hz), 7.54-7.47 (m,2H), 7.45-7.41 (m,2H), 4.09-4.06 (m,4H), 3.43 (t,2H, J = 7.0 Hz), 1.67-1.61 (m,2H), 1.58-1.53 (m,2H), 1.35-1.29 (m,2H), 1.22-1.15 (m,2H).

<Step 2: Synthesis of Intermediate M1 (Still another example of the compound represented by formula (IV))>

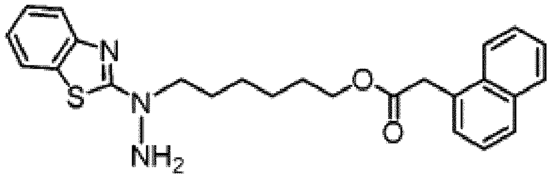

・・・Intermediate M1

6.00 g (36.32 mmol) of 2-hydrazinobenzothiazole was dissolved in 65 mL of N-N-dimethylformamide in a three-necked reactor equipped with a thermometer under a nitrogen stream. 23.67 g (72.63 mmol) of cesium carbonate, 20 g of the brown oil contained in Intermediate L1 synthesized in Step 1 was added to the solution, and the solution was stirred at 25°C for 15 hours. After completion of the reaction, the reaction solution was charged with 250 mL of distilled water, followed by extraction twice with 250 mL of ethyl acetate. After drying the ethyl acetate layer with anhydrous sodium sulfate, the sodium sulfate was filtered off. The organic layer was collected, dried with anhydrous sodium sulfate, and the sodium sulfate was filtered off. After the solvent was evaporated from the filtrate using a rotary evaporator, the obtained residue was purified by silica gel column chromatography (hexane:THF = 80:20 (volume ratio)) to obtain 8.0 g of Intermediate M1 was a white solid. The yield was 51.0 mol%. The structure of Intermediate M1 was identified by $^1$H-NMR. The $^1$H-NMR spectral data is shown below.

$^1$H-NMR (500 MHz, $CDCl_3$, TMS, δppm):8.00 (d,1H, J = 8.5 Hz), 7.85 (dd,1H, J = 1.0 Hz,8.0 Hz), 7.78 (dd,1H, J = 1.5 Hz, 7.5 Hz), 7.60 (dd,1H, J = 1.0 Hz,7.5 Hz), 7.54-7.51 (m,2H), 7.49-7.40 (m,3H), 7.28 (ddd,1H, J = 1.0 Hz, 7.5 Hz,7.5 Hz), 7.07 (ddd,1H, J = 1.0 Hz,7.5 Hz,7.5 Hz), 4.16 (br,2H), 4.08 (t,2H, J = 6.5 Hz), 4.06 (s,2H), 3.66 (t,2H, J = 7.0 Hz), 1.63-1.54 (m,4H), 1.32-1.22 (m,4H).

<Step 3: Synthesis of Polymerizable compound 21 (Still another example of the compound represented by formula (III-1))>

3 g (7.17 mmol) of Intermediate A synthesized in the Synthesis Step 1 of Synthesis Example 1, 30 g of chloroform, 1.0 g (13.7 mmol) of N-N-dimethylformamide were added in a three-necked reactor equipped with a thermometer, under a nitrogen stream, and cooled to 10°C or less. 0.98 g (8.24 mmol) of thionyl chloride was dropped therein while maintaining the reaction temperature at 10°C or less. After the dropwise addition, the reaction solution was returned to 25°C and stirred for 1 hour. After completion of the reaction, 20 g of chloroform was extracted and concentrated using an evaporator to synthesize a chloroform solution (1).

0.45 g (3.26 mmol) of 2,5-dihydroxybenzaldehyde and 2.09 g (19.5 mmol) of 2,6-lutidine were dissolved in 20 g of chloroform in a separately prepared three-necked reactor equipped with a thermometer, under a nitrogen stream, and the obtained solution was cooled to 10°C or less. The entire amount of the chloroform solution (1) was gradually dropped in the solution while maintaining the reaction temperature at 10°C or less. After the dropwise addition, the solution was further stirred at 5 to 10°C for 1 hour. After completion of the reaction, while maintaining at 10°C or less, 12 g of a 1.0N aqueous hydrochloric acid and 1.84 g (4.24 mmol) of Intermediate M1 synthesized in Step 2 were added to the reaction solution. Then, the temperature of the reaction solution was raised to 40°C and stirred for 3 hours. After completion of the reaction, the water later was extracted. Furthermore, 10 g of distilled water was charged in the organic layer to wash the organic layer. After washing the obtained organic layer with anhydrous sodium sulfate, the sodium sulfate was filtered off. The chloroform was removed from the filtrate under reduced pressure using a rotary evaporator to obtain a yellow solid. The yellow solid was purified by silica gel column chromatography (chloroform:THF = 95:5) to obtain 3.0 g of polymerizable compound 21 as a yellow solid. The yield was 67.9%. The structure of polymerizable compound 21 was identified by 1H-NMR. The 1H-NMR spectral data is shown below.

$^1$H-NMR (500 MHz, $CDCl_3$, TMS, δppm):7.97 (dd,1H, J = 0.5 Hz,8.5 Hz), 7.80 (ddd,1H, J = 0.5 Hz,0.5 Hz,8.0 Hz), 7.73-7.76 (m,2H), 7.67-7.71 (m,2H), 7.61 (s,1H), 7.49 (ddd,1H, J = 1.0 Hz,6.5 Hz,8.5 Hz), 7.42 (ddd,1H, J = 1.5 Hz,7.0 Hz,7.0 Hz), 7.33-7.39 (m,3H), 7.18 (ddd,1H, J = 1.0 Hz,7.5 Hz,8.0 Hz), 7.10-7.14 (m,2H), 6.95-7.01 (m,4H), 6.85-6.90 (m,4H), 6.405 (dd,1H, J = 1.5 Hz,17.5 Hz), 6.402 (dd,1H, J = 1.5 Hz,17.5 Hz), 6.127 (dd,1H, J = 10.5 Hz,17.5 Hz), 6.124 (dd,1H, J = 10.5 Hz,17.5 Hz), 5.822 (dd,1H, J = 1.5 Hz,10.5 Hz), 5.819 (dd,1H, J = 1.5 Hz,10.5 Hz), 4.16-4.22 (m,6H), 4.08 (t,2H, J = 6.5 Hz), 4.03 (s,2H), 3.95 (t,2H, J = 6.5 Hz), 3.93 (t,2H, J = 6.5 Hz), 2.56-2.67 (m,4H), 2.28-2.36 (m,8H), 1.59-1.83 (m,20H), 1.42-1.56 (m,8H), 1.24-1.36 (m,4H).

(Comparative Synthesis Example 1) Synthesis of Polymerizable compound X

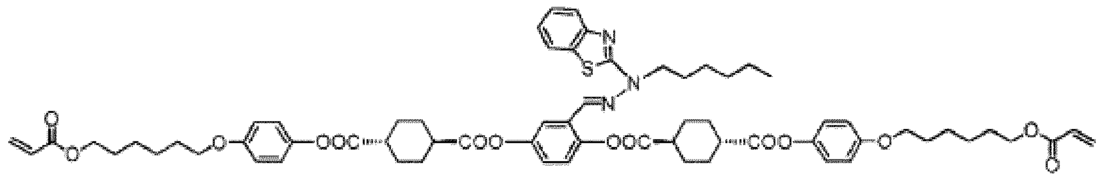

・・・Polymerizable compound X

<Step 1: Synthesis of Intermediate $\alpha$>

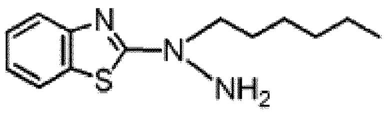

・・・Intermediate $\alpha$

2.00 g (12.1 mmol) of 2-hydrazinobenzothiazole was dissolved in 20 mL of dimethylformamide in a four-necked reactor equipped with a thermometer, under a nitrogen stream. 8.36 g (60.5 mmol) of potassium carbonate and 3.08 g of 1-iodohexane (14.5 mmol) were added to the solution, and the solution was stirred at 50°C for 7 hours. after completion of the reaction, the reaction solution was cooled to 20°C, the reaction solution was charged in 200 mL of water, and extracted with 300 mL of ethyl acetate. The ethyl acetate layer was dried with anhydrous sodium sulfate. After the sodium sulfate was filtered off, and ethyl acetate was evaporated under reduced pressure using a rotary evaporator to obtain a yellow solid. The yellow solid was purified by silica gel column chromatography (hexane:ethyl acetate = 75:25 (volume ratio)) to obtain 2.10 g of Intermediate $\alpha$ as a white solid. The yield was 69.6 mol%. The structure of Intermediate $\alpha$ was identified by $^1$H-NMR. The $^1$H-NMR spectral data is shown below.

$^1$H-NMR (500 MHz, $CDCl_3$, TMS, $\delta$ppm):7.60 (dd, 1H, J = 1.0,8.0 Hz), 7.53 (dd,1H, J = 1.0,8.0 Hz), 7.27 (ddd,1H, J = 1.0,8.0,8.0 Hz), 7.06 (ddd,1H, J = 1.0,8.0,8.0 Hz), 4.22 (s,2H), 3.74 (t,2H, J = 7.5 Hz), 1.69-1.76 (m,2H), 1.29-1.42 (m,6H), 0.89 (t,3H, J = 7.0 Hz).

<Step 2: Synthesis of Polymerizable compound X>

697 mg (2.37 mmol) of Intermediate $\alpha$ synthesized in Step 1 and 2.00 g (2.13 mmol) of Intermediate B synthesized in the Synthesis Step 2 of Synthesis Example 1 were dissolved in 50 mL of chloroform in a four-necked reactor equipped with a thermometer, under a nitrogen stream. 49 mg (0.21 mmol) of ($\pm$)-10-camphorsulfonic acid was added to the solution and the solution was stirred at 50°C for 3 hours. after completion of the reaction, the reaction solution was charged in a mixed water of 100 mL and 50 mL of 5% aqueous solution of sodium hydrogen carbonate, followed by extraction with 250 mL of ethyl acetate. The ethyl acetate layer was dried with anhydrous sodium sulfate. After the sodium sulfate was filtered off, and ethyl acetate was evaporated under reduced pressure using a rotary evaporator to obtain a white solid. The white solid was purified by silica gel column chromatography (toluene:ethyl acetate = 88:12 (volume ratio)) to obtain 2.33 g of polymerizable compound X as a white solid. The yield was 93.5 mol%. The structure of the target product (polymerizable compound X) was identified by $^1$H-NMR. The $^1$H-NMR spectral data is shown below.

$^1$H-NMR (400 MHz, $CDCl_3$, TMS, $\delta$ppm): 7.75 (d,1H, J = 2.5 Hz), 7.67-7.70 (m,3H), 7.34 (ddd,1H, J = 1.0 Hz,7.0 Hz,7.5 Hz), 7.17 (ddd,1H, J = 1.0 Hz,7.5 Hz,7.5 Hz), 7.12 (d,1H, J = 9.0 Hz), 7.10 (dd,1H, J = 2.5 Hz,9.0 Hz), 6.99 (d,2H, J = 9.0 Hz), 6.98 (d,2H, J = 9.0 Hz), 6.88 (d,4H, J = 9.0 Hz), 6.40 (dd,2H, J = 1.5 Hz,17.0 Hz), 6.13 (dd,2H, J = 10.5 Hz,17.5 Hz), 5.82 (dd,2H, J = 1.5 Hz,10.5 Hz), 4.30 (t,2H, J = 8.0 Hz), 4.18 (t,4H, J = 6.5 Hz), 3.95 (t,4H, J = 6.5 Hz), 2.58-2.70 (m,4H), 2.31-2.35 (m,8H), 1.66-1.82 (m,18H), 1.31-1.54 (m,14H), 0.90 (t,3H, J = 7.0 Hz).

<Measurement of Phase Transition Temperature>

5 mg of each of polymerizable compounds 1 to 21 and Polymerizable compound X was weighed, and placed in a solid state between two glass substrates provided with a polyimide alignment film subjected to a rubbing treatment (manufactured by E. H. C Co., Ltd., Product name: Alignment Treatment Glass Substrate).The substrates were placed on a hot plate, heated from 50°C to 200°C, and cooled to 50°C. A change in the structure during a change in the temperature was observed using a polarizing microscope (ECLIPSE LV100 POL manufactured by Nikon Corporation).

The measured phase transition temperatures are shown in the following Tables 1-1 to 1-4.

In Tables 1-1 to 1-4, "C" refers to "crystal", "N" refers to "nematic", and "I" refers to "isotropic". Here, the term

"crystal" means that the test compound was in a solid phase, the term "nematic" means that the test compound was in a nematic liquid crystal phase, and the term "isotropic" means that the test compound was in an isotropic liquid phase.

Table 1-1

| Compound number | Phase transition temperature |
|---|---|
| Compound 1 | 110°C 180°C<br>C ⇄ N ⇄ I<br>50°C or below 177°C |
| Compoud 2 | 105°C 200°C or above<br>C ⇄ N → I<br>50°C or below |
| Compound 3 | 112°C 200°C or above<br>C ⇄ N → I<br>50°C or below |
| Compound 4 | 111°C 190°C<br>C ⇄ N ⇄ I<br>50°C or below 182°C |
| Compound 5 | 120°C 200°C or above<br>C ⇄ N → I<br>50°C or below |
| Compound 6 | 123°C 186°C<br>C ⇄ N ⇄ I<br>50°C or below 183°C |
| Compound 7 | 126°C 200°C or above<br>C ⇄ N → I<br>50°C or below |
| Compound 8 | 104°C 200°C or above<br>C ⇄ N → I<br>50°C or below |
| Compound X | 96°C 200°C or above<br>C ⇄ N → I<br>50°C or below |

Table 1-2

| Compound Number | Phase transition temperature |
|---|---|
| Compound 9 | 106°C 200°C or above<br>C ⇄ N → I<br>50°C or below |
| Compound 10 | 145°C 200°C or above<br>C ⇄ N → I<br>50°C or below |
| Compound 11 | 120°C 200°C or above<br>C ⇄ N → I<br>50°C or below |
| Compound 12 | 94°C<br>C ⇄ I<br>50°C or below |

Table 1-3

| Compound number | Phase transition temperature |
|---|---|
| Compound 13 | $C \underset{50^\circ C\ \text{or below}}{\overset{148^\circ C}{\rightleftarrows}} N \underset{177^\circ C}{\overset{189^\circ C}{\rightleftarrows}} I$ |
| Compound 14 | $C \underset{50^\circ C\ \text{or below}}{\overset{125^\circ C}{\rightleftarrows}} N \xrightarrow{200^\circ C\ \text{or above}} I$ |
| Compound 15 | $C \underset{50^\circ C\ \text{or below}}{\overset{123^\circ C}{\rightleftarrows}} N \underset{180^\circ C}{\overset{185^\circ C}{\rightleftarrows}} I$ |
| Compound 16 | $C \underset{50^\circ C\ \text{or below}}{\overset{153^\circ C}{\rightleftarrows}} N \underset{185^\circ C}{\overset{191^\circ C}{\rightleftarrows}} I$ |
| Compound 17 | $C \underset{50^\circ C\ \text{or below}}{\overset{135^\circ C}{\rightleftarrows}} N \underset{181^\circ C}{\overset{185^\circ C}{\rightleftarrows}} I$ |
| Compound 18 | $C \underset{50^\circ C\ \text{or below}}{\overset{130^\circ C}{\rightleftarrows}} N \underset{168^\circ C}{\overset{170^\circ C}{\rightleftarrows}} I$ |
| Compound 19 | $C \underset{50^\circ C\ \text{or below}}{\overset{127^\circ C}{\rightleftarrows}} N \xrightarrow{200^\circ C\ \text{or above}} I$ |
| Compound 20 | $C \underset{50^\circ C\ \text{or below}}{\overset{129^\circ C}{\rightleftarrows}} N \underset{175^\circ C}{\overset{185^\circ C}{\rightleftarrows}} I$ |
| Compound 21 | $C \underset{50^\circ C\ \text{or below}}{\overset{125^\circ C}{\rightleftarrows}} N \xrightarrow{200^\circ C\ \text{or above}} I$ |

Table 1-4

| Compound number | Phase transition temperature |
|---|---|
| Compound 21 | $C \underset{50^\circ C\ \text{or below}}{\overset{125^\circ C}{\rightleftarrows}} N \xrightarrow{200^\circ C\ \text{or above}} I$ |

<Preparation of Polymerizable Liquid Crystal Composition>

(Examples 1 to 8, Examples 17 to 19, and, Examples 25 to 33-2)

2 g of each of polymerizable compounds 1 to 11 and polymerizable compounds 13 to 21 obtained Synthesis Examples 1 to 11 and Synthesis Examples 13 to 22, 86 mg of Adeka Arkly N-1919T (manufactured by ADEKA Corporation) as the photopolymerization initiator, and 600 mg of a mixed solvent (mixing ratio (mass ratio): cyclopentanone / 1,3-dioxolane = 4/6) of cyclopentanone and 1,3-dioxolane containing 1 mass% of MEGAFACE F-562 (manufactured by DIC Corporation) as the surfactant were prepared separately, and dissolved in a mixed solvent of 4.1 g of 1,3-dioxolane and 2.74 g of cyclopentanone. The solution was filtered through a disposable filter having a pore size of 0.45 μm to obtain polymerizable compositions 1 to 11 and polymerizable compositions 13 to 22.

(Example 20)

1.0 g of polymerizable compound 12 obtained in Synthesis Example 12, 1.0 g of polymerizable compound X obtained in Comparative Synthesis Example 1, 86 mg of Adeka Arkly N-1919T (manufactured by ADEKA) as the photopolymerization initiator, and 600 mg of a mixed solvent (mixing ratio (mass ratio): cyclopentanone / 1,3-dioxolane = 4/6) of cyclopentanone and 1,3-dioxolane containing 1 mass% of MEGAFACE F-562 (manufactured by DIC Corporation) as the surfactant were prepared separately, and dissolved in a mixed solvent of 4.1 g of 1,3-dioxolane and 2.74 g of cyclopentanone. The solution was filtered through a disposable filter having a pore size of 0.45 μm to obtain polymerizable composition 12.

(Comparative Example 1)

2.0 g of polymerizable compound X obtained in Comparative Synthesis Example 1, 86 mg of Adeka Arkly N-1919T (manufactured by ADEKA Corporation) as the photopolymerization initiator, and 600 mg of a mixed solvent (mixing ratio (mass ratio): cyclopentanone / 1,3-dioxolane = 4/6) of cyclopentanone and 1,3-dioxolane containing 1 mass% of MEGAFACE F-562 (manufactured by DIC Corporation) as the surfactant, were prepared separately, and dissolved in a mixed solvent of 4.1 g of 1,3-dioxolane and 2.74 g of cyclopentanone. The solution was filtered through a disposable filter having a pore size of 0.45 μm to obtain polymerizable composition 1r.

<Evaluation of Optical Property>

(i) Formation of the liquid crystal layer by the polymerizable composition

Each of the polymerizable compositions 1 to 22 and 1r obtained as stated above was applied to a polyimide alignment film and subjected to a rubbing treatment (manufactured by E.H.C Co., Ltd., Product name: Alignment Treatment Glass Substrate) using a #6 bar coater to obtain the coating film. The obtained coating film was dried for 1 minute at the temperatures shown in the following Table 2-1, Table 2-2, Table 2-3 and Table 2-4, and subjected to an alignment treatment for 1 minute at 23°C, or, the temperatures shown in Table 2-2, Table 2-3 and Table 2-4 to form the liquid crystal layer.

(ii) Formation of the optically anisotropic body

UV rays were applied to the coated surface side of the liquid crystal layer produced as stated above at a dose of 2000 mJ/cm$^2$ at 60°C, or, at the temperatures shown in Table 2-2, Table 2-3 and Table 2-4 to effect polymerization to obtain an optically anisotropic body with a transparent glass substrate which is the wavelength dispersion measurement sample. Here, the film thickness of the optically anisotropic body (liquid crystal polymer film) was measured by scratching the optically anisotropic body of the optically anisotropic body with a transparent glass substrate with a needle, and the step height was measured by DEKTAK 150 surface profilometer (manufactured by ULVAC, Inc). The results are shown in Table 2-1, Table 2-2, Table 2-3 and Table 2-4.

(iii) Measurement of retardation

The retardation between wavelengths of 400 nm and 800 nm was measured using the sample obtained in the aforementioned (ii) utilizing a Mueller Matrix Polarimeter Axoscan (manufactured by Axometrics Inc). The retardation at a wavelength of 550 nm is shown in Table 2-1, Table 2-2, Table 2-3 and Table 2-4.

(iv) Evaluation of wavelength dispersion

The wavelength dispersion was evaluated based on the values of the wavelength dispersion ratios α and β that were calculated as described below using the measured retardation. The results are shown in Table 2-1, Table 2-2, Table 2-3 and Table 2-4.

$$\alpha = (450\ \text{nm}) / (\text{retardation value at } 550\ \text{nm})$$

$$\beta = (650\ \text{nm}) / (\text{retardation value at } 550\ \text{nm})$$

(v) Calculation of $\Delta n$ at 550 nm

The $\Delta n$ at 550 nm was calculated by the following formula. The results are shown in Table 2-1, Table 2-2, Table 2-3 and Table 2-4.

$$\Delta n = \text{(retardation value at 550 nm)} / \text{(film thickness of optically anisotropic body; } \mu\text{m)}/1000$$

Table 2-1

| | Polymerizable composition | Polymerizable compound | Drying temperature (°C) | Film thickness (μm) | Phase difference (nm) at 550 nm | Δn at 550 nm | Wavelength dispersion ratio | |
|---|---|---|---|---|---|---|---|---|
| | | | | | | | α | β |
| Example 1 | 1 | 1 | 120 | 2.30 | 109.14 | 0.0475 | 0.832 | 1.042 |
| Example 2 | 2 | 2 | 120 | 2.34 | 142.67 | 0.0610 | 0.856 | 1.033 |
| Example 3 | 3 | 3 | 120 | 2.02 | 112.02 | 0.0556 | 0.834 | 1.031 |
| Example 4 | 4 | 4 | 120 | 2.12 | 108.37 | 0.0511 | 0.841 | 1.033 |
| Example 5 | 5 | 5 | 130 | 2.17 | 137.11 | 0.0633 | 0.868 | 1.024 |
| Example 6 | 6 | 6 | 130 | 2.46 | 114.01 | 0.0464 | 0.797 | 1.043 |
| Example 7 | 7 | 7 | 135 | 2.07 | 132.85 | 0.0640 | 0.864 | 1.026 |
| Example 8 | 8 | 8 | 110 | 2.04 | 133.58 | 0.0654 | 0.860 | 1.026 |
| Comparative Example 1 | 1r | X | 110 | 2.03 | 149.63 | 0.0737 | 0.809 | 1.037 |

Table 2-2

| | Polymerizable composition | Polymerizable compound | Drying temperature (°C) | Alignment treatment temperature (°C) | Exposure temperature (°C) | Film thickness (μm) | Phase difference (nm) at 550 nm | Δn at 550 nm | Wavelength dispersion ratio | |
|---|---|---|---|---|---|---|---|---|---|---|
| | | | | | | | | | α | β |
| Example 17 | 9 | 9 | 115 | 23 | 60 | 2.14 | 139.84 | 0.0655 | 0.881 | 1.020 |
| Example 18 | 10 | 10 | 155 | 23 | 60 | 2.31 | 142.41 | 0.0617 | 0.829 | 1.035 |
| Example 19 | 11 | 11 | 130 | 23 | 60 | 2.19 | 140.34 | 0.0641 | 0.823 | 1.034 |
| Example 20 | 12 | 12, X | 130 | 90 | 90 | 2.33 | 144.18 | 0.0618 | 0.923 | 1.007 |
| Comparative Example 1 | 1r | X | 110 | 23 | 60 | 2.03 | 149.63 | 0.0737 | 0.809 | 1.037 |

Table 2-3

| | Polymerizable composition | Polymerizable compound | Drying temperature (°C) | Alignment treatment temperature (°C) | Exposure temperature (°C) | Film thickness (μm) | Phase difference (nm) at 550 nm | Δn at 550 nm | Wavelength dispersion ratio | |
|---|---|---|---|---|---|---|---|---|---|---|
| | | | | | | | | | α | β |
| Example 25 | 13 | 13 | 150 | 23 | 60 | 2.05 | 109.47 | 0.0534 | 0.870 | 1.027 |
| Example 26 | 14 | 14 | 130 | 23 | 60 | 2.06 | 145.57 | 0.0708 | 0.873 | 1.028 |
| Example 27 | 15 | 15 | 130 | 23 | 60 | 2.24 | 101.94 | 0.0455 | 0.700 | 1.067 |
| Example 28 | 16 | 16 | 160 | 23 | 60 | 2.21 | 104.37 | 0.0473 | 0.759 | 1.056 |
| Example 29 | 17 | 17 | 145 | 23 | 60 | 2.26 | 106.99 | 0.0474 | 0.704 | 1.070 |
| Example 30 | 18 | 18 | 130 | 23 | 60 | 2.20 | 102.26 | 0.0466 | 0.710 | 1.065 |
| Example 31 | 19 | 19 | 140 | 60 | 60 | 2.04 | 131.46 | 0.0644 | 0.826 | 1.035 |
| Example 32 | 20 | 20 | 130 | 60 | 60 | 2.16 | 97.73 | 0.0453 | 0.789 | 1.048 |
| Example 33 | 21 | 21 | 130 | 23 | 60 | 2.00 | 113.68 | 0.0570 | 0.842 | 1.030 |

Table 2-4

| | Polymerizable composition | Polymerizable compound | Drying temperature (°C) | Alignment treatment temperature (°C) | Exposure temperature (°C) | Film thickness (μm) | Phase difference (nm) at 550 nm | Δn at 550 nm | Wavelength dispersion ratio | |
|---|---|---|---|---|---|---|---|---|---|---|
| | | | | | | | | | α | β |
| Example 33-2 | 22 | 21 | 130 | 23 | 60 | 2.00 | 113.68 | 0.0570 | 0.842 | 1.030 |

α is smaller than 1, and β is larger than 1 when an ideal wideband wavelength dispersion i.e., a reverse wavelength dispersion is achieved. It is understood from Table 2-1, Table 2-2, Table 2-3 and Table 2-4 that the optically anisotropic bodies produced from the polymerizable compounds of Examples 1 to 8, Examples 17 to 20 and Examples 25 to 33-2 have an ideal wideband wavelength dispersion i.e., a reverse wavelength dispersion.

Further, the polymerizable compounds of Examples 1 to 8, Examples 17 to 20 and Examples 25 to 33-2 have a smaller Δn, and in order to obtain the same retardation, it is necessary to make the application of the polymerizable compound of Comparative Example 1 thicker, and as a result, it is anticipated that control of retardation and uniformity of film thickness can be more easily performed.

<Evaluation of retardation and in-plane variation of film thickness>

(Examples 9 to 16, Examples 21 to 24, Examples 34 to 43 and Comparative Example 2)

(i) Formation of liquid crystal layer by polymerizable composition

Each of the polymerizable compositions 1 to 21 and 1r obtained as described above was applied to a polyimide alignment film and subjected to a rubbing treatment (manufactured by E.H.C Co., Ltd., Product name: Alignment Treatment Glass Substrate using a spin coater so as to make the retardation at 550 nm to 138 nm in Region A (refer to Fig. 1) to obtain a coating film. The obtained coating film was dried for 1 minute at the temperatures shown in the following Table 3-1, Table 3-2, Table 3-3 and Table 3-4 and subjected to an alignment treatment for 1 minute at 23°C, or at the temperatures shown in Table 3-2, Table 3-3 and Table 3-4 to form the liquid crystal layer.

(ii) Formation of the optically anisotropic body

UV rays were applied to the coated surface side of the liquid crystal layer produced as stated above at a dose of 2000 mJ/cm$^2$ at 60°C (temperature shown Table 3-3) to effect polymerization so as to obtain an optically anisotropic body with a transparent glass substrate which is the evaluation sample.

(iii) Measurement of retardation and film thickness

The retardation at a 550 nm in Regions A to E illustrated in Fig. 1 was measured using the obtained samples utilizing a MuellerMatrix Polarimeter Axoscan (manufactured by Axometrics Inc). The results are shown in Table 3-1, Table 3-2, Table 3-3 and Table 3-4.

After the retardation was measured, the film thickness of the optically anisotropic body (liquid crystal polymer film) was measured by scratching the optically anisotropic body of the optically anisotropic body with a transparent glass substrate using a needle, and measuring the step height by a DEKTAK 150 surface profilometer (manufactured by ULVAC, Inc). The results are shown in Table 3-1, Table 3-2, Table 3-3 and Table 3-4.
Note that, the standard deviation was calculated by the following formula (I).

$$\sqrt{\frac{\sum (x-\bar{x})^2}{(n-1)}} \quad \cdots \text{Formula (1)}$$

In formula (1), x represents the sample, $\bar{x}$ represents the sample average, and n represents the number of samples.

Table 3-1

| | Polymerizable composition | Drying temperature (°C) | Region A | | Region B | | Region C | | Region D | | Region E | | Standard deviation | |
|---|---|---|---|---|---|---|---|---|---|---|---|---|---|---|
| | | | Phase difference (nm) | Film thickness (μm) | Phase difference (nm) | Film thickness (μm) | Phase difference (nm) | Film thickness (μm) | Phase difference (nm) | Film thickness (μm) | Phase difference (nm) | Film thickness (μm) | Phase difference | Film thickness |
| Example 9 | 1 | 120 | 138.3 | 2.91 | 140.2 | 2.95 | 139.9 | 2.94 | 139.2 | 2.93 | 140.1 | 2.95 | 0.796 | 0.017 |
| Example 10 | 2 | 120 | 138.2 | 2.27 | 139.2 | 2.28 | 139.4 | 2.28 | 140.5 | 2.30 | 139.9 | 2.29 | 0.856 | 0.014 |
| Example 11 | 3 | 120 | 138.0 | 2.48 | 138.8 | 2.50 | 140.0 | 2.52 | 139.5 | 2.51 | 139.7 | 2.51 | 0.803 | 0.014 |
| Example 12 | 4 | 120 | 138.4 | 2.71 | 140.2 | 2.75 | 140.3 | 2.75 | 140.5 | 2.75 | 140.3 | 2.75 | 0.868 | 0.017 |
| Example 13 | 5 | 130 | 138.3 | 2.18 | 140.3 | 2.22 | 140.5 | 2.22 | 140.5 | 2.22 | 140.6 | 2.22 | 0.979 | 0.015 |
| Example 14 | 6 | 130 | 138.1 | 2.98 | 139.9 | 3.01 | 139.4 | 3.00 | 140.2 | 3.02 | 140.2 | 3.02 | 0.879 | 0.019 |
| Example 15 | 7 | 135 | 138.0 | 2.16 | 139.5 | 2.18 | 138.5 | 2.16 | 139.7 | 2.18 | 139.9 | 2.18 | 0.826 | 0.013 |
| Example 16 | 8 | 110 | 138.4 | 2.12 | 140.5 | 2.15 | 139.2 | 2.13 | 140.0 | 2.14 | 138.8 | 2.12 | 0.861 | 0.013 |
| Comparative Example 2 | 1r | 110 | 138.2 | 1.87 | 141.6 | 1.92 | 141.8 | 1.92 | 142.6 | 1.93 | 142.7 | 1.94 | 1.842 | 0.025 |

Table 3-2

| | Polymerizable composition | Drying temperature (°C) | Alignment treatment temperature (°C) | Region A | | Region B | | Region C | | Region D | | Region E | | Standard deviation | |
|---|---|---|---|---|---|---|---|---|---|---|---|---|---|---|---|
| | | | | Phase difference (nm) | Film thickness (μm) | Phase difference (nm) | Film thickness (μm) | Phase difference (nm) | Film thickness (μm) | Phase difference (nm) | Film thickness (μm) | Phase difference (nm) | Film thickness (μm) | Phase difference | Film thickness |
| Example 21 | 9 | 115 | 23 | 138.0 | 2.11 | 139.8 | 2.14 | 140.1 | 2.14 | 140.2 | 2.14 | 138.8 | 2.12 | 0.950 | 0.015 |
| Example 22 | 10 | 155 | 23 | 138.3 | 2.24 | 138.2 | 2.24 | 139.7 | 2.26 | 137.9 | 2.24 | 139.1 | 2.25 | 0.740 | 0.012 |
| Example 23 | 11 | 130 | 23 | 138.1 | 2.15 | 138.0 | 2.15 | 138.8 | 2.17 | 139.8 | 2.18 | 138.9 | 2.17 | 0.726 | 0.011 |
| Example 24 | 12 | 130 | 90 | 138.2 | 2.23 | 140.1 | 2.27 | 139.5 | 2.26 | 138.4 | 2.24 | 139.1 | 2.25 | 0.783 | 0.013 |
| Comparative Example 2 | 1r | 110 | 23 | 138.2 | 1.87 | 141.6 | 1.92 | 141.8 | 1.92 | 142.6 | 1.93 | 142.7 | 1.94 | 1.842 | 0.025 |

Table 3-3

| | Polymerizable composition | Drying temperature (°C) | Alignment treatment temperature (°C) | Exposure temperature (°C) | Region A | | Region B | | Region C | | Region D | | Region E | | Standard deviation | |
|---|---|---|---|---|---|---|---|---|---|---|---|---|---|---|---|---|
| | | | | | Phase difference (nm) | Film thickness (μm) | Phase difference (nm) | Film thickness (μm) | Phase difference (nm) | Film thickness (μm) | Phase difference (nm) | Film thickness (μm) | Phase difference (nm) | Film thickness (μm) | Phase difference | Film thickness |
| Example 34 | 13 | 150 | 23 | 60 | 138.1 | 2.58 | 139.2 | 2.60 | 140.5 | 2.63 | 140.3 | 2.63 | 139.5 | 2.61 | 0.960 | 0.018 |
| Example 35 | 14 | 130 | 23 | 60 | 138.2 | 1.95 | 140 | 1.98 | 140.4 | 1.98 | 138.5 | 1.96 | 139.9 | 1.98 | 0.982 | 0.014 |
| Example 36 | 15 | 130 | 23 | 60 | 138.3 | 3.04 | 138.9 | 3.05 | 139.8 | 3.07 | 139.9 | 3.08 | 140 | 3.08 | 0.746 | 0.016 |
| Example 37 | 16 | 160 | 23 | 60 | 137.9 | 2.91 | 138.8 | 2.93 | 139.2 | 2.94 | 139.8 | 2.95 | 139.9 | 2.96 | 0.817 | 0.017 |
| Example 38 | 17 | 145 | 23 | 60 | 138.0 | 2.91 | 139.2 | 2.93 | 139.9 | 2.95 | 138.8 | 2.93 | 138.3 | 2.92 | 0.750 | 0.016 |
| Example 39 | 18 | 130 | 23 | 60 | 138.3 | 2.97 | 140.2 | 3.01 | 139.8 | 3.00 | 139.4 | 2.99 | 139.7 | 3.00 | 0.719 | 0.015 |
| Example 40 | 19 | 140 | 60 | 60 | 138.1 | 2.14 | 138.5 | 2.15 | 139.8 | 2.17 | 140.2 | 2.18 | 139.1 | 2.16 | 0.873 | 0.014 |
| Example 41 | 20 | 130 | 60 | 60 | 137.8 | 3.04 | 138.2 | 3.05 | 139.2 | 3.08 | 139.9 | 3.09 | 138.7 | 3.06 | 0.826 | 0.018 |
| Example 42 | 21 | 130 | 23 | 60 | 138.0 | 2.42 | 139.6 | 2.45 | 139.9 | 2.46 | 140.0 | 2.46 | 139.7 | 2.45 | 0.820 | 0.014 |
| Comparative Example 2 | 1r | 110 | 23 | 60 | 138.2 | 1.87 | 141.6 | 1.92 | 141.8 | 1.92 | 142.6 | 1.93 | 142.7 | 1.94 | 1.842 | 0.025 |

Table 3-4

| | Polymerizable composition | Drying temperature (°C) | Alignment treatment temperature (°C) | Exposure temperature (°C) | Region A | | Region B | | Region C | | Region D | | Region E | | Standard deviation | |
|---|---|---|---|---|---|---|---|---|---|---|---|---|---|---|---|---|
| | | | | | Phase difference (nm) | Film thickness (μm) | Phase difference (nm) | Film thickness (μm) | Phase difference (nm) | Film thickness (μm) | Phase difference (nm) | Film thickness (μm) | Phase difference (nm) | Film thickness (μm) | Phase difference | Film thickness |
| Example 43 | 22 | 130 | 23 | 60 | 136.2 | 2.39 | 136.9 | 2.40 | 137.5 | 2.41 | 137.9 | 2.42 | 138.3 | 2.43 | 0.823 | 0.014 |

It is understood from Table 3-1, Table 3-2, Table 3-3 and Table 3-4 that the optically anisotropic bodies of Examples 9 to 16, Examples 21 to 24 and Examples 34 to 43 produced from the polymerizable compositions 1 to 22 have a small retardation and in-plane variation (standard deviation) of the film thickness compared to the optically anisotropic body of Comparative Example 2 produced from the polymerizable composition 1r, and the production of an optically anisotropic body having excellent in-plane thickness uniformity and improved in-plane uniformity in optical properties is possible.

## Claims

**1.** A polymerizable compound represented by the following formula (I):

$$P^1-Y^7-G^1-Y^5\left[B^1-Y^3\right]_n A^1-Y^1-Ar-Y^2-A^2\left[Y^4-B^2\right]_m Y^6-G^2-Y^8-P^2 \quad \text{(I)}$$

where in the formula (I), Ar is represented by the following formula (II-1) or (II-2):

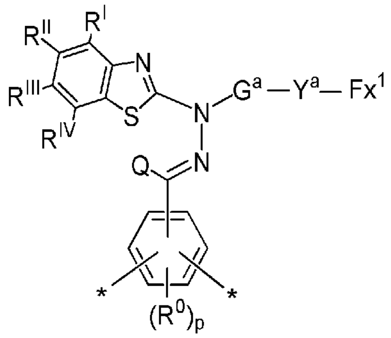
(II-1)

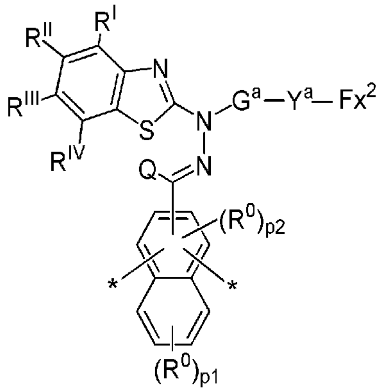
(II-2)

where in the formulas (II-1) and (II-2),

$Fx^1$ and $Fx^2$ each independently represent an organic group having at least one of an aromatic hydrocarbon ring and an aromatic heterocyclic ring,
$Y^a$ represents a chemical single bond, -O-, -C(=O)-, -C(=O)-O-, -O-C(=O)-, -O-C(=O)-O-, -C(=O)-S-, -S-C(=O)-, -$NR^{11}$-C(=O)-, -C (=O)-$NR^{11}$-, -O-C (=O)-$NR^{11}$-, -$NR^{11}$-C(=O)-O-, -S-, -N=N-, or -C=C-, where $R^{11}$ represents a hydrogen atom or an alkyl group having 1 to 6 carbon atoms,
$G^a$ is an organic group having 3 to 30 carbon atoms which may have a substituent,
Q represents a hydrogen atom, or an alkyl group having 1 to 6 carbon atoms which may have a substituent,
$R^I$ to $R^{IV}$ each independently represent a hydrogen atom, a halogen atom, an alkyl group having 1 to 6 carbon atoms, a cyano group, a nitro group, an alkyl group having 1 to 6 carbon atoms in which at least one hydrogen atom is substituted with a halogen atom, an alkoxy group having 1 to 6 carbon atoms, -OCF3, -C(=O)-O-$R^a$, or -O-C(=O)-$R^a$, where $R^a$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring having 5 to 12 carbon atoms which may have a substituent, $R^I$ to $R^{IV}$ may be the same or different, and one or more ring constituents C-$R^I$ to C-$R^{IV}$ may be replaced by a nitrogen atom,
$R^0$ represents halogen atom, an alkyl group having 1 to 6 carbon atoms, a cyano group, a nitro group, an alkyl group having 1 to 6 carbon atoms in which at least one hydrogen atom is substituted with a halogen atom, an alkoxy group having 1 to 6 carbon atoms, -OCF3, -C(=O)-O-$R^a$, or, -O-C(=O)-$R^a$, where $R^a$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring having 5 to 12 carbon atoms which may have a substituent, and when there

is a plurality of $R^0$, the plurality of $R^0$ may be the same or different from each other,
* represents a bond with $Y^1$ or $Y^2$, and
p represents an integer from 0 to 3, p1 represents an integer from 0 to 4, and p2 represents 0 or 1;
$Y^1$ to $Y^8$ each independently represent a chemical single bond, -O-, -O-$CH_2$-, -$CH_2$-O-, -O-$CH_2$-$CH_2$-, -$CH_2$-$CH_2$-O-, -C(=O)-O-, -O-C(=O)-, -O-C(=O)-O-, -C(=O)-S-, -S-C(=O)-, -$NR^{13}$-C(=O)-, -C(=O)-$NR^{13}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-C(=O)-O-, -O-C(=O)-$CH_2$-, -$CH_2$-O-C(=O)-, -C(=O)-O-$CH_2$-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, or -C=C-, where $R^{13}$ represents a hydrogen atom or an alkyl group having 1 to 6 carbon atoms;
$A^1$, $A^2$, $B^1$ and $B^2$ each independently represent a cyclic aliphatic group which may have a substituent, or, an aromatic group which may have a substituent;
$G^1$ and $G^2$ each independently represent an organic group which is either a divalent aliphatic hydrocarbon group having 1 to 30 carbon atoms, or a divalent aliphatic hydrocarbon group having 3 to 30 carbon atoms in which at least one -$CH_2$- contained in the divalent aliphatic hydrocarbon group is substituted with -O-, -S-, -O-C(=O)-, -C(=O)-O-, -O-C(=O)-O-, -$NR^{14}$-C(=O)-, -C(=O)-$NR^{14}$-, -$NR^{14}$-, or -C(=O)-, with the proviso that cases where there are two or more contiguous -O- or -S- are excluded, where $R^{14}$ represents a hydrogen atom or an alkyl group having 1 to 6 carbon atoms, and the hydrogen atoms included in the organic group of $G^1$ and $G^2$ may be substituted by an alkyl group having 1 to 5 carbon atoms, an alkoxy group having 1 to 5 carbon atoms, a cyano group, or a halogen atom;
$P^1$ and $P^2$ each independently represent an alkenyl group having 2 to 10 carbon atoms which may be substituted by a halogen atom or a methyl group; and
n and m each independently represent 0 or 1.

**2.** The polymerizable compound according to claim 1, wherein the number of $\pi$ electrons included in the ring structure in Ar is 22 or more.

**3.** The polymerizable compound according to any one of claim 1 or 2, wherein the number of $\pi$ electrons included in the ring structure in $Fx^1$ is 8 or more, and the number of $\pi$ electrons included in the ring structure in $Fx^2$ is 4 or more.

**4.** The polymerizable compound according to any one of claims 1 to 3, wherein $G^a$ is an organic group which is either a divalent aliphatic hydrocarbon group having 3 to 30 carbon atoms, or a divalent aliphatic hydrocarbon group having 3 to 30 carbon atoms in which at least one -$CH_2$-contained in the divalent aliphatic hydrocarbon group is substituted with -O-, -S-, -O-C(=O)-, -C(=O)-O-, -O-C(=O)-O-, -$NR^{12}$-C(=O)-, -C(=O)-$NR^{12}$-, -$NR^{12}$-, or -C(=O)-, with the proviso that cases where there are two or more contiguous -O- or -S- are excluded, where $R^{12}$ represents a hydrogen atom or an alkyl group having 1 to 6 carbon atoms, and the hydrogen atoms included in the organic group of $G^a$ may be substituted by an alkyl group having 1 to 5 carbon atoms, an alkoxy group having 1 to 5 carbon atoms, a cyano group, or a halogen atom.

**5.** The polymerizable compound according to any one of claims 1 to 4, wherein $G^a$ is an organic group which is either a divalent chain aliphatic hydrocarbon group having 3 to 18 carbon atoms, or a divalent chain aliphatic hydrocarbon group having 3 to 18 carbon atoms in which at least one -$CH_2$-contained in the divalent chain aliphatic hydrocarbon group is substituted with -O-, -S-, -O-C(=O)-, -C(=O)-O-, or -C(=O)-, with the proviso that cases where there are two or more contiguous -O- or -S- are excluded, and the hydrogen atoms included in the organic group of $G^a$ may be substituted by an alkyl group having 1 to 5 carbon atoms, an alkoxy group having 1 to 5 carbon atoms, a cyano group, or, a halogen atom.

**6.** The polymerizable compound according to any one of claims 1 to 5, wherein $G^a$ is an alkylene group having 3 to 18 carbon atoms.

**7.** The polymerizable compound according to any one of claims 1 to 6 represented by the following formula (III-1) or (III-2):

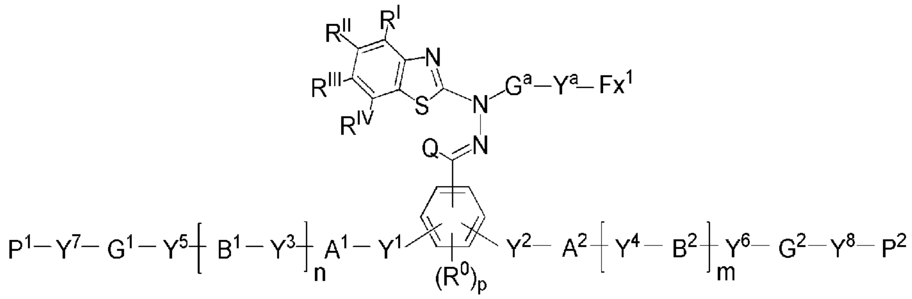

(III-1)

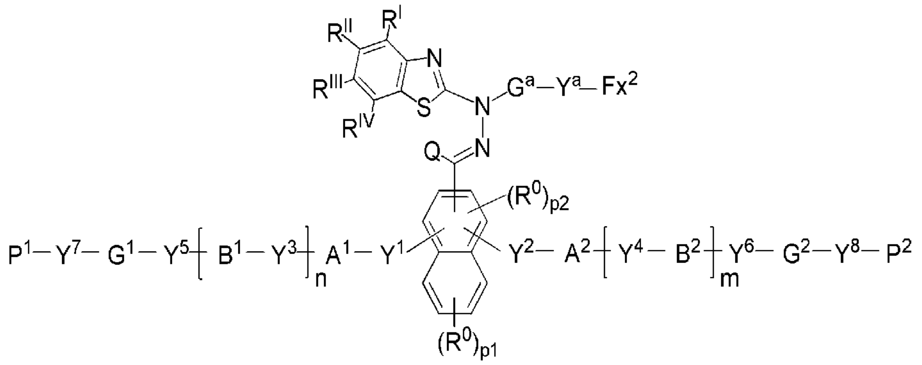

(III-2)

where in the formulas (III-1) and (III-2), $Y^1$ to $Y^8$, $A^1$, $A^2$, $B^1$, $B^2$, $G^1$, $G^2$, $P^1$, $P^2$, $R^I$ to $R^{IV}$, Q, $R^0$, n, m, p, p1, and p2 are the same as defined above;
$G^a$ represents an organic group which is either an alkylene group having 3 to 18 carbon atoms which may have a substituent, or an alkylene group having 3 to 18 carbon atoms in which at least one $-CH_2-$ contained in the alkylene group is substituted with -O-, -S-, -O-C(=O)-, -C(=O)-O-, or -C(=O)-, with the proviso that cases where there are two or more contiguous -O- or -S- are excluded;
$Y^a$ represents a chemical single bond, -O-, -C(=O)-, -C(=O)-O-, -O-C(=O)-, -O-C(=O)-O-, -C(=O)-S-, -S-C(=O)-, $-NR^{11}-C(=O)-$, $-C(=O)-NR^{11}-$, $-O-C(=O)-NR^{11}-$, $-NR^{11}-C(=O)-O-$, or -S-, where $R^{11}$ represents a hydrogen atom or an alkyl group having 1 to 6 carbon atoms;
$Fx^1$ and $Fx^2$ each independently represent an organic group having 2 to 30 carbon atoms having one of an aromatic hydrocarbon ring and an aromatic heterocyclic ring, and
the number of $\pi$ electrons included in the ring structure in $Fx^1$ is 8 or more, and the number of $\pi$ electrons included in the ring structure in $Fx^2$ is 4 or more.

**8.** The polymerizable compound according to any one of claims 1 to 7, wherein the number of $\pi$ electrons included in the ring structure in $Fx^1$ is 10 or more, the number of $\pi$ electrons included in the ring structure in $Fx^2$ is 6 or more.

**9.** The polymerizable compound according to any one of claims 1 to 8, wherein the $Fx^1$ and $Fx^2$ are each independently an alkyl group having 1 to 18 carbon atoms in which at least one hydrogen atom is substituted with a ring-containing group having at least one of an aromatic hydrocarbon ring and an aromatic heterocyclic ring and which may have a substituent other than the ring-containing group; or a cyclic group having 2 to 20 carbon atoms having at least one of an aromatic hydrocarbon ring and an aromatic heterocyclic ring and which may have a substituent.

**10.** The polymerizable compound according to any one of claims 1 to 9, wherein $Fx^1$ is represented by any of the following formulas (i-1) to (i-9), $Fx^2$ is represented by any of the following formulas (i-1) to (i-11), and the groups represented by the following formulas (i-1) to (i-11) may have a substituent:

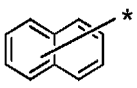
(i-1)
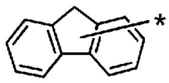
(i-2)
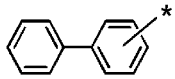
(i-3)
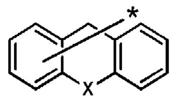
(i-4)
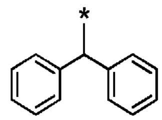
(i-5)
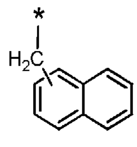
(i-6)
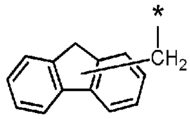
(i-7)
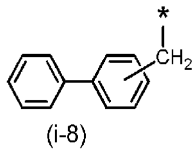
(i-8)
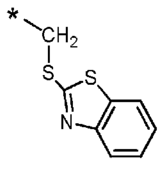
(i-9)
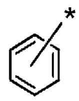
(i-10)
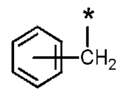
(i-11)

where in the formula (i-4), X represents $-CH_2-$, $-NR^d-$, an oxygen atom, a sulfur atom, -SO- or $-SO_2-$, and $R^d$ represents a hydrogen atom or an alkyl group having 1 to 6 carbon atoms.

**11.** The polymerizable compound according to any one of claims 1 to 10, wherein $G^1$ and $G^2$ each independently represent an organic group which is either a divalent aliphatic hydrocarbon group having 1 to 18 carbon atoms, or a divalent aliphatic hydrocarbon group having 3 to 18 carbon atoms in which at least one $-CH_2-$ contained in the divalent aliphatic hydrocarbon group is substituted with -O-, -S-, -O-C(=O)-, -C(=O)-O-, -O-C(=O)-O-, $-NR^{14}-C(=O)-$, $-C(=O)-NR^{14}-$, $-NR^{14}-$, or -C(=O)-, with the proviso that cases where there are two or more contiguous -O- or -S- are excluded, where $R^{14}$ represents a hydrogen atom or an alkyl group having 1 to 6 carbon atoms, the hydrogen atoms included in the organic group of $G^1$ and $G^2$ may be substituted by an alkyl group having 1 to 5 carbon atoms, an alkoxy group having 1 to 5 carbon atoms, a cyano group, or a halogen atom.

**12.** The polymerizable compound according to any one of claims 1 to 11, wherein $G^1$ and $G^2$ are each independently an alkylene group having 1 to 18 carbon atoms which may have at least one substituent selected from the group consisting of an alkyl group having 1 to 5 carbon atoms, an alkoxy group having 1 to 5 carbon atoms, a cyano group, and a halogen atom.

**13.** The polymerizable compound according to any one of claims 1 to 12, wherein $P^1$ and $P^2$ are each independently $CH_2=CH-$, $CH_2=C(CH_3)-$, or $CH_2=C(Cl)-$.

**14.** The polymerizable compound according to any one of claims 1 to 13, wherein $Y^1$ to $Y^8$ are each independently a chemical single bond, -O-, -O-C(=O)-, -C(=O)-O-, or -O-C(=O)-O-.

**15.** The polymerizable compound according to any one of claims 1 to 14, wherein $A^1$ and $A^2$ are each independently a trans-1,4-cyclohexelene group which may have a substituent, and
$B^1$ and $B^2$ are each independently a 1,4-phenylene group.

**16.** The polymerizable compound according to any one of claims 1 to 6 represented by the following formula (iii-1):

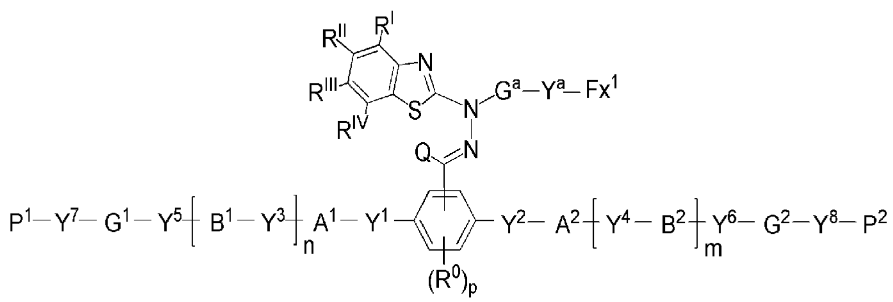
（ｉｉｉ－１）

where in the formula (iii-1), $Y^1$ to $Y^8$, $A^1$, $A^2$, $B^1$, $B^2$, $G^1$, $G^2$, $P^1$, $P^2$, $R^I$ to $R^{IV}$, Q, $G^a$, $Y^a$, $Fx^1$, $R^0$, m, n and p are the same as defined above.

**17.** The polymerizable compound according to any one of claims 1 to 16 represented by any of the following formulas

(1) to (21):

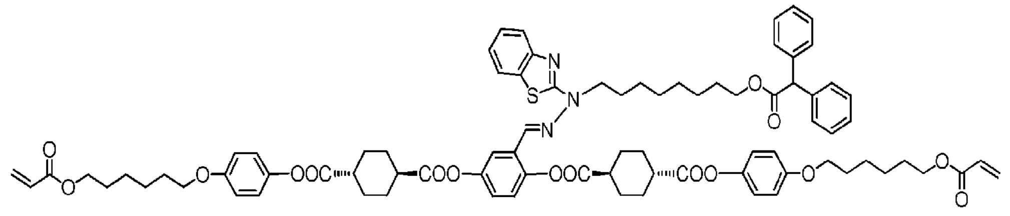
(1)

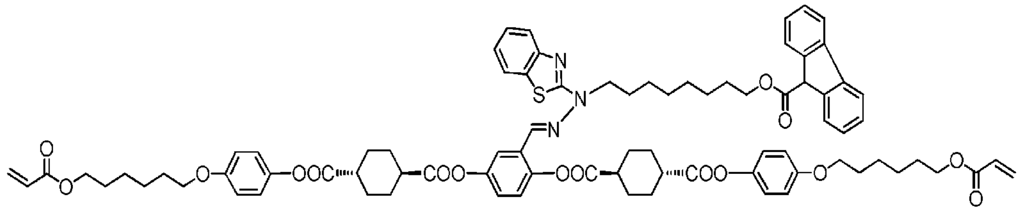
(2)

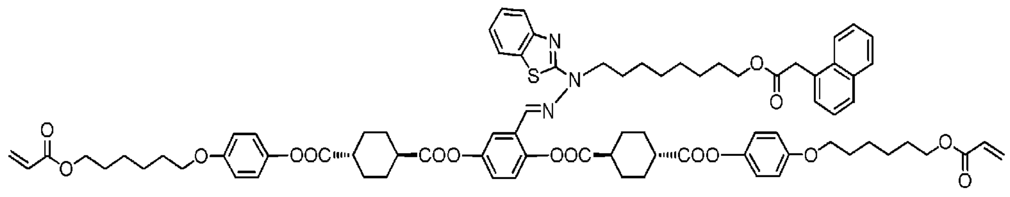
(3)

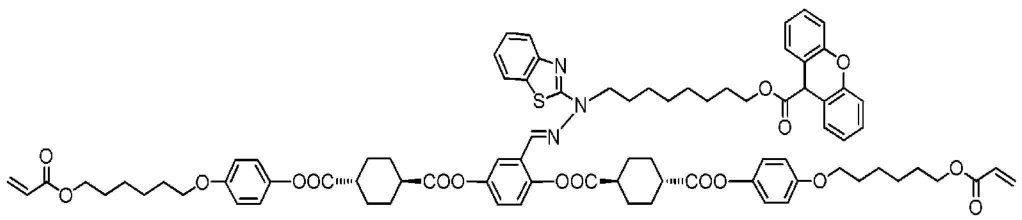
(4)

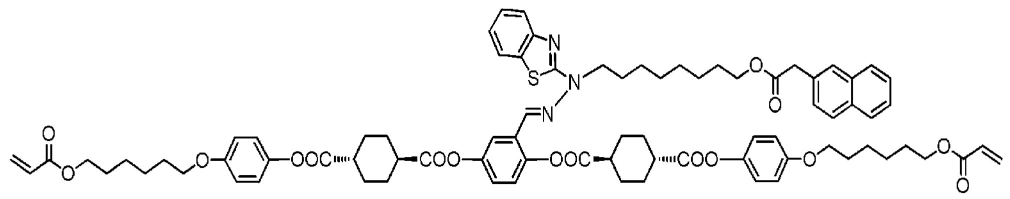
(5)

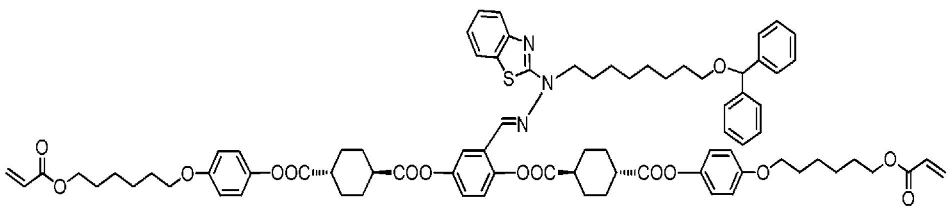
(6)

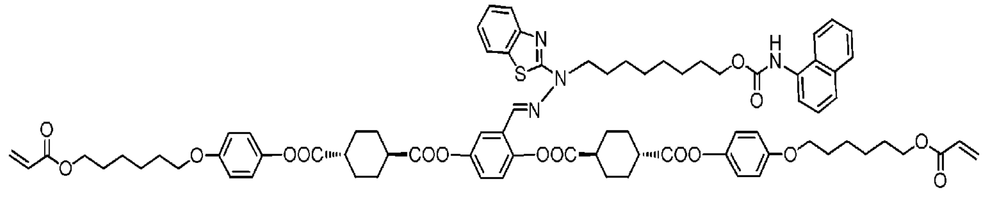
(7)

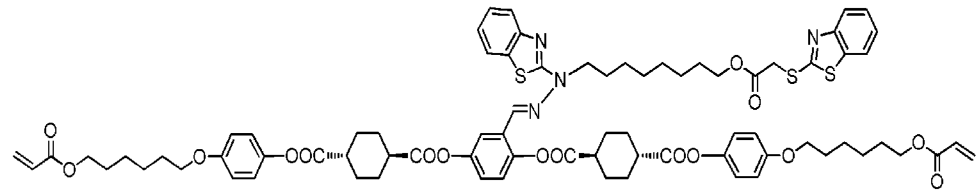
(8)

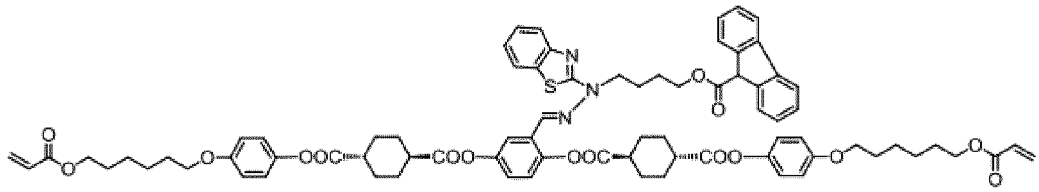
(9)

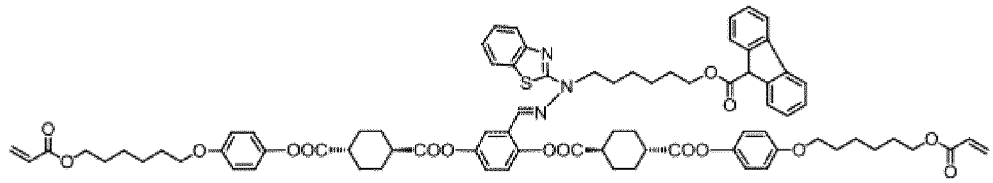
(10)

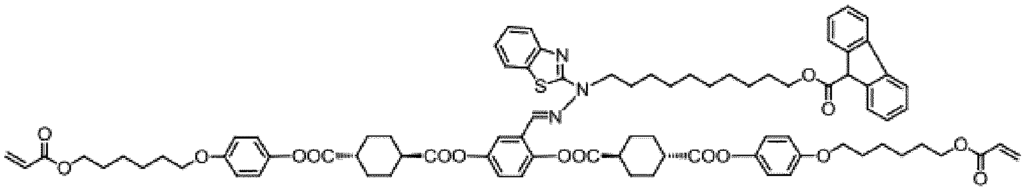
(11)

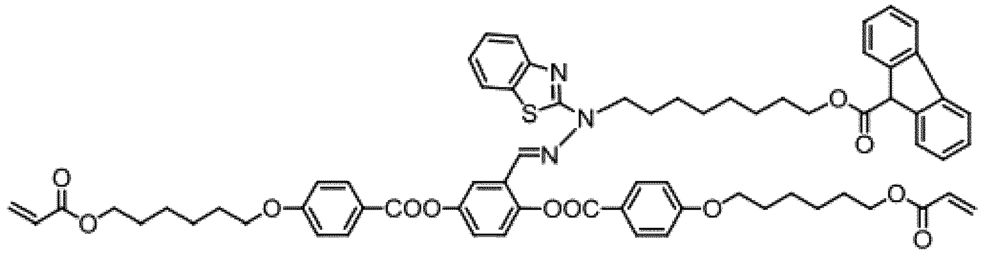
(12)

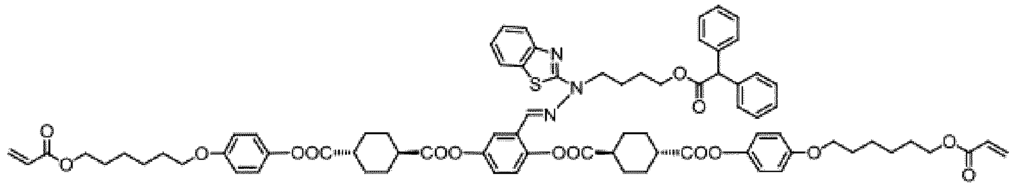
(13)

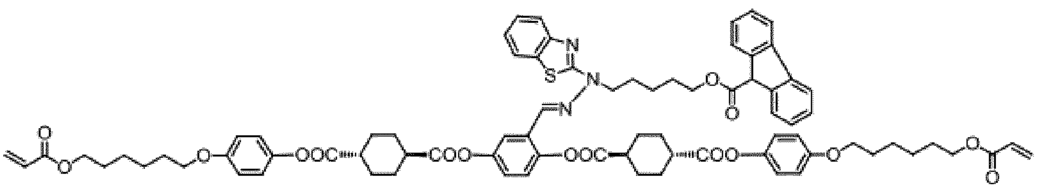
(14)

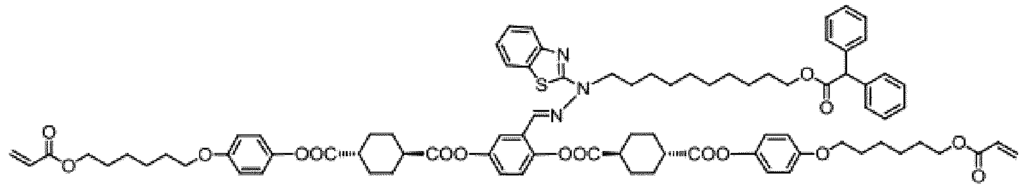
(1 5)

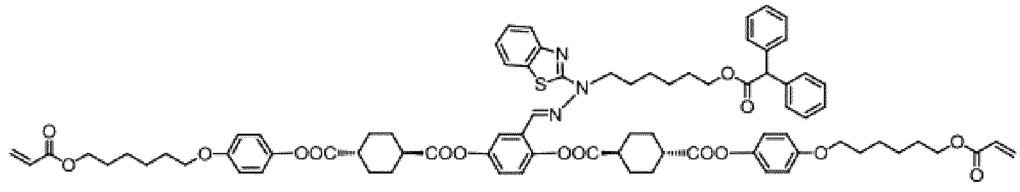
(1 6)

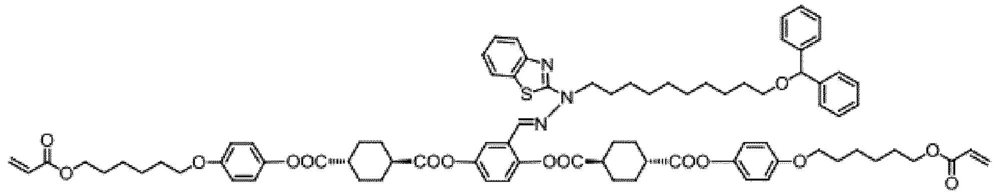
(1 7)

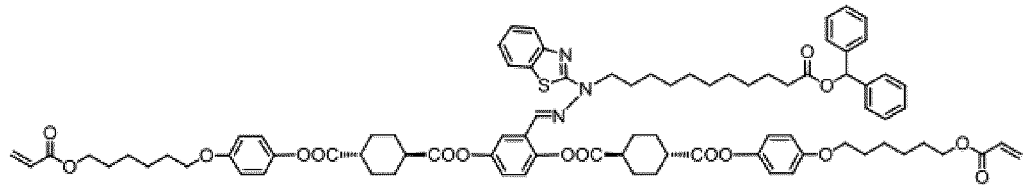
(1 8)

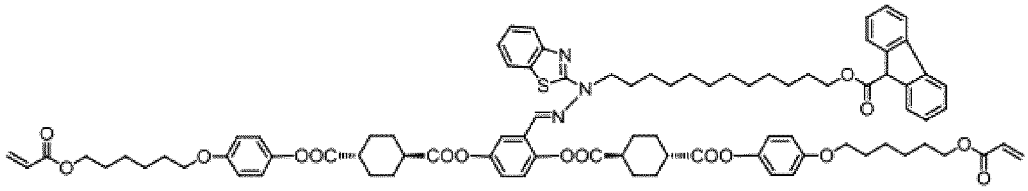
(1 9)

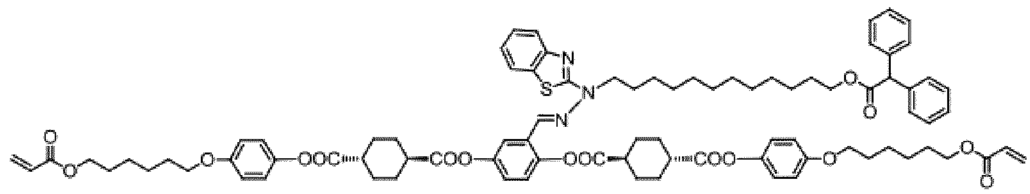
(2 0)

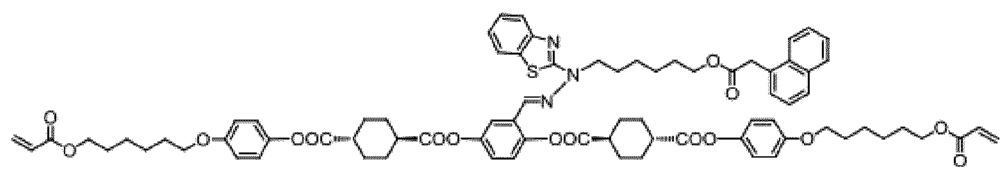
(2 1)

**18.** A polymerizable composition comprising the polymerizable compound according to any one of claims 1 to 17.

**19.** A polymer obtainable by polymerizing the polymerizable compound according to any one of claims 1 to 17.

**20.** An optical film comprising the polymer according to claim 19 as a constituent material.

**21.** An optically anisotropic body comprising a layer which comprises the polymer according to claim 19 as a constituent material.

**22.** A polarizing plate comprising the optically anisotropic body according to claim 21 and a polarizing film.

**23.** A display device comprising the polarizing plate according to claim 22.

**24.** An antireflection film comprising the polarizing plate according to claim 22.

**25.** A compound represented by the following formula (IV):

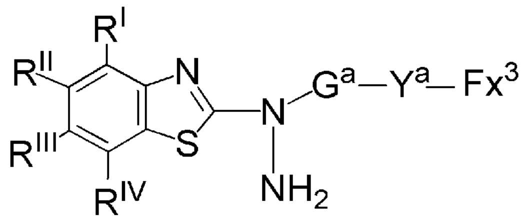

(I V)

where in the formula (IV), $R^I$ to $R^{IV}$ represent each independently a hydrogen atom, a halogen atom, an alkyl group having 1 to 6 carbon atoms, a cyano group, a nitro group, an alkyl group having 1 to 6 carbon atoms in which at least one hydrogen atom is substituted with a halogen atom, an alkoxy group having 1 to 6 carbon atoms, -OCF3, -C(=O)-O-$R^a$, or -O-C(=O)-$R^a$, where $R^a$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring having 5 to 12 carbon atoms which may have a substituent, where $R^I$ to $R^{IV}$ may be the same or different, and one or more ring constituents C-$R^I$ to C-$R^{IV}$ may be replaced by a nitrogen atom;

$Y^a$ represents a chemical single bond, -O-, -C(=O)-, -C(=O)-O-, -O-C(=O)-, -O-C(=O)-O-, -C(=O)-S-, -S-C(=O)-, -$NR^{11}$-C(=O)-, -C(=O)-$NR^{11}$-, -O-C(=O)-$NR^{11}$-, -$NR^{11}$-C(=O)-O-, -S-, -N=N-, or -C=C-, where $R^{11}$ represents a hydrogen atom or an alkyl group having 1 to 6 carbon atoms;

$G^a$ represents an organic group which is either an alkylene group having 3 to 18 carbon atoms which may have a substituent, or an alkylene group having 3 to 18 carbon atoms in which at least one -$CH_2$- contained in the alkylene group is substituted with -O-, -S-, -O-C(=O)-, -C(=O)-O-, or -C(=O)-, with the proviso that cases where there are two or more contiguous -O- or -S- are excluded;

$Fx^3$ is a hydrogen atom, or an organic group having 2 to 30 carbon atoms having at least one of an aromatic hydrocarbon ring and an aromatic heterocyclic ring; and

when $Fx^3$ has a ring structure, the number of $\pi$ electrons included in the ring structure in $Fx^3$ is 4 or more.

**26.** The compound according to claim 25, wherein $G^a$ is an alkylene group having 3 to 18 carbon atoms which may have a substituent, and $Y^a$ represents a chemical single bond, -O-, -C(=O)-, -C(=O)-O-, -O-C(=O)-, -O-C(=O)-O-, -O-C(=O)-$NR^{11}$-, -$NR^{11}$-C(=O)-O-, or, -S-, where $R^{11}$ represents a hydrogen atom or an alkyl group having 1 to 6 carbon atoms.

**27.** The compound according to claim 25 or 26 represented by any of the following formulas (A) to (O):

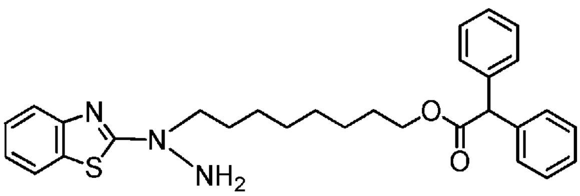

(A)

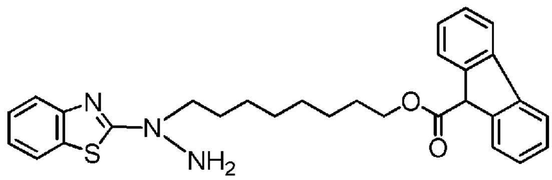

(B)

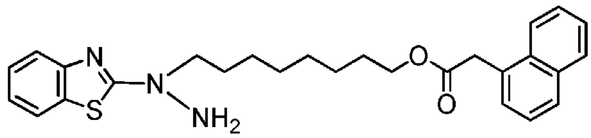

(C)

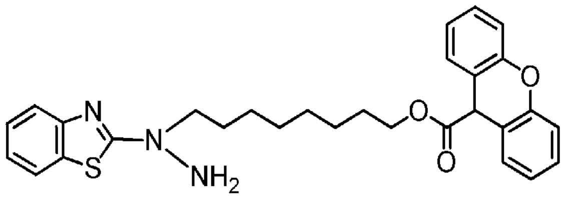

(D)

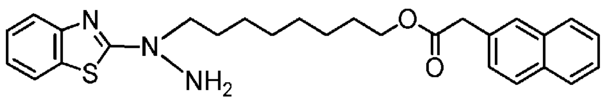

(E)

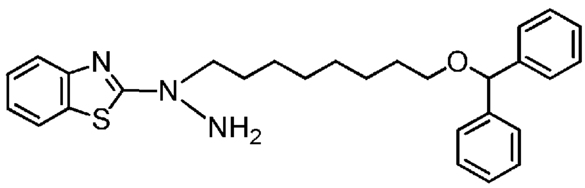

(F)

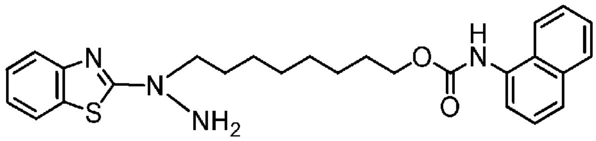

(G)

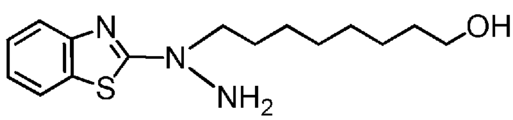

(H)

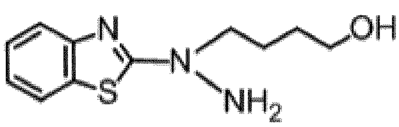

(I)

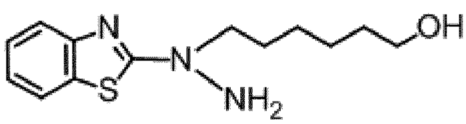

(J)

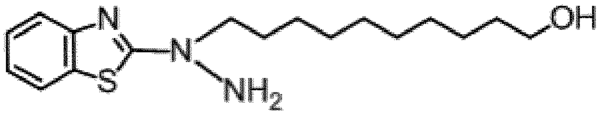

(K)

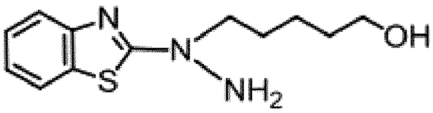

(L)

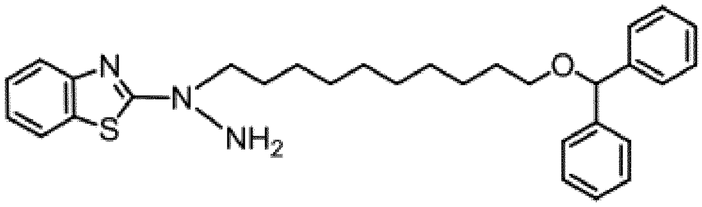

(M)

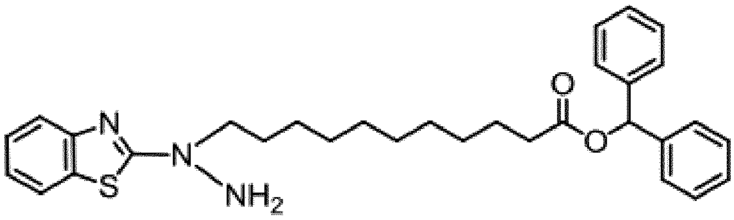

(N)

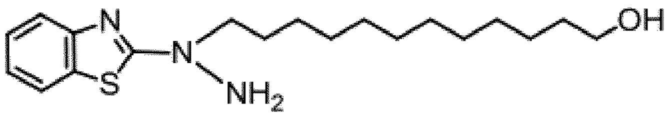

(O)

**28.** A method for producing a polymerizable compound, comprising:

reacting the compound according to any one of claims 25 to 27 with a compound represented by the following formula (V-1) or (V-2):

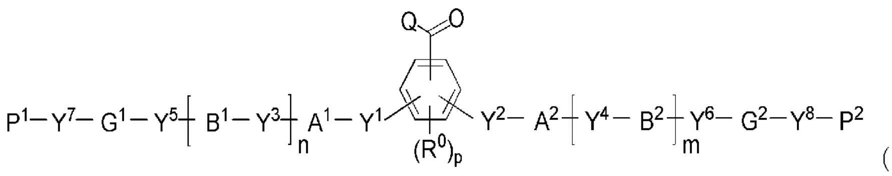

(V − 1)

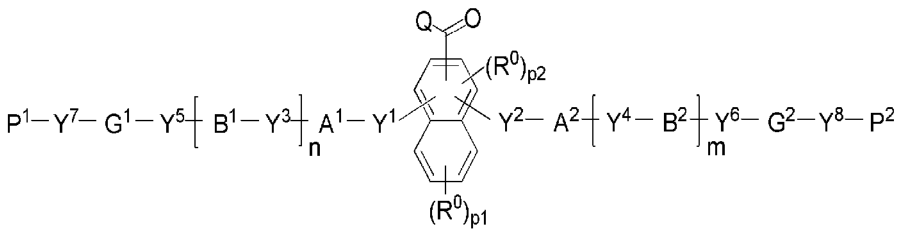

(V − 2)

where in the formulas (V-1) and (V-2),
Q represents a hydrogen atom, or, an alkyl group having 1 to 6 carbon atoms which may have a substituent;
$R^0$ represents a halogen atom, an alkyl group having 1 to 6 carbon atoms, a cyano group, a nitro group, an alkyl group having 1 to 6 carbon atoms in which at least one hydrogen atom is substituted with a halogen atom,

an alkoxy group having 1 to 6 carbon atoms, -OCF3, -C(=O)-O-$R^a$, or -O-C(=O)-$R^a$, where $R^a$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring having 5 to 12 carbon atoms which may have a substituent, and when there is a plurality of $R^0$, the plurality of $R^0$ may be the same or different from each other;

p represents an integer from 0 to 3, p1 represents an integer from 0 to 4, and p2 represents 0 or 1;

$Y^1$ to $Y^8$ each independently represent a chemical single bond, -O-, -O-$CH_2$-, -$CH_2$-O-, -O-$CH_2$-$CH_2$-, -$CH_2$-$CH_2$-O-, -C(=O)-O-, -O-C(=O)-, -O-C(=O)-O-, -C(=O)-S-, -S-C(=O)-, -$NR^{13}$-C(=O)-, -C(=O)-$NR^{13}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-C(=O)-O-, -O-C(=O)-$CH_2$-, -$CH_2$-O-C(=O)-, -C(=O)-O-$CH_2$-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, or -C=C-, where $R^{13}$ represents a hydrogen atom or an alkyl group having 1 to 6 carbon atoms;

$A^1$, $A^2$, $B^1$ and $B^2$ each independently represent a cyclic aliphatic group which may have a substituent, or, an aromatic group which may have a substituent;

$G^1$ and $G^2$ each independently represent an organic group which is either a divalent aliphatic hydrocarbon group having 1 to 30 carbon atoms, or a divalent aliphatic hydrocarbon group having 3 to 30 carbon atoms in which at least one -$CH_2$- contained in the divalent aliphatic hydrocarbon group is substituted with -O-, -S-, -O-C(=O)-, -C(=O)-O-, -O-C(=O)-O-, -$NR^{14}$-C(=O)-, -C(=O)-$NR^{14}$-, -$NR^{14}$-, or, -C(=O)-, with the proviso that cases where there are two or more contiguous -O- or -S- are excluded, where $R^{14}$ represents a hydrogen atom or an alkyl group having 1 to 6 carbon atoms, and the hydrogen atoms included in the organic group of $G^1$ and $G^2$ may be substituted by an alkyl group having 1 to 5 carbon atoms, an alkoxy group having 1 to 5 carbon atoms, a cyano group, or, a halogen atom;

$P^1$ and $P^2$ each independently represent an alkenyl group having 2 to 10 carbon atoms which may be substituted by a halogen atom or a methyl group; and

n and m each independently represent 0 or 1.

**29.** A method for using the compound according to any one of claims 25 to 27 to obtain a polymerizable compound.

**30.** A compound represented by the following formula (V-3) or (V-4):

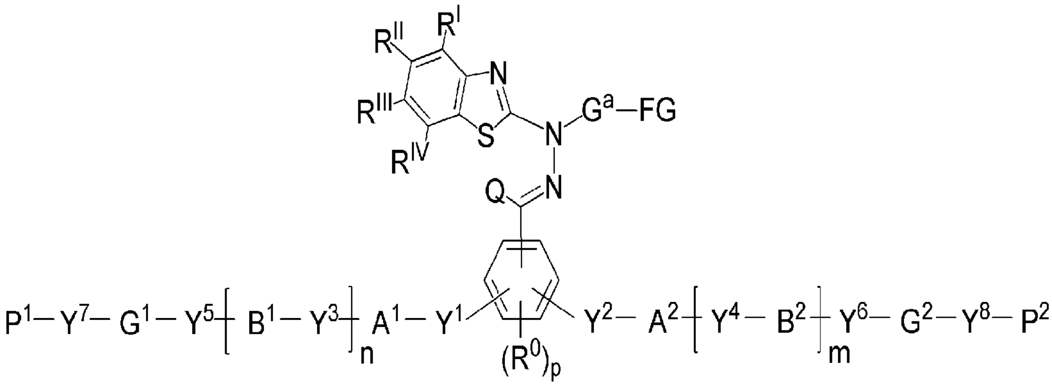

(V－3)

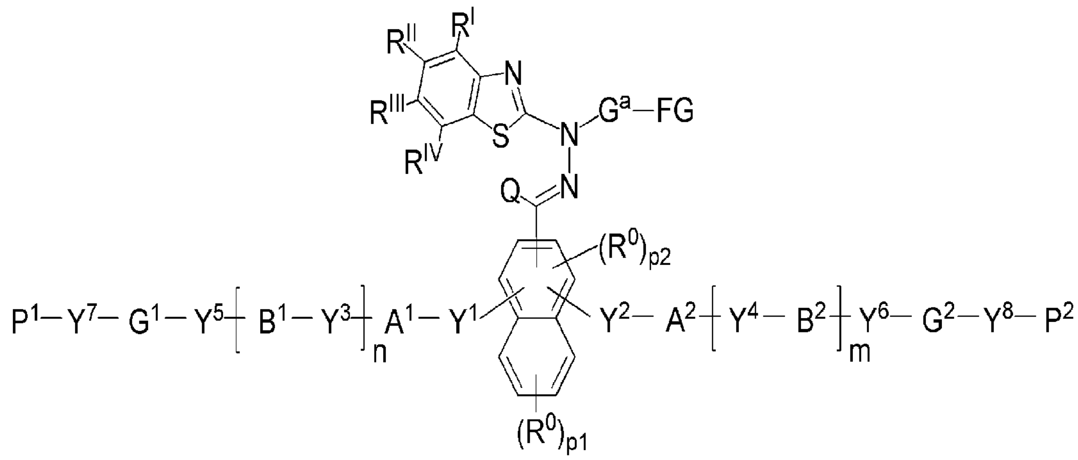

(V－4)

where in the formulas (V-3) and (V-4),

Q represents a hydrogen atom, or, an alkyl group having 1 to 6 carbon atoms which may have a substituent;
$R^0$ represents a halogen atom, an alkyl group having 1 to 6 carbon atoms, a cyano group, a nitro group, an alkyl group having 1 to 6 carbon atoms in which at least one hydrogen atom is substituted with a halogen atom, an alkoxy group having 1 to 6 carbon atoms, -OCF3, -C(=O)-O-$R^a$, or -O-C(=O)-$R^a$, where $R^a$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring having 5 to 12 carbon atoms which may have a substituent, and when there is a plurality of $R^0$, the plurality of $R^0$ may be the same or different from each other;
p represents an integer from 0 to 3, p1 represents an integer from 0 to 4, and p2 represents 0 or 1;
$Y^1$ to $Y^8$ each independently represent a chemical single bond, -O-, -O-$CH_2$-, -$CH_2$-O-, -O-$CH_2$-$CH_2$-, -$CH_2$-$CH_2$-O-, -C(=O)-O-, -O-C(=O)-, -O-C(=O)-O-, -C(=O)-S-, -S-C(=O)-, -$NR^{13}$-C(=O)-, -C(=O)-$NR^{13}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-C(=O)-O-, -O-C(=O)-$CH_2$-, -$CH_2$-O-C(=O)-, -C(=O)-O-$CH_2$-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, or -C=C-, where $R^{13}$ represents a hydrogen atom or an alkyl group having 1 to 6 carbon atoms;
$A^1$, $A^2$, $B^1$ and $B^2$ each independently represent a cyclic aliphatic group which may have a substituent, or, an aromatic group which may have a substituent;
$G^1$ and $G^2$ each independently represent an organic group which is either a divalent aliphatic hydrocarbon group having 1 to 30 carbon atoms, or a divalent aliphatic hydrocarbon group having 3 to 30 carbon atoms in which at least one -$CH_2$- contained in the divalent aliphatic hydrocarbon group is substituted with -O-, -S-, -O-C(=O)-, -C(=O)-O-, -O-C(=O)-O-, -$NR^{14}$-C(=O)-, -C(=O)-$NR^{14}$-, -$NR^{14}$-, or -C(=O)-, with the proviso that cases where there are two or more contiguous -O- or -S- are excluded, where $R^{14}$ represents a hydrogen atom or an alkyl group having 1 to 6 carbon atoms, and the hydrogen atoms included in the organic group of $G^1$ and $G^2$ may be substituted by an alkyl group having 1 to 5 carbon atoms, an alkoxy group having 1 to 5 carbon atoms, a cyano group, or, a halogen atom;
$P^1$ and $P^2$ each independently represent an alkenyl group having 2 to 10 carbon atoms which may be substituted by a halogen atom or a methyl group;
n and m each independently represent 0 or 1;
$R^I$ to $R^{IV}$ each independently represent a hydrogen atom, a halogen atom, an alkyl group having 1 to 6 carbon atoms, a cyano group, a nitro group, an alkyl group having 1 to 6 carbon atoms in which at least one hydrogen atom is substituted with a halogen atom, an alkoxy group having 1 to 6 carbon atoms, -OCF3, -C (=O)-O-$R^a$, or -O-C(=O)-$R^a$, where $R^a$ represents an alkyl group having 1 to 20 carbon atoms which may have a substituent, an alkenyl group having 2 to 20 carbon atoms which may have a substituent, a cycloalkyl group having 3 to 12 carbon atoms which may have a substituent, or an aromatic hydrocarbon ring having 5 to 12 carbon atoms which may have a substituent, where $R^I$ to $R^{IV}$ may be the same or different, and one or more ring constituents C-$R^I$ to C-$R^{IV}$ may be replaced by a nitrogen atom;
$G^a$ represents an organic group which is either an alkylene group having 3 to 18 carbon atoms which may have a substituent, or an alkylene group having 3 to 18 carbon atoms in which at least one -$CH_2$- contained in the alkylene group is substituted with -O-, -S-, -O-C(=O)-, -C(=O)-O-, or -C(=O)-, with the proviso that cases where

there are two or more contiguous -O- or -S- are excluded; and
FG represents -OH, -C(=O)-OH, -SH, or -NR*R**, where R* and R** each independently represent a hydrogen atom or an alkyl group having 1 to 6 carbon atoms, with the proviso that R* and R** are not simultaneously an alkyl group having 1 to 6 carbon atoms.

**31.** The compound according to claim 30 represented by any of the following formulas (a) to (g):

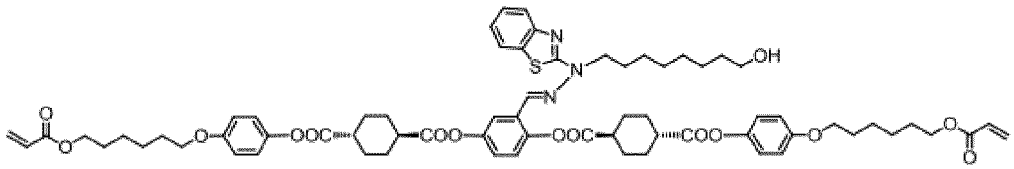

( a )

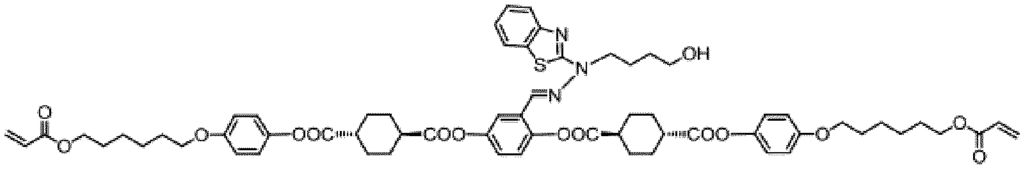

( b )

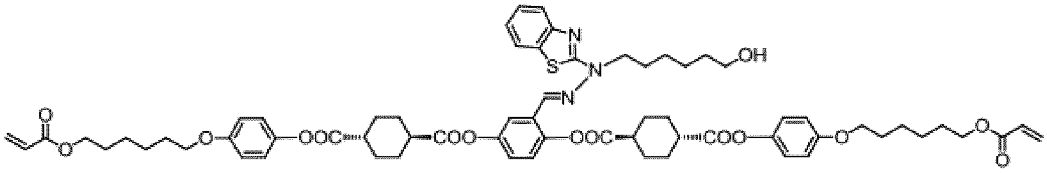

( c )

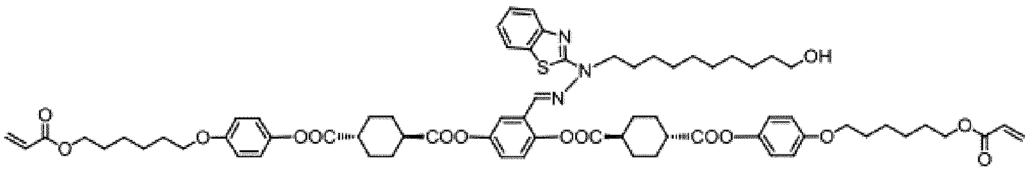

( d )

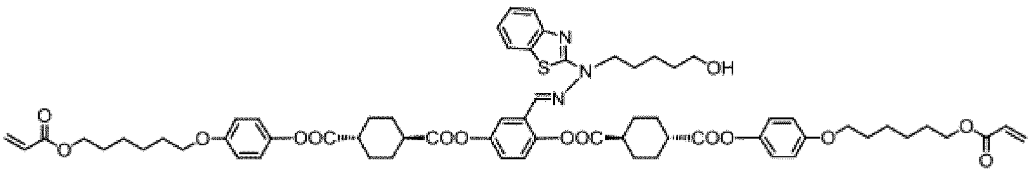

( e )

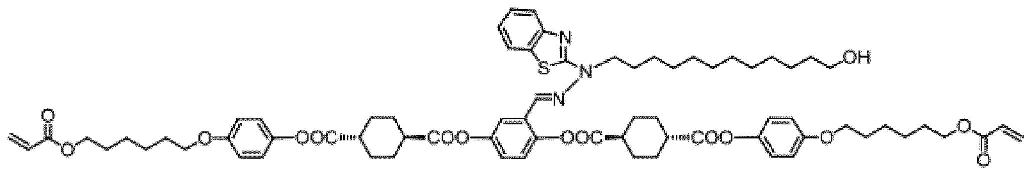

( f )

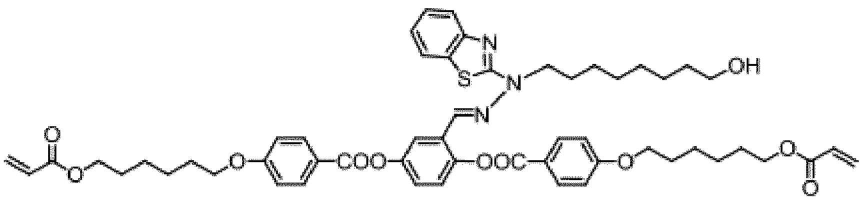

( g )

FIG. 1

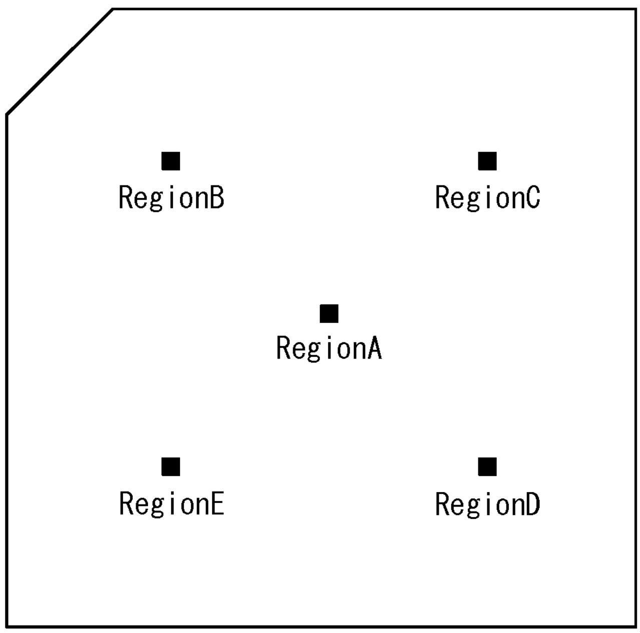

## INTERNATIONAL SEARCH REPORT

International application No.
PCT/JP2018/010481

A. CLASSIFICATION OF SUBJECT MATTER
Int.Cl. C08F20/38(2006.01), C08F20/34(2006.01)i, C09K19/38(2006.01)i, G02B1/11(2015.01)i, G02B5/30(2006.01)i, G02F1/1335(2006.01)i, G02F1/13363(2006.01)i

According to International Patent Classification (IPC) or to both national classification and IPC

B. FIELDS SEARCHED

Minimum documentation searched (classification system followed by classification symbols)
Int.Cl. C08F20/38, C08F20/34, C09K19/38, G02B1/11, G02B5/30, G02F1/1335, G02F1/13363

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 2016/114211 A1 (DIC CORP.) 21 July 2016, claims, paragraphs [0012], [0095]-[0111], [0113], [0186], [0203] & US 2018/0002276 A1, claims, paragraphs [0015], [0072], [0074], [0140], [0162] & CN 107108473 A & KR 10-2017-0102242 A | 25-31<br>1-24 |
| P, X<br>P, A | WO 2017/169839 A1 (DIC CORP.) 05 October 2017, claims, paragraphs [0085], [0096]-[0119], [0122]-[0123], [0129], [0133]-[0134], [0137]-[0143], [0166] (Family: none) | 25-31<br>1-24 |
| P, X<br>P, A | WO 2017/098952 A1 (DIC CORP.) 15 June 2017, claims, paragraphs [0033]-[0035], [0143]-[0144], [0153] (Family: none) | 25-31<br>1-24 |
| P, X<br>P, A | WO 2017/068860 A1 (DIC CORP.) 27 April 2017, claims, paragraphs [0104], [0109], [0123]-[0176], [0183], [0197], [0209], [0215], [0250] (Family: none) | 25-31<br>1-24 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 31 May 2018 (31.05.2018) | 12 June 2018 (12.06.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/010481

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☒ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (January 2015)

**INTERNATIONAL SEARCH REPORT**

International application No. PCT/JP2018/010481

Continuation of Box III

Document 1: WO 2016/114211 A1 (DIC CORP.) 21 July 2016, claims, paragraph [0186], [0203] & CN 107108473 A & KR 10-2017-0102242 A

The claims are classified into the 3 inventions below.

(Invention 1) Claims 1-24

Claims 1-24 have the special technical feature in which Fx1 and Fx2 of formula (I) are each independently organic groups having at least one among an aromatic hydrocarbon ring and an aromatic heterocyclic ring, and thus are classified as invention 1.

(Invention 2) Claims 25-29

Claims 25-29 share the technical feature of using a compound represented by formula (IV) with claim 1 classified as invention 1. However, said technical feature does not make a contribution over the prior art in light of the disclosure of document 1, and thus cannot be said to be a special technical feature. In addition, there do not exist other identical or corresponding technical features between these inventions.

In addition, claims 25-29 are not dependent on claim 1. In addition, claims 25-29 are not substantially identical or equivalent to any of the claims classified as invention 1.

Accordingly, claims 25-29 cannot be classified as invention 1.

In addition, claims 25-29 share the technical feature of a compound represented by formula (IV), and thus are classified as invention 2.

(Invention 3) Claims 30-31

Claims 30-31 share the technical feature in which Ar of formula (I) is

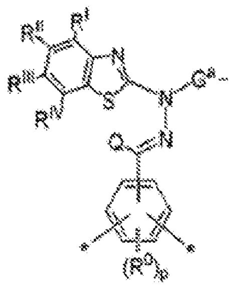

(II-1)

or

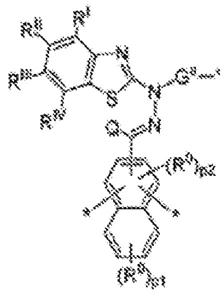

(II-2)

with claim 1 classified as invention 1, and share the technical feature of using a compound represented by formula (IV) with claim 25 classified as invention 2. However, said technical feature does not make a contribution over the prior art in light of the disclosure of document 1, and thus cannot be said to be a special technical feature. In addition, there do not exist other identical or corresponding technical features between these inventions.

In addition, claims 30-31 are not dependent on claims 1 and 25. In addition, claims 30-31 are not substantially identical or equivalent to any of the claims classified as invention 1 or 2.

Accordingly, claims 30-31 cannot be classified as invention 1 or 2.

In addition, claims 30-31 share the technical feature of having a specific FG in formula (I), and thus are classified as invention 3.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014010325 A
- WO 2012141245 A
- WO 2012147904 A
- WO 2013046781 A
- WO 2014061709 A
- WO 2014126113 A
- WO 2015064698 A
- WO 2015140302 A
- WO 2015129654 A
- WO 2015141784 A
- WO 2016159193 A
- WO 2012169424 A
- WO 2012176679 A
- WO 2015122385 A
- JP 2007002208 A
- JP 2009173893 A
- JP 2009274984 A
- JP 2010030979 A
- JP 2010031223 A
- JP 2011006360 A
- JP 2010024438 A
- WO 201276679 A
- WO 2013180217 A
- WO 2014065176 A
- WO 2015025793 A
- WO 2015122384 A
- JP H05310845 A
- US 5179171 A
- JP H0597978 A
- US 5202388 A
- JP H11124429 A
- WO 9920676 A